# EUROPÄISCHE PATENTSCHRIFT

(11) **EP 2 875 003 B1**
(45) Veröffentlichungstag und Bekanntmachung des Hinweises auf die Patenterteilung: **16.11.2016**
(21) Anmeldenummer: 13744464.2
(22) Anmeldetag: 16.07.2013
(51) Int. Cl.: C07D 217/26, C07D 401/12, C07D 413/12, A61K 31/47, A61P 9/00

(54) **NEUE 5-AMINOTETRAHYDROCHINOLIN-2-CARBONSÄUREN UND IHRE VERWENDUNG**
NEW 5-AMINOTETRAHYDROQUINOLINE-2-CARBOXYLIC ACIDS UND THEIR USE
NOUVEAUX ACIDES CARBOXYLIQUES DE 5-AMINOTETRAHYDROQUINOLINE-2-YL ET LEUR UTILISATION

(30) Priorität: 20.07.2012 EP 12177284; 16.05.2013 EP 13167967
(43) Veröffentlichungstag der Anmeldung: 27.05.2015
(73) Patentinhaber: Bayer Pharma Aktiengesellschaft, 13353 Berlin (DE)
(72) Erfinder: HAHN, Michael, 40764 Langenfeld (DE); FOLLMANN, Markus, 50859 Köln (DE); HÜBSCH, Walter, 42113 Wuppertal (DE); BECKER-PELSTER, Eva-Maria, 42327 Wuppertal (DE); STASCH, Johannes-Peter, 42651 Solingen (DE); KELDENICH, Joerg, deceased (DE); DELBECK, Martina, 42579 Heiligenhaus (DE); TINEL, Hanna, 42113 Wuppertal (DE); WUNDER, Frank, 42117 Wuppertal (DE); MITTENDORF, Joachim, 42113 Wuppertal (DE); TEREBESI, Ildiko, 14195 Berlin (DE); LANG, Dieter, 42553 Velbert (DE); MARTIN, René, 01157 Dresden (DE)
(74) Vertreter: BIP Patents
(86) Internationale Anmeldenummer: PCT/EP2013/065017
(87) Internationale Veröffentlichungsnummer: WO 2014/012934

(56) Entgegenhaltungen:
- EP-A1- 2 840 076
- WO-A2-02/070510
- STASCH JOHANNES-PETER ET AL: "TARGETING THE HEME-OXIDIZED NITRIC OXIDE RECEPTOR FOR SELECTIVE VASODILATATION OF DISEASED BLOOD VESSELS", JOURNAL OF CLINICAL INVESTIGATION, AMERICAN SOCIETY FOR CLINICAL INVESTIGATION, US, Bd. 116, Nr. 9, 1. September 2006 (2006-09-01), Seiten 2552-2561, XP008079104, ISSN: 0021-9738, DOI: 10.1172/JCI28371 in der Anmeldung erwähnt

## Beschreibung

Die vorliegende Anmeldung betrifft neue 5-Amino-5,6,7,8-tetrahydrochinolin-2-carbonsäuren, Verfahren zu ihrer Herstellung, besagte Verbindungen zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention kardiovaskulärer und kardiopulmonaler Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, am Eisen-Zentralatom des Häms anzugreifen, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu pulmonaler Hypertonie, Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall und Myokardinfarkt führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisen-Zentralatom des Häms. Neben den Nebenwirkungen gehört die Toleranzentwicklung zu den entscheidenden Nachteilen dieser Behandlungsweise [O.V. Evgenov et al., Nature Rev. Drug Disc. 5 (2006), 755].

In den letzten Jahren wurden Substanzen identifiziert, die die lösliche Guanylatcyclase direkt, d.h. ohne vorherige Freisetzung von NO stimulieren. Mit dem Indazolderivat YC-1 wurde erstmals ein NO-unabhängiger, jedoch Häm-abhängiger Stimulator der sGC beschrieben [Evgenov et al., *ibid*.]. Ausgehend von YC-1 wurden weitere Substanzen gefunden, welche eine höhere Potenz als YC-1 besitzen und keine relevante Hemmung von Phosphodiesterasen (PDE) aufweisen. Dies führte zur Identifizierung der Pyrazolopyridin-Derivate BAY 41-2272, BAY 41-8543 und BAY 63-2521 (Riociguat). Diese Verbindungen bilden gemeinsam mit den kürzlich publizierten, strukturell diversen Substanzen CMF-1571 und A-350619 die neue Klasse der sGC-Stimulatoren [Evgenov et al., *ibid*.], die eine NO-unabhängige und selektive Aktivierung der hämhaltigen sGC bewirken. Darüber hinaus zeigen sGC-Stimulatoren in Kombination mit NO einen synergistischen Effekt auf die sGC-Aktivierung, welcher auf einer Stabilisierung des Nitrosyl-Häm-Komplexes basiert. Entfernt man von der löslichen Guanylatcyclase die Häm-Gruppe, zeigt das Enzym immer noch eine nachweisbare katalytische Basalaktivität, d.h. es wird nach wie vor cGMP gebildet. Die verbleibende katalytische Basalaktivität des Häm-freien Enzyms ist durch keinen der vorstehend genannten Stimulatoren stimulierbar [Evgenov et al., *ibid*.].

Darüber hinaus wurden NO- und Häm-unabhängige sGC-Aktivatoren, mit BAY 58-2667 (Cinaciguat) als Prototyp dieser Klasse, identifiziert. Gemeinsame Charakteristika dieser Substanzen sind, dass sie in Kombination mit NO nur einen additiven Effekt auf die Enzymaktivierung ausüben und dass die Aktivierung des oxidierten oder hämfreien Enzyms im Vergleich zum hämhaltigen Enzym deutlich stärker ist [Evgenov et al., *ibid*.; J.P. Stasch et al., Br. J. Pharmacol. 136 (2002), 773; J.P. Stasch et al., J. Clin. Invest. 116 (2006), 2552]. Spektroskopische Untersuchungen lassen erkennen, dass BAY 58-2667 die oxidierte Hämgruppe verdrängt, die durch Schwächung der Eisen-Histidin-Bindung nur schwach an der sGC gebunden ist. Auch wurde gezeigt, dass das charakteristische sGC-Hämbindungsmotiv Tyr-x-Ser-x-Arg sowohl für die Interaktion der negativ geladenen Propionsäuren der Hämgruppe als auch für die Wirkung von BAY 58-2667 zwingend erforderlich ist. Vor diesem Hintergrund wird angenommen, dass die Bindungsstelle von BAY 58-2667 an der sGC identisch zur Bindungsstelle der Hämgruppe ist [J.P. Stasch et al., J. Clin. Invest. 116 (2006), 2552]. Kristallisationsuntersuchungen mit der *Nostoc* H-NOX-Domäne, einer prokaryotischen Hämbindungsdomäne mit hoher Sequenzhomologie zur sGC, haben vor kurzem gezeigt, dass BAY 58-2667 in der Hämbindungstasche bindet [F. van den Akker et al., J. Biol. Chem. 285 (2010), 22651].

Die Pulmonale Hypertonie (PH) ist eine progrediente Lungenerkrankung, die unbehandelt innerhalb von wenigen Jahren nach Diagnosestellung zum Tode führt. Definitionsgemäß liegt bei einer chronischen pulmonalen Hypertonie ein pulmonal-arterieller Mitteldruck (mPAP) von > 25 mmHg in Ruhe oder > 30 mmHg unter Belastung vor (Normalwert < 20 mmHg). Die Pathophysiologie der pulmonalen Hypertonie ist gekennzeichnet durch Vasokonstriktion und ein Remodeling der Pulmonalgefäße. Bei der chronischen PH kommt es zu einer Neomuskularisierung primär nicht muskularisierter Lungengefäße, und die Gefäßmuskulatur der bereits muskularisierten Gefäße nimmt an Umfang zu. Durch diese zunehmende Obliteration der Lungenstrombahn kommt es zu einer progredienten Belastung des rechten Herzens, die zu einer verminderten Auswurfleistung des rechten Herzens führt und letztlich in einem Rechtsherzversagen endet [M. Humbert et al., J. Am. Coll. Cardiol. 2004, 43, 13S-24S]. Auch wenn die idiopathische (oder primäre) pulmonal-arterielle Hypertonie (IPAH) eine sehr seltene Erkrankung ist, so ist die sekundäre pulmonale Hypertonie (non-PAH PH) weit verbreitet, und es wird angenommen, dass letztere derzeit die dritthäufigste Gruppe von kardiovaskulären Erkrankungen nach koronarer Herzkrankheit und systemischem Bluthochdruck darstellt. Die Einteilung der pulmonalen Hypertonie in verschiedene Untergruppen gemäß der jeweiligen Ätiologie erfolgt seit 2008 nach der Dana Point-Klassifikation [M. Humbert und V.V. McLaughlin, J. Am. Coll. Cardiol. 2009, 54 (1), S1-S2; D. Montana und G. Simonneau, in: A.J. Peacock et al. (Eds.), Pulmonary Circulation. Diseases and their treatment, 3rd edition, Hodder Arnold Publ., 2011, S. 197-206].

Trotz aller Fortschritte in der Therapie der PH gibt es bisher keine Aussicht auf Heilung dieser schwerwiegenden Erkrankung. Auf dem Markt befindliche Standardtherapien (z.B. Prostacyclin-Analoga, Endothelinrezeptor-Antagonisten, Phosphodiesterase-Inhibitoren) sind in der Lage, die Lebensqualität, die körperliche Belastbarkeit und die Prognose der Patienten zu verbessern. Hierbei handelt es sich um systemisch applizierte, primär hämodynamisch wirkende Therapieprinzipien, die den Gefäßtonus beeinflussen. Die Anwendbarkeit dieser Medikamente ist durch die z.T. gravierenden Nebenwirkungen und/oder aufwendigen Applikationsformen eingeschränkt. Der Zeitraum, über den unter einer spezifischen Monotherapie die klinische Situation der Patienten stabilisiert oder verbessert werden kann, ist begrenzt (z.B. aufgrund einer Toleranzentwicklung). Es erfolgt schließlich eine Therapieeskalation und somit eine Kombinationstherapie, bei der mehrere Medikamente gleichzeitig gegeben werden müssen. Zur Zeit sind diese Standardtherapeutika nur zur Behandlung der pulmonal-arteriellen Hypertonie (PAH) zugelassen. Bei sekundären Formen der PH, wie z.B. PH-COPD, scheiterten diese Therapieprinzipien (z.B. Sildenafil, Bosentan) in klinischen Studien, da sie infolge einer unselektiven Vasodilatation zu einer Absenkung (Entsättigung) des arteriellen Sauerstoffgehalts bei den Patienten führten. Ursache hierfür ist wahrscheinlich eine ungünstige Beeinflussung der Ventilations-Perfusions-Anpassung innerhalb der Lunge bei heterogenen Lungenerkrankungen aufgrund der systemischen Gabe unselektiver Vasodilatatoren [I. Blanco et al., Am. J. Respir. Crit. Care Med. 2010, 181, 270-278; D. Stolz et al., Eur. Respir. J. 2008, 32, 619-628].

Neue Kombinationstherapien sind eine der aussichtsreichsten zukünftigen Therapieoptionen zur Behandlung der pulmonalen Hypertonie. In diesem Zusammenhang ist die Erkundung neuer pharmakologischer Mechanismen zur Behandlung der PH von besonderem Interesse [Ghofrani et al., Herz 2005, 30, 296-302; E.B. Rosenzweig, Expert Opin. Emerging Drugs 2006, 11, 609-619; T. Ito et al., Curr. Med. Chem. 2007, 14, 719-733]. Vor allem solche neuen Therapieansätze, die mit den bereits auf dem Markt befindlichen Therapiekonzepten kombinierbar sind, könnten Grundlage einer effizienteren Behandlung sein und somit einen großen Vorteil für die Patienten bringen. Darüber hinaus könnte eine lungenselektive Anwendbarkeit eines solchen neuen Wirkprinzips die Möglichkeit bieten, dieses nicht nur in PAH einzusetzen, sondern vor allem auch eine erste Therapieoption für Patienten mit sekundären PH-Formen zu eröffnen.

In einem Tiermodell für pulmonale Hypertonie konnte gezeigt werden, dass der sGC-Aktivator BAY 58-2667 (Cinaciguat) nach inhalativer Gabe in Form von Mikropartikeln zu einer dosisabhängigen selektiven Reduktion des pulmonal-arteriellen Drucks führt. Die intravenöse Gabe von 1*H*-1,2,4-Oxadiazolo[4,3-a]chinoxalin-1-on (ODQ), das die prosthetische Häm-Gruppe der sGC oxidiert, reduzierte in diesem Modell den vasodilatierenden Effekt von inhaliertem NO (iNO), wohingegen BAY 58-2267 ihn verstärkte. Diese Ergebnisse gaben zu der Hypothese Anlass, dass die inhalative Applikation eines sGC-Aktivators eine neue effektive Behandlungsmethode für Patienten mit pulmonaler Hypertonie darstellen könnte, insbesondere dann, wenn das Ansprechen dieser Patienten auf iNO und/oder auf PDE5-Inhibitoren infolge eines Mangels an NO oder einer Oxidation der sGC abgeschwächt ist [O.V. Evgenov et al., Am. J. Respir. Crit. Care Med. 2007, 176, 1138-1145]. Cinaciguat selbst zeigt jedoch in diesem Modell eine nicht hinreichende Wirkdauer und führt zudem in höheren Dosierungen zu unerwünschten systemischen Nebenwirkungen.

Aufgabe der vorliegenden Erfindung war daher die Bereitstellung neuer Verbindungen, welche in der oben beschriebenen Weise als Aktivatoren der löslichen Guanylatcyclase wirken und als solche insbesondere zur Behandlung und zur Prävention kardiovaskulärer Erkrankungen eingesetzt werden können. Darüber hinaus sollten diese neuen Verbindungen eine verbesserte pulmonale Wirkselektivität aufweisen und damit insbesondere zur Behandlung der pulmonalen Hypertonie und ihrer sekundären Formen geeignet sein. Zu diesem Zweck sollten die neuen Verbindungen mit der Standardtherapie in PAH, aber auch mit den Basistherapeutika in sekundären PH-Formen kombinierbar sein.

Aus den Patentanmeldungen WO 01/19780-A2, WO 02/070459-A1, WO 02/070460-A1, WO 02/070461-A1, WO 02/070462-A1 und WO 02/070510-A2 sind verschiedene Aminodicarbonsäure-Derivate zur Behandlung von Herz-Kreislauf-Erkrankungen bekannt. In WO 2009/ 023669-A1 werden substituierte 5,6,7,8-Tetrahydrochinoline als C5a-Rezeptor-Modulatoren zur Behandlung von Entzündungs- und Immunerkrankungen offenbart. In WO 95/18617-A1 und WO 00/35882-A1 werden 1-Amino-1,2,3,4-tetrahydronaphthalin-Derivate zur Behandlung neurologischer Erkrankungen beschrieben. In WO 2006/104826-A2 werden acylierte 5-Amino-5,6,7,8-tetrahydronaphthalin-2-carboxamide als Glukagon-Rezeptor-Antagonisten zur Behandlung von Diabetes offenbart.

Gegenstand der vorliegenden Erfindung sind Verbindungen der allgemeinen Formel (I) 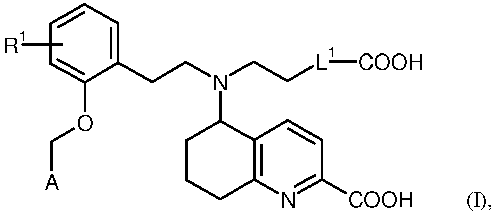 in welcher
- R¹: für Wasserstoff oder Fluor steht,
- L¹: für Ethan-1,2-diyl oder 1,4-Phenylen steht,
und
- A: für eine Gruppe der Formel 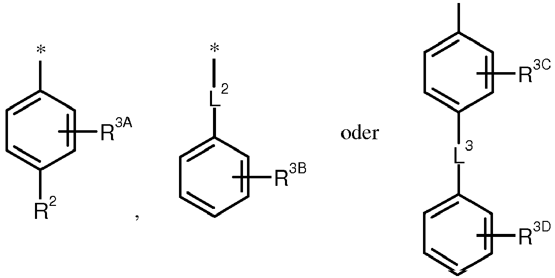
steht, (wie in den Ansprüchen definiert) worin * die jeweilige Verknüpfungsstelle mit dem Rest des Moleküls kennzeichnet,
L³ eine Bindung, -O-, -CH₂-, -CH₂-CH₂- oder -CH=CH- bedeutet,
und
R^{3C} einen Substituenten ausgewählt aus der Reihe Fluor, Chlor, Brom, Cyano, (C₁-C₄)-Alkyl, Difluormethyl, Trifluormethyl, (C₁-C₄)-Alkoxy, Difluormethoxy und Trifluormethoxy bedeutet und R 3D einen Substituenten ausgewählt aus der Reihe Wasserstoff, Fluor, Chlor, Brom, Cyano, (C1-C4)-Alkyl, Difluormethyl, Trifluormethyl, (C1-C4)-Alkoxy, Difluormethoxy und Trifluormethoxy bedeutet,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung, Reinigung oder Lagerung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Benzolsulfonsäure, Toluolsulfonsäure, Naphthalindisulfonsäure, Ameisensäure, Essigsäure, Trifluoressigsäure, Propionsäure, Bernsteinsäure, Fumarsäure, Maleinsäure, Milchsäure, Weinsäure, Äpfelsäure, Zitronensäure, Gluconsäure, Benzoesäure und Embonsäure. Physiologisch unbedenkliche Salze der erfindungsgemäßen Verbindungen umfassen auch von üblichen Basen abgeleitete Salze, wie beispielhaft und vorzugsweise Alkalimetallsalze (z.B. Natrium- und Kaliumsalze), Erdalkalisalze (z.B. Calcium- und Magnesiumsalze), Zinksalze sowie Ammoniumsalze abgeleitet von Ammoniak oder organischen Aminen mit 1 bis 16 C-Atomen, wie beispielhaft und vorzugsweise Ethylamin, Diethylamin, Triethylamin, *N,N*-Diisopropylethylamin, Monoethanolamin, Diethanolamin, Triethanolamin, Tromethamin, Dimethylaminoethanol, Diethylaminoethanol, Cholin, Procain, Dicyclohexylamin, Dibenzylamin, *N*-Methylmorpholin, *N*-Methylpiperidin, Arginin, Lysin und 1,2-Ethylendiamin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise zu einer Verlängerung der Halbwertszeit im Körper oder zu einer Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach allgemein gebräuchlichen, dem Fachmann bekannten Verfahren hergestellt werden, so beispielsweise nach den weiter unten beschriebenen Methoden und den bei den Ausführungsbeispielen wiedergegebenen Vorschriften, indem hierbei entsprechende isotopische Modifikationen der jeweiligen Reagentien und/oder Ausgangsverbindungen eingesetzt werden.

Im Rahmen der vorliegenden Erfindung haben die Substituenten, soweit nicht anders spezifiziert, die folgende Bedeutung:
(C1-C4)-Alkyl steht im Rahmen der Erfindung für einen geradkettigen oder verzweigten, monovalenten Alkylrest mit 1 bis 4 Kohlenstoffatomen. Beispielhaft und vorzugsweise seien genannt: Methyl, Ethyl, *n*-Propyl, Isopropyl, *n*-Butyl, *iso*-Butyl, *sec*.-Butyl und *tert*.-Butyl.
(C₁-C₆)-Alkandiyl und (C₃-C₅)-Alkandiyl stehen im Rahmen der Erfindung für einen geradkettigen, α,ω-divalenten Alkylrest mit 1 bis 6 bzw. 3 bis 5 Kohlenstoffatomen. Beispielhaft und vorzugsweise seien genannt: Methylen, Ethan-1,2-diyl (1,2-Ethylen), Propan-1,3-diyl (1,3-Propylen), Butan-1,4-diyl (1,4-Butylen), Pentan-1,5-diyl (1,5-Pentylen) und Hexan-1,6-diyl (1,6-Hexylen).
(C₁-C₄)-Alkylcarbonyl steht im Rahmen der Erfindung für einen geradkettigen oder verzweigten Alkylrest mit 1 bis 4 Kohlenstoffatomen, der über eine Carbonyl-Gruppe [-C(=O)-] mit dem Rest des Moleküls verknüpft ist. Beispielhaft und vorzugsweise seien genannt: Acetyl, Propionyl, *n*-Butyryl, Isobutyryl, *n*-Pentanoyl und Pivaloyl.
(C₁-C₄)-Alkoxy steht im Rahmen der Erfindung für einen geradkettigen oder verzweigten Alkoxyrest mit 1 bis 4 Kohlenstoffatomen. Beispielhaft und vorzugsweise seien genannt: Methoxy, Ethoxy, *n*-Propoxy, Isopropoxy, *n*-Butoxy, Isobutoxy, *sec*.-Butoxy und *tert*.-Butoxy.
(C₃-C₆)-Cycloalkyl steht im Rahmen der Erfindung für einen monocyclischen gesättigten Carbocyclus mit 3 bis 6 Ring-Kohlenstoffatomen. Beispielhaft und vorzugsweise seien genannt: Cyclopropyl, Cyclobutyl, Cyclopentyl und Cyclohexyl.
4- bis 6-gliedriges Heterocyclyl steht im Rahmen der Erfindung für einen monocyclischen gesättigten Heterocyclus mit insgesamt 4 bis 6 Ringatomen, welcher ein oder zwei gleiche oder verschiedene Ring-Heteroatome aus der Reihe N, O, S und/oder S(O)₂ enthält und über ein Ring-Kohlenstoffatom oder gegebenenfalls über ein Ring-Stickstoffatom verknüpft ist. Bevorzugt ist 5- oder 6-gliedriges Heterocyclyl, das ein Ring-Stickstoffatom enthält und darüber hinaus ein weiteres Ring-Heteroatom aus der Reihe N oder O enthalten kann. Beispielhaft seien genannt: Azetidinyl, Oxetanyl, Thietanyl, Pyrrolidinyl, Pyrazolidinyl, Tetrahydrofuranyl, Thiolanyl, 1,2-Oxazolidinyl, 1,3-Oxazolidinyl, 1,3-Thiazolidinyl, Piperidinyl, Piperazinyl, Tetrahydropyranyl, Tetrahydrothiopyranyl, 1,3-Dioxanyl, 1,4-Dioxanyl, 1,2-Oxazinanyl, Morpholinyl und Thiomorpholinyl. Bevorzugt sind Pyrrolidinyl, Piperidinyl, Piperazinyl und Morpholinyl.
5-gliedriges Heteroaryl steht im Rahmen der Erfindung für einen aromatischen Heterocyclus (Heteroaromaten) mit insgesamt 5 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Bevorzugt ist 5-gliedriges Heteroaryl, das ein Ring-Stickstoffatom und darüber hinaus ein oder zwei weitere Ring-Heteroatome aus der Reihe N, O und/oder S enthält. Beispielhaft seien genannt: Furyl, Pyrrolyl, Thienyl, Pyrazolyl, Imidazolyl, 1,2-Oxazolyl (Isoxazolyl), 1,3-Oxazolyl, 1,2-Thiazolyl (Isothiazolyl), 1,3-Thiazolyl, 1,2,3-Triazolyl, 1,2,4-Triazolyl, 1,2,4-Oxadiazolyl, 1,3,4-Oxadiazolyl, 1,2,4-Thiadiazolyl und 1,3,4-Thiadiazolyl. Bevorzugt sind 1,2-Oxazolyl (Isoxazolyl), 1,3-Oxazolyl, 1,2-Thiazolyl (Isothiazolyl), 1,3-Thiazolyl, 1,2,4-Oxadiazolyl, 1,3,4-Oxadiazolyl, 1,2,4-Thiadiazolyl und 1,3,4-Thiadiazolyl.

Im Rahmen der vorliegenden Erfindung gilt, dass für alle Reste, die mehrfach auftreten, deren Bedeutung unabhängig voneinander ist. Wenn Reste in den erfindungsgemäßen Verbindungen substituiert sind, können die Reste, soweit nicht anders spezifiziert, ein- oder mehrfach substituiert sein. Eine Substitution mit einem oder zwei oder drei gleichen oder verschiedenen Substituenten ist bevorzugt. Besonders bevorzugt ist die Substitution mit einem oder zwei gleichen oder verschiedenen Substituenten.

Eine besondere Ausführungsform der vorliegenden Erfindung umfasst Verbindungen der Formel (I), in welcher
R¹ für Wasserstoff steht,
sowie ihre Salze, Solvate und Solvate der Salze.
Eine weitere besondere Ausführungsform der vorliegenden Erfindung umfasst Verbindungen der Formel (I), in welcher
   - R¹: für Fluor steht, das sich in *para*-Position relativ zur ACH₂O-Gruppe befindet,
   sowie ihre Salze, Solvate und Solvate der Salze.

Eine weitere besondere Ausführungsform der vorliegenden Erfindung umfasst Verbindungen der Formel (I), in welcher
- L¹: für Ethan-1,2-diyl steht,
sowie ihre Salze, Solvate und Solvate der Salze.

Eine weitere besondere Ausführungsform der vorliegenden Erfindung umfasst Verbindungen der Formel (I), in welcher
- L¹: für 1,4-Phenylen steht,
sowie ihre Salze, Solvate und Solvate der Salze.

Bevorzugt im Rahmen der vorliegenden Erfindung sind Verbindungen der Formel (I), in welcher
- R¹: für Wasserstoff oder Fluor steht,
- L¹: für Ethan-1,2-diyl oder 1,4-Phenylen steht,
und
- A: für eine Gruppe der Formel 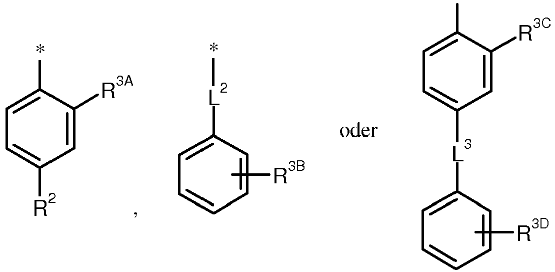
steht, (wie in den Ansprüchen definiert), worin * die jeweilige Verknüpfungsstelle mit dem Rest des Moleküls kennzeichnet,
- L³: eine Bindung, -CH₂-CH₂- oder -CH=CH- bedeutet,

- R^{3C}: Fluor, Chlor, Methyl oder Trifluormethyl bedeutet,
und
- R^{3D}: Wasserstoff, Fluor, Chlor, Cyano, Methyl, Trifluormethyl, Methoxy oder Trifluor-methoxy bedeutet,
sowie ihre Salze, Solvate und Solvate der Salze.

Besonders bevorzugt im Rahmen der vorliegenden Erfindung sind Verbindungen der Formel (I), in welcher
- R¹: für Wasserstoff oder Fluor steht,
- L¹: für Ethan-1,2-diyl oder 1,4-Phenylen steht,
- und A: für eine Gruppe der Formel 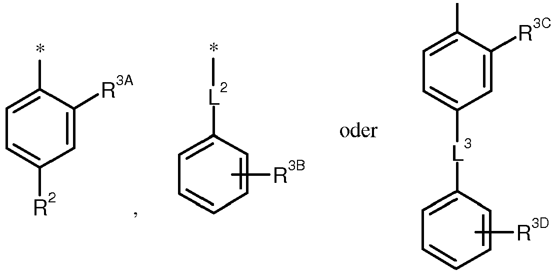
steht, (wie in den Ansprüchen definiert), worin * die jeweilige Verknüpfungsstelle mit dem Rest des Moleküls kennzeichnet,
- L³: eine Bindung, -CH₂-CH₂- oder -CH=CH- bedeutet,

- R^{3C}: Fluor, Chlor, Methyl oder Trifluormethyl bedeutet,
und
- R^{3D}: Wasserstoff, Fluor, Chlor, Cyano, Methyl, Trifluormethyl oder Trifluormethoxy bedeutet,
sowie ihre Salze, Solvate und Solvate der Salze.

Ganz besonders bevorzugt im Rahmen der vorliegenden Erfindung sind Verbindungen der Formel (I), in welcher
- R¹: für Wasserstoff oder Fluor steht,
- L¹: für Ethan-1,2-diyl oder 1,4-Phenylen steht,
- und A: für eine Gruppe der Formel 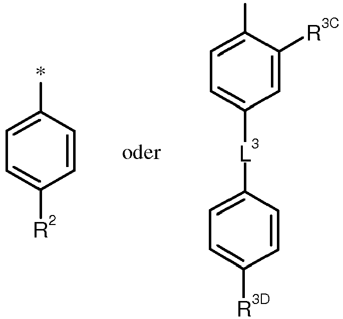
steht, (wie in den Ansprüchen definiert), worin * die jeweilige Verknüpfungsstelle mit dem Rest des Moleküls kennzeichnet,
- L³: eine Bindung oder -CH₂-CH₂- bedeutet,

- R^{3C}: Chlor bedeutet,
- und R^{3D}: Wasserstoff, Fluor oder Trifluormethyl bedeutet,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt. Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), dadurch gekennzeichnet, dass man entweder

### [A] eine Verbindung der Formel (II)

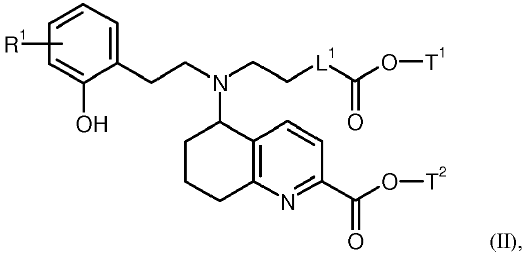

in welcher R¹ und L¹ die oben angegebenen Bedeutungen haben
und
T¹ und T² gleich oder verschieden sind und für (C₁-C₄)-Alkyl stehen,
in Gegenwart einer Base mit einer Verbindung der Formel (III) 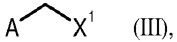
in welcher A die oben angegebenen Bedeutungen hat und
   - X¹: für eine Abgangsgruppe wie beispielsweise Chlor, Brom, Iod, Mesylat, Triflat oder Tosylat steht,
   umsetzt
   oder

### [B] eine Verbindung der Formel (IV)

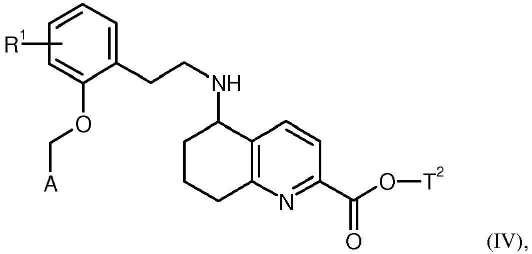

in welcher R¹ und A die oben angegebenen Bedeutungen haben und
- T²: für (C₁-C₄)-Alkyl steht,
in Gegenwart einer Base mit einer Verbindung der Formel (V) 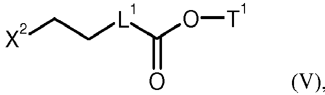 in welcher L¹ die oben angegebenen Bedeutungen hat,
- T¹: für (C₁-C₄)-Alkyl steht,
und
- X²: für eine Abgangsgruppe wie beispielsweise Chlor, Brom, Iod, Mesylat, Triflat oder Tosylat steht,
umsetzt,
und die jeweils resultierende Verbindung der Formel (VI) 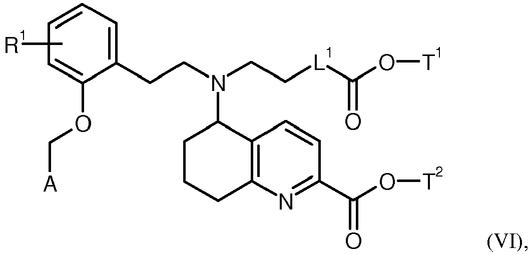 in welcher R¹, A, L¹, T¹ und T² die oben angegebenen Bedeutungen haben,
dann durch Hydrolyse der Ester-Gruppierungen -C(O)OT¹ und -C(O)OT² in die entsprechende Dicarbonsäure der Formel (I) überführt
und die so erhaltenen Verbindungen der Formel (I) gegebenenfalls in ihre Enantiomere und/oder Diastereomere trennt und/oder gegebenenfalls mit den entsprechenden (i) Lösungsmitteln und/oder (ii) Basen oder Säuren zu ihren Solvaten, Salzen und/oder Solvaten der Salze umsetzt.

Als inerte Lösungsmittel für die Verfahrensschritte (II) + (III) → (VI) und (IV) + (V) → (VI) eignen sich beispielsweise Ether wie Diethylether, Diisopropylether, Methyl-*tert*.-butylether, Tetrahydrofuran, 1,4-Dioxan, 1,2-Dimethoxyethan oder Bis-(2-methoxyethyl)-ether, Kohlenwasserstoffe wie Benzol, Toluol, Xylol, Pentan, Hexan, Heptan, Cyclohexan oder Erdölfraktionen, oder dipolar-aprotische Lösungsmittel wie Aceton, Methylethylketon, Acetonitril, *N,N*-Dimethylformamid (DMF), *N,N*-Dimethylacetamid (DMA), Dimethylsulfoxid (DMSO), *N*,*N'*-Dimethylpropylenharnstoff (DMPU) oder N-Methylpyrrolidinon (NMP). Ebenso ist es möglich, Gemische solcher Lösungsmittel einzusetzen. Bevorzugt wird Acetonitril oder Dimethylformamid verwendet.

Für die Verfahrensschritte (II) + (III) → (VI) und (IV) + (V) → (VI) geeignete Basen sind insbesondere Alkalicarbonate wie Natrium-, Kalium- oder Cäsiumcarbonat, Alkali-Alkoholate wie Natrium- oder Kaliummethanolat, Natrium- oder Kaliumethanolat oder Natrium- oder Kalium-*tert*.-butylat, Alkalihydride wie Natrium- oder Kaliumhydrid, Amide wie Natriumamid, Lithium-oder Kalium-bis(trimethylsilyl)amid oder Lithiumdiisopropylamid, oder metallorganische Verbindungen wie *n*-Butyllithium oder Phenyllithium. Bevorzugt wird Natrium-, Kalium- oder Cäsiumcarbonat als Base eingesetzt. Gegebenenfalls ist der Zusatz eines Alkylierungskatalysators, wie beispielsweise Lithiumbromid, Natrium- oder Kaliumiodid, Tetra-*n*-butylammoniumbromid oder Benzyltriethylammoniumchlorid, von Vorteil.

Die Umsetzungen (II) + (III) → (VI) und (IV) + (V) → (VI) werden im Allgemeinen in einem Temperaturbereich von 0°C bis +150°C, bevorzugt bei +50°C bis +100°C durchgeführt.

Die Hydrolyse der Ester-Gruppen -C(O)OT¹ und -C(O)OT² im Verfahrensschritt (VI) → (I) wird nach üblichen Methoden durchgeführt, indem man die Ester in inerten Lösungsmitteln mit Säuren oder Basen behandelt, wobei bei letzterer Variante die zunächst entstehenden Salze durch Behandeln mit Säure in die freien Carbonsäuren überführt werden. Im Falle der *tert*.-Butylester erfolgt die Esterspaltung bevorzugt mit Säuren.

Bei unterschiedlichen Gruppen T¹ und T² kann die Hydrolyse gegebenenfalls simultan in einer Eintopf-Reaktion oder in zwei separaten Reaktionsschritten durchgeführt werden.

Als inerte Lösungsmittel eignen sich für diese Reaktionen Wasser oder die für eine Esterspaltung üblichen organischen Lösungsmittel. Hierzu gehören bevorzugt Alkohole wie Methanol, Ethanol, *n*-Propanol, Isopropanol, *n*-Butanol oder *tert*.-Butanol, oder Ether wie Diethylether, Tetrahydrofuran, 1,4-Dioxan oder 1,2-Dimethoxyethan, oder andere Lösungsmittel wie Dichlormethan, Aceton, Methylethylketon, *N,N*-Dimethylformamid oder Dimethylsulfoxid. Ebenso ist es möglich, Gemische dieser Lösungsmittel einzusetzen. Im Falle einer basischen Ester-Hydrolyse werden bevorzugt Gemische von Wasser mit Dioxan, Tetrahydrofuran, Methanol, Ethanol, Dimethylformamid und/oder Dimethylsulfoxid verwendet. Im Falle der Umsetzung mit Trifluoressigsäure wird bevorzugt Dichlormethan und im Falle der Umsetzung mit Chlorwasserstoff bevorzugt Tetrahydrofuran, Diethylether, Dioxan oder Wasser eingesetzt.

Als Basen sind die üblichen anorganischen Basen geeignet. Hierzu gehören insbesondere Alkali- oder Erdalkalihydroxide wie beispielsweise Lithium-, Natrium-, Kalium- oder Bariumhydroxid, oder Alkali- oder Erdalkalicarbonate wie Natrium-, Kalium- oder Calciumcarbonat. Bevorzugt sind Lithium-, Natrium- oder Kaliumhydroxid.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der *tert*.-Butylester und Salzsäure im Falle der Methylester.

Die Esterspaltung erfolgt im Allgemeinen in einem Temperaturbereich von -20°C bis +120°C, bevorzugt bei 0°C bis +80°C.

Die zuvor beschriebenen Verfahrensschritte können bei normalem, bei erhöhtem oder bei erniedrigtem Druck durchgeführt werden (z.B. im Bereich von 0.5 bis 5 bar); im Allgemeinen arbeitet man jeweils bei Normaldruck.

Die Verbindungen der Formel (II) ihrerseits können dadurch hergestellt werden, dass man 5-Oxo-5,6,7,8-tetrahydrochinolin-2-carbonitril (VII) 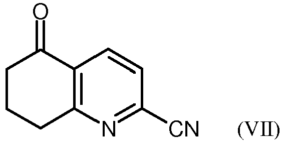 über eine reduktive Aminierung mit einem 2-(2-Methoxyphenyl)ethylamin der Formel (VIII) 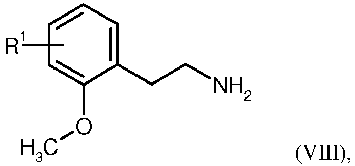 in welcher R¹ die oben angegebenen Bedeutungen hat,
zu einem sekundären Amin der Formel (IX) 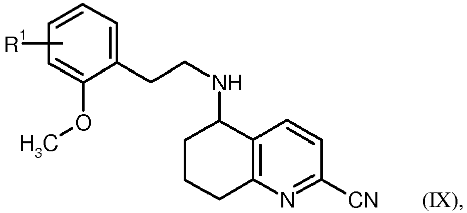 in welcher R¹ die oben angegebenen Bedeutungen hat,
umsetzt, nachfolgend in Gegenwart einer Base mit einer Verbindung der Formel (V) 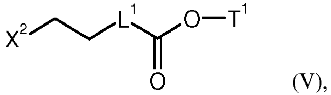 in welcher L¹, T¹ und X² die oben angegebenen Bedeutungen haben,
zu einem tertiären Amin der Formel (X) 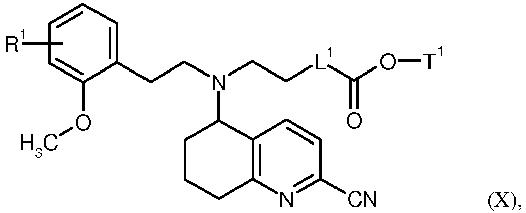 in welcher L¹, R¹ und T¹ die oben angegebenen Bedeutungen haben,
alkyliert, dann durch Behandlung mit Bortribromid oder Bromwasserstoff die phenolische Methylether-Gruppierung abspaltet und die resultierende Verbindung der Formel (XI) 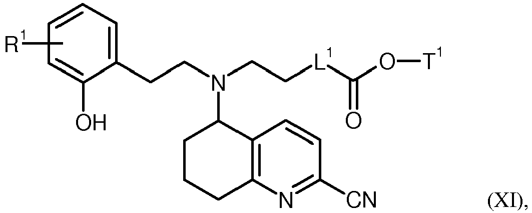 in welcher L¹, R¹ und T¹ die oben angegebenen Bedeutungen haben,
schließlich durch säurekatalysierte Solvolyse der Nitril-Gruppe mit einem Alkohol der Formel (XII)

T²-OH (XII),

in welcher T² die oben angegebene Bedeutung hat,
in den Dicarbonsäureester der Formel (II) überführt.

Die Umsetzung (VII) + (VIII) → (IX) erfolgt in einem für reduktive Aminierungen üblichen, unter den Reaktionsbedingungen inerten Lösungsmittel, gegebenenfalls in Gegenwart einer Säure und/ oder eines wasserentziehenden Mittels als Katalysator. Zu diesen Lösungsmitteln gehören beispielsweise Tetrahydrofuran, Toluol, Dichlormethan, 1,2-Dichlorethan, *N,N*-Dimethylformamid und Alkohole wie Methanol, Ethanol, *n*-Propanol oder Isopropanol; auch ist es möglich, Gemische solcher Lösungsmittel einzusetzen. Bevorzugt werden Toluol, Methanol und/oder Ethanol verwendet. Als Katalysator kommen gebräuchliche organische Säuren wie Essigsäure oder p-Toluolsulfonsäure in Betracht.

Als Reduktionsmittel für diese Aminierungsreaktion eignen sich insbesondere Borhydride wie beispielsweise Natriumborhydrid, Natriumcyanoborhydrid, Natriumtriacetoxyborhydrid oder Tetra-*n*-butylammoniumborhydrid; bevorzugt wird Natriumborhydrid eingesetzt.

Die Umsetzung (VII) + (VIII) → (IX) wird vorzugsweise in einem zweistufigen Prozess zunächst in einem Temperaturbereich von +50°C bis +120°C (zur Imin-Kondensation) und dann bei 0°C bis +30°C (zur Borhydrid-Reduktion) durchgeführt.

Die Alkylierung im Verfahrensschritt (IX) + (V) → (X) erfolgt unter analogen Reaktionsbedingungen bezüglich Lösungsmittel, Base und Temperatur, wie zuvor bei der Umsetzung (IV) + (V) → (VI) beschrieben.

Die Spaltung der phenolischen Methylether-Gruppe im Verfahrensschritt (X) → (XI) erfolgt nach üblichen Methoden durch Behandlung mit Bortribromid in Dichlormethan bei -20°C bis +10°C oder durch Erhitzen mit einer Lösung von Bromwasserstoff in Eisessig oder Wasser auf +100°C bis +130°C. Sollte unter diesen Reaktionsbedingungen gleichzeitig auch - ganz oder teilweise - die Ester-Gruppierung -C(O)OT¹ und/oder die Nitril-Gruppe hydrolysiert werden, so kann die hierdurch entstehende Dicarbonsäure der Formel (XIII) 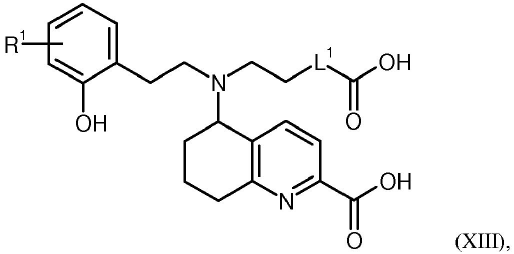 in welcher L¹ und R¹ die oben angegebenen Bedeutungen haben,
beispielsweise durch nachfolgende Behandlung mit Methanol oder Ethanol in Gegenwart von Chlorwasserstoff oder Thionylchlorid wieder zum Dicarbonsäureester der Formel (II) rückverestert werden [T¹ = T² = Methyl bzw. Ethyl in (II)].

Die Verbindungen der Formel (IV) können hergestellt werden, indem man die oben beschriebene Verbindung der Formel (IX) 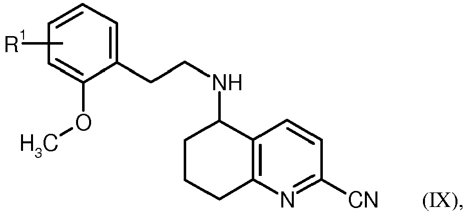 in welcher R¹ die oben angegebenen Bedeutungen hat,
zunächst mit Hilfe von wässriger Bromwasserstoffsäure in die Hydroxy-Carbonsäure der Formel (XIV) 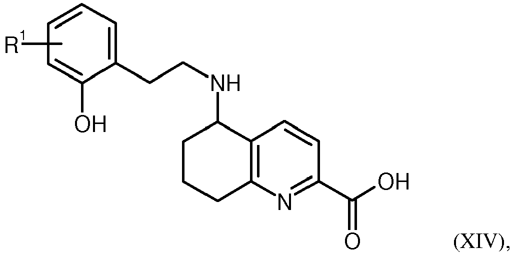 in welcher R¹ die oben angegebenen Bedeutungen hat,
überführt, anschließend unter Säure-Katalyse mit einem Alkohol der Formel (XII)

T²-OH (XII),

in welcher T² die oben angegebene Bedeutung hat,
zu einer Verbindung der Formel (XV) 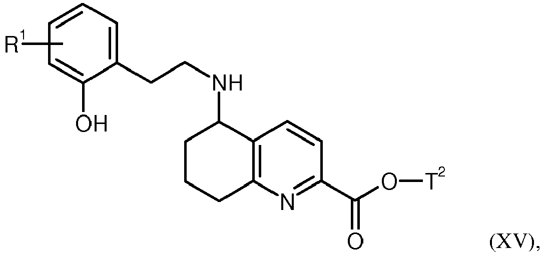 in welcher R¹ und T² die oben angegebenen Bedeutungen haben,
verestert, die Amin-Verbindung (XV) dann in ein geschütztes Derivat der Formel (XVI) 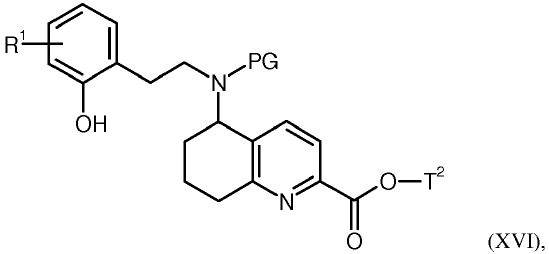 in welcher R¹ und T² die oben angegebenen Bedeutungen haben und
- PG: für eine geeignete temporäre Amino-Schutzgruppe wie beispielsweise *tert*.-Butoxycarbonyl steht,
überführt, nachfolgend in Gegenwart einer Base mit einer Verbindung der Formel (III) 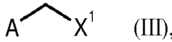 in welcher A und X¹ die oben angegebenen Bedeutungen haben,
zu einer Verbindung der Formel (XVII) 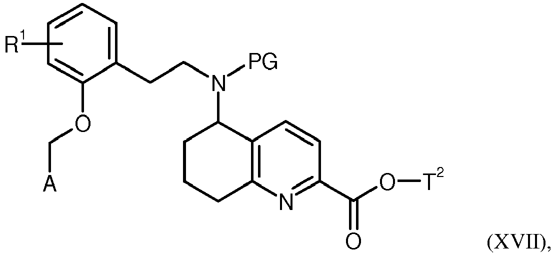 in welcher A, PG, R¹ und T² die oben angegebenen Bedeutungen haben,
alkyliert und schließlich die temporäre Schutzgruppe PG wieder abspaltet.

Die Transformation (IX) → (XIV) → (XV) wird auf analoge Weise durchgeführt, wie zuvor für die Reaktionssequenz (X) → (XI) [oder (XIII)] → (II) beschrieben.

Als Schutzgruppe PG in Verbindung (XVI) eignen sich gebräuchliche Amino-Schutzgruppen, insbesondere solche vom nicht-benzylischen Carbamat-Typ wie beispielsweise Allyloxycarbonyl (Alloc), *tert*.-Butoxycarbonyl (Boc) oder 9-Fluorenylmethoxycarbonyl (Fmoc). Die Schutzgruppe PG wird hierbei so ausgewählt, dass die Bedingungen ihrer Abspaltung im Verfahrensschritt (XVII) → (IV) kompatibel mit dem jeweils eingesetzten Ester-Rest T² sind. Einführung und Entfernung der Schutzgruppe erfolgen nach üblichen Methoden [siehe z.B. T.W. Greene und P.G.M. Wuts, Protective Groups in Organic Synthesis, Wiley, New York, 1999]. Bevorzugt wird die *tert*.-Butoxycarbonyl-Gruppe (Boc) verwendet.

Die Alkylierung im Verfahrensschritt (XVI) + (III) → (XVII) erfolgt unter analogen Reaktionsbedingungen bezüglich Lösungsmittel, Base und Temperatur, wie zuvor bei der Umsetzung (II) + (III) → (VI) beschrieben.

Die oben aufgeführte Verbindung der Formel (VII) 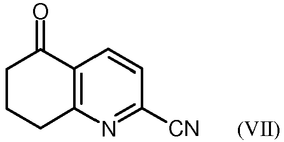 ist als solche neu und kann durch Palladium-katalysierten Halogen/Cyanid-Austausch ausgehend von der literaturbekannten Chlorverbindung (XVIII) 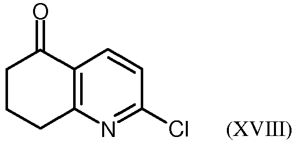 hergestellt werden (siehe nachfolgendes Reaktionsschema 1). Die Reaktion wird vorzugsweise mit Zinkcyanid mit Hilfe von Tetrakis(triphenylphosphin)palladium als Katalysator in einem dipolaraprotischen Lösungsmittel wie *N,N*-Dimethylformamid oder *N,N*-Dimethylacetamid in einem Temperaturbereich von +80°C bis +150°C durchgeführt.

Die zuvor beschriebenen Reaktionen können bei normalem, bei erhöhtem oder bei erniedrigtem Druck durchgeführt werden (z.B. im Bereich von 0.5 bis 5 bar); im Allgemeinen arbeitet man jeweils bei Normaldruck.

Eine Trennung der erfindungsgemäßen Verbindungen in die entsprechenden Enantiomere und/ oder Diastereomere kann gegebenenfalls, je nach Zweckmäßigkeit, auch bereits auf der Stufe der Verbindungen (II), (IV), (VI), (IX), (X), (XI), (XIII), (XIV), (XV), (XVI) oder (XVII) erfolgen, welche dann in separierter Form entsprechend den zuvor beschriebenen Verfahrenssequenzen weiter umgesetzt werden. Eine solche Auftrennung der Stereoisomeren läßt sich nach üblichen, dem Fachmann bekannten Methoden durchführen. Im Rahmen der vorliegenden Erfindung werden vorzugsweise chromatographische Verfahren an achiralen bzw. chiralen Trennphasen angewandt; im Falle von Carbonsäuren als Zwischen- oder Endprodukten kann alternativ auch eine Trennung über diastereomere Salze mit Hilfe chiraler Basen erfolgen.

Die Verbindungen der Formeln (III), (V), (VIII), (XII) und (XVIII) sind entweder kommerziell erhältlich oder als solche in der Literatur beschrieben, oder sie können auf für den Fachmann offenkundigem Wege in Analogie zu in der Literatur publizierten Methoden hergestellt werden. Zahlreiche detaillierte Vorschriften sowie Literaturangaben zur Herstellung der Ausgangsmaterialien befinden sich auch im Experimentellen Teil im Abschnitt zur Herstellung der Ausgangsverbindungen und Intermediate.

Die Herstellung der erfindungsgemäßen Verbindungen kann durch die folgenden Reaktionsschemata beispielhaft veranschaulicht werden:

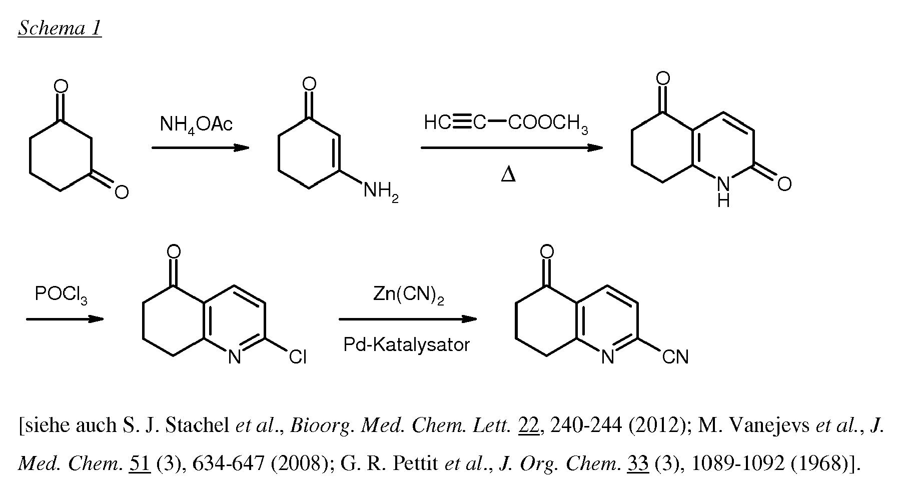

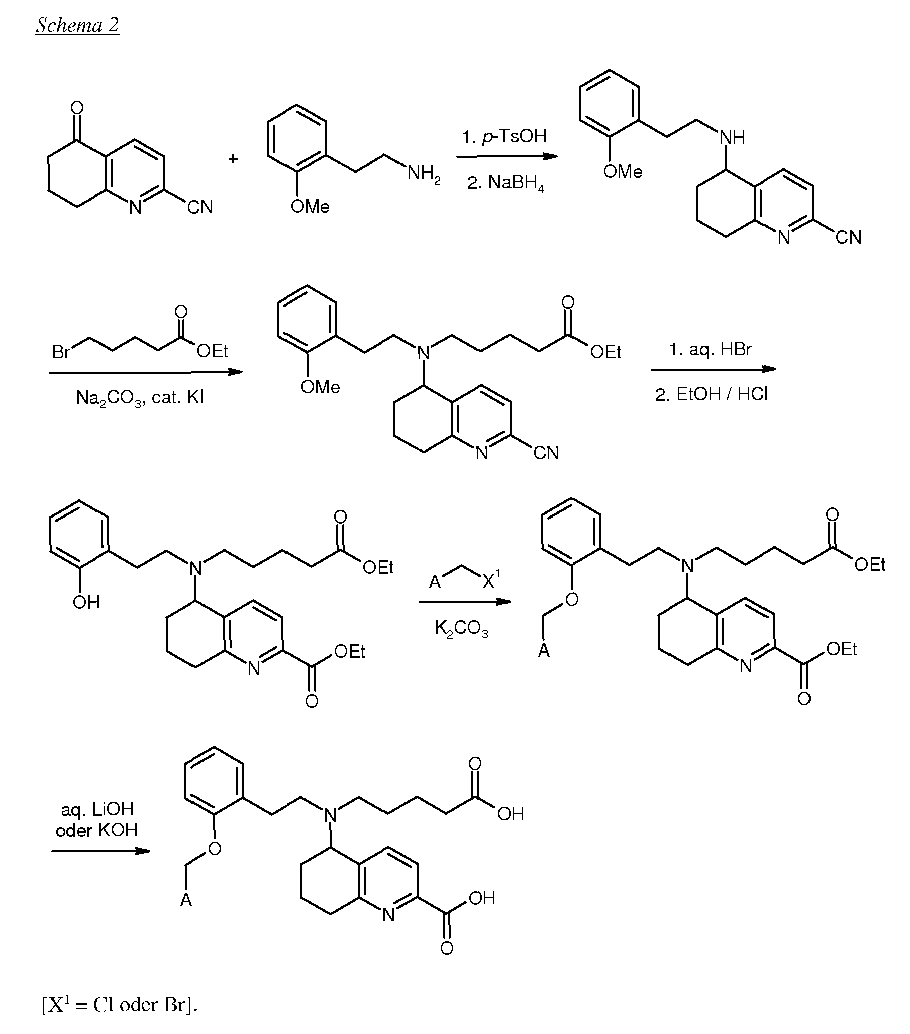

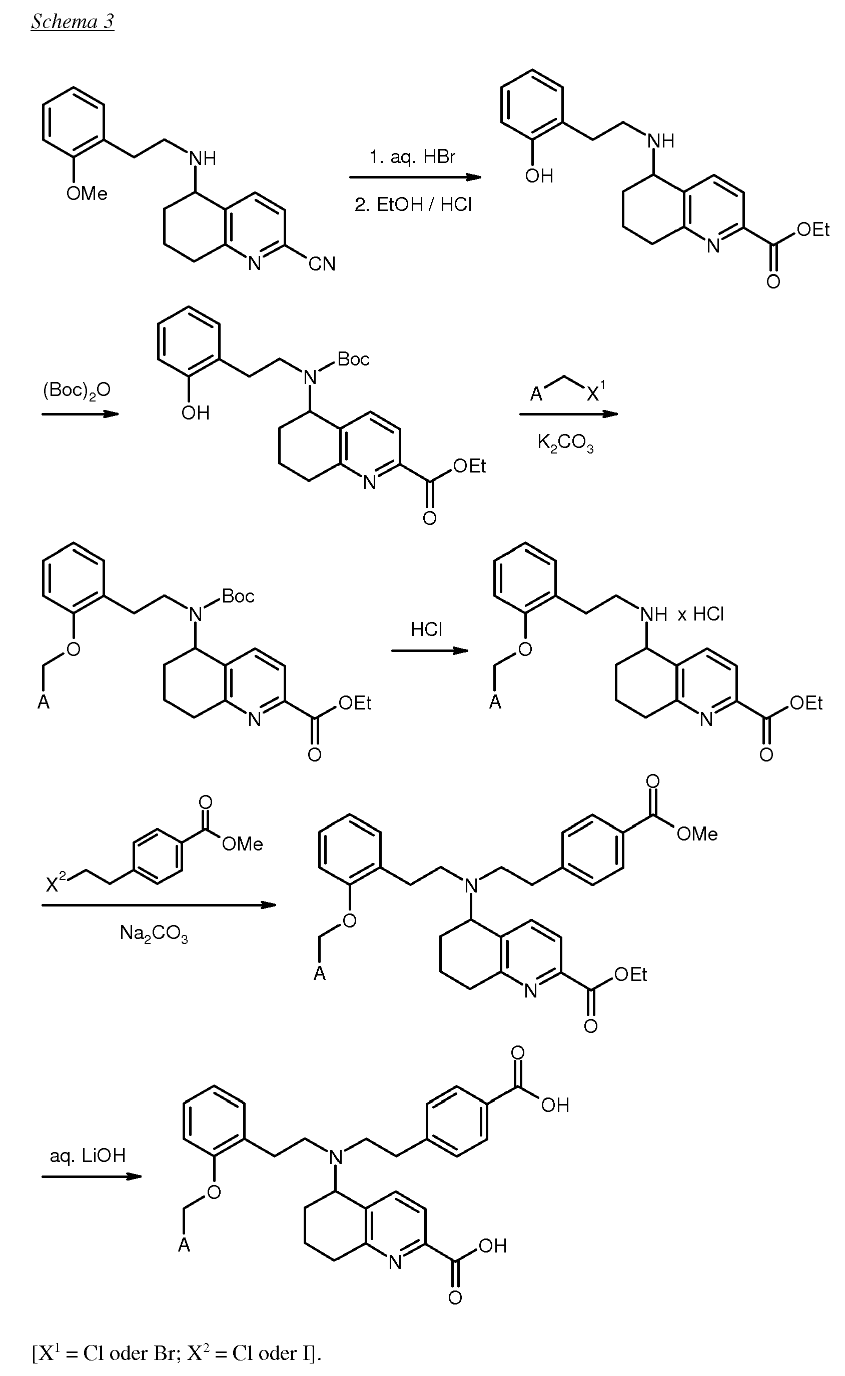

Die erfindungsgemäßen Verbindungen besitzen wertvolle pharmakologische Eigenschaften und können zur Vorbeugung und Behandlung von Erkrankungen bei Menschen und Tieren verwendet werden.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Die erfindungsgemäßen Verbindungen stellen potente Aktivatoren der löslichen Guanylatcyclase dar. Sie führen zu einer Gefäßrelaxation, zu einer Thrombozytenaggregationshemmung und zu einer Blutdrucksenkung sowie zu einer Steigerung des koronaren Blutflusses und der Mikrozirkulation. Diese Wirkungen sind über eine direkte, Häm-unabhängige Aktivierung der löslichen Guanylatcyclase und einen intrazellulären cGMP-Anstieg vermittelt.

Darüber hinaus weisen die erfindungsgemäßen Verbindungen weitere vorteilhafte Eigenschaften auf, insbesondere hinsichtlich ihrer pulmoselektiven Wirkung (gegenüber einer systemischen), ihrer Lungenretentionszeit und/oder ihrer Wirkdauer nach intrapulmonaler Gabe.

Die erfindungsgemäßen Verbindungen eignen sich in besonderem Maße zur Behandlung und/oder Prävention von kardiovaskulären, kardiopulmonalen, thromboembolischen, fibrotischen und pulmonalen Erkrankungen.

Die erfindungsgemäßen Verbindungen können daher in Arzneimitteln eingesetzt werden zur Behandlung und/oder Prävention von kardiovaskulären und kardiopulmonalen Erkrankungen, wie beispielsweise Bluthochdruck (Hypertonie), Herzinsuffizienz, koronare Herzerkrankung, stabile und instabile Angina pectoris, pulmonale arterielle Hypertonie (PAH) und sekundäre Formen der pulmonalen Hypertonie (PH), renale Hypertonie, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden des Grades I-III, supraventrikuläre Tachyarrhythmie, Vorhofflimmern, Vorhofflattern, Kammerflimmern, Kammerflattern, ventrikuläre Tachyarrhythmie, Torsade de pointes-Tachykardie, Extrasystolen des Vorhofs und des Ventrikels, AVjunktionale Extrasystolen, Sick-Sinus-Syndrom, Synkopen, AV-Knoten-Reentry-Tachykardie, Wolff-Parkinson-White-Syndrom, akutes Koronarsyndrom (ACS), autoimmune Herzerkrankungen (Perikarditis, Endokarditis, Valvolitis, Aortitis, Kardiomyopathien), Boxerkardiomyopathie, Aneurysmen, Schock wie kardiogener Schock, septischer Schock und anaphylaktischer Schock, ferner zur Behandlung und/oder Prävention von thromboembolischen Erkrankungen und Ischämien, wie myokardiale Ischämie, Myokardinfarkt, Hirnschlag, Herzhypertrophie, transistorische und ischämische Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, periphere Durchblutungsstörungen, Reperfusionsschäden, arterielle und venöse Thrombosen, Mikroalbuminurie, Herzmuskelschwäche, endotheliale Dysfunktion, mikro- und makrovaskuläre Schädigungen (Vaskulitis), sowie zur Verhinderung von Restenosen beispielsweise nach Thrombolyse-Therapien, percutan-transluminalen Angioplastien (PTA), percutan-transluminalen Koronarangioplastien (PTCA), Herztransplantationen und Bypass-Operationen.

Im Sinne der vorliegenden Erfindung umfasst der Begriff pulmonale Hypertonie sowohl primäre als auch sekundäre Unterformen hiervon, wie sie nach der Dana Point-Klassifikation gemäß ihrer jeweiligen Ätiologie definiert worden sind [siehe D. Montana und G. Simonneau, in: A.J. Peacock et al. (Eds.), Pulmonary Circulation. Diseases and their treatment, 3rd edition, Hodder Arnold Publ., 2011, S. 197-206; M.M. Hoeper et al., J. Am. Coll. Cardiol. 2009, 54 (1), S85-S96]. Hierzu gehört insbesondere in Gruppe 1 die pulmonal-arterielle Hypertonie (PAH), zu der unter anderem die idiopathischen und die familiären Formen zählen (IPAH bzw. FPAH). Des weiteren umfasst PAH auch die persistierende pulmonale Hypertonie bei Neugeborenen sowie die assoziierte pulmonal-arterielle Hypertonie (APAH), welche assoziiert ist mit Kollagenosen, kongenitalen systemisch-pulmonalen Shuntvitien, portaler Hypertension, HIV-Infektionen, der Einnahme bestimmter Drogen und Medikamente (z.B. von Appetitzüglern), mit Erkrankungen mit einer signifikanten venösen/kapillären Beteiligung wie der pulmonal-venookklusiven Erkrankung und der pulmonal-kapillären Hämangiomatose, oder mit anderen Erkrankungen wie Schilddrüsenerkrankungen, Glykogenspeicherkrankheiten, Morbus Gaucher, hereditärer Teleangiektasie, Hämoglobinopathien, myeloproliferativen Erkrankungen und Splenektomie. In Gruppe 2 der Dana Point-Klassifikation werden PH-Patienten mit einer ursächlichen Linksherzerkrankung, wie ventrikulären, atrialen oder valvulären Erkrankungen, zusammengefasst. Gruppe 3 umfasst Formen der pulmonalen Hypertonie, die mit einer Lungenerkrankung, wie z.B. chronisch-obstruktiver Lungenerkrankung (COPD), interstitieller Lungenkrankheit (ILD), Lungenfibrose (IPF), und/oder einer Hypoxämie (z.B. Schlafapnoe-Syndrom, alveoläre Hypoventilation, chronische Höhenkrankheit, anlagebedingte Fehlbildungen) assoziiert sind. Zur Gruppe 4 zählen PH-Patienten mit chronischthrombotischen und/oder embolischen Erkrankungen, z.B. bei thromboembolischer Obstruktion von proximalen und distalen Lungenarterien (CTEPH) oder bei nicht-thrombotischen Embolisierungen (z.B. infolge von Tumorerkrankungen, Parasiten, Fremdkörpern). Seltenere Formen der pulmonalen Hypertonie, wie z.B. bei Patienten mit Sarkoidose, Histiozytose X oder Lymphangiomatose, sind in der Gruppe 5 zusammengefasst.

Im Sinne der vorliegenden Erfindung umfasst der Begriff Herzinsuffizienz sowohl akute als auch chronische Erscheinungsformen der Herzinsuffizienz wie auch spezifische oder verwandte Krankheitsformen hiervon, wie akute dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen sowie diastolische und systolische Herzinsuffizienz.

Darüber hinaus können die erfindungsgemäßen Verbindungen auch zur Behandlung und/oder Prävention von Arteriosklerose, Lipidstoffwechselstörungen, Hypolipoproteinämien, Dyslipidämien, Hypertriglyceridämien, Hyperlipidämien, kombinierten Hyperlipidämien, Hypercholesterolämien, Abetalipoproteinämie, Sitosterolämie, Xanthomatose, Tangier-Krankheit, Fettsucht (Adipositas), Fettleibigkeit (Obesitas) sowie des Metabolischen Syndroms eingesetzt werden.

Weiterhin können die erfindungsgemäßen Verbindungen zur Behandlung und/oder Prävention von primärem und sekundärem Raynaud-Phänomen, Mikrozirkulationsstörungen, Claudicatio, Tinnitus, peripheren und autonomen Neuropathien, diabetischen Mikroangiopathien, diabetischer Retinopathie, diabetischen Geschwüren an den Extremitäten, Gangrän, CREST-Syndrom, Erythematose, Onychomykose sowie von rheumatischen Erkrankungen verwendet werden.

Die erfindungsgemäßen Verbindungen können darüber hinaus zur Verhinderung von ischämie- und/oder reperfusionsbedingten Schädigungen von Organen oder Geweben sowie als Zusatzstoffe für Perfusions- und Konservierungslösungen von Organen, Organteilen, Geweben oder Gewebeteilen menschlichen oder tierischen Ursprungs, insbesondere bei chirurgischen Eingriffen oder im Bereich der Transplantationsmedizin, Verwendung finden.

Die erfindungsgemäßen Verbindungen eignen sich außerdem zur Behandlung und/oder Prävention von Nierenerkrankungen, insbesondere von Niereninsuffizienz und Nierenversagen. Im Sinne der vorliegenden Erfindung umfassen die Begriffe Niereninsuffizienz und Nierenversagen sowohl akute als auch chronische Erscheinungsformen hiervon wie auch diesen zugrundeliegende oder verwandte Nierenerkrankungen, wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantat-Abstoßung und Immunkomplex-induzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prävention von Folgeerscheinungen einer Niereninsuffizienz, wie beispielsweise Hypertonie, Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Darüber hinaus sind die erfindungsgemäßen Verbindungen zur Behandlung und/oder Prävention von Erkrankungen des Urogenitalsystems geeignet, wie beispielsweise benignes Prostata-Syndrom (BPS), benigne Prostatahyperplasie (BPH), benigne Prostatavergrößerung (BPE), Blasenentleerungsstörungen (BOO), untere Harnwegssyndrome (LUTS), neurogene überaktive Blase (OAB), Inkontinenz wie beispielsweise Misch-, Drang-, Stress- oder Überlauf-Inkontinenz (MUI, UUI, SUI, OUI), Beckenschmerzen sowie erektile Dysfunktion und weibliche sexuelle Dysfunktion.

Die erfindungsgemäßen Verbindungen eignen sich auch zur Behandlung und/oder Prävention von asthmatischen Erkrankungen, chronisch-obstruktiven Atemwegserkrankungen (COPD), des akuten Atemwegssyndroms (ARDS) und der akuten Lungenschädigung (ALI), alpha-1-Antitrypsin-Defizienz (AATD), Lungenfibrose, Lungenemphysem (z.B. durch Zigarettenrauch induziertes Lungenemphysem) und zystischer Fibrose (CF).

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierte Lern- und Gedächtnisstörungen, altersassoziierte Gedächtnisverluste, vaskuläre Demenz, Schädel-Hirn-Trauma, Schlaganfall, Demenz, die nach Schlaganfällen auftritt ("post stroke dementia"), post-traumatisches Schädel-Hirn-Trauma, allgemeine Konzentrationsstörungen, Konzentrationsstörungen bei Kindern mit Lern- und Gedächtnisproblemen, Alzheimer'sche Krankheit, Demenz mit Lewy-Körperchen, Demenz mit Degeneration der Frontallappen einschließlich des Pick's-Syndroms, Parkinson'sche Krankheit, progressiver nuclear palsy, Demenz mit corticobasaler Degeneration, Amyolateralsklerose (ALS), Huntington'sche Krankheit, Demyelinisation, Multiple Sklerose, thalamische Degeneration, Creutzfeld-Jacob-Demenz, HIV-Demenz, Schizophrenie mit Demenz oder Korsakoff-Psychose. Sie eignen sich auch zur Behandlung und/oder Prävention von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen eingesetzt werden.

Zudem besitzen die erfindungsgemäßen Verbindungen anti-inflammatorische Wirkung und können daher als entzündungshemmende Mittel zur Behandlung und/oder Prävention von Sepsis (SIRS), multiplem Organversagen (MODS, MOF), entzündlichen Erkrankungen der Niere, chronischen Darmentzündungen (IBD, Morbus Crohn, Colitis ulcerosa), Pankreatitis, Peritonitis, rheumatoiden Erkrankungen, entzündlichen Hauterkrankungen und entzündlichen Augenerkrankungen eingesetzt werden.

Die erfindungsgemäßen Verbindungen sind ferner zur Behandlung und/oder Prävention von fibrotischen Erkrankungen der inneren Organe, wie beispielsweise der Lunge, des Herzens, der Niere, des Knochenmarks und insbesondere der Leber, sowie von dermatologischen Fibrosen und fibrotischen Erkrankungen des Auges geeignet. Im Sinne der vorliegenden Erfindung umfasst der Begriff fibrotische Erkrankungen insbesondere solche Erkrankungen wie Leberfibrose, Leberzirrhose, Lungenfibrose, Endomyokardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung, Naevi, diabetische Retinopathie, proliferative Vitroretinopathie und Erkrankungen des Bindegewebes (z.B. Sarkoidose). Die erfindungsgemäßen Verbindungen können ebenso verwendet werden zur Förderung der Wundheilung, zur Bekämpfung postoperativer Narbenbildung, z.B. nach Glaukom-Operationen, und zu kosmetischen Zwecken bei alternder oder verhornender Haut.

Aufgrund ihres Wirkprofils eignen sich die erfindungsgemäßen Verbindungen insbesondere zur Behandlung und/oder Prävention von kardiovaskulären und kardiopulmonalen Erkrankungen wie primären und sekundären Formen der pulmonalen Hypertonie, Herzinsuffizienz, Angina pectoris und Hypertonie sowie von thromboembolischen Erkrankungen, Ischämien, Gefäßerkrankungen, Mikrozirkulationsstörungen, Niereninsuffizienz, fibrotischen Erkrankungen und Arteriosklerose.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Arzneimittel, enthaltend mindestens eine der erfindungsgemäßen Verbindungen, zur Behandlung und/oder Prävention von Erkrankungen, insbesondere der zuvor genannten Erkrankungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 4-Inhibitoren wie Roflumilast oder Revamilast und PDE 5-Inhibitoren wie Sildenafil, Vardenafil, Tadalafil, Udenafil, Dasantafil, Avanafil, Mirodenafil oder Lodenafil;
- NO-unabhängige, jedoch Häm-abhängige Stimulatoren der Guanylatcyclase, wie insbesondere Riociguat sowie die in WO 00/06568, WO 00/06569, WO 02/42301, WO 03/095451, WO 2011/147809, WO 2012/004258, WO 2012/028647 und WO 2012/059549 beschriebenen Verbindungen;
- Prostacyclin-Analoga und IP-Rezeptor-Agonisten, wie beispielhaft und vorzugsweise Iloprost, Beraprost, Treprostinil, Epoprostenol oder NS-304;
- Endothelin-Rezeptor-Antagonisten, wie beispielhaft und vorzugsweise Bosentan, Darusentan, Ambrisentan oder Sitaxsentan;
- Verbindungen, die die humane neutrophile Elastase (HNE) inhibieren, wie beispielhaft und vorzugsweise Sivelestat oder DX-890 (Reltran);
- die Signaltransduktionskaskade inhibierende Verbindungen, insbesondere aus der Gruppe der Tyrosinkinase-Inhibitoren, wie beispielhaft und vorzugsweise Dasatinib, Nilotinib, Bosutinib, Regorafenib, Sorafenib, Sunitinib, Cediranib, Axitinib, Telatinib, Imatinib, Brivanib, Pazopanib, Vatalanib, Gefitinib, Erlotinib, Lapatinib, Canertinib, Lestaurtinib, Pelitinib, Semaxanib, Masitinib oder Tandutinib;
- die Rho-Kinase inhibierende Verbindungen, wie beispielhaft und vorzugsweise Fasudil, Y-27632, SLx-2119, BF-66851, BF-66852, BF-66853, KI-23095 oder BA-1049;
- anti-obstruktiv wirkende Mittel, wie sie z.B. zur Therapie einer chronisch-obstruktiven Lungenerkrankung (COPD) oder eines Asthma bronchiale eingesetzt werden, wie beispielhaft und vorzugsweise inhalativ oder systemisch angewendete beta-Rezeptor-Mimetika (z.B. Bedoradrine) oder inhalativ angewendete anti-muscarinerge Substanzen;
- entzündungshemmmende und/oder immunsuppressive Mittel, wie sie z.B. zur Therapie einer chronisch-obstruktiven Lungenerkrankung (COPD), eines Asthma bronchiale oder einer Lungenfibrose eingesetzt werden, wie beispielhaft und vorzugsweise systemisch oder inhalativ angewendete Corticosteroide, Flutiform, Pirfenidon, Acetylcystein, Azathioprin oder BIBF-1120;
- Chemotherapeutika, wie sie z.B. zur Therapie von Neubildungen (Neoplasien) der Lunge oder anderer Organe eingesetzt werden;
- Wirkstoffe, die zur systemischen und/oder inhalativen Behandlung von Lungenerkrankungen eingesetzt werden, wie beispielsweise bei zystischer Fibrose (alpha-1-Antitrypsin, Aztreonam, Ivacaftor, Lumacaftor, Ataluren, Amikacin, Levofloxacin), chronisch-obstruktiven Atemwegserkrankungen (COPD) (LAS40464, PT003, SUN-101), akutem Atemwegssyndrom (ARDS) und akuter Lungenschädigung (ALI) (Interferon-beta-1a, Traumakine), obstruktiver Schlafapnoe (VI-0521), Bronchiektasie (Mannitol, Ciprofloxacin), Bronchiolitis obliterans (Cyclosporin, Aztreonam) und Sepsis (Pagibaximab, Voluven, ART-123);
- Wirkstoffe, die zur Behandlung von Muskeldystrophie eingesetzt werden, wie beispielsweise Idebenone;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Dabigatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, Apixaban, Fidexaban, Razaxaban, Fondaparinux, Idraparinux, DU-176b, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, intrapulmonal, nasal, sublingual, lingual, buccal, rectal, dermal, transdermal, conjunctival, otisch oder als Implantat oder Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer, Dosieraerosole), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale, die intrapulmonale (inhalative) und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht. Bei intrapulmonaler Applikation beträgt die Menge im Allgemeinen etwa 0.1 bis 50 mg je Inhalation.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- abs.: absolut
- Ac: Acetyl
- aq.: wässrig, wässrige Lösung
- Boc: *tert*.-Butoxycarbonyl
- Bsp.: Beispiel
- Bu: Butyl
- c: Konzentration
- ca.: *circa,* ungefähr
- cat.: katalytisch
- CI: chemische Ionisation (bei MS)
- d: Tag(e)
- DC: Dünnschichtchromatographie
- DCI: direkte chemische Ionisation (bei MS)
- de: Diastereomerenüberschuss
- DMA: *N,N*-Dimethylacetamid
- DMF: *N,N*-Dimethylformamid
- DMSO: Dimethylsulfoxid
- d. Th.: der Theorie (bei Ausbeute)
- ee: Enantiomerenüberschuss
- EI: Elektronenstoß-Ionisation (bei MS)
- ent: enantiomerenrein, Enantiomer
- eq.: Äquivalent(e)
- ESI: Elektrospray-Ionisation (bei MS)
- Et: Ethyl
- GC: Gaschromatographie
- ges.: gesättigt
- h: Stunde(n)
- HPLC: Hochdruck-, Hochleistungsflüssigchromatographie
- iPr: Isopropyl
- konz.: konzentriert
- LC-MS: Flüssigchromatographie-gekoppelte Massenspektroskopie
- Me: Methyl
- min: Minute(n)
- MS: Massenspektroskopie
- NMR: Kernresonanzspektroskopie
- p: para
- Ph: Phenyl
- Pr: Propyl
- rac: racemisch, Racemat
- R_{f}: Retentionsindex (bei DC)
- RP: reverse phase (Umkehrphase, bei HPLC)
- RT: Raumtemperatur
- Rₜ: Retentionszeit (bei HPLC oder GC)
- s.o.: siehe oben
- tBu: *tert*.-Butyl
- TFA: Trifluoressigsäure
- THF: Tetrahydrofuran
- Ts: Toluolsulfonyl (Tosyl)
- UV: Ultraviolett-Spektroskopie
- v/v: Volumen zu Volumen-Verhältnis (einer Lösung)
- zus.: zusammen

### GC-MS- und LC-MS-Methoden:

### Methode 1 (LC-MS):

Instrument: Waters Acquity SQD UPLC System; Säule: Waters Acquity UPLC HSS T3 1.8 µ, 50 mm x 1 mm; Eluent A: 1 1 Wasser + 0.25 ml 99%-ige Ameisensäure, Eluent B: 1 1 Acetonitril + 0.25 ml 99%-ige Ameisensäure; Gradient: 0.0 min 90% A → 1.2 min 5% A → 2.0 min 5% A; Fluss: 0.40 ml/min; Ofen: 50°C; UV-Detektion: 210-400 nm.

### Methode 2 (LC-MS):

Instrument: Micromass Quattro Premier mit Waters UPLC Acquity; Säule: Thermo Hypersil GOLD 1.9 µ, 50 mm x 1 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 97% A → 0.5 min 97% A → 3.2 min 5% A → 4.0 min 5% A; Fluss: 0.3 ml/min; Ofen: 50°C; UV-Detektion: 210 nm.

### Methode 3 (LC-MS):

Instrument: Waters Acquity SQD UPLC System; Säule: Waters Acquity UPLC HSS T3 1.8 µ, 50 mm x 1 mm; Eluent A: 1 1 Wasser + 0.25 ml 99%-ige Ameisensäure, Eluent B: 1 1 Acetonitril + 0.25 ml 99%-ige Ameisensäure; Gradient: 0.0 min 90% A → 1.2 min 5% A → 2.0 min 5% A; Fluss: 0.40 ml/min; Ofen: 50°C; UV-Detektion: 208-400 nm.

### Methode 4 (LC-MS):

Instrument: Waters Acquity SQD UPLC System; Säule: Waters Acquity UPLC HSS T3 1.8 µ, 30 mm x 2 mm; Eluent A: 1 1 Wasser + 0.25 ml 99%-ige Ameisensäure, Eluent B: 1 1 Acetonitril + 0.25 ml 99%-ige Ameisensäure; Gradient: 0.0 min 90% A → 1.2 min 5% A → 2.0 min 5% A; Fluss: 0.60 ml/min; Ofen: 50°C; UV-Detektion: 208-400 nm.

### Methode 5 (GC-MS):

Instrument: Thermo DFS, Trace GC Ultra; Säule: Restek RTX-35, 15 m x 200 µm x 0.33 µm; konstanter Fluss mit Helium: 1.20 ml/min; Ofen: 60°C; Inlet: 220°C; Gradient: 60°C, 30°C/min → 300°C (3.33 min halten).

### Methode 6 (LC-MS):

Instrument: Waters Acquity SQD UPLC System; Säule: Waters Acquity UPLC HSS T3 1.8 µ, 50 mm x 1 mm; Eluent A: 1 1 Wasser + 0.25 ml 99%-ige Ameisensäure, Eluent B: 1 1 Acetonitril + 0.25 ml 99%-ige Ameisensäure; Gradient: 0.0 min 95% A → 6.0 min 5% A → 7.5 min 5% A; Fluss: 0.35 ml/min; Ofen: 50°C; UV-Detektion: 210-400 nm.

### Ausgangsverbindungen und Intermediate:

### Beispiel 1A

(nur Beispiele, welche unter die Ansprüche fallen, sind erfindtingsgemäss) 3-Aminocyclohex-2-en-1-on 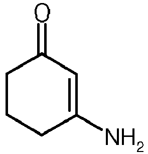 Eine Lösung von 250 g (2.2 mol) Cyclohexan-1,3-dion und 180.45 g (2.3 mol) Ammoniumacetat in 1.3 Liter Toluol wurde 2 Stunden unter Verwendung eines Wasserabscheiders mit Rückflusskühler unter Rückfluss erhitzt. Anschließend wurde der Ansatz bis zur Trockene eingeengt. Der Rückstand wurde in 1.3 Liter Essigsäureethylester und 100 ml Methanol aufgenommen und auf 110°C erhitzt. Die Lösung wurde danach heiß filtriert und langsam auf Raumtemperatur abgekühlt. Anschließend wurde die Lösung über Nacht bei ca. 4°C im Kühlschrank gelagert. Der erhaltene kristalline Niederschlag wurde abfiltriert und im Vakuum getrocknet. Es wurden 66.59 g (0.60 mol) als erste Charge des Zielprodukts erhalten. Das zurückgewonnene Filtrat wurde im Vakuum auf ein Volumen von ca. 800 ml eingeengt, mit etwas kristallinem Produkt angeimpft und dann 12 Tage lang bei ca. 4°C gelagert. Der erhaltene kristalline Niederschlag wurde abfiltriert und im Vakuum getrocknet. Es wurden so weitere 13.28 g (0.12 mol) des Zielprodukts erhalten. Das zurückgewonnene Filtrat wurde im Vakuum bis zur Trockene eingeengt. Der Rückstand wurde in 100 ml eines Gemischs aus Essigsäureethylester und Methanol (10:1) gelöst, auf Kieselgel aufgezogen und chromatographisch an Kieselgel gereinigt (Laufmittel: Essigsäureethylester/Methanol 10:1). Es wurden hierdurch weitere 113.79 g (1.02 mol) des gewünschten Produkts als gelber Feststoff isoliert. Insgesamt wurden so 193.66 g (1.74 mol, 78% d. Th.) des Zielprodukts erhalten.
¹H-NMR (400 MHz, DMSO-*d*₆, δ/ppm): 1.71-1.84 (m, 2H), 2.01 (t, 2H), 2.25 (t, 2H), 4.91 (s, 1H), 6.39-6.99 (br. s, 2H).

### Beispiel 2A

### 7,8-Dihydrochinolin-2,5(1H,6H)-dion

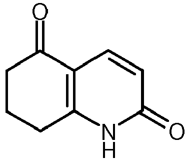

113.79 g (1.02 mol) 3-Aminocyclohex-2-en-1-on und 114.37 ml (1.19 mol) Propiolsäuremethylester wurden 1 Stunde unter Rühren auf 105°C erhitzt. Die entstandene homogene dunkle Lösung wurde anschließend langsam weiter auf 170°C erhitzt. Nach 20 min (Temperatur: 135°C) entstand eine zähe Masse und es kam zu einer deutlichen Gasentwicklung. Nach weiteren 15 min (Temperatur: 160°C) wurde die Reaktionsmasse noch zäher, während die Gasentwicklung abnahm. Nach insgesamt 42 min wurde die Temperatur von 170°C erreicht. Nach weiteren 13 min bei dieser Temperatur wurde die Reaktionsmasse auf Raumtemperatur abgekühlt. Anschließend wurde der Ansatz mit 200 ml Dichlormethan versetzt, kurz erhitzt, ins Ultraschallbad gestellt und der entstandene kristalline Rückstand abfiltriert. Diese Prozedur wurde mit weiteren 200 ml Dichlormethan noch einmal wiederholt. Die so erhaltenen kristallinen Rückstände wurden vereinigt, in 1.6 Liter Methanol aufgenommen und dann unter Rühren erhitzt, bis der Feststoff vollständig in Lösung ging. Diese Lösung wurde danach langsam auf Raumtemperatur abgekühlt und anschließend über 2 Tage bei ca. 4°C im Kühlschrank gelagert. Der kristalline Niederschlag wurde abfiltriert und im Vakuum getrocknet. Es wurden 47.65 g (0.29 mol, 29% d. Th.) des Zielprodukts erhalten.

¹H-NMR (400 MHz, DMSO-*d*₆, δ/ppm): 1.90-2.07 (m, 2H), 2.42 (t, 2H), 2.78 (t, 2H), 6.23 (d, 1H), 7.76 (d, 1H), 12.06 (br. s, 1H).

### Beispiel 3A

### 2-Chlor-7,8-dihydrochinolin-5(6H)-on

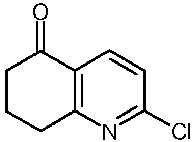

Unter Stickstoff wurden 21.02 g (0.13 mol) 7,8-Dihydrochinolin-2,5(1*H*,6*H*)-dion in 100 ml Acetonitril (wasserfrei, < 30 ppm H₂O) suspendiert und mit 135.28 ml (Dichte 1.46 g/ml, 1.29 mol) Phosphoroxychlorid versetzt. Die gelbliche Suspension wurde dann auf 75°C erhitzt und 1.25 Stunden bei dieser Temperatur gerührt. Anschließend wurde die gelbe, klare Lösung auf Raumtemperatur abgekühlt und mit 150 ml Toluol versetzt. Die Lösung wurde danach am Rotationsverdampfer auf ca. 100 ml eingeengt und nochmals mit 150 ml Toluol versetzt. Anschließend wurde die Lösung am Rotationsverdampfer bis zur Trockene eingeengt. Das erhaltene orangefarbene Öl wurde mit 300 ml Essigsäureethylester versetzt. Die Lösung wurde nachfolgend vorsichtig (Gasentwicklung) auf 500 ml gesättigte wässrige Natriumhydrogencarbonat-Lösung gegeben und 15 min gerührt. Die Phasen wurden getrennt, und die wässrige Phase wurde mit 200 ml Essigsäureethylester extrahiert. Die vereinigten organischen Phasen wurden zweimal mit 250 ml Wasser und einmal mit 100 ml gesättigter Natriumchlorid-Lösung gewaschen, über Natriumsulfat getrocknet, filtriert und im Vakuum bis zur Trockene eingeengt. Es wurden 22.58 g (0.12 mmol, 96% d. Th.) der Zielverbindung als leicht gelblicher Feststoff erhalten.

¹H-NMR (400 MHz, DMSO-*d*₆, δ/ppm): 2.06-2.17 (m, 2H), 2.61-2.70 (m, 2H), 3.05 (t, 2H), 7.51 (d, 1H), 8.18 (d, 1H).

### Beispiel 4A

### 5-Oxo-5,6,7,8-tetrahydrochinolin-2-carbonitril

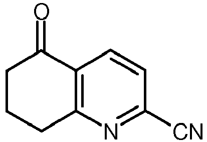

Unter Stickstoff wurden 42.25 g (0.23 mol) 2-Chlor-7,8-dihydrochinolin-5(6*H*)-on, 54.64 g (0.47 mol) Zinkcyanid und 13.44 g (0.01 mol) Tetrakis(triphenylphosphin)palladium in 200 ml wasserfreien *N,N*-Dimethylacetamid (Wassergehalt < 0.01%, zuvor mit Stickstoff entgast) suspendiert, auf 100°C erhitzt und 2 Stunden bei dieser Temperatur gerührt. Nach vollständiger Umsetzung (DC-Kontrolle, Laufmittel Petrolether/Essigsäureethylester 2:1) wurde das Reaktionsgemisch (graue Suspension) auf Raumtemperatur abgekühlt, über Celite filtriert und der Filterkuchen mit 500 ml Essigsäureethylester gewaschen. Die erhaltene organische Lösung wurde nachfolgend mit 200 ml gesättigter wässriger Natriumchlorid-Lösung versetzt. Dabei entstand ein weißer Niederschlag, der abfiltriert und verworfen wurde. Die organische Phase wurde abgetrennt, dreimal mit jeweils 200 ml gesättigter Natriumchlorid-Lösung gewaschen, über Natriumsulfat getrocknet, filtriert und bis zur Trockene eingeengt. Der erhaltene Rückstand wurde auf 20 g Kieselgel aufgezogen und durch Säulenchromatographie an Kieselgel gereinigt (80 g-Kartusche; Fluss: 60 ml/min; Laufmittel: Petrolether/Essigsäureethylester 95:5 → 60:40 innerhalb von 40 min, danach isokratisch Petrolether/Essigsäureethylester 60:40 für 30 min). Es wurden 26.35 g (0.15 mmol, 66% d. Th.) der Zielverbindung erhalten.

MS (EI): m/z = 172 (M)⁺.

¹H-NMR (400 MHz, CDCl₃, δ/ppm): 2.19-2.30 (m, 2H), 2.70-2.79 (m, 2H), 3.20 (t, 2H), 7.67 (d, 1H), 8.39 (d, 1H).

### Beispiel 5A

### rac-5-{[2-(2-Methoxyphenyl)ethyl]amino}-5,6,7,8-tetrahydrochinolin-2-carbonitril

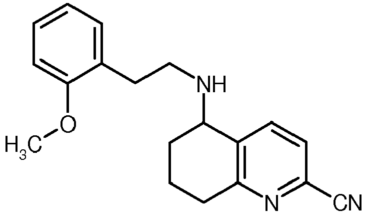

41.10 g (0.24 mol) 5-Oxo-5,6,7,8-tetrahydrochinolin-2-carbonitril wurden in 500 ml Toluol gelöst und mit 35.51 ml (0.25 mol) 2-(2-Methoxyphenyl)ethylamin und 4.54 g (0.024 mol) *p*-Toluolsulfonsäure-Monohydrat versetzt. Danach wurde die Reaktionslösung 5 Stunden unter Rückfluss (unter Einsatz eines Wasserabscheiders) gerührt. Anschließend wurde die Reaktionslösung bis zur Trockene eingedampft, der Rückstand in 500 ml Ethanol (wasserfrei) aufgenommen und unter Rühren auf 0°C abgekühlt. Die Reaktionslösung wurde dann portionsweise mit 18.06 g (0.48 mol) Natriumborhydrid versetzt (Vorsicht: Reaktionsgemisch schäumt) und über Nacht gerührt. Nachfolgend wurde das Reaktionsgemisch am Rotationsverdampfer auf ca. 100 ml eingeengt und mit 300 ml Wasser und 300 ml Essigsäureethylester versetzt. Die Phasen wurden getrennt und die wässrige Phase zweimal mit jeweils 150 ml Essigsäureethylester extrahiert. Die vereinigten organischen Phasen wurden zweimal mit jeweils 250 ml gesättigter Natriumchlorid-Lösung gewaschen, über Natriumsulfat getrocknet, filtriert und bis auf ein Volumen von ca. 150 ml am Rotationsverdampfer eingeengt. Die so erhaltene Lösung wurde auf 50 g Kieselgel aufgezogen und durch Säulenchromatographie an Kieselgel gereinigt (80 g-Kartusche; Fluss: 75 ml/min; Laufmittel: Petrolether/Essigsäureethylester 85:15 → 50:50 innerhalb von 45 min). Es wurden 39.03 g (0.10 mol, Gehalt 80%, 43% d. Th.) der Zielverbindung erhalten.

¹H-NMR (400 MHz, CDCl₃, δ/ppm): 1.68-1.88 (m, 2H), 1.98-2.10 (m, 2H), 2.76-3.02 (m, 6H), 3.80 (s, 3H), 3.81-3.91 (m, 1H), 6.81-6.93 (m, 2H), 7.15 (dd, 1H), 7.24 (tt, 1H), 7.43 (d, 1H), 7.82 (d, 1H).

### Beispiel 6A

### rac-Ethyl-5-{(2-cyano-5,6,7,8-tetrahydrochinolin-5-yl)[2-(2-methoxyphenyl)ethyl]amino}-pentanoat

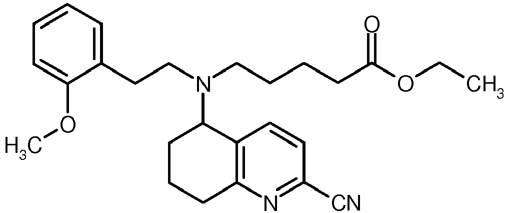

Eine Lösung von 31.22 g (0.10 mol) 5-{[2-(2-Methoxyphenyl)ethyl]amino}-5,6,7,8-tetrahydro-chinolin-2-carbonitril in 300 ml trockenem Acetonitril wurde mit 17.07 ml (0.11 mol) Ethyl-5-brompentanoat, 8.43 g (0.05 mol) Kaliumiodid und 22.61 g (0.21 mol) wasserfreiem Natriumcarbonat versetzt und 4 Tage unter Rückfluss erhitzt. Anschließend wurde der Ansatz bis auf ein Volumen von ca. 50 ml am Rotationsverdampfer eingeengt. Die erhaltene Lösung wurde in 250 ml Essigsäureethylester und 400 ml gesättigter wässriger Natriumchlorid-Lösung aufgenommen und nachfolgend die organische Phase abgetrennt. Die wässrige Phase wurde zweimal mit jeweils 150 ml Essigsäureethylester extrahiert. Die vereinigten organischen Phasen wurden über Natriumsulfat getrocknet, filtriert und bis zur Trockene eingeengt. Der erhaltene Rückstand wurde auf 25 g Kieselgel aufgezogen und durch Säulenchromatographie an Kieselgel gereinigt (80 g-Kartusche; Fluss: 60 ml/min; Laufmittel: Petrolether/Essigsäureethylester 95:5 → 80:20 innerhalb von 30 min). Es wurden 28.89 g (0.05 mol, Gehalt 80%, 52% d. Th.) der Zielverbindung als orangefarbenes Öl erhalten.

¹H-NMR (400 MHz, DMSO-*d*₆, δ/ppm): 1.11-1.19 (m, 1H), 1.16 (t, 3H), 1.33-1.60 (m, 5H), 1.61-1.79 (m, 1H), 1.93-2.09 (m, 3H), 2.23 (t, 2H), 2.39-2.55 (m, 1H, teilweise verdeckt durch DMSO-Signal), 2.56-2.75 (m, 2H), 2.77-2.88 (m, 2H), 3.64 (s, 3H), 3.96-4.09 (m, 4H), 6.84 (t, 1H), 6.88 (d, 1H), 7.07 (d, 1H), 7.17 (t, 1H), 7.65 (d, 1H), 7.84 (d, 1H).

### Beispiel 7A

### rac-Ethyl-5-{(5-ethoxy-5-oxopentyl)[2-(2-hydroxyphenyl)ethyl]amino}-5,6,7,8-tetrahydrochino-lin-2-carboxylat

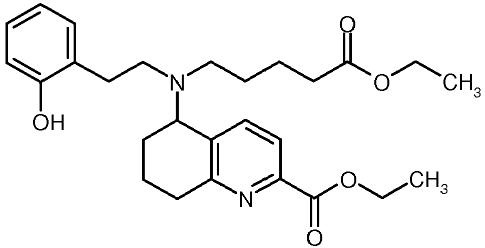

Unter Stickstoff wurden 23.23 g (0.05 mol) Ethyl-5-{(2-cyano-5,6,7,8-tetrahydrochinolin-5-yl)-[2-(2-methoxyphenyl)ethyl]amino}pentanoat in 175 ml Bromwasserstoffsäure (48% in Wasser) aufgenommen. Anschließend wurde die sirupartige Lösung auf 120°C erhitzt und 5 Stunden bei dieser Temperatur gerührt. Danach wurde die klare, gelbe Reaktionslösung auf Raumtemperatur abgekühlt und bis zur Trockene eingeengt. Der erhaltene Rückstand wurde nachfolgend mit 350 ml wasserfreiem Ethanol und 25 ml einer 4 N Lösung von Chlorwasserstoff in Dioxan versetzt und über Nacht bei 65°C gerührt. Das Reaktionsgemisch wurde dann am Rotationsverdampfer bis auf ca. 50 ml eingeengt, vorsichtig mit 550 ml gesättigter wässriger Natriumhydrogencarbonat-Lösung versetzt und dreimal mit jeweils 150 ml Essigsäureethylester extrahiert. Die vereinigten organischen Phasen wurden über Natriumsulfat getrocknet, filtriert und bis zur Trockene eingeengt. Der erhaltene Rückstand (braunes Öl) wurde in 100 ml Essigsäureethylester gelöst, mit 65 g Kieselgel versetzt und wiederum bis zur Trockene eingedampft. Anschließend wurde der Rückstand durch Säulenchromatographie an Kieselgel gereinigt (Metallsäule 58 x 8 cm, 1600 ml Kieselgel; Laufmittel: Essigsäureethylester/Petrolether 1:5, nach ca. 3 Liter 1:4, nach ca. 3.5 Liter 1:3). Es wurden 9.43 g (0.02 mol, 38% d. Th.) der Zielverbindung als farbloses Öl erhalten.

MS (EI): m/z = 468 (M)⁺.

¹H-NMR (400 MHz, DMSO-*d*₆, δ/ppm): 1.12-1.19 (m, 1H), 1.15 (t, 3H), 1.31 (t, 3H), 1.35-1.61 (m, 5H), 1.61-1.79 (m, 1H), 1.93-2.09 (m, 3H), 2.22 (t, 2H), 2.40-2.62 (m, 2H, teilweise verdeckt durch DMSO-Signal), 2.62-2.78 (m, 1H), 2.78-2.88 (m, 2H), 3.97-4.09 (m, 4H), 4.32 (q, 2H), 6.62-6.75 (m, 2H), 6.92-7.02 (m, 2H), 7.71 (d, 1H), 7.92 (d, 1H), 9.14 (s, 1H).

### Beispiel 8A

### rac-5-{[2-(2-Hydroxyphenyl)ethyl]amino}-5,6,7,8-tetrahydrochinolin-2-carbonsäure

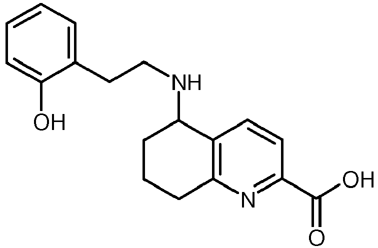

14.6 g (47.5 mmol) 5-{[2-(2-Methoxyphenyl)ethyl]amino}-5,6,7,8-tetrahydrochinolin-2-carbonitril wurden in 100 ml Bromwasserstoffsäure (48% in Wasser) aufgenommen und 5 Stunden bei Siedetemperatur gerührt. Danach wurde die Reaktionslösung auf Raumtemperatur abgekühlt, mit Wasser verdünnt und mit gesättigter Natriumhydrogencarbonat-Lösung auf pH 6 gestellt. Die entstandenen Kristalle wurden abgesaugt, mit Wasser gewaschen und an der Luft getrocknet. Es wurden 14.6 g (46.76 mmol, 98% d. Th.) der Zielverbindung erhalten.

LC-MS (Methode 2): Rₜ = 1.08 min; m/z = 313 (M+H)⁺.

### Beispiel 9A

### rac-Ethyl-5-{[2-(2-hydroxyphenyl)ethyl]amino}-5,6,7,8-tetrahydrochinolin-2-carboxylat

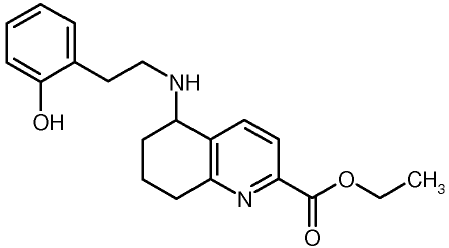

25.8 g (82.59 mmol) 5-{[2-(2-Hydroxyphenyl)ethyl]amino}-5,6,7,8-tetrahydrochinolin-2-carbonsäure wurden mit 645 ml wasserfreiem Ethanol und 52 ml einer 4 N Lösung von Chlorwasserstoff in Dioxan versetzt und über Nacht unter Rückfluss gerührt. Anschließend wurde die Reaktionslösung auf Raumtemperatur abgekühlt und zunächst mit Essigsäureethylester und dann langsam mit gesättigter wässriger Natriumhydrogencarbonat-Lösung versetzt. Nachfolgend wurde die organische Phase abgetrennt, über Natriumsulfat getrocknet, filtriert und bis zur Trockene eingeengt. Es wurden 23.9 g (70.21 mmol, 85% d. Th.) der Zielverbindung erhalten.

LC-MS (Methode 4): Rₜ = 0.57 min; m/z = 341 (M+H)⁺.

¹H-NMR (400 MHz, CDCl₃, δ/ppm): 1.42 (t, 3H), 1.85-2.02 (m, 4H), 2.77-2.86 (m, 2H), 2.86-3.05 (m, 2H), 3.06-3.23 (m, 2H), 3.92-4.00 (m, 1H), 4.46 (q, 2H), 6.77 (t, 1H), 6.91 (d, 1H), 7.00 (d, 1H), 7.14 (t, 1H), 7.89 (d, 1H), 7.96 (d, 1H).

### Beispiel 10A

### rac-Ethyl-5-{(tert.-butoxycarbonyl)[2-(2-hydroxyphenyl)ethyl]amino}-5,6,7,8-tetrahydrochinolin-2-carboxylat

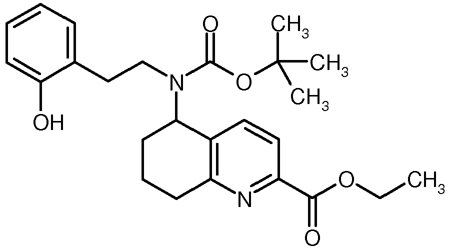

23.85 g (70.06 mmol) Ethyl-5-{[2-(2-hydroxyphenyl)ethyl]amino}-5,6,7,8-tetrahydrochinolin-2-carboxylat wurden in 530 ml Dichlormethan gelöst und unter Rühren auf 0°C gekühlt. Anschließend wurde eine Lösung von 16.06 g (73.56 mmol) Di-*tert*.-butyldicarbonat in 30 ml Dichlormethan langsam zugetropft und das Reaktionsgemisch über Nacht bei Raumtemperatur gerührt. Danach wurde die Reaktionslösung bis zur Trockene eingeengt und der Rückstand mit Ethanol verrührt. Nach Filtration wurde der Filterkuchen mehrmals mit Ethanol gewaschen und abschließend an der Luft getrocknet. Es wurden 27.2 g (61.74 mmol, 88% d. Th.) der Zielverbindung erhalten.

LC-MS (Methode 4): Rₜ = 1.19 min; m/z = 441 (M+H)⁺.

¹H-NMR (400 MHz, CDCl₃, δ/ppm): 1.01-1.24 (m, 4H), 1.24-1.37 (m, 3H), 1.39-1.58 (m, 5H), 1.65-1.90 (m, 1H), 1.90-2.12 (m, 3H), 2.64-3.00 (m, 5H), 3.14-3.55 (m, 1H, teilweise verdeckt durch H₂O-Signal), 4.32 (q, 2H), 4.63-4.85 (m, 0.5H), 5.08-5.30 (m, 0.5H), 6.59-6.83 (m, 2H), 6.91-7.14 (m, 2H), 7.40-7.64 (m, 1H), 7.79-7.87 (m, 1H), 9.31 (s, 1H).

### Beispiel 11A

### 4-(Chlormethyl)-N-(2-hydroxy-5-methylphenyl)benzamid

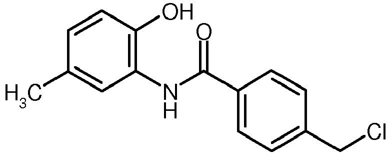

50 g (406 mmol) 2-Amino-4-methylphenol in 250 ml 2-Methoxyethanol wurden unter Rühren mit 37.52 g (446.6 mmol) Natriumhydrogencarbonat versetzt. Anschließend wurden der Lösung 84.4 g (446.6 mmol) 4-Chlormethylbenzoylchlorid, gelöst in 250 ml 2-Methoxyethanol, innerhalb von 15 min zugetropft. Während dieser Zeit wurde ein Anstieg der Reaktionstemperatur von Raumtemperatur auf 40°C beobachtet. Nach 4 Stunden Rühren wurde dem Reaktionsgemisch 1 Liter Wasser und 10 ml konzentrierte Salzsäure zugesetzt. Die entstandenen Kristalle wurden abfiltriert und im Vakuum getrocknet. Es wurden 116 g der Zielverbindung erhalten, die ohne weitere Aufreinigung weiter umgesetzt wurden.

LC-MS (Methode 3): Rₜ = 1.10 min; m/z = 276 (M+H)⁺.

### Beispiel 12A

### 2-[4-(Chlormethyl)phenyl]-5-methyl-1,3-benzoxazol

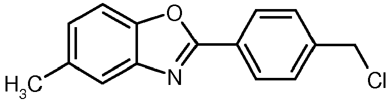

116 g (ca. 406 mmol) 4-(Chlormethyl)-N-(2-hydroxy-5-methylphenyl)benzamid in 700 ml 1,2-Dichlorbenzol wurden unter Rühren mit 5 g (26.3 mmol) *p*-Toluolsulfonsäure-Monohydrat versetzt. Anschließend wurde die Reaktionslösung auf 175°C (Ölbadtemperatur) erhitzt und 3 Stunden bei dieser Temperatur am Wasserabscheider gerührt. Danach wurde die Reaktionslösung auf Raumtemperatur abgekühlt, mit 200 ml Hexan versetzt und das Gemisch ca. 1 Stunde nachgerührt. Der ausgefallene Feststoff wurde abfiltriert, mit Hexan gewaschen und an der Luft getrocknet. Es wurden 56 g (217.29 mmol, 53% d. Th.) der Zielverbindung erhalten.

LC-MS (Methode 3): Rₜ = 1.29 min; m/z = 258 (M+H)⁺.

¹H-NMR (400 MHz, CDCl₃, δ/ppm): 2.45 (s, 3H), 4.88 (s, 2H), 7.26 (dd, 1H), 7.61 (s, 1H), 7.67 (dd, 3H), 8.20 (d, 2H).

### Beispiel 13A

### 1-{4-[4-(Chlormethyl)phenyl]piperidin-1-yl}propan-1-on

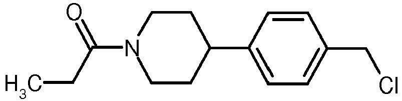

5 g (23 mmol) 1-(4-Phenylpiperidin-1-yl)propan-1-on, 4.84 g (161 mmol) Paraformaldehyd und 4.7 g (34.5 mmol) Zinkchlorid wurden in 200 ml Dichlormethan vorgelegt. Anschließend wurde unter starkem Rühren für 30 min Chlorwasserstoff-Gas durch das Reaktionsgemisch geleitet. Nach Abschluss der Einleitung wurde das Reaktionsgemisch über Nacht bei Raumtemperatur nachgerührt. Die Reaktionslösung wurde dann mit Wasser versetzt, die organische Phase abgetrennt und die wässrige Phase mit Essigsäureethylester extrahiert. Die vereinigten organischen Phasen wurden über Natriumsulfat getrocknet, filtriert und am Rotationsverdampfer bis zur Trockene eingeengt. Der erhaltene Rückstand wurde mittels präparativer HPLC gereinigt. Während des Einengens hydrolysierte das Produkt teilweise zur analogen 4-(Hydroxymethyl)-Verbindung. Daraufhin wurde das erhaltene Produktgemisch (3.68 g) unter Rühren in 100 ml THF aufgenommen und mit 500 mg Zinkchlorid und anschließend 2 ml Thionylchlorid versetzt. Dieses Gemisch wurde dann 1 Stunde bei Raumtemperatur gerührt. Nach Zugabe von Wasser und Essigsäureethylester zur Reaktionslösung wurde die organische Phase abgetrennt, über Natriumsulfat getrocknet, filtriert und bis zur Trockene eingeengt. Es wurden 3.4 g (12.79 mmol, 56% d. Th.) der Zielverbindung erhalten.

LC-MS (Methode 2): Rₜ = 2.18 min; m/z = 266 (M+H)⁺.

¹H-NMR (400 MHz, DMSO-*d*₆, δ/ppm): 1.00 (t, 3H), 1.36-1.61 (m, 2H), 1.69-1.84 (m, 2H), 2.35 (q, 2H), 2.52-2.63 (m, 1H, teilweise verdeckt durch DMSO-Signal), 2.70-2.82 (m, 1H), 3.02-3.14 (m, 1H), 3.91-3.99 (m, 1H), 4.50-4.60 (m, 1H), 4.73 (s, 2H), 7.25 (d, 2H), 7.36 (d, 2H).

### Beispiel 14A

### 1-(Brommethyl)-4-[trans-4-(trifluormethyl)cyclohexyl]benzol

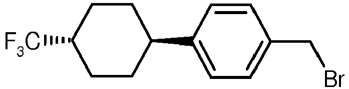

Unter Argon wurden 2 g (7.74 mmol) {4-[*trans*-4-(Trifluormethyl)cyclohexyl]phenyl}methanol [zur Herstellung siehe Patentanmeldung WO 2009/032249-A1, Example 8 / Steps C-E] in 40 ml THF gelöst und nacheinander mit 2.437 g (9.29 mmol) Triphenylphosphin und 3.081 g (9.29 mmol) Tetrabromkohlenstoff versetzt. Das Reaktionsgemisch wurde über Nacht bei Raumtemperatur gerührt. Anschließend wurde zunächst Wasser und dann Essigsäureethylester hinzugesetzt. Nach Abtrennung der organischen Phase wurde diese über Magnesiumsulfat getrocknet, filtriert und bis zur Trockene eingeengt. Der erhaltene Rückstand wurde chromatographisch an Kieselgel gereinigt (Laufmittel: Cyclohexan/Essigsäureethylester 10:1). Es wurden 2.07 g (6.44 mmol, 83% d. Th.) der Zielverbindung erhalten.

GC-MS (Methode 5): Rₜ = 6.14 min; m/z = 422 (M+H)⁺.

¹H-NMR (400 MHz, CDCl₃, δ/ppm): 1.32-1.59 (m, 4H), 1.68-1.78 (m, 1H), 1.81-1.91 (m, 2H), 1.91-2.01 (m, 2H), 2.27-2.42 (m, 1H), 4.68 (s, 2H), 7.22 (d, 2H), 7.37 (d, 2H).

### Beispiel 15A

### rac-Ethyl-5-{(5-ethoxy-5-oxopentyl)[2-(2-{[4-(2-phenylethyl)benzyl]oxy}phenyl)ethyl]amino}-5,6,7,8-tetrahydrochinolin-2-carboxylat

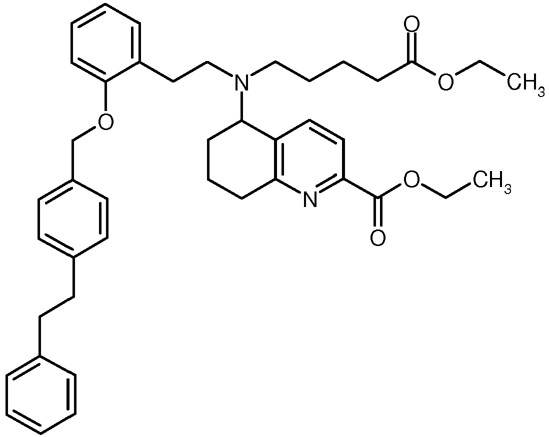

Unter Argon wurden 500 mg (1.07 mmol) Ethyl-5-{(5-ethoxy-5-oxopentyl)[2-(2-hydroxyphenyl)-ethyl]amino}-5,6,7,8-tetrahydrochinolin-2-carboxylat, 246 mg (1.07 mmol) 1-(Chlormethyl)-4-(2-phenylethyl)benzol sowie 295 mg (2.13 mmol) Kaliumcarbonat in 5 ml DMF auf 80°C erhitzt und 6 Stunden bei dieser Temperatur gerührt. Nach Abkühlen wurde das Reaktionsgemisch mit Wasser und Essigsäureethylester versetzt und nachfolgend die Phasen getrennt. Die organische Phase wurde zweimal mit Wasser und einmal mit gesättigter Natriumchlorid-Lösung gewaschen und dann bis zur Trockene eingeengt. Es wurden 760 mg (1.01 mmol, Gehalt 88%, 94% d. Th.) der Zielverbindung erhalten.

LC-MS (Methode 1): Rₜ = 1.37 min; m/z = 663 (M+H)⁺.

Analog zu Beispiel 15A wurden die folgenden Verbindungen aus den jeweils angegebenen Edukten hergestellt:

| **Beispiel** | **Name / Struktur / Edukte** | **Analytische Daten** |
|---|---|---|
| **16A** | *rac*-Ethyl-5-[(5-ethoxy-5-oxopentyl){2-[2-({4-[2-(4-fluorphenyl)ethyl]benzyl}oxy)phenyl]ethyl}-amino]-5,6,7,8-tetrahydrochinolin-2-carboxylat | LC-MS (Methode 1): |
| | | Rₜ = 1.36 min; m/z = 681 (M+H)⁺. |
| | 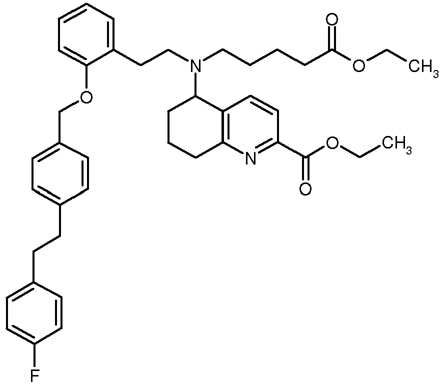 | |
| | aus Ethyl-5-{(5-ethoxy-5-oxopentyl)[2-(2-hydroxy-phenyl)ethyl]amino}-5,6,7,8-tetrahydrochinolin-2-carboxylat und 1-(Chlormethyl)-4-[2-(4-fluorphenyl)ethyl]benzol | |
| **17A** | *rac*-Ethyl-5-{(5-ethoxy-5-oxopentyl)[2-(2-{[4'-(tri-fluormethyl)biphenyl-4-yl]methoxy}phenyl)ethyl]-amino}-5,6,7,8-tetrahydrochinolin-2-carboxylat | LC-MS (Methode 4): |
| | | Rₜ = 1.42 min; m/z = 703 (M+H)⁺. |
| | 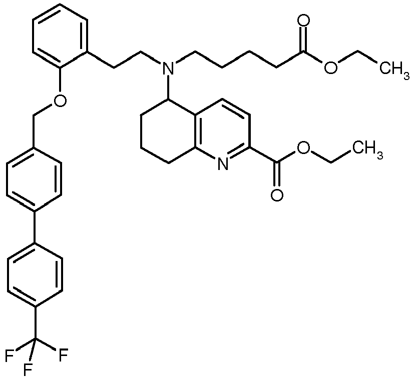 | |
| | aus Ethyl-5-{(5-ethoxy-5-oxopentyl)[2-(2-hydroxy-phenyl)ethyl]amino}-5,6,7,8-tetrahydrochinolin-2-carboxylat und 4-(Brommethyl)-4'-(trifluormethyl)biphenyl | |

### Beispiel 18A

### rac-Ethyl-5-{(tert.-butoxycarbonyl)[2-(2-{[4-(5-methyl-1,3-benzoxazol-2-yl)benzyl]oxy}phenyl)-ethyl]amino}-5,6,7,8-tetrahydrochinolin-2-carboxylat

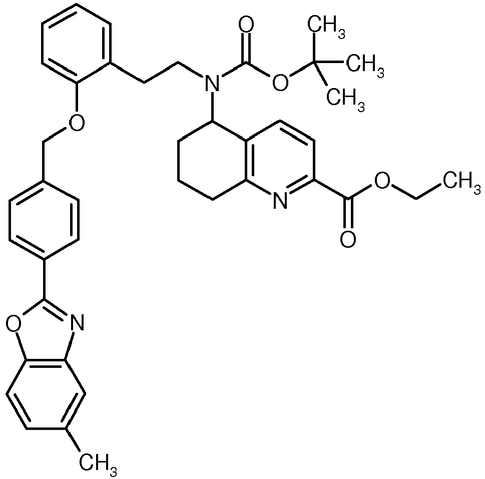

5 g (11.35 mmol) Ethyl-5-{(*tert*.-butoxycarbonyl)[2-(2-hydroxyphenyl)ethyl]amino}-5,6,7,8-tetra-hydrochinolin-2-carboxylat, 3.51 g (13.62 mmol) 2-[4-(Chlormethyl)phenyl]-5-methyl-1,3-benz-oxazol und 3.92 g (28.37 mmol) Kaliumcarbonat in 50 ml Acetonitril wurden auf 110°C erhitzt und über Nacht bei dieser Temperatur gerührt. Nach Abkühlen wurde das Reaktionsgemisch filtriert, der Filterkuchen mehrmals mit Acetonitril nachgewaschen und die vereinigten Filtrate bis zur Trockene am Rotationsverdampfer eingeengt. Der erhaltene Rückstand wurde chromatographisch an Kieselgel gereinigt (Laufmittel: Cyclohexan/Essigsäureethylester 4:1 → 2:1). Es wurden 6.59 g (9.96 mmol, 87% d. Th.) der Zielverbindung erhalten.

LC-MS (Methode 3): Rₜ = 1.62 min; m/z = 662 (M+H)⁺.

¹H-NMR (400 MHz, CDCl₃, δ/ppm): 1.01-1.21 (m, 4H), 1.22-1.35 (m, 3H), 1.37-1.59 (m, 5.5H), 1.60-1.74 (m, 0.5H), 1.74-1.97 (m, 3H), 2.46 (s, 3H), 2.57-2.79 (m, 2H), 2.79-3.04 (m, 3H), 3.16-3.30 (m, 0.5H), 3.40-3.54 (m, 0.5H), 4.27 (q, 2H), 4.44-4.64 (m, 0.5H), 5.03-5.28 (m, 2.5H), 6.83-6.95 (m, 1H), 6.97-7.04 (m, 0.5H), 7.04-7.14 (m, 1H), 7.14-7.29 (m, 3H), 7.40-7.49 (m, 0.5H), 7.49-7.72 (m, 4H), 7.82 (d, 1H), 8.06 (d, 1H), 8.14 (d, 1H).

Analog zu Beispiel 18A wurden die folgenden Verbindungen aus den jeweils angegebenen Edukten hergestellt:

| **Beispiel** | **Name / Struktur / Edukte** | **Analytische Daten** |
|---|---|---|
| **19A** | *rac*-Ethyl-5-{(*tert.*-butoxycarbonyl)[2-(2-{[4-(1-propionylpiperidin-4-yl)benzyl]oxy}phenyl)ethyl]-amino}-5,6,7,8-tetrahydrochinolin-2-carboxylat | LC-MS (Methode 3): Rₜ = 1.45 min; m/z = 670 (M+H)⁺. |
| | 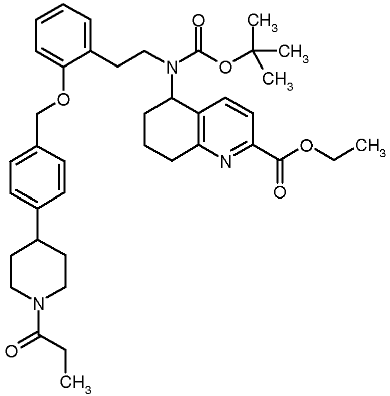 | ¹H-NMR (400 MHz, DMSO-*d*₆): δ [ppm] = 1.02 (t, 3H), 1.07-1.22 (m, 4H), 1.26-1.34 (m, 3H), 1.34-1.62 (m, 7H), 1.64-1.95 (m, 5H), 2.29-2.41 (m, 2H), 2.47-2.98 (m, 7H, teilweise verdeckt durch DMSO-Signal), 3.00-3.14 (m, 1H), 3.14-3.33 (m, 1H, teilweise verdeckt durch H₂O-Signal), 3.37-3.49 (m, 1H), 3.87-4.03 (m, 1H), 4.24-4.38 (m, 2H), 4.45-4.63 (m, 1H), 4.92-5.19 (m, 3H), 6.80-6.93 (m, 1H), 6.95-7.58 (m, 8H), 7.82 (d, 1H). |
| | aus Ethyl-5-{(*tert.*-butoxycarbonyl)[2-(2-hydroxy-phenyl)ethyl]amino}-5,6,7,8-tetrahydrochinolin-2-carboxylat und 1-{4-[4-(Chlormethyl)phenyl]-piperidin-1-yl}propan-1-on | |
| **20A** | *rac*-Ethyl-5-[(*tert.*-butoxycarbonyl)(2-{2-[(4*-tert.-*butylbenzyl)oxy]phenyl}ethyl)amino]-5,6,7,8-tetrahydrochinolin-2-carboxylat | LC-MS (Methode 4): Rₜ = 1.64 min; m/z = 587 (M+H)⁺. |
| | 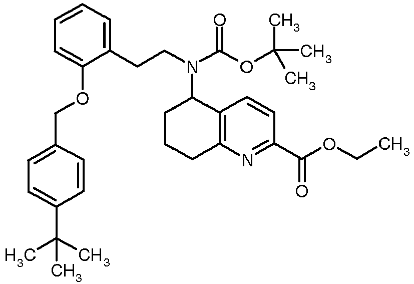 | |
| | aus Ethyl-5-{(*tert.*-butoxycarbonyl)[2-(2-hydroxyphenyl)ethyl]amino}-5,6,7,8-tetrahydrochinolin-2-carboxylat und 4-*tert*. -Butylbenzylbromid | |
| **21A** | *rac*-Ethyl-5-[(*tert.*-butoxycarbonyl)(2-{2-[(4-cyclo-hexylbenzyl)oxy]phenyl}ethyl)amino]-5,6,7,8-tetrahydrochinolin-2-carboxylat | LC-MS (Methode 3): Rₜ = 1.72 min; m/z = 613 (M+H)⁺. |
| | 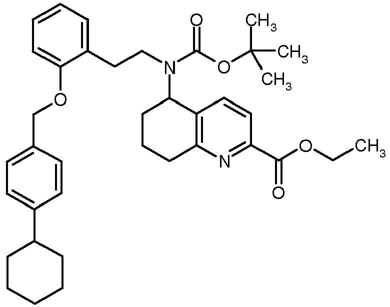 | |
| | aus Ethyl-5-{(*tert*.-butoxycarbonyl)[2-(2-hydroxy-phenyl)ethyl]amino}-5,6,7,8-tetrahydrochinolin-2-carboxylat und 1-(Chlormethyl)-4-cyclohexylbenzol | |

### Beispiel 22A

*rac*-Ethyl-5-{(*tert*.-butoxycarbonyl)[2-(2-{[3-chlor-4'-(trifluormethyl)biphenyl-4-yl]methoxy}-phenyl)ethyl]amino}-5,6,7,8-tetrahydrochinolin-2-carboxylat

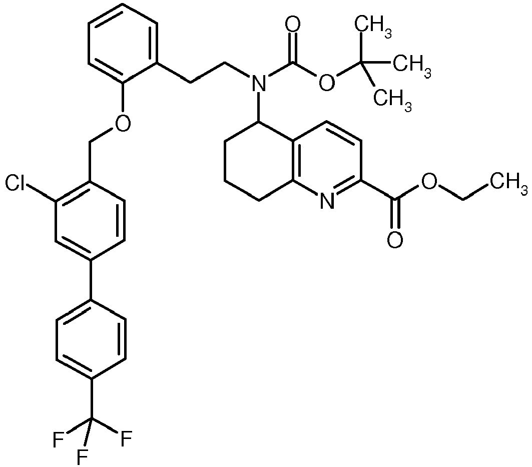

250 mg (0.57 mmol) *rac*-Ethyl-5-{(*tert*.-butoxycarbonyl)[2-(2-hydroxyphenyl)ethyl]amino}-5,6,7,8-tetrahydrochinolin-2-carboxylat (Beispiel 10A), 277 mg (0.68 mmol) 4-(Brommethyl)-3-chlor-4'-(trifluormethyl)biphenyl und 118 mg (0.85 mmol) Kaliumcarbonat in 10 ml Acetonitril wurden auf 110°C erhitzt und 4 h bei dieser Temperatur gerührt. Nach dem Abkühlen wurde das Reaktionsgemisch filtriert, der Filterkuchen mehrmals mit Acetonitril nachgewaschen und die vereinigten Filtrate am Rotationsverdampfer bis zur Trockene eingeengt. Der erhaltene Rückstand wurde chromatographisch an Kieselgel gereinigt (Laufmittel: Cyclohexan/Essigsäureethylester 10:1 → 4:1). Das so gewonnene Produkt wurde nochmals mittels präparativer HPLC nachgereinigt (Eluent: Methanol/Wasser 9:1). Es wurden 182 mg (0.26 mmol, 45% d. Th.) der Zielverbindung erhalten.

LC-MS (Methode 3): Rₜ = 1.70 min; m/z = 709/711 (M+H)⁺.

Analog zu Beispiel 18A wurden auch die folgenden Verbindungen aus den jeweils angegebenen Edukten hergestellt:

| **Beispiel** | **Name / Struktur / Edukte** | **Analytische Daten** |
|---|---|---|
| **23A** | *rac*-Ethyl-5-[(*tert*.-butoxycarbonyl)(2-{2-[(5-phenyl-pentyl)oxy]phenyl}ethyl)amino]-5,6,7,8-tetrahydro-chinolin-2-carboxylat | LC-MS (Methode 3): Rₜ = 1.64 min; m/z = 587 (M+H)⁺. |
| | 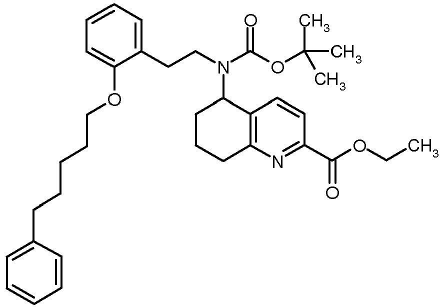 | |
| | aus Ethyl-5-{(*tert*.-butoxycarbonyl)[2-(2-hydroxy-phenyl)ethyl]amino}-5,6,7,8-tetrahydrochinolin-2-carboxylat und (5-Brompentyl)benzol | |
| **24A** | *rac*-Ethyl-5-[(*tert*.-butoxycarbonyl){2-[2-({4-[*trans-*4-(trifluormethyl)cyclohexyl]benzyl}oxy)phenyl]-ethyl}amino]-5,6,7,8-tetrahydrochinolin-2-carboxylat | LC-MS (Methode 3): Rₜ = 1.66 min; m/z = 681 (M+H)⁺. |
| | 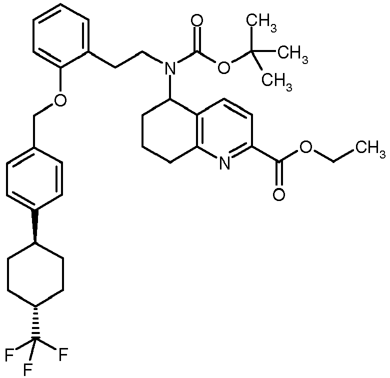 | |
| | aus Ethyl-5-{(*tert*.-butoxycarbonyl)[2-(2-hydroxyphenyl)ethyl]amino}1-5,6,7,8-tetrahydrochinolin-2-carboxylat und 1-(Brommethyl)-4-[*trans*-4-(trifluormethyl)cyclohexyl]benzol | |

### Beispiel 25A und Beispiel 26A

### Ethyl-5-{(tert.-butoxycarbonyl)[2-(2-{[4-(5-methyl-1,3-benzoxazol-2-yl)benzyl]oxy}phenyl)-ethyl]amino}-5,6,7,8-tetrahydrochinolin-2-carboxylat (Enantiomer 1 und 2)

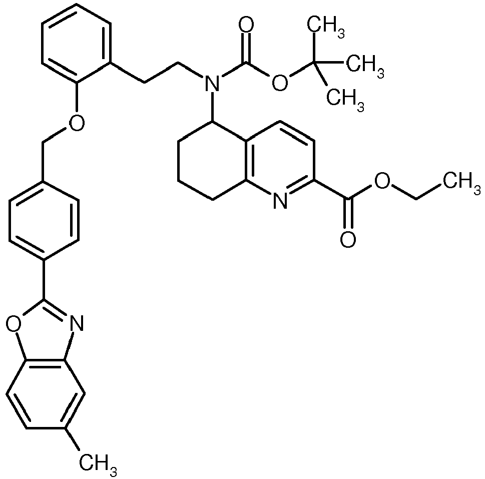

6.59 g (9.96 mmol) des racemischen Ethyl-5-{(*tert*.-butoxycarbonyl)[2-(2-{[4-(5-methyl-1,3-benz-oxazol-2-yl)benzyl]oxy}phenyl)ethyl]amino}-5,6,7,8-tetrahydrochinolin-2-carboxylats (Beispiel 18A) wurden durch superkritische Flüssigchromatographie (SFC) an chiraler Phase in die Enantiomere aufgetrennt [Säule: Daicel Chiracel OD-H, 5 µm, 250 mm x 20 mm; Elutionsmittel: Kohlendioxid/Ethanol 75:25 (v/v); Fluss: 125 ml/min; Druck: 150 bar; UV-Detektion: 210 nm; Temperatur: 38°C]:

### Beispiel 25A (Enantiomer 1):

### (+)-Ethyl-5- {(tert.-butoxycarbonyl)[2-(2-{[4-(5-methyl-1,3-benzoxazol-2-yl)benzyl]oxy}phenyl)-ethyl] amino}-5,6,7,8-tetrahydrochinolin-2-carboxylat

Ausbeute: 2864 mg
Rₜ = 2.92 min; chemische Reinheit >99%; >99.9% ee
[Säule: Chiralpak OD-H, 5 µm, 250 mm x 4.6 mm; Elutionsmittel: Kohlendioxid/Ethanol 75:25 (v/v); Fluss: 4 ml/min; Temperatur: 34.3°C; UV-Detektion: 210 nm].
[α]_{D}²⁰ = +6.345°, c = 0.415, Methanol.
LC-MS (Methode 3): Rₜ = 1.62 min; m/z = 662 (M+H)⁺.

¹H-NMR (400 MHz, CDCl₃, δ/ppm): 1.01-1.21 (m, 4H), 1.22-1.35 (m, 3H), 1.37-1.59 (m, 5.5H), 1.60-1.74 (m, 0.5H), 1.74-1.97 (m, 3H), 2.46 (s, 3H), 2.57-2.79 (m, 2H), 2.79-3.04 (m, 3H), 3.16-3.30 (m, 0.5H), 3.40-3.54 (m, 0.5H), 4.27 (q, 2H), 4.44-4.64 (m, 0.5H), 5.03-5.28 (m, 2.5H), 6.83-6.95 (m, 1H), 6.97-7.04 (m, 0.5H), 7.04-7.14 (m, 1H), 7.14-7.29 (m, 3H), 7.40-7.49 (m, 0.5H), 7.49-7.72 (m, 4H), 7.82 (d, 1H), 8.06 (d, 1H), 8.14 (d, 1H).

### Beispiel 26A (Enantiomer 2):

### (-)-Ethyl-5-{(tert.-butoxycarbonyl)[2-(2-{[4-(5-methyl-1,3-benzoxazol-2-yl)benzyl]oxy}phenyl)-ethyl]amino}-5,6,7,8-tetrahydrochinolin-2-carboxylat

Ausbeute: 2359 mg
Rₜ = 4.52 min; chemische Reinheit >99%; >99.9% ee
[Säule: Chiralpak OD-H, 5 µm, 250 mm x 4.6 mm; Elutionsmittel: Kohlendioxid/Ethanol 75:25 (v/v); Fluss: 4 ml/min; Temperatur: 34.3°C; UV-Detektion: 210 nm].
[α]_{D}²⁰ = -6.082°, c = 0.589, Methanol.
LC-MS (Methode 3): Rₜ = 1.62 min; m/z = 662 (M+H)⁺.

¹H-NMR (400 MHz, CDCl₃, δ/ppm): 0.98-1.20 (m, 4H), 1.21-1.33 (m, 3H), 1.37-1.59 (m, 5.5H), 1.60-1.74 (m, 0.5H), 1.74-1.98 (m, 3H), 2.46 (s, 3H), 2.58-2.79 (m, 2H), 2.79-3.03 (m, 3H), 3.17-3.30 (m, 0.5H), 3.40-3.54 (m, 0.5H), 4.27 (q, 2H), 4.44-4.64 (m, 0.5H), 5.02-5.27 (m, 2.5H), 6.83-6.96 (m, 1H), 6.96-7.04 (m, 0.5H), 7.04-7.13 (m, 1H), 7.14-7.30 (m, 3H), 7.40-7.49 (m, 0.5H), 7.49-7.72 (m, 4H), 7.82 (d, 1H), 8.06 (d, 1H), 8.14 (d, 1H).

### Beispiel 27A

### Ethyl-5-{[2-(2-{[4-(5-methyl-1,3-benzoxazol-2-yl)benzyl]oxy}phenyl)ethyl]amino}-5,6,7,8-tetra-hydrochinolin-2-carboxylat-Dihydrochlorid (Enantiomer 1)

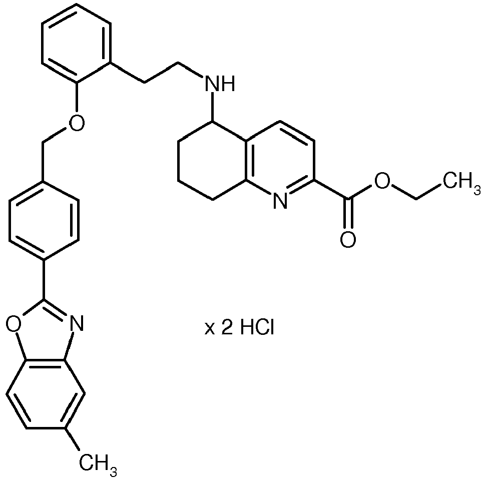

581 mg (0.88 mmol) (+)-Ethyl-5-{(*tert*.-butoxycarbonyl)[2-(2-{[4-(5-methyl-1,3-benzoxazol-2-yl)-benzyl]oxy}phenyl)ethyl]amino}-5,6,7,8-tetrahydrochinolin-2-carboxylat (Beispiel 25A) wurden mit 5 ml einer 4 N Lösung von Chlorwasserstoff in Dioxan versetzt und 4 h bei Raumtemperatur gerührt. Die Reaktionslösung wurde danach bis zur Trockene eingeengt und der Rückstand über Nacht im Hochvakuum getrocknet. Es wurden 564 mg (0.88 mmol, 100% d. Th.) des Zielprodukts als beiger Festsoff erhalten.

LC-MS (Methode 3): Rₜ = 0.96 min; m/z = 562 (M+H)⁺.

¹H-NMR (400 MHz, DMSO-*d₆*, δ/ppm): 1.28 (t, 3H), 1.76-1.89 (m, 1H), 1.97-2.10 (m, 2H), 2.10-2.19 (m, 1H), 2.80-2.92 (m, 1H), 2.92-3.03 (m, 1H), 3.05-3.14 (m, 2H), 3.14-3.72 (m, 2H), 3.54-3.61 (m, 1H), 3.57 (s, 3H), 4.29 (q, 2H), 4.62-4.71 (m, 1H), 5.26 (s, 2H), 6.96 (t, 1H), 7.12 (d, 1H), 7.22-7.32 (m, 3H), 7.62 (s, 1H), 7.65 (m, 3H), 7.84 (d, 1H), 8.20 (d, 3H), 9.29-9.46 (br. s, 2H).

### Beispiel 28A

### Ethyl-5-{[2-(2-{[4-(5-methyl-1,3-benzoxazol-2-yl)benzyl]oxy}phenyl)ethyl]amino}-5,6,7,8-tetra-hydrochinolin-2-carboxylat-Dihydrochlorid (Enantiomer 2)

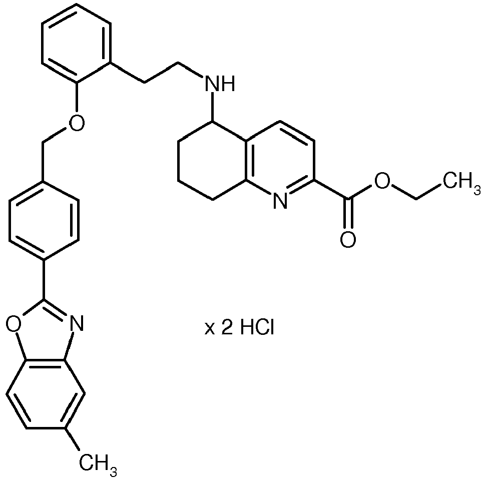

620 mg (0.94 mmol) (-)-Ethyl-5-{(*tert*.-butoxycarbonyl)[2-(2-{[4-(5-methyl-1,3-benzoxazol-2-yl)-benzyl]oxy}phenyl)ethyl]amino}-5,6,7,8-tetrahydrochinolin-2-carboxylat (Beispiel 26A) wurden mit 6.1 ml einer 4 N Lösung von Chlorwasserstoff in Dioxan versetzt und 4 h bei Raumtemperatur gerührt. Die Reaktionslösung wurde danach bis zur Trockene eingeengt und der Rückstand über Nacht im Hochvakuum getrocknet. Es wurden 604 mg (ca. 0.95 mmol, ca. 100% d. Th.) des Zielprodukts als beiger Festsoff erhalten.

LC-MS (Methode 3): Rₜ = 1.05 min; m/z = 562 (M+H)⁺.

Analog zu den Beispielen 27A und 28A wurden die folgenden Verbindungen hergestellt:

| **Beispiel** | **Name / Struktur / Edukt** | **Analytische Daten** |
|---|---|---|
| **29A** | *rac*-Ethyl-5-{[2-(2-{[4-(1-propionylpiperidin-4-yl)-benzyl]oxy}phenyl)ethyl]amino-5,6,7,8-tetra-hydrochinolin-2-carboxylat-Dihydrochlorid | LC-MS (Methode 3): Rₜ = 0.89 min; m/z = 570 (M+H)⁺. |
| | 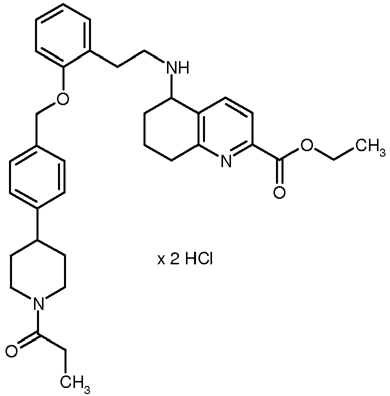 | |
| | aus Ethyl-5-{(*tert.*-butoxycarbonyl)[2-(2-{[4-(1-propionylpiperidin-4-yl)benzyl]oxy}phenyl)ethyl]-amino}-5,6,7,8-tetrahydrochinolin-2-carboxylat | |
| **30A** | *rac*-Ethyl-5-[(2-{2-[(4-*tert*.-butylbenzyl)oxy]-phenyl}ethyl)amino]-5,6,7,8-tetrahydrochinolin-2-carboxylat-Dihydrochlorid | LC-MS (Methode 1): Rₜ = 0.98 min; m/z = 487 (M+H)⁺. |
| | 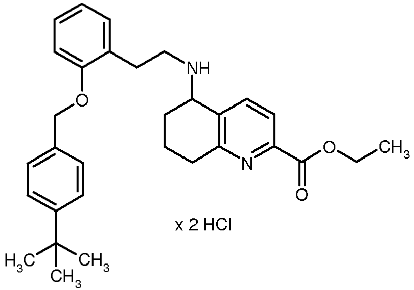 | |
| | aus Ethyl-5-[(*tert*.-butoxycarbonyl)(2-{2-[(4-*tert*.-butylbenzyl)oxy]phenyl}ethyl)amino]-5,6,7,8-tetrahydrochinolin-2-carboxylat | |
| **31A** | *rac*-Ethyl-5-[(2-{2-[(4-cyclohexylbenzyl)oxy]-phenyl}ethyl)amino]-5,6,7,8-tetrahydrochinolin-2-carboxylat-Dihydrochlorid | LC-MS (Methode 3): Rₜ = 1.03 min; m/z = 513 (M+H)⁺. |
| | 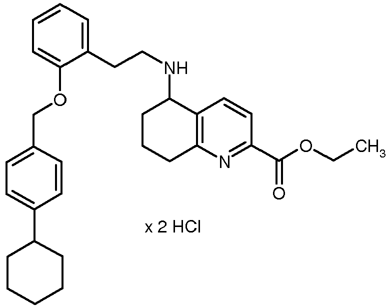 | |
| | aus Ethyl-5-[(*tert*.-butoxycarbonyl)(2-{2-[(4-cyclo-hexylbenzyl)oxy]phenyl}ethyl)amino]-5,6,7,8-tetrahydrochinolin-2-carboxylat | |
| **32A** | *rac*-Ethyl-5-{[2-(2-{[3-chlor-4'-(trifluormethyl)-biphenyl-4-yl]methoxy}phenyl)ethyl]amino}-5,6,7,8-tetrahydrochinolin-2-carboxylat-Dihydrochlorid | LC-MS (Methode 3): Rₜ = 1.05 min; m/z = 609 (M+H)⁺. |
| | 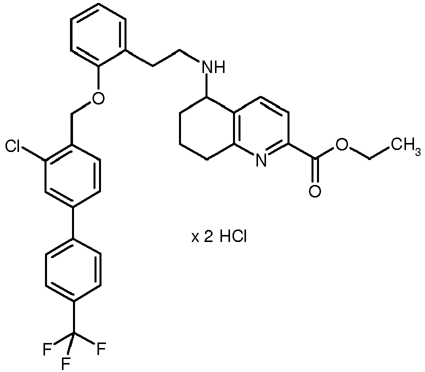 | |
| | aus Ethyl-5-{(*tert*.-butoxycarbonyl)[2-(2-{[3-chlor-4'-(trifluormethyl)biphenyl-4-yl]methoxy}phenyl)-ethyl]amino}-5,6,7,8-tetrahydrochinolin-2-carboxylat | |
| **33A** | *rac*-Ethyl-5-[(2-{2-[(5-phenylpentyl)oxy]phenyl}-ethyl)amino]-5,6,7,8-tetrahydrochinolin-2-carboxylat-Dihydrochlorid | LC-MS (Methode 3): Rₜ = 0.97 min; m/z = 487 (M+H)⁺. |
| | 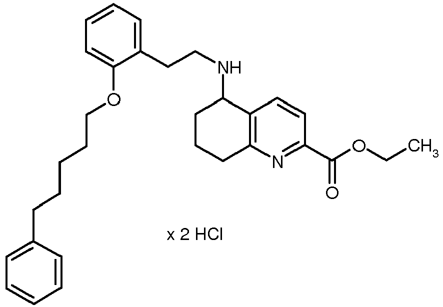 | |
| | aus Ethyl-5-[(*tert*.-butoxycarbonyl)(2-{2-[(5-phenyl-pentyl)oxy]phenyl}ethyl)amino]-5,6,7,8-tetrahydro-chinolin-2-carboxylat | |
| **34A** | *rac*-Ethyl-5-({2-[2-({4-[*trans*-4-(trifluormethyl)-cyclohexyl]benzyl}oxy)phenyl]ethyl}amino)-5,6,7,8-tetrahydrochinolin-2-carboxylat-Dihydrochlorid | Die Titelverbindung wurde ohne weitere Charakterisierung weiter umgesetzt. |
| | 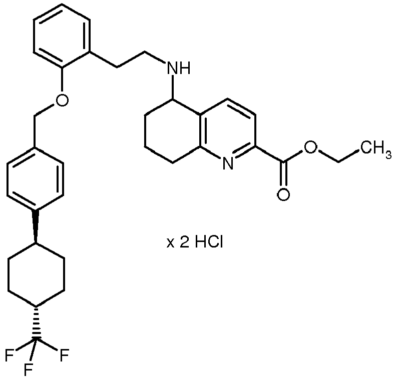 | |
| | aus Ethyl-5-[(*tert*.-butoxycarbonyl){2-[2-({4-[*trans-*4-(trifluormethyl)cyclohexyl]benzyl}oxy)phenyl]-ethyl}ammo]-5,6,7,8-tetrahydrochinolin-2-carboxylat | |

### Beispiel 35A

### (-)-Ethyl-5-{(5-ethoxy-5-oxopentyl)[2-(2-{[4-(5-methyl-,3-benzoxazol-2-yl)benzyl]oxy}phenyl)-ethyl]amino}-5,6,7,8-tetrahydrochinolin-2-carboxylat (Enantiomer 1)

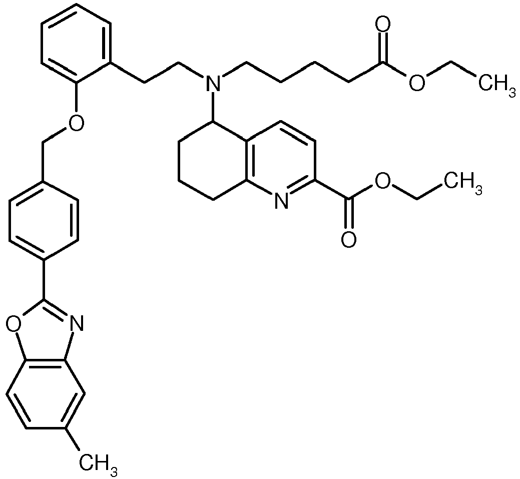

Eine Lösung von 543 mg (0.86 mmol) Ethyl-5-{[2-(2-{[4-(5-methyl-1,3-benzoxazol-2-yl)benzyl]-oxy}phenyl)ethyl]amino}-5,6,7,8-tetrahydrochinolin-2-carboxylat-Dihydrochlorid (Enantiomer 1, Beispiel 27A) in 10 ml trockenem Acetonitril wurde mit 0.27 ml (1.70 mmol) Ethyl-5-brom-pentanoat, 14 mg (0.09 mmol) Kaliumiodid sowie 372 mg (2.57 mmol) wasserfreiem Natriumcarbonat versetzt und über Nacht unter Rückfluss erhitzt. Anschließend wurden weitere 0.2 ml Ethyl-5-brompentanoat zugesetzt und das Gemisch 8 Stunden unter Rückfluss nachgerührt. Danach wurden erneut 0.2 ml Ethyl-5-brompentanoat sowie ca. 14 mg Kaliumiodid zugegeben und weiter über Nacht unter Rückfluss erhitzt. Nach nochmaliger Zugabe von 0.2 ml Ethyl-5-brom-pentanoat wurde erneut für 8 Stunden unter Rückfluss gerührt. Abschließend wurden nochmals ca. 14 mg Kaliumiodid zugegeben und das Gemisch weiter über Nacht unter Rückfluss erhitzt. Der Ansatz wurde dann filtriert, der Filterkuchen mit Acetonitril nachgewaschen und das Filtrat bis zur Trockene eingeengt. Der erhaltene Rückstand wurde chromatographisch an Kieselgel gereinigt (Laufmittel: Cyclohexan/Essigsäureethylester 3:1). Es wurden 396 mg (0.57 mmol, 67% d. Th.) der Titelverbindung als farbloses Öl erhalten.

LC-MS (Methode 3): Rₜ = 1.36 min; m/z = 690 (M+H)⁺.

¹H-NMR (400 MHz, DMSO-*d₆*, δ/ppm): 1.10 (t, 3H), 1.26 (t, 3H), 1.30-1.70 (m, 7H), 1.89-2.04 (m, 2H), 2.09-2.20 (m, 2H), 2.35-2.64 (m, 3H, teilweise verdeckt durch DMSO-Signal), 2.45 (s, 3H), 2.65-2.86 (m, 4H), 3.92-4.02 (m, 3H), 4.26 (q, 2H), 5.04-5.15 (m, 2H), 6.87 (t, 1H), 6.99 (d, 1H), 7.09-7.21 (m, 2H), 7.25 (d, 1H), 7.52 (d, 2H), 7.61 (s, 1H), 7.63-7.68 (m, 2H), 7.87 (d, 1H), 8.15 (d, 2H).

[α]_{D}²⁰ = -52.70°, c = 0.420, Methanol.

### Beispiel 36A

### (+)-Ethyl-5-{(5-ethoxy-5-oxopentyl)[2-(2-{[4-(5-methyl-1,3-benzoxazol-2-yl)benzyl]oxy}phenyl)-ethyl]amino}-5,6,7,8-tetrahydrochinolin-2-carboxylat (Enantiomer 2)

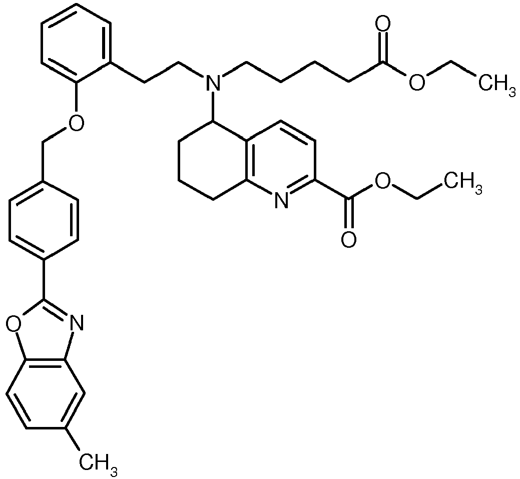

Eine Lösung von 564 mg (0.89 mmol) Ethyl-5-{[2-(2-{[4-(5-methyl-1,3-benzoxazol-2-yl)benzyl]-oxy}phenyl)ethyl]amino}-5,6,7,8-tetrahydrochinolin-2-carboxylat-Dihydrochlorid (Enantiomer 2, Beispiel 28A) in 10 ml trockenem Acetonitril wurde mit 0.28 ml (1.78 mmol) Ethyl-5-brom-pentanoat, 15 mg (0.09 mmol) Kaliumiodid sowie 283 mg (2.67 mmol) wasserfreiem Natriumcarbonat versetzt und über Nacht unter Rückfluss erhitzt. Anschließend wurden weitere 0.2 ml Ethyl-5-brompentanoat zugesetzt und das Gemisch 8 Stunden unter Rückfluss nachgerührt. Danach wurden erneut 0.2 Il Ethyl-5-brompentanoat sowie ca. 14 mg Kaliumiodid zugegeben und weiter über Nacht unter Rückfluss erhitzt. Nach nochmaliger Zugabe von 0.2 ml Ethyl-5-brom-pentanoat wurde erneut für 8 Stunden unter Rückfluss gerührt. Abschließend wurden nochmals ca. 14 mg Kaliumiodid zugegeben und das Gemisch weiter über Nacht unter Rückfluss erhitzt. Der Ansatz wurde dann filtriert, der Filterkuchen mit Acetonitril nachgewaschen und das Filtrat bis zur Trockene eingeengt. Der erhaltene Rückstand wurde chromatographisch an Kieselgel gereinigt (Laufmittel: Cyclohexan/Essigsäureethylester 3:1). Es wurden 320 mg (0.46 mmol, 52% d. Th.) der Titelverbindung als farbloses Öl erhalten.

LC-MS (Methode 3): Rₜ = 1.37 min; m/z = 690 (M+H)⁺.

¹H-NMR (400 MHz, DMSO-*d₆*, δ/ppm): 1.10 (t, 3H), 1.26 (t, 3H), 1.30-1.70 (m, 7H), 1.89-2.04 (m, 2H), 2.10-2.19 (m, 2H), 2.38-2.64 (m, 3H, teilweise verdeckt durch DMSO-Signal), 2.45 (s, 3H), 2.65-2.87 (m, 4H), 3.91-4.03 (m, 3H), 4.26 (q, 2H), 5.04-5.15 (m, 2H), 6.87 (t, 1H), 6.99 (d, 1H), 7.09-7.21 (m, 2H), 7.25 (d, 1H), 7.52 (d, 2H), 7.61 (s, 1H), 7.66 (dd, 2H), 7.87 (d, 1H), 8.15 (d, 2H).

[α]_{D}²⁰ = +54.95°, c = 0.330, Methanol.

Analog zu den Beispielen 35A und 36A wurden die folgenden Verbindungen hergestellt:

| **Beispiel** | **Name / Struktur / Edukte** | **Analytische Daten** |
|---|---|---|
| **37A** | *rac*-Ethyl-5-{(5-ethoxy-5-oxopentyl)[2-(2-{[4-(1-propionylpiperidin-4-yl)benzyl]oxy}phenyl)ethyl]-amino}-5,6,7,8-tetrahydrochinolin-2-carboxylat | LC-MS (Methode 3): Rₜ = 1.11 min; m/z = 698 (M+H)⁺. |
| | 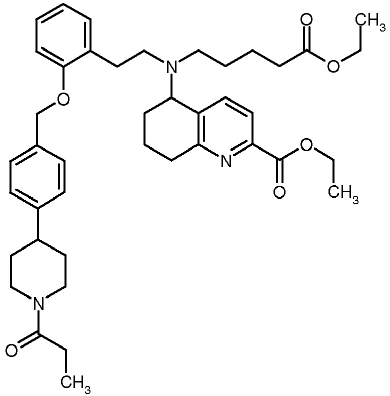 | |
| | aus Ethyl-5-{[2-(2-{[4-(1-propionylpiperidin-4-yl)-benzyl]oxy}phenyl)ethyl]amino}-5,6,7,8-tetrahydro-chinolin-2-carboxylat-Dihydrochlorid und Ethyl-5 -brompentanoat | |
| **38A** | *rac*-Ethyl-5-[(2-{2-[(4-*tert*.-butylbenzyl)oxy]-phenyl}ethyl)(5-ethoxy-5-oxopentyl)amino]-5,6,7,8-tetrahydrochinolin-2-carboxylat | LC-MS (Methode 4): Rₜ = 1.40 min; m/z = 615 (M+H)⁺. |
| | 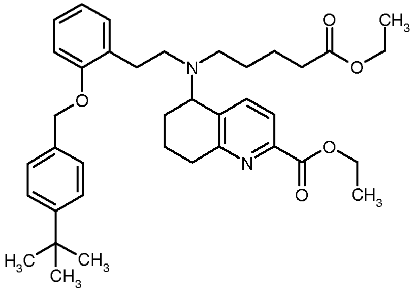 | |
| | aus Ethyl-5-[(2-{2-[(4-*tert*.-butylbenzyl)oxy]-phenyl}ethyl)amino]-5,6,7,8-tetrahydrochinolin-2-carboxylat-Dihydrochlorid und Ethyl-5-brom-pentanoat | |
| **39A** | *rac*-Ethyl-5-[(2-{2-[(4-cyclohexylbenzyl)oxy]-phenyl ethyl)(5-ethoxy-5-oxopentyl)amino]-5,6,7,8-tetrahydrochinolin-2-carboxylat | LC-MS (Methode 3): Rₜ = 1.49 min; m/z = 641 (M+H)⁺. |
| | 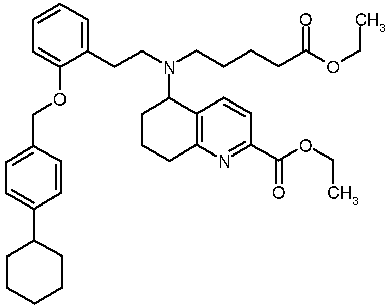 | |
| | aus Ethyl-5-[(2-{2-[(4-cyclohexylbenzyl)oxy]-phenyl}ethyl)amino]-5,6,7,8-tetrahydrochinolin-2-carboxylat-Dihydrochlorid und Ethyl-5-brom-pentanoat | |
| **40A** | *rac*-Ethyl-5-{[2-(2-{[3-chlor-4'-(trifluormethyl)-biphenyl-4-yl]methoxy}phenyl)ethyl](5-ethoxy-5-oxopentyl)amino}-5,6,7,8-tetrahydrochinolin-2-carboxylat | LC-MS (Methode 3): Rₜ = 1.49 min; m/z = 737 (M+H)⁺. |
| | 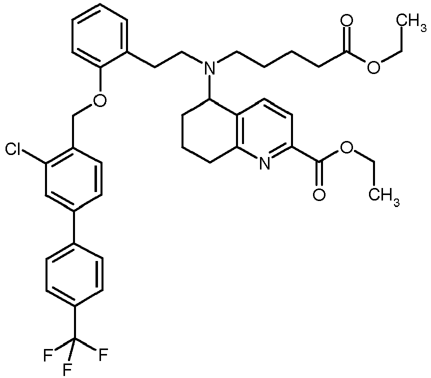 | |
| | aus Ethyl-5-{[2-(2-{[3-chlor-4'-(trifluormethyl)-biphenyl-4-yl]methoxy}phenyl)ethyl]amino}-5,6,7,8-tetrahydrochinolin-2-carboxylat-Dihydrochlorid und Ethyl-5-brompentanoat | |
| **41A** | *rac*-Ethyl-5-[(5-ethoxy-5-oxopentyl)(2-{2-[(5-phenylpentyl)oxy]phenyl}ethyl)amino]-5,6,7,8-tetrahydrochinolin-2-carboxylat | LC-MS (Methode 3): Rₜ = 1.35 min; m/z = 615 (M+H)⁺. |
| | 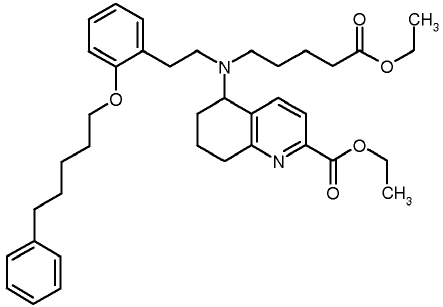 | |
| | aus Ethyl-5-[(2-{2-[(5-phenylpentyl)oxy]phenyl}-ethyl)amino]-5,6,7,8-tetrahydrochinolin-2-carboxylat-Dihydrochlorid und Ethyl-5-brom-pentanoat | |
| **42A** | *rac*-Ethyl-5-[(5-ethoxy-5-oxopentyl){2-[2-({4-[*trans*-4-(trifluormethyl)cyclohexyl]benzyl}oxy)-phenyl]ethyl}amino]-5,6,7,8-tetrahydrochinolin-2-carboxylat | LC-MS (Methode 3): Rₜ = 1.45 min; m/z = 709 (M+H)⁺. |
| | 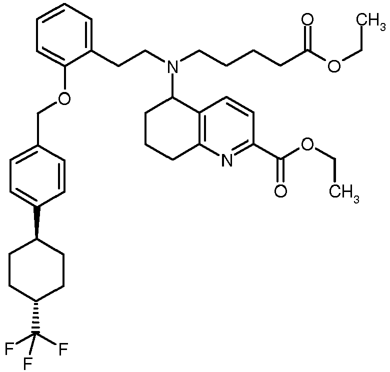 | |
| | aus Ethyl-5-({2-[2-({4-[*trans*-4-(trifluormethyl)-cyclohexyl]benzyl}oxy)phenyl]ethyl}amino)-5,6,7,8-tetrahydrochinolin-2-carboxylat-Dihydrochlorid und Ethyl-5-brompentanoat | |

### Beispiel 43A

### rac-Ethyl-5-[(2-{2-[(4-tert.-butylbenzyl)oxy]phenyl}ethyl){2-[4-(methoxycarbonyl)phenyl]ethyl}-amino]-5,6,7,8-tetrahydrochinolin-2-carboxylat

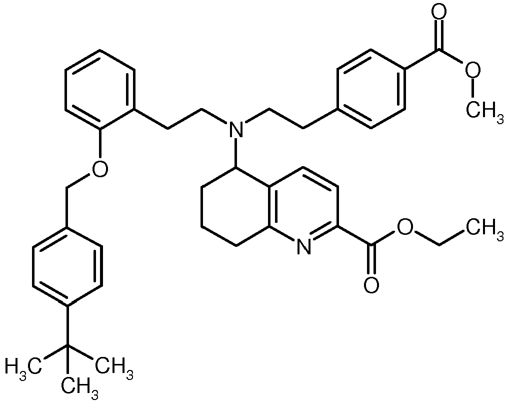

Eine Lösung von 210 mg (0.43 mmol) Ethyl-5-[(2-{2-[(4-*tert*.-butylbenzyl)oxy]phenyl}ethyl)-amino]-5,6,7,8-tetrahydrochinolin-2-carboxylat-Dihydrochlorid in 4 ml trockenem Acetonitril wurde mit 129 mg (0.65 mmol) Methyl-4-(2-chlorethyl)benzoat sowie 91 mg (0.86 mmol) wasserfreiem Natriumcarbonat versetzt und zunächst 4 Stunden unter Rückfluss erhitzt. Anschließend wurden weitere 0.1 ml Methyl-4-(2-chlorethyl)benzoat zugegeben und das Gemisch 4 Stunden unter Rückfluss nachgerührt. Es wurden nochmals 0.1 ml Methyl-4-(2-chlorethyl)benzoat zudosiert und das Gemisch dann über Nacht unter Rückfluss erhitzt. Danach wurden erneut 0.1 ml Methyl-4-(2-chlorethyl)benzoat sowie 100 mg wasserfreies Natriumcarbonat hinzugefügt und weitere 5 Stunden unter Rückfluss gerührt. Abschließend wurden nochmals 0.1 ml Methyl-4-(2-chlorethyl)benzoat sowie 0.2 ml Methyl-4-(2-iodethyl)benzoat hinzugegeben und das Reaktionsgemisch 2 Tage unter Rückfluss nachgerührt. Danach wurde der Ansatz bis zur Trockene eingeengt. Der erhaltene Rückstand wurde mittels präparativer HPLC gereinigt. Es wurden 38 mg (0.06 mmol, Gehalt 92%, 14% d. Th.) der Titelverbindung als farbloses Öl erhalten.

LC-MS (Methode 4): Rₜ = 1.66 min; m/z = 649 (M+H)⁺.

### Beispiel 44A und Beispiel 45A

### Ethyl-5-[(2-{2-[(4-tert.-butylbenzyl)oxy]phenyl}ethyl)(5-ethoxy-5-oxopentyl)amino]-5,6,7,8-tetrahydrochinolin-2-carboxylat (Enantiomer 1 und 2)

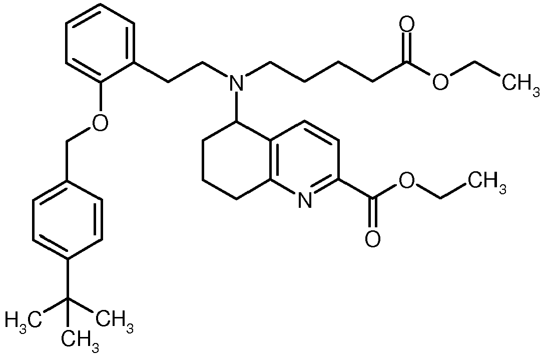

765 mg (1.24 mmol) des racemischen Ethyl-5-[(2-{2-[(4-*tert*.-butylbenzyl)oxy]phenyl}ethyl)-(5-ethoxy-5-oxopentyl)amino]-5,6,7,8-tetrahydrochinolin-2-carboxylats (Beispiel 38A) wurden durch präparative HPLC an chiraler Phase in die Enantiomere aufgetrennt [Säule: chirale Kieselgel-Phase basierend auf dem Selektor Poly(*N*-methacryloyl-L-phenylalanin-D-neomenthylamid) an sphärischem SH-Kieselgel, 10 µm, 250 mm x 20 mm; Elutionsmittel: Essigsäureethylester/*iso-*Hexan 20:80 (v/v); Fluss: 20 ml/min; UV-Detektion: 270 nm; Temperatur: 25°C]:

### Beispiel 44A (Enantiomer 1):

Ausbeute: 318 mg
Rₜ = 2.84 min; chemische Reinheit >98%; >99.9% ee
[Säule: chirale Kieselgel-Phase basierend auf dem Selektor Poly(N-methacryloyl-L-phenylalanin-D-neomenthylamid) an sphärischem SH-Kieselgel, 5 µm, 250 mm x 4 mm; Elutionsmittel: Essigsäureethylester/*iso*-Hexan 20:80 (v/v); Fluss: 1.5 ml/min; UV-Detektion: 260 nm; Temperatur: 25°C].

### Beispiel 45A (Enantiomer 2):

Ausbeute: 316 mg
Rₜ = 3.50 min; chemische Reinheit >98%; >99% ee
[Säule: chirale Kieselgel-Phase basierend auf dem Selektor Poly(N-methacryloyl-L-phenylalanin-D-neomenthylamid) an sphärischem SH-Kieselgel, 5 µm, 250 mm x 4 mm; Elutionsmittel: Essigsäureethylester/*iso*-Hexan 20:80 (v/v); Fluss: 1.5 ml/min; UV-Detektion: 260 nm; Temperatur: 25°C].

### Beispiel 46A und Beispiel 47A

### Ethyl-5-{[2-(2-{[3-chlor-4'-(trifluormethyl)biphenyl-4-yl]methoxy}phenyl)ethyl](5-ethoxy-5-oxo-pentyl)amino}-5,6,7,8-tetrahydrochinolin-2-carboxylat (Enantiomer 1 und 2)

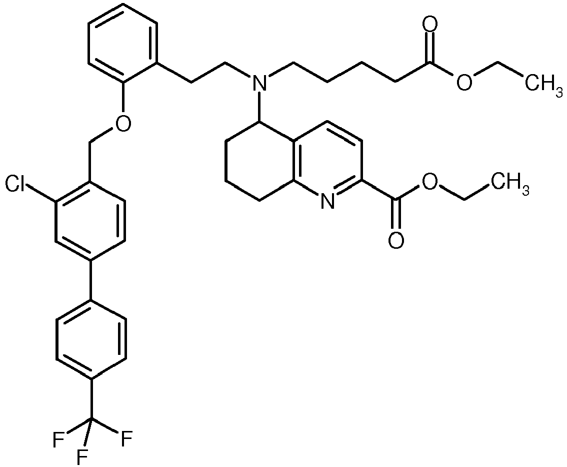

69 mg (0.09 mmol) des racemischen Ethyl-5-{[2-(2-{[3-chlor-4'-(trifluormethyl)biphenyl-4-yl]-methoxy}phenyl)ethyl](5-ethoxy-5-oxopentyl)amino}-5,6,7,8-tetrahydrochinolin-2-carboxylats (Beispiel 40A) wurden durch präparative HPLC an chiraler Phase in die Enantiomere aufgetrennt [Säule: Daicel Chiralcel OZ-H, 5 µm, 250 mm x 20 mm; Elutionsmittel: Ethanol/*iso*-Hexan 50:50 + 0.2% Diethylamin (v/v); Fluss: 15 ml/min; UV-Detektion: 220 nm; Temperatur: 40°C]:

### Beispiel 46A (Enantiomer 1):

Ausbeute: 28 mg
Rₜ = 4.34 min; chemische Reinheit >99%; >99% ee
[Säule: Daicel Chiralcel OZ-H, 5 µm, 250 mm x 4.6 mm; Elutionsmittel: Ethanol/*iso*-Hexan 50:50 + 0.2% Diethylamin (v/v); Fluss: 1 ml/min; UV-Detektion: 220 nm; Temperatur: 40°C].
LC-MS (Methode 3): Rₜ = 1.51 min; m/z = 737 (M+H)⁺.
[α]_{D}²⁰ = +61.09°, c = 0.275, Methanol.

### Beispiel 47A (Enantiomer 2):

Ausbeute: 29 mg
Rₜ = 5.14 min; chemische Reinheit >99%; >99% ee
[Säule: Daicel Chiralcel OZ-H, 5 µm, 250 mm x 4.6 mm; Elutionsmittel: Ethanol/*iso*-Hexan 50:50 + 0.2% Diethylamin (v/v); Fluss: 1 ml/min; UV-Detektion: 220 nm; Temperatur: 40°C].
LC-MS (Methode 3): Rₜ = 1.50 min; m/z = 737 (M+H)⁺.
[α]_{D}²⁰ = -81.21°, c = 0.330, Methanol.

### Beispiel 48A und Beispiel 49A

### Ethyl-5-[(5-ethoxy-5-oxopentyl)(2-{2-[(5-phenylpentyl)oxy]phenyl}ethyl)amino]-5,6,7,8-tetra-hydrochinolin-2-carboxylat (Enantiomer 1 und 2)

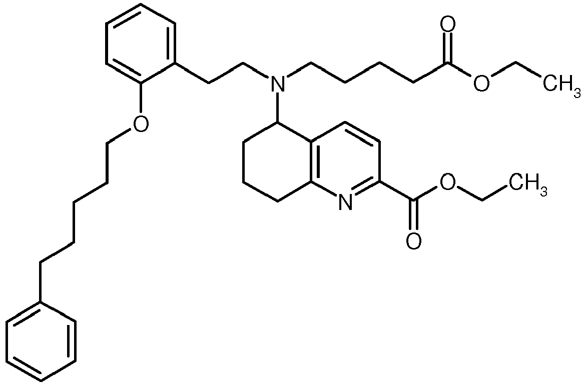

67 mg (0.11 mmol) des racemischen Ethyl-5-[(5-ethoxy-5-oxopentyl)(2-{2-[(5-phenylpentyl)oxy]-phenyl}ethyl)amino]-5,6,7,8-tetrahydrochinolin-2-carboxylats (Beispiel 41A) wurden durch präparative HPLC an chiraler Phase in die Enantiomere aufgetrennt [Säule: Daicel Chiralcel OZ-H, 5 µm, 250 mm x 20 mm; Elutionsmittel: Ethanol/*iso*-Hexan 15:85 (v/v); Fluss: 15 ml/min; UV-Detektion: 220 nm; Temperatur: 40°C]:

### Beispiel 48A (Enantiomer 1):

Ausbeute: 14 mg
Rₜ = 5.84 min; chemische Reinheit >99%; >99% ee
[Säule: Daicel Chiralcel OZ-H, 5 µm, 250 mm x 4.6 mm; Elutionsmittel: Ethanol/*iso*-Hexan 15:85 + 0.2% Diethylamin (v/v); Fluss: 1 ml/min; UV-Detektion: 220 nm; Temperatur: 40°C].
LC-MS (Methode 3): Rₜ = 1.38 min; m/z = 615 (M+H)⁺.

### Beispiel 49A (Enantiomer 2):

Ausbeute: 10 mg
Rₜ = 7.30 min; chemische Reinheit >99%; >99% ee
[Säule: Daicel Chiralcel OZ-H, 5 µm, 250 mm x 4.6 mm; Elutionsmittel: Ethanol/*iso*-Hexan 15:85 + 0.2% Diethylamin (v/v); Fluss: 1 ml/min; UV-Detektion: 220 nm; Temperatur: 40°C].
LC-MS (Methode 3): Rₜ = 1.39 min; m/z = 615 (M+H)⁺.

### Beispiel 50A und Beispiel 51A

### Ethyl-5-{(5-ethoxy-5-oxopentyl)[2-(2-{[4-(2-phenylethyl)benzyl]oxy}phenyl)ethyl]amino}-5,6,7,8-tetrahydrochinolin-2-carboxylat (Enantiomer 1 und 2)

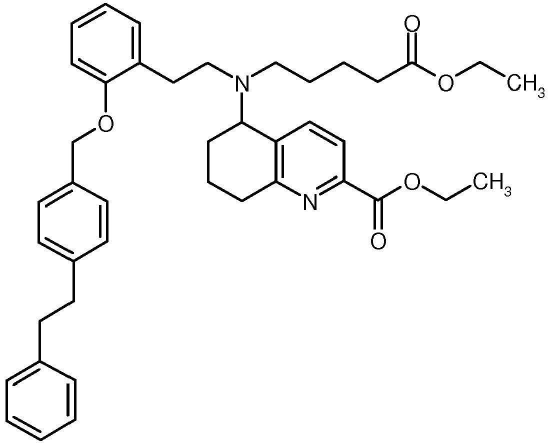

760 mg (1.15 mmol) des racemischen Ethyl-5-{(5-ethoxy-5-oxopentyl)[2-(2-{[4-(2-phenylethyl)-benzyl]oxy}phenyl)ethyl]amino}-5,6,7,8-tetrahydrochinolin-2-carboxylats (Beispiel 15A) wurden durch präparative HPLC an chiraler Phase in die Enantiomere aufgetrennt [Säule: Daicel Chiralpak AD-H, 5 µm, 250 mm x 20 mm; Elutionsmittel: *iso*-Propanol (+ 0.2% Diethylamin)/*iso*-Hexan 50:50 (v/v); Fluss: 20 ml/min; UV-Detektion: 210 nm; Temperatur: 20°C]:

### Beispiel 50A (Enantiomer 1):

Ausbeute: 261 mg
Rₜ = 8.78 min; chemische Reinheit >98%; >99% ee
[Säule: Daicel Chiralpak AD-H, 5 µm, 250 mm x 4.6 mm; Elutionsmittel: *iso*-Propanol/*iso*-Hexan 15:85 (v/v); Fluss: 1 ml/min; UV-Detektion: 230 nm; Temperatur: 20°C].
LC-MS (Methode 4): Rₜ = 1.40 min; m/z = 663 (M+H)⁺.

### Beispiel 51A (Enantiomer 2):

Ausbeute: 276 mg
Rₜ = 9.89 min; chemische Reinheit >86%; >98.5% ee
[Säule: Daicel Chiralpak AD-H, 5 µm, 250 mm x 4.6 mm; Elutionsmittel: *iso*-Propanol/*iso*-Hexan 15:85 (v/v); Fluss: 1 ml/min; UV-Detektion: 230 nm; Temperatur: 20°C].
LC-MS (Methode 4): Rₜ = 1.40 min; m/z = 663 (M+H)⁺.

### Beispiel 52A und Beispiel 53A

### Ethyl-5-[(5-ethoxy-5-oxopentyl){2-[2-({4-[2-(4-fluorphenyl)ethyl]benzyl}oxy)phenyl]ethyl}-amino]-5,6,7,8-tetrahydrochinolin-2-carboxylat (Enantiomer 1 und 2)

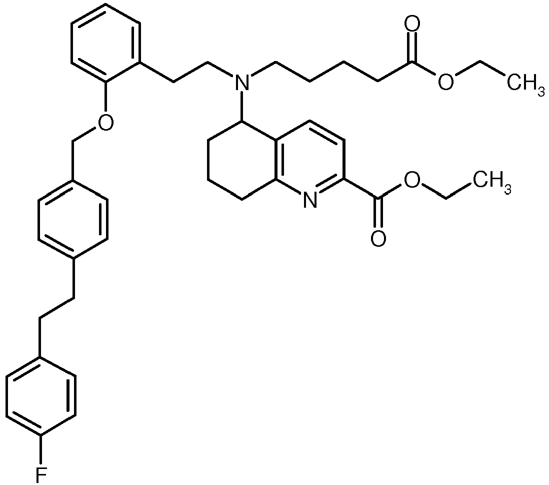

603 mg (0.89 mmol) des racemischen Ethyl-5-[(5-ethoxy-5-oxopentyl){2-[2-({4-[2-(4-fluor-phenyl)ethyl]benzyl}oxy)phenyl]ethyl}amino]-5,6,7,8-tetrahydrochinolin-2-carboxylats (Beispiel 16A) wurden durch präparative HPLC an chiraler Phase in die Enantiomere aufgetrennt [Säule: Daicel Chiralpak AD-H, 5 µm, 250 mm x 20 mm; Elutionsmittel: *iso*-Propanol/*iso*-Hexan 10:90 (v/v); Fluss: 20 ml/min; UV-Detektion: 230 nm; Temperatur: 25°C]:

### Beispiel 52A (Enantiomer 1):

Ausbeute: 70 mg
Rₜ = 10.83 min; chemische Reinheit >97.5%; >99% ee
[Säule: Daicel Chiralpak AD-H, 5 µm, 250 mm x 4.6 mm; Elutionsmittel: *iso*-Propanol (+ 0.2% Diethylamin)/*iso*-Hexan 10:90 (v/v); Fluss: 1 ml/min; UV-Detektion: 230 nm; Temperatur: 40°C].
LC-MS (Methode 4): Rₜ = 1.40 min; m/z = 681 (M+H)⁺.

### Beispiel 53A (Enantiomer 2):

Ausbeute: 72 mg
Rₜ = 12.69 min; chemische Reinheit >93.5%; >98% ee
[Säule: Daicel Chiralpak AD-H, 5 µm, 250 mm x 4.6 mm; Elutionsmittel: *iso*-Propanol (+ 0.2% Diethylamin)/*iso*-Hexan 10:90 (v/v); Fluss: 1 ml/min; UV-Detektion: 230 nm; Temperatur: 40°C].
LC-MS (Methode 4): Rₜ = 1.40 min; m/z = 681 (M+H)⁺.

### Beispiel 54A und Beispiel 55A

### Ethyl-5-{(5-ethoxy-5-oxopentyl)[2-(2-{[4'-(trifluormethyl)biphenyl-4-yl]methoxy}phenyl)ethyl]-amino}-5,6,7,8-tetrahydrochinolin-2-carboxylat (Enantiomer 1 und 2)

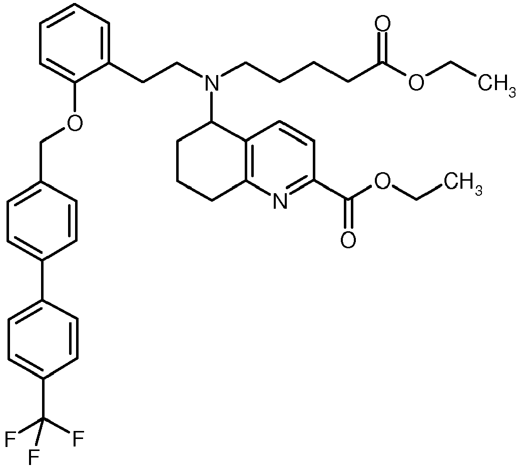

642 mg (0.91 mmol) des racemischen Ethyl-5-{(5-ethoxy-5-oxopentyl)[2-(2-{[4'-(trifluormethyl)-biphenyl-4-yl]methoxy}phenyl)ethyl]amino}-5,6,7,8-tetrahydrochinolin-2-carboxylats (Beispiel 17A) wurden durch präparative HPLC an chiraler Phase in die Enantiomere aufgetrennt [Säule: Daicel Chiralpak AD-H, 5 µm, 250 mm x 20 mm; Elutionsmittel: *iso*-Propanol/*iso*-Hexan 20:80 (v/v); Fluss: 15 ml/min; UV-Detektion: 220 nm; Temperatur: 40°C]:

### Beispiel 54A (Enantiomer 1):

Ausbeute: 161 mg
Rₜ = 5.50 min; chemische Reinheit >99%; >99% ee
[Säule: Daicel Chiralpak AD-H, 5 µm, 250 mm x 4.6 mm; Elutionsmittel: *iso*-Propanol (+ 0.2% Diethylamin)/*iso*-Hexan 20:80 (v/v); Fluss: 1 ml/min; UV-Detektion: 220 nm; Temperatur: 40°C].
LC-MS (Methode 4): Rₜ = 1.44 min; m/z = 703 (M+H)⁺.

### Beispiel 55A (Enantiomer 2):

Ausbeute: 168 mg
Rₜ = 7.01 min; chemische Reinheit >97.5%; >99% ee
[Säule: Daicel Chiralpak AD-H, 5 µm, 250 mm x 4.6 mm; Elutionsmittel: *iso*-Propanol (+ 0.2% Diethylamin)/*iso*-Hexan 20:80 (v/v); Fluss: 1 ml/min; UV-Detektion: 220 nm; Temperatur: 40°C].
LC-MS (Methode 4): Rₜ = 1.44 min; m/z = 703 (M+H)⁺.

Analog zu Beispiel 11A wurde die folgende Verbindung aus den angegebenen Edukten hergestellt:

| **Beispiel** | **Name / Struktur / Edukte** | **Analytische Daten** |
|---|---|---|
| **56A** | *N*-(5-Chlor-2-hydroxyphenyl)-4-(chlormethyl)-benzamid | LC-MS (Methode 3): Rₜ = 1.05 min; m/z = 296/298 (M+H)⁺. |
| | 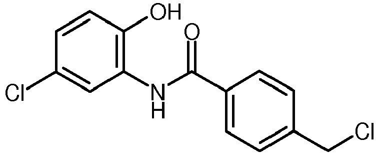 | ¹H-NMR (400 MHz, DMSO-*d*₆): δ [ppm] = 4.85 (s, 2H), 6.93 (d, 1H), 7.08 (dd, 1H), 7.60 (d, 2H), 7.83 (d, 1H), 7.96 (d, 2H), 9.53 (s, 1H), 10.17 (s, 1H). |
| | aus 2-Amino-4-chlorphenol und 4-(Chlormethyl)benzoylchlorid | |

Analog zu Beispiel 12A wurde die folgende Verbindung aus dem angegebenen Edukt hergestellt:

| **Beispiel** | **Name / Struktur / Edukt** | **Analytische Daten** |
|---|---|---|
| **57A** | 5-Chlor-2-[4-(chlormethyl)phenyl]-1,3-benzoxazol | LC-MS (Methode 3): Rₜ = 1.34 min; m/z = 278/280 (M+H)⁺. |
| | 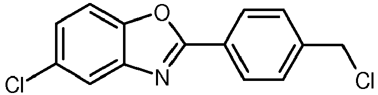 | ¹H-NMR (400 MHz, DMSO-*d*₆): δ [ppm] = 4.89 (s, 2H), 7.50 (dd, 1H), 7.70 (d, 2H), 7.86 (d, 1H), 7.95 (d, 1H), 8.22 (d, 2H). |
| | aus *N*-(5-Chlor-2-hydroxyphenyl)-4-(chlormethyl)benzamid | |

Analog zu Beispiel 18A wurde die folgende Verbindung aus den angegebenen Edukten hergestellt:

| **Beispiel** | **Name / Struktur / Edukte** | **Analytische Daten** |
|---|---|---|
| **58A** | *rac*-Ethyl-5-{(*tert*.-butoxycarbonyl)[2-(2-{[4-(5-chlor-1,3-benzoxazol-2-yl)benzyl]oxy}phenyl)-ethyl]amino}-5,6,7,8-tetrahydrochinolin-2-carboxylat | LC-MS (Methode 3): Rₜ = 1.64 min; m/z = 682/684 (M+H)⁺. |
| | 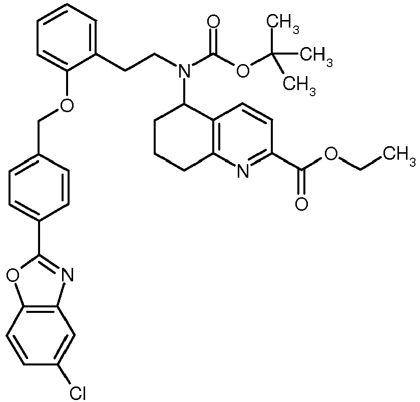 | |
| | aus Ethyl-5-{(*tert*.-butoxycarbonyl)[2-(2-hydroxy-phenyl)ethyl]amino}-5,6,7,8-tetrahydrochinolin-2-carboxylat und 5-Chlor-2-[4-(chlormethyl)-phenyl]-1,3-benzoxazol | |

### Beispiel 59A und Beispiel 60A

### Ethyl-5- {(tert.-butoxycarbonyl)[2-(2-{[4-(5-chlor-1,3-benzoxazol-2-yl)benzyl]oxy}phenyl)ethyl]-amino}-5,6,7,8-tetrahydrochinolin-2-carboxylat (Enantiomer 1 und 2)

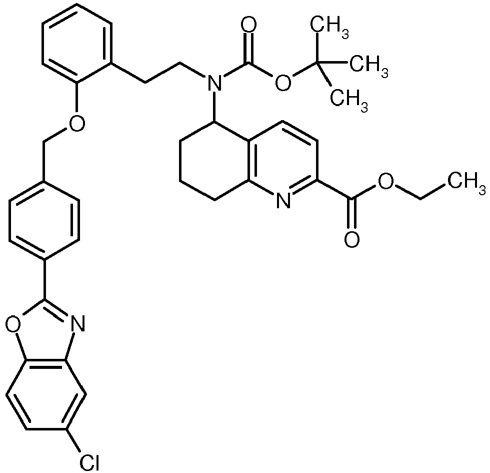

494 mg (0.72 mmol) des racemischen Ethyl-5-{(*tert*.-butoxycarbonyl)[2-(2-{[4-(5-chlor-1,3-benz-oxazol-2-yl)benzyl]oxy}phenyl)ethyl]amino}-5,6,7,8-tetrahydrochinolin-2-carboxylats (Beispiel 58A) wurden durch superkritische Flüssigchromatographie (SFC) an chiraler Phase in die Enantiomere aufgetrennt [Säule: Daicel Chiracel OD-H, 5 µm, 250 mm x 20 mm; Elutionsmittel: Kohlendioxid/Ethanol 70:30 (v/v); Fluss: 100 ml/min; Druck: 100 bar; UV-Detektion: 210 nm; Temperatur: 40°C]:

### Beispiel 59A (Enantiomer 1):

Ausbeute: 247 mg
Rₜ = 4.47 min; chemische Reinheit >99.9%; >99% ee
[Säule: Chiralpak OD-H, 5 µm, 250 mm x 4.6 mm; Elutionsmittel: Kohlendioxid/Ethanol 70:30 (v/v); Fluss: 3 ml/min; UV-Detektion: 210 nm].
LC-MS (Methode 3): Rₜ = 1.62 min; m/z = 682/684 (M+H)⁺.

### Beispiel 60A (Enantiomer 2):

Ausbeute: 213 mg
Rₜ = 9.22 min; chemische Reinheit >99%; >99% ee
[Säule: Chiralpak OD-H, 5 µm, 250 mm x 4.6 mm; Elutionsmittel: Kohlendioxid/Ethanol 70:30 (v/v); Fluss: 3 ml/min; UV-Detektion: 210 nm].
LC-MS (Methode 3): Rₜ = 1.62 min; m/z = 682/684 (M+H)⁺.

### Beispiel 61A

### Ethyl-5-{[2-(2-{[4-(5-chlor-1,3-benzoxazol-2-yl)benzyl]oxy}phenyl)ethyl]amino}-5,6,7,8-tetra-hydrochinolin-2-carboxylat (Enantiomer 1)

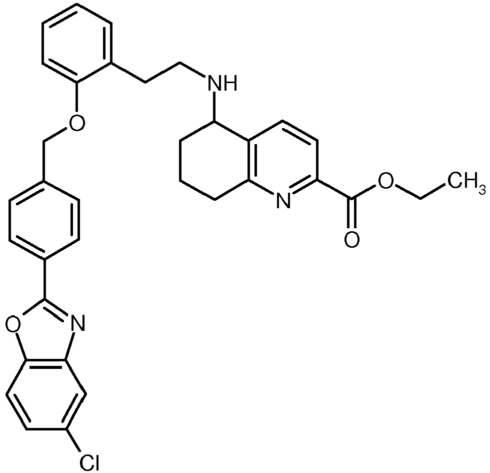

247 mg (0.36 mmol) Ethyl-5-{(*tert*.-butoxycarbonyl)[2-(2-{[4-(5-chlor-1,3-benzoxazol-2-yl)-benzyl]oxy}phenyl)ethyl]amino}-5,6,7,8-tetrahydrochinolin-2-carboxylat (Enantiomer 1, Beispiel 59A) wurden mit 10 ml einer 4 N Lösung von Chlorwasserstoff in Dioxan versetzt und 4 h bei Raumtemperatur gerührt. Die Reaktionslösung wurde danach bis zur Trockene eingeengt und der Rückstand über Nacht im Hochvakuum getrocknet. Es wurden 210 mg der Titelverbindung als Hydrochlorid in Form eines Feststoffs erhalten. Dieser Feststoff wurde in 5 ml THF aufgenommen, mit 0.13 ml Triethylamin versetzt und eine Stunde bei Raumtemperatur gerührt. Das Gemisch wurde danach mit Wasser und Essigsäureethylester versetzt und die Phasen getrennt. Die wässrige Phase wurde zweimal mit Essigsäureethylester extrahiert, und anschließend wurden die vereinigten organischen Phasen über Magnesiumsulfat getrocknet, filtriert und dann bis zur Trockene eingeengt. Es wurden 149 mg (0.26 mmol, 72% d. Th.) der Zielverbindung erhalten.

LC-MS (Methode 3): Rₜ = 1.06 min; m/z = 582/584 (M+H)⁺.

¹H-NMR (400 MHz, DMSO-*d*₆, δ/ppm): 1.27 (t, 3H), 1.63-1.77 (m, 2H), 1.81-2.02 (m, 2H), 2.71-2.91 (m, 6H), 3.44-3.54 (m, 0.5H), 3.64-3.74 (m, 0.5H), 3.75-3.84 (br. s, 1H), 4.27 (q, 2H), 5.23 (s, 2H), 6.90 (t, 1H), 7.05 (d, 1H), 7.14-7.24 (m, 2H), 7.49 (dd, 1H), 7.67 (d, 2H), 7.74 (d, 1H), 7.82-7.90 (m, 2H), 7.95 (d, 1H), 8.20 (d, 2H).

Analog zu Beispiel 61A wurde die folgende Verbindung hergestellt:

| **Beispiel** | **Name / Struktur / Edukt** | **Analytische Daten** |
|---|---|---|
| **62A** | Ethyl-5-{[2-(2-{[4-(5-chlor-1,3-benzoxazol-2-yl)-benzyl]oxy}phenyl)ethyl]amino}-5,6,7,8-tetra-hydrochinolin-2-carboxylat (*Enantiomer* 2) | LC-MS (Methode 3): Rₜ = 1.05 min; m/z = 582/584 (M+H)⁺. |
| | 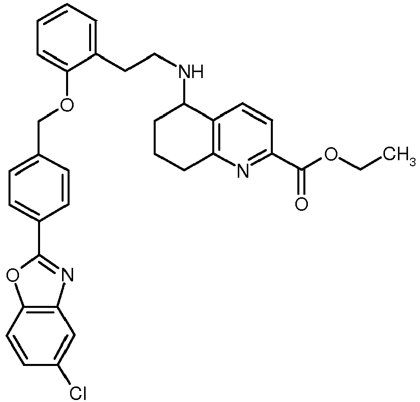 | |
| | aus Ethyl-5-{(*tert*.-butoxycarbonyl)[2-(2-{[4-(5-chlor-1,3-benzoxazol-2-yl)benzyl]oxy}phenyl)-ethyl]amino}-5,6,7,8-tetrahydrochinolin-2-carboxylat (Enantiomer 2, Beispiel 60A) | |

### Beispiel 63A

### Ethyl-5-([2-(2-{[4-(5-chlor-1,3-benzoxazol-2-yl)benzyl]oxy}phenyl)ethyl]{2-[4-(methoxy-carbonyl)phenyl]ethyl}amino)-5,6,7,8-tetrahydrochinolin-2-carboxylat (Enantiomer 1)

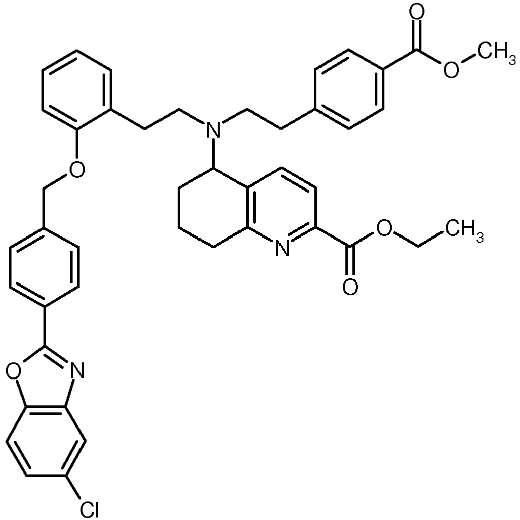

Eine Lösung von 149 mg (0.26 mmol) Ethyl-5-{[2-(2-{[4-(5-chlor-1,3-benzoxazol-2-yl)benzyl]-oxy}phenyl)ethyl]amino}-5,6,7,8-tetrahydrochinolin-2-carboxylat (Enantiomer 1, Beispiel 61A) in 10 ml trockenem Acetonitril wurde mit 112 mg (0.39 mmol) Methyl-4-(2-iodethyl)benzoat und 41 mg (0.39 mmol) wasserfreiem Natriumcarbonat versetzt und über Nacht unter Rückfluss erhitzt. Anschließend wurden weitere 112 mg Methyl-4-(2-iodethyl)benzoat zugesetzt und das Gemisch nochmals über Nacht unter Rückfluss erhitzt. Der Ansatz wurde dann bis zur Trockene eingeengt, der Rückstand in Wasser und Essigsäureethylester aufgenommen und die Phasen getrennt. Die organische Phase wurde bis zur Trockene eingeengt und der erhaltene Rückstand mittels präparativer HPLC gereinigt. Es wurden 72 mg (0.10 mmol, 38% d. Th.) der Titelverbindung erhalten.

LC-MS (Methode 3): Rₜ = 1.60 min; m/z = 744/746 (M+H)⁺.

Analog zu Beispiel 63A wurde die folgende Verbindung hergestellt:

| **Beispiel** | **Name / Struktur / Edukte** | **Analytische Daten** |
|---|---|---|
| **64A** | Ethyl-5-([2-(2-{[4-(5-chlor-1,3-benzoxazol-2-yl)-benzyl]oxy}phenyl)ethyl]{2-[4-(methoxycarbonyl)-phenyl]ethyl}amino)-5,6,7,8-tetrahydrochinolin-2-carboxylat *(Enantiomer 2*) | LC-MS (Methode 3): Rₜ = 1.61 min; m/z = 744/746 (M+H)⁺. |
| | 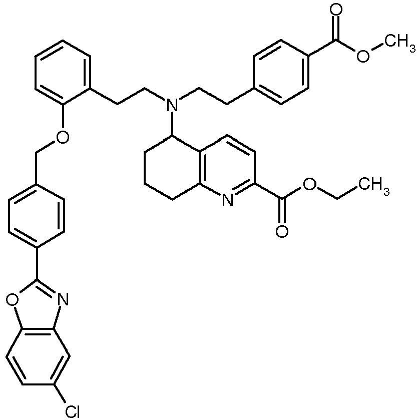 | ¹H-NMR (400 MHz, DMSO-*d₆,* δ/ppm): 1.26 (t, 3H), 1.42-1.55 (m, 1H), 1.55-1.70 (m, 1H), 1.89-2.08 (m, 2H), 2.44-2.84 (m, 10H, teilweise verdeckt durch DMSO-Signal), 3.76 (s, 3H), 4.01-4.12 (m, 1H), 4.26 (q, 2H), 5.04-5.15 (m, 2H), 6.86 (t, 1H), 7.02 (d, 1H), 7.05-7.15 (m, 3H), 7.21 (t, 1H), 7.43 (d, 1H), 7.46-7.57 (m, 4H), 7.72 (d, 2H), 7.83 (d, 1H), 7.92 (d, 1H), 8.09 (d, 2H). |
| | aus Ethyl-5-{[2-(2-{[4-(5-chlor-1,3-benzoxazol-2-yl)benzyl]oxy}phenyl)ethyl]amino}-5,6,7,8-tetrahydrochinolin-2-carboxylat (Enantiomer 2, Beispiel 62A) und Methyl-4-(2-iodethyl)benzoat | |

### Beispiel 65A

### Ethyl-5-{[2-(2-{[4-(5-methyl-1,3-benzoxazol-2-yl)benzyl]oxy}phenyl)ethyl]amino}-5,6,7,8-tetra-hydrochinolin-2-carboxylat (Enantiomer 1)

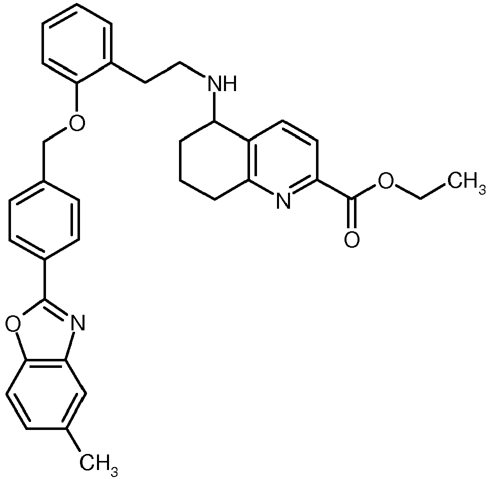

3.8 g (5.99 mmol) Ethyl-5-{[2-(2-{[4-(5-methyl-1,3-benzoxazol-2-yl)benzyl]oxy}phenyl)ethyl]-amino}-5,6,7,8-tetrahydrochinolin-2-carboxylat-Dihydrochlorid (Enantiomer 1, Beispiel 27A) wurden in 50 ml THF gelöst, mit 2.5 ml Triethylamin versetzt und 1 h bei Raumtemperatur gerührt. Das Gemisch wurde danach mit Wasser und Essigsäureethylester versetzt und die Phasen getrennt. Die wässrige Phase wurde zweimal mit Essigsäureethylester extrahiert, anschließend wurden die vereinigten organischen Phasen über Magnesiumsulfat getrocknet, filtriert und bis zur Trockene eingeengt. Es wurden 2.48 g (4.42 mmol, 74% d. Th.) der Zielverbindung erhalten.

LC-MS (Methode 3): Rₜ = 1.06 min; m/z = 562 (M+H)⁺.

¹H-NMR (400 MHz, DMSO-*d*₆, δ/ppm): 1.27 (t, 3H), 1.59-1.79 (m, 2H), 1.99 (s, 3H), 2.01-2.16 (m, 1H), 2.69-2.92 (m, 6H), 3.42-3.55 (m, 1H), 3.64-3.87 (m, 1H), 3.98-4.07 (m, 1H), 4.28 (q, 2H), 5.22 (s, 2H), 6.84-6.95 (m, 1H), 7.00-7.09 (m, 1H), 7.20 (s, 3H), 7.58-7.70 (m, 4H), 7.71-7.79 (m, 1H), 7.83-7.94 (m, 1H), 8.18 (d, 2H).

Analog zu Beispiel 65A wurde die folgende Verbindung hergestellt:

| **Beispiel** | **Name / Struktur / Edukt** | **Analytische Daten** |
|---|---|---|
| **66A** | Ethyl-5-{[2-(2-{[4-(5-methyl-1,3-benzoxazol-2-yl)-benzyl]oxy}phenyl)ethyl]amino}-5,6,7,8-tetra-hydrochinolin-2-carboxylat (*Enantiomer* 2) | LC-MS (Methode 6): Rₜ = 3.24 min; m/z = 562 (M+H)⁺. |
| | 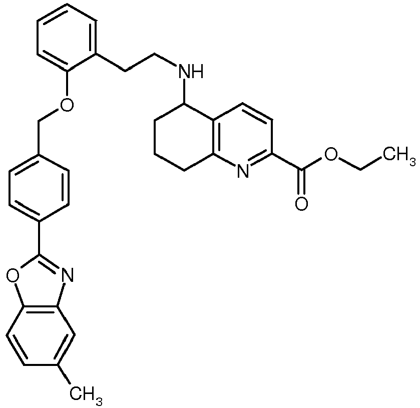 | |
| | aus Ethyl-5-{[2-(2-{[4-(5-methyl-1,3-benzoxazol-2-yl)benzyl]oxy}phenyl)ethyl]amino}-5,6,7,8-tetrahydrochinolin-2-carboxylat-Dihydrochlorid (Enantiomer 2, Beispiel 28A) | |

Analog zu Beispiel 63A wurden die folgenden Verbindungen hergestellt:

| **Beispiel** | **Name / Struktur / Edukte** | **Analytische Daten** |
|---|---|---|
| **67A** | Ethyl-5-({2-[4-(methoxycarbonyl)phenyl]ethyl}-[2-(2-{[4-(5-methyl-1,3-benzoxazol-2-yl)-benzyl]oxy}phenyl)ethyl]amino)-5,6,7,8-tetrahydrochinolin-2-carboxylat (*Enantiomer 1*) | LC-MS (Methode 3): Rₜ = 1.61 min; m/z = 724 (M+H)⁺. |
| | 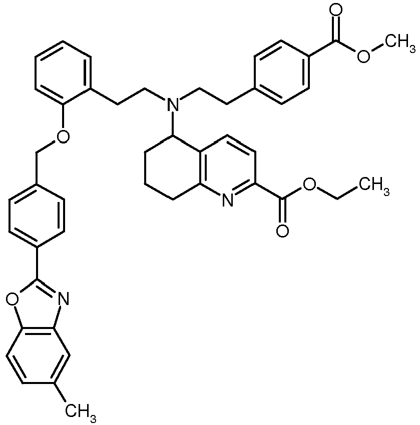 | |
| | aus Ethyl-5-{[2-(2-{[4-(5-methyl-1,3-benzoxazol-2-yl)benzyl]oxy}phenyl)ethyl]amino}-5,6,7,8-tetrahydrochinolin-2-carboxylat (Enantiomer 1, Beispiel 65A) und Methyl-4-(2-iodethyl)benzoat | |
| **68A** | Ethyl-5-({2-[4-(methoxycarbonyl)phenyl]ethyl}-[2-(2-{[4-(5-methyl-1,3-benzoxazol-2-yl)-benzyl]oxy}phenyl)ethyl]amino)-5,6,7,8-tetrahydrochinolin-2-carboxylat (*Enantiomer* 2) | LC-MS (Methode 3): Rₜ = 1.58 min; m/z = 724 (M+H)⁺. |
| | 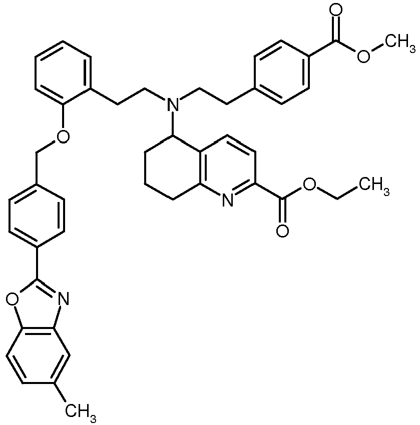 | ¹H-NMR (400 MHz, DMSO-*d₆,* δ/ppm): 1.26 (t, 3H), 1.42-1.54 (m, 1H), 1.55-1.71 (m, 1H), 1.89-2.09 (m, 2H), 2.42-2.57 (m, 3H, teilweise verdeckt durch DMSO-Signal) 2.57-2.86 (m, 10H), 3.76 (s, 3H), 4.02-4.13 (m, 1H), 4.26 (q, 2H), 5.08 (m, 2H), 6.79-6.92 (m, 1H), 6.98-7.09 (m, 2H), 7.10-7.16 (m, 2H), 7.17-7.29 (m, 2H), 7.42 (d, 1H), 7.48-7.56 (m, 3H), 7.57-7.69 (m, 2H), 7.74 (d, 2H), 8.09 (d, 2H). |
| | aus Ethyl-5-{[2-(2-{[4-(5-methyl-1,3-benzoxazol-2-yl)benzyl]oxy}phenyl)ethyl]amino}-5,6,7,8-tetrahydrochinolin-2-carboxylat (Enantiomer 2, Beispiel 66A) und Methyl-4-(2-iodethyl)benzoat | |

### Beispiel 69A

### Ethyl-5-{(tert.-butoxycarbonyl)[2-(2-hydroxyphenyl)ethyl]amino}-5,6,7,8-tetrahydrochinolin-2-carboxylat (Enantiomer 2)

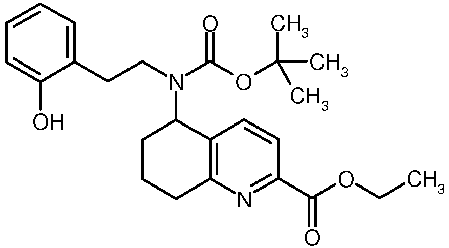

10 g (15.11 mmol) (-)-Ethyl-5-{(*tert*.-butoxycarbonyl)[2-(2-{[4-(5-methyl-1,3-benzoxazol-2-yl)-benzyl]oxy}phenyl)ethyl]amino}-5,6,7,8-tetrahydrochinolin-2-carboxylat (Enantiomer 2, Beispiel 26A) wurden in 500 ml Ethanol gelöst und mit 9.53 g (151.10 mmol) Ammoniumformiat sowie 161 mg (1.51 mmol) 10% Palladium auf Aktivkohle versetzt. Anschließend wurde das Reaktionsgemisch auf 80°C erhitzt und über Nacht bei dieser Temperatur gerührt. Danach wurde das Gemisch auf Raumtemperatur abgekühlt, nochmals mit 100 mg des Palladium-Katalysators versetzt und weitere 6 h bei 80°C gerührt. Das Reaktionsgemisch wurde dann wieder auf Raumtemperatur abgekühlt, filtriert und das Filtrat bis zur Trockene eingedampft. Der erhaltene Rückstand wurde chromatographisch an Kieselgel gereinigt (Laufmittel: Cyclohexan/Essigsäureethylester 20:1 → 2:1). Es wurden 6.55 g (14.87 mmol, 98% d. Th.) der Titelverbindung erhalten.

LC-MS (Methode 3): Rₜ = 1.17 min; m/z = 441 (M+H)⁺.

Ein alternativer Darstellungsweg zu Beispiel 69A ist in Zusammenhang mit der Beschreibung von Beispiel 147A wiedergegeben (siehe dort).

Analog zu Beispiel 11A wurden die folgenden Verbindungen aus den angegebenen Edukten hergestellt:

| **Beispiel** | **Name / Struktur / Edukte** | **Analytische Daten** |
|---|---|---|
| **70A** | 4-(Chlormethyl)-*N*-[2-hydroxy-5-(trifluormethyl)-phenyl]benzamid | LC-MS (Methode 3): Rₜ = 1.12 min; m/z = 330 (M+H)⁺. |
| | 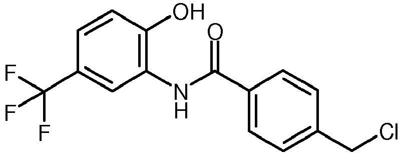 | ¹H-NMR (400 MHz, DMSO-*d*₆): δ [ppm] = 4.85 (s, 2H), 7.09 (d, 1H), 7.40 (dd, 1H), 7.60 (d, 2H), 7.98 (d, 2H), 8.12 (d, 1H), 9.62 (s, 1H), 10.81 (s, 1H). |
| | aus 2-Amino-4-(trifluormethyl)phenol und 4-(Chlormethyl)benzoylchlorid | |
| **71A** | 4-(Chlormethyl)-*N*-[2-hydroxy-5-(trifluormethoxy)-phenyl]benzamid | LC-MS (Methode 3): Rₜ = 1.14 min; m/z = 346 (M+H)⁺. |
| | 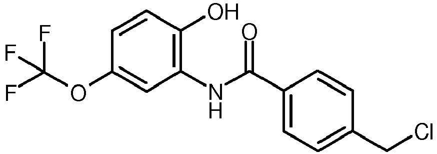 | ¹H-NMR (400 MHz, DMSO-*d*₆): δ [ppm] = 4.85 (s, 2H), 6.96-7.01 (m, 1H), 7.02-7.08 (m, 1H), 7.60 (d, 2H), 7.78-7.90 (m, 1H), 7.97 (d, 2H), 9.54 (s, 1H), 10.31 (s, 1H). |
| | aus 2-Amino-4-(trifluormethoxy)phenol und 4-(Chlormethyl)benzoylchlorid | |

Analog zu Beispiel 12A wurden die folgenden Verbindungen aus dem angegebenen Edukt hergestellt:

| **Beispiel** | **Name / Struktur / Edukt** | **Analytische Daten** |
|---|---|---|
| **72A** | 2-[4-(Chlormethyl)phenyl]-5-(trifluormethyl)-1,3-benzoxazol | LC-MS (Methode 3): Rₜ = 1.32 min; m/z = 312 (M+H)⁺. |
| | 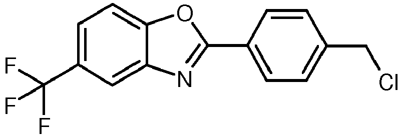 | ¹H-NMR (400 MHz, DMSO-*d*₆): δ [ppm] = 4.90 (s, 2H), 7.71 (d, 2H), 7.82 (dd, 1H), 8.04 (d, 1H), 8.19-8.35 (m, 3H). |
| | aus 4-(Chlormethyl)-*N*-[2-hydroxy-5-(trifluor-methyl)phenyl]benzamid | |
| **73A** | 2-[4-(Chlormethyl)phenyl]-5-(trifluormethoxy)-1,3-benzoxazol | LC-MS (Methode 3): Rₜ = 1.37 min; m/z = 328 (M+H)⁺. |
| | 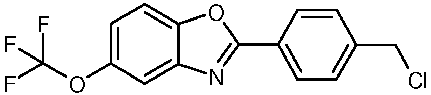 | ¹H-NMR (400 MHz, DMSO-*d*₆): δ [ppm] = 4.89 (s, 2H), 7.47 (dd, 1H), 7.70 (d, 2H), 7.87-8.01 (m, 2H), 8.23 (d, 2H). |
| | aus 4-(Chlormethyl)-*N*-[2-hydroxy-5-(trifluor-methoxy)phenyl]benzamid | |

### Beispiel 74A

### Ethyl-5-{(tert.-butoxycarbonyl)[2-(2-{[3-chlor-4'-(trifluormethyl)biphenyl-4-yl]methoxy}phenyl)-ethyl]amino}-5,6,7,8-tetrahydrochinolin-2-carboxylat (Enantiomer 2)

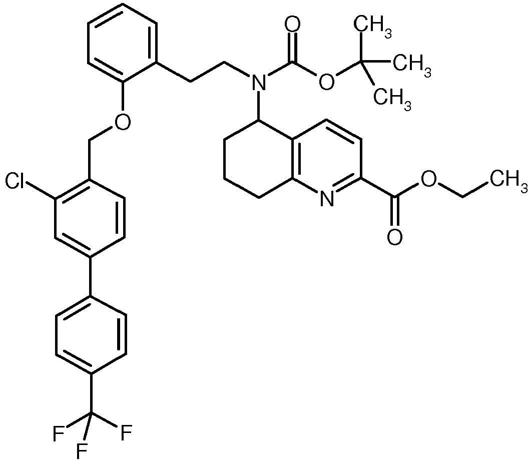

Eine Suspension aus 51.21 g (116.24 mmol) Ethyl-5-{(*tert*.-butoxycarbonyl)[2-(2-hydroxyphenyl)-ethyl]amino}-5,6,7,8-tetrahydrochinolin-2-carboxylat (Enantiomer 2, Beispiel 69A), 44.70 g (127.86 mmol) 4-(Brommethyl)-3-chlor-4'-(trifluormethyl)biphenyl und 40.16 g (290.60 mmol) Kaliumcarbonat in 1420 ml Acetonitril wurde auf 110°C erhitzt und über Nacht bei dieser Temperatur gerührt. Nach dem Abkühlen wurde das Reaktionsgemisch filtriert, der Filterkuchen mehrmals mit Acetonitril nachgewaschen und die vereinigten Filtrate bis zur Trockene am Rotationsverdampfer eingeengt. Der erhaltene Rückstand wurde chromatographisch an Kieselgel (2.5 kg) gereinigt (Laufmittel: Petrolether/Essigsäureethylester 4:1). Es wurden 79 g (111.39 mmol, 96% d. Th.) der Zielverbindung erhalten.

LC-MS (Methode 3): Rₜ = 1.69 min; m/z = 709 (M+H)⁺.

¹H-NMR (400 MHz, DMSO-*d*₆, δ/ppm): 1.05-1.20 (m, 4H), 1.21-1.34 (m, 4H), 1.45 (s, 6H), 1.56-1.74 (m, 2H), 1.75-1.93 (m, 2H), 2.76-2.99 (m, 3H), 4.30 (q, 2H), 5.00-5.24 (m, 3H), 6.86-6.99 (m, 1H), 7.03-7.16 (m, 1.5H), 7.17-7.29 (m, 1.5H), 7.38-7.45 (m, 0.5H), 7.50-7.56 (m, 0.5H), 7.58-7.68 (m, 1H), 7.69-7.78 (m, 1.5H), 7.79-7.93 (m, 5H), 8.01-8.12 (m, 1.5H).

Ein alternativer Darstellungsweg zu Beispiel 74A ist in Zusammenhang mit der Beschreibung von Beispiel 148A wiedergegeben (siehe dort).

Analog zum zuvor beschriebenen Beispiel 74A wurden die folgenden Verbindungen aus den jeweils angegebenen Edukten hergestellt:

| **Beispiel** | **Name / Struktur / Edukte** | **Analytische Daten** |
|---|---|---|
| **75A** | Ethyl-5-[(*tert*.-butoxycarbonyl){2-[2-({4-[5-(tri-fluormethyl)-1,3-benzoxazol-2-yl]benzyl}oxy)-phenyl]ethyl}amino]-5,6,7,8-tetrahydrochinolin-2-carboxylat (*Enantiomer 2*) | LC-MS (Methode 3): Rₜ = 1.62 min; m/z = 716 (M+H)⁺. |
| | 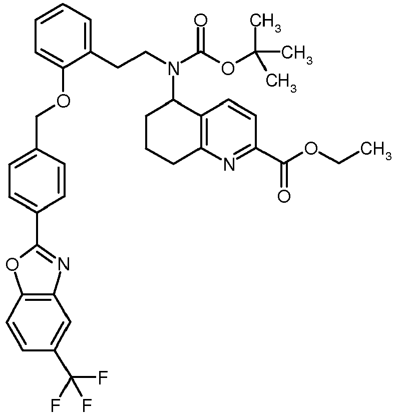 | |
| | aus Ethyl-5-{(*tert*.-butoxycarbonyl)[2-(2-hydroxyphenyl)ethyl]amino}-5,6,7,8-tetrahydrochinolin-2-carboxylat (Enantiomer 2, Beispiel 69A) und 2-[4-(Chlormethyl)phenyl]-5-(trifluormethyl)-1,3-benzoxazol | |
| **76A** | Ethyl-5-{[2-(2-{[4-(1,3-benzoxazol-2-yl)benzyl]-oxy}phenyl)ethyl](*tert*.-butoxycarbonyl)amino}-5,6,7,8-tetrahydrochinolin-2-carboxylat (*Enantiomer 2*) | LC-MS (Methode 3): Rₜ = 1.53 min; m/z = 648 (M+H)⁺. |
| | 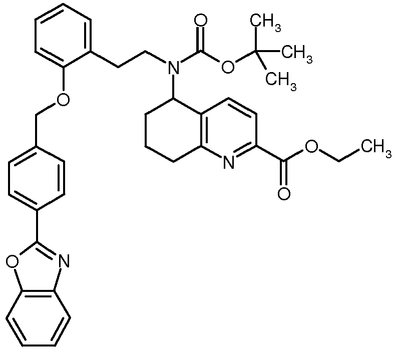 | |
| | aus Ethyl-5-{(*tert*.-butoxycarbonyl)[2-(2-hydroxyphenyl)ethyl]amino}-5,6,7,8-tetrahydrochinolin-2-carboxylat (Enantiomer 2, Beispiel 69A) und 2-[4-(Chlormethyl)phenyl]-1,3-benzoxazol | |
| **77A** | Ethyl-5-[(*tert*.-butoxycarbonyl){2-[2-({4-[5-(trifluormethoxy)-1,3-benzoxazol-2-yl]benzyl}oxy)-phenyl]ethyl}amino]-5,6,7,8-tetrahydrochinolin-2-carboxylat (*Enantiomer 2*) | LC-MS (Methode 3): Rₜ = 1.63 min; m/z = 732 (M+H)⁺. |
| | 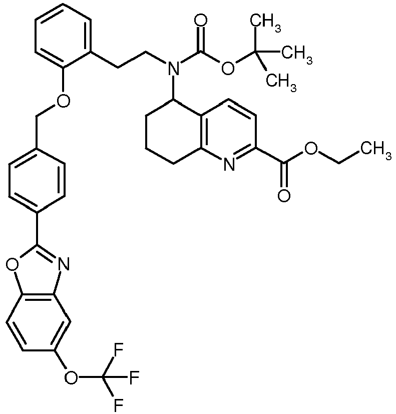 | |
| | aus Ethyl-5-{(*tert*.-butoxycarbonyl)[2-(2-hydroxyphenyl)ethyl]amino}-5,6,7,8-tetrahydrochinolin-2-carboxylat (Enantiomer 2, Beispiel 69A) und 2-[4-(Chlormethyl)phenyl]-5-(trifluormethoxy)-1,3-benzoxazol | |
| **78A** | Ethyl-5-{(*tert*.-butoxycarbonyl)[2-(2-{[4-(5-cyano-1,3-benzoxazol-2-yl)benzyl]oxy}phenyl)-ethyl]amino}-5,6,7,8-tetrahydrochinolin-2-carboxylat (*Enantiomer 2*) | LC-MS (Methode 3): Rₜ = 1.47 min; m/z = 673 (M+H)⁺. |
| | 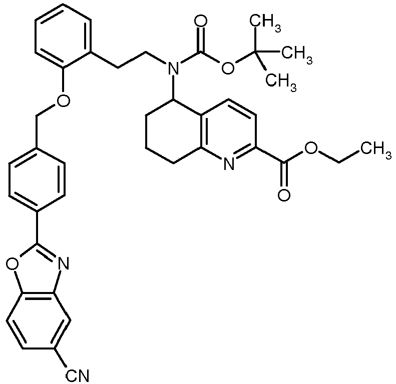 | |
| | aus Ethyl-5-{(*tert*.-butoxycarbonyl)[2-(2-hydroxyphenyl)ethyl]amino}-5,6,7,8-tetrahydrochinolin-2-carboxylat (Enantiomer 2, Beispiel 69A) und 2-[4-(Chlormethyl)phenyl]-1,3-benzoxazol-5-carbonitril [CAS Reg.-Nr. 885050-65-7] | |
| **79A** | Ethyl-5-{(*tert*.-butoxycarbonyl)[2-(2-{[4-(2-phenyl-ethyl)benzyl]oxy}phenyl)ethyl]amino}-5,6,7,8-tetrahydrochinolin-2-carboxylat (*Enantiomer 2*) | LC-MS (Methode 3): Rₜ = 1.58 min; m/z = 635 (M+H)⁺. |
| | 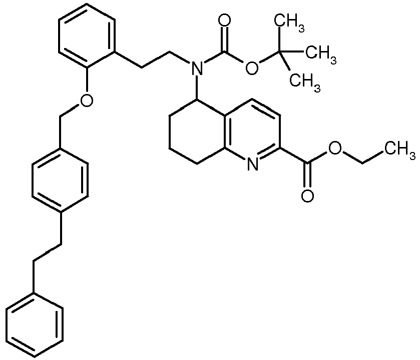 | |
| | aus Ethyl-5-{(*tert*.-butoxycarbonyl)[2-(2-hydroxyphenyl)ethyl]amino}-5,6,7,8-tetrahydrochinolin-2-carboxylat (Enantiomer 2, Beispiel 69A) und 1-(Chlormethyl)-4-(2-phenylethyl)benzol | |

### Beispiel 80A

### Ethyl-5-{[2-(2-{[3-chlor-4'-(trifluormethyl)biphenyl-4-yl]methoxy}phenyl)ethyl]amino}-5,6,7,8-tetrahydrochinolin-2-carboxylat-Dihydrochlorid (Enantiomer 2)

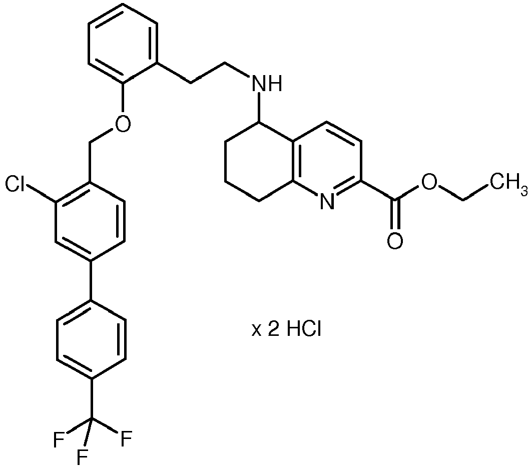

79 g (111.39 mmol) Ethyl-5-{(*tert*.-butoxycarbonyl)[2-(2-{[3-chlor-4'-(trifluormethyl)biphenyl-4-yl]methoxy}phenyl)ethyl]amino}-5,6,7,8-tetrahydrochinolin-2-carboxylat (Enantiomer 2, Beispiel 74A) wurden mit 557 ml einer 4 N Lösung von Chlorwasserstoff in Dioxan, verdünnt mit weiteren 389 ml Dioxan, versetzt und über Nacht bei Raumtemperatur gerührt. Die Reaktionslösung wurde danach bis zur Trockene eingeengt und der Rückstand über Nacht im Hochvakuum getrocknet. Es wurden 78 g (111.39 mmol, ca. 100% d. Th.) des Zielprodukts erhalten.

LC-MS (Methode 3): Rₜ = 1.07 min; m/z = 609/611 (M+H)⁺.

Analog zu Beispiel 80A wurden die folgenden Verbindungen hergestellt:

| **Beispiel** | **Name / Struktur / Edukt** | **Analytische Daten** |
|---|---|---|
| **81A** | Ethyl-5-({2-[2-({4-[5-(trifluormethyl)-1,3-benzoxazol-2-yl]benzyl}oxy)phenyl]ethyl}amino)-5,6,7,8-tetrahydrochinolin-2-carboxylat-Dihydrochlorid (*Enantiomer 2*) | LC-MS (Methode 3): Rₜ = 1.01 min; m/z = 616 (M+H)⁺. |
| | 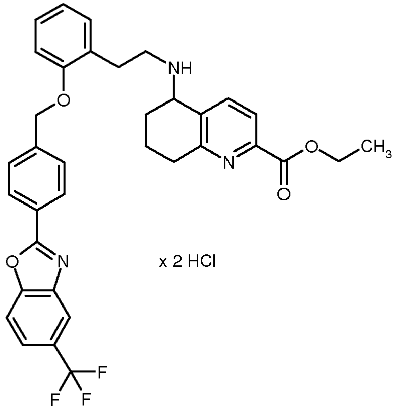 | |
| | aus Ethyl-5-[(*tert*.-butoxycarbonyl){2-[2-({4-[5-(trifluormethyl)-1,3-benzoxazol-2-yl]benzyl}-oxy)phenyl]ethyl}amino]-5,6,7,8-tetrahydrochinolin-2-carboxylat (*Enantiomer 2*) | |
| **82A** | Ethyl-5-{[2-(2-{[4-(1,3-benzoxazol-2-yl)benzyl]-oxy}phenyl)ethyl]amino}-s,6,7,S-tetrahydro-chinolin-2-carboxylat-Dihydrochlorid (*Enantiomer 2*) | |
| | 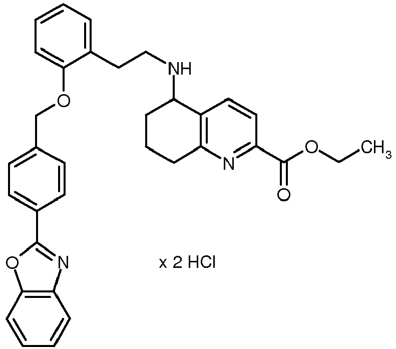 | |
| | aus Ethyl-5-{[2-(2-{[4-(1,3-benzoxazol-2-yl)-benzyl]oxy}phenyl)ethyl](*tert*.-butoxycarbonyl)-amino}-5,6,7,8-tetrahydrochinolin-2-carboxylat (*Enantiomer 2*) | |
| **83A** | Ethyl-5-({2-[2-({4-[5-(trifluormethoxy)-1,3-benzoxazol-2-yl]benzyl}oxy)phenyl]ethyl}amino)-5,6,7,8-tetrahydrochinolin-2-carboxylat-Dihydrochlorid (*Enantiomer 2*) | LC-MS (Methode 3): Rₜ = 1.01 min; m/z = 632 (M+H)⁺. |
| | 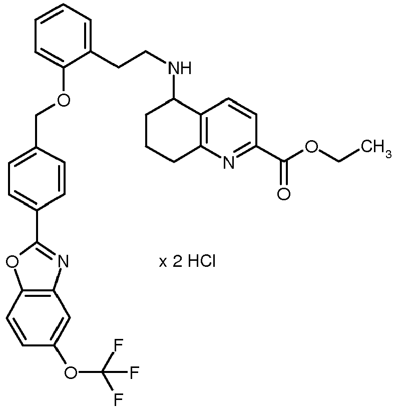 | |
| | aus Ethyl-5-[(*tert*.-butoxycarbonyl){2-[2-({4-[5-(trifluormethoxy)-1,3-benzoxazol-2-yl]benzyl}-oxy)phenyl]ethyl}amino]-5,6,7,8-tetrahydro-chinolin-2-carboxylat (*Enantiomer 2*) | |
| **84A** | Ethyl-5-{[2-(2-{[4-(5-cyano-1,3-benzoxazol-2-yl)-benzyl]oxy}phenyl)ethyl]amino}-5,6,7,8-tetra-hydrochinolin-2-carboxylat-Dihydrochlorid (*Enantiomer 2*) | LC-MS (Methode 3): Rₜ = 0.92 min; m/z = 573 (M+H)⁺. |
| | 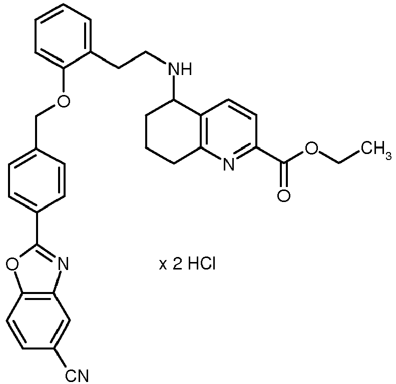 | |
| | aus Ethyl-5-{(*tert*.-butoxycarbonyl)[2-(2-{[4-(5-cyano-1,3-benzoxazol-2-yl)benzyl]oxy}-phenyl)ethyl]amino}-5,6,7,8-tetrahydrochinolin-2-carboxylat (*Enantiomer 2*) | |
| **85A** | Ethyl-5-{[2-(2-{[4-(2-phenylethyl)benzyl]oxy}-phenyl)ethyl]amino}-5,6,7,8-tetrahydrochinolin-2-carboxylat-Dihydrochlorid (*Enantiomer 2*) | LC-MS (Methode 3): Rₜ = 1.03 min; m/z = 535 (M+H)⁺. |
| | 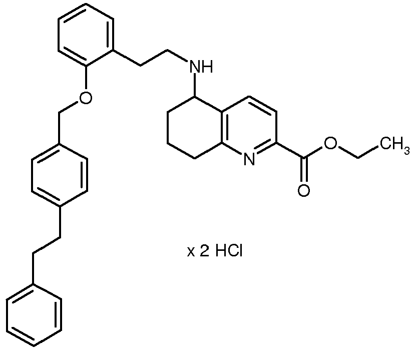 | |
| | aus Ethyl-5-{(*tert*.-butoxycarbonyl)[2-(2-{[4-(2-phenylethyl)benzyl]oxy}phenyl)ethyl]amino}-5,6,7,8-tetrahydrochinolin-2-carboxylat (*Enantiomer 2*) | |

### Beispiel 86A

### Ethyl-5-{[2-(2-{[3-chlor-4'-(trifluormethyl)biphenyl-4-yl]methoxy}phenyl)ethyl]amino}-5,6,7,8-tetrahydrochinolin-2-carboxylat (Enantiomer 2)

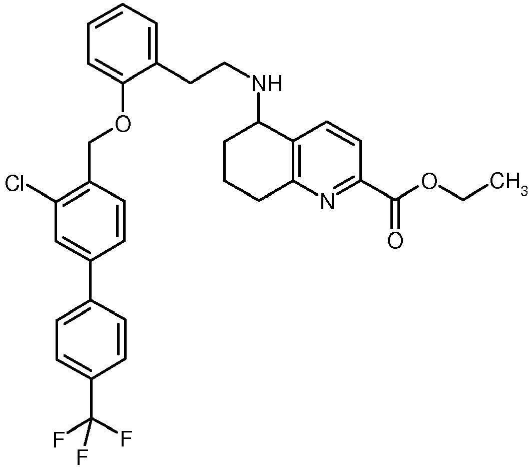

78 g (111.39 mmol) Ethyl-5-{[2-(2-{[3-chlor-4'-(trifluormethyl)biphenyl-4-yl]methoxy}phenyl)-ethyl]amino}-5,6,7,8-tetrahydrochinolin-2-carboxylat-Dihydrochlorid (Enantiomer 2, Beispiel 80A) wurden in 1200 ml THF aufgenommen, mit 47 ml Triethylamin versetzt und 1 h bei Raumtemperatur gerührt. Anschließend wurden die ausgefallenen Triethylammoniumchlorid-Kristalle abfiltriert und mit THF nachgewaschen. Das erhaltene Filtrat wurde bis zur Trockene eingedampft. Der Rückstand wurde in Essigsäureethylester gelöst, zweimal mit 10%-iger wässriger Natriumchlorid-Lösung gewaschen, über Magnesiumsulfat getrocknet, filtriert und wieder bis zur Trockene eingedampft. Es wurden 69 g (111.24 mmol, 99.9% d. Th.) der Zielverbindung erhalten.

LC-MS (Methode 3): Rₜ = 1.07 min; m/z = 609/611 (M+H)⁺.

¹H-NMR (400 MHz, DMSO-*d₆*, δ/ppm): 1.27 (t, 3H), 1.59-1.71 (m, 2H), 1.76-1.87 (m, 1H), 1.87-1.95 (m, 1H), 1.96-2.06 (m, 1H), 2.66-2.89 (m, 6H), 3.75 (br. s, 1H), 4.27 (q, 2H), 5.19 (s, 2H), 6.91 (t, 1H), 7.07 (d, 1H), 7.16-7.27 (m, 2H), 7.65-7.77 (m, 3H), 7.83 (d, 3H), 7.88 (s, 1H), 7.94 (d, 2H).

Analog zu Beispiel 86A wurden die folgenden Verbindungen hergestellt:

| **Beispiel** | **Name / Struktur / Edukt** | **Analytische Daten** |
|---|---|---|
| **87A** | Ethyl-5-({2-[2-({4-[5-(trifluormethyl)-1,3-benzoxazol-2-yl]benzyl}oxy)phenyl]ethyl}amino)-5,6,7,8-tetrahydrochinolin-2-carboxylat (*Enantiomer 2*) | LC-MS (Methode 3): Rₜ = 1.06 min; m/z = 616 (M+H)⁺. |
| | 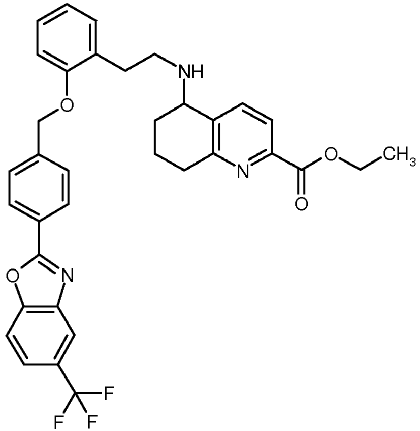 | ¹H-NMR (400 MHz, DMSO-*d₆,* δ/ppm): 1.27 (t, 3H), 1.61-1.79 (m, 2H), 1.81-2.03 (m, 3H), 2.72-2.92 (m, 6H), 3.73-3.86 (m, 1H), 4.27 (q, 2H), 5.24 (s, 2H), 6.90 (t, 1H), 7.05 (d, 1H), 7.15-7.27 (m, 2H), 7.69 (d, 1H), 7.73 (d, 1H), 7.82 (d, 1H), 7.87 (d, 1H), 8.04 (d, 1H), 8.18-8.30 (m, 3H). |
| | aus Ethyl-5-({2-[2-({4-[5-(trifluormethyl)-1,3-benzoxazol-2-yl]benzyl}oxy)phenyl]ethyl}amino)-5,6,7,8-tetrahydrochinolin-2-carboxylat-Dihydrochlorid (Enantiomer 2, Beispiel 81A) | |
| **88A** | Ethyl-5-{[2-(2-{[4-(1,3-benzoxazol-2-yl)benzyl]-oxy}phenyl)ethyl]amino}-5,6,7,8-tetrahydro-chinolin-2-carboxylat (*Enantiomer 2*) | |
| | 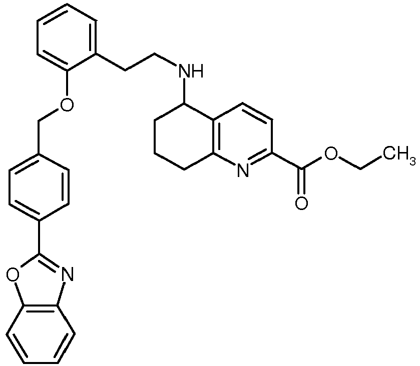 | |
| | aus Ethyl-5-{[2-(2-{[4-(1,3-benzoxazol-2-yl)-benzyl]oxy}phenyl)ethyl]ammo}-5,6,7,8-tetrahydrochinolin-2-carboxylat-Dihydrochlorid (Enantiomer 2, Beispiel 82A) | |
| **89A** | Ethyl-5-({2-[2-({4-[5-(trifluormethoxy)-1,3-benzoxazol-2-yl]benzyl}oxy)phenyl]ethyl}amino)-5,6,7,8-tetrahydrochmolin-2-carboxylat (*Enantiomer 2*) | LC-MS (Methode 3): Rₜ = 1.09 min; m/z = 632 (M+H)⁺. |
| | 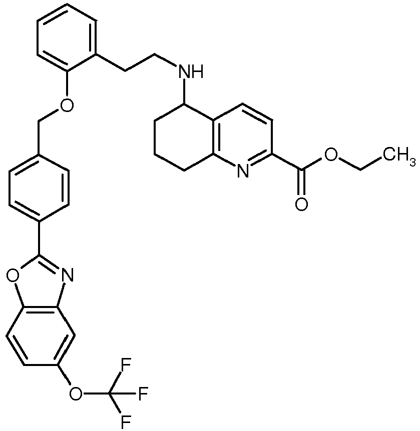 | |
| | aus Ethyl-5-({2-[2-({4-[5-(trifluormethoxy)-1,3-benzoxazol-2-yl]benzyl}oxy)phenyl]ethyl}amino)-5,6,7,8-tetrahydrochinolin-2-carboxylat-Dihydrochlorid (Enantiomer 2, Beispiel 83A) | |
| **90A** | Ethyl-5-{[2-(2-{[4-(5-cyano-1,3-benzoxazol-2-yl)-benzyl]oxy}phenyl)ethyl]amino}-5,6,7,8-tetrahydrochinolin-2-carboxylat (*Enantiomer 2*) | LC-MS (Methode 3): Rₜ = 0.93 min; m/z = 573 (M+H)⁺. |
| | 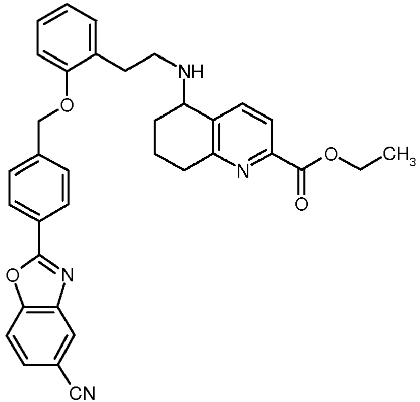 | ¹H-NMR (400 MHz, DMSO-*d₆,* δ/ppm): 1.27 (t, 3H), 1.62-1.76 (m, 2H), 1.81-2.02 (m, 2H), 2.02-2.13 (m, 1H), 2.69-2.91 (m, 6H), 3.78 (br. s, 1H), 4.27 (q, 2H), 5.24 (s, 2H), 6.90 (t, 1H), 7.05 (d, 1H), 7.14-7.26 (m, 2H), 7.65-7.77 (m, 3H), 7.83-7.95 (m, 2H), 8.03 (d, 1H), 8.22 (d, 2H), 8.43 (d, 1H). |
| | aus Ethyl-5-{[2-(2-{[4-(5-cyano-1,3-benzoxazol-2-yl)benzyl]oxy}phenyl)ethyl]ammo}-5,6,7,8-tetrahydrochinolin-2-carboxylat-Dihydrochlorid (Enantiomer 2, Beispiel 84A) | |
| **91A** | Ethyl-5-{[2-(2-{[4-(2-phenylethyl)benzyl]oxy}-phenyl)ethyl]amino}-5,6,7,8-tetrahydrochinolin-2-carboxylat (*Enantiomer 2*) | LC-MS (Methode 3): Rₜ = 1.02 min; m/z = 535 (M+H)⁺. |
| | 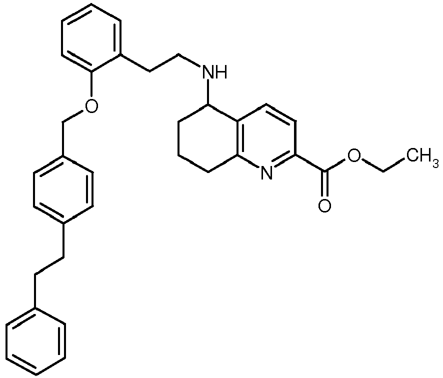 | |
| | aus Ethyl-5-{[2-(2-{[4-(2-phenylethyl)benzyl]oxy}-phenyl)ethyl]amino}-5,6,7,8-tetrahydrochinolin-2-carboxylat-Dihydrochlorid (Enantiomer 2, Beispiel 85A) | |

### Beispiel 92A

### Ethyl-5-([2-(2-{[3-chlor-4'-(trifluormethyl)biphenyl-4-yl]methoxy}phenyl)ethyl]{2-[4-(methoxy-carbonyl)phenyl]ethyl}amino)-5,6,7,8-tetrahydrochinolin-2-carboxylat (Enantiomer 2)

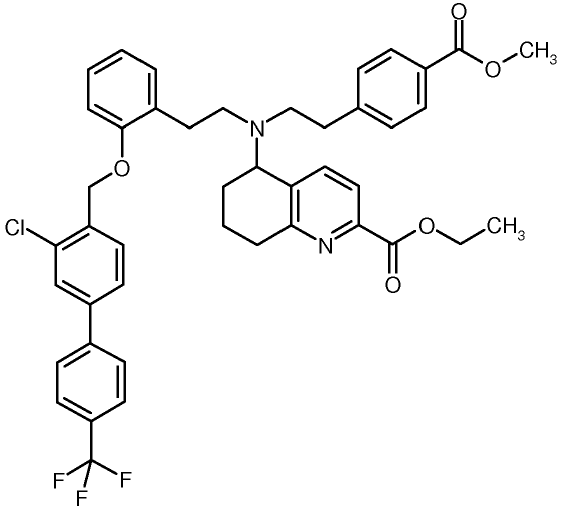

Eine Suspension aus 69 g (111.24 mmol) Ethyl-5-{[2-(2-{[3-chlor-4'-(trifluormethyl)biphenyl-4-yl]methoxy}phenyl)ethyl]amino}-5,6,7,8-tetrahydrochinolin-2-carboxylat (Enantiomer 2, Beispiel 86A), 129 g (444.98 mmol) Methyl-4-(2-iodethyl)benzoat und 17.68 g (166.87 mmol) wasserfreiem Natriumcarbonat in 1500 ml trockenem Acetonitril wurde bei einer Badtemperatur von 110°C über Nacht gerührt. Anschließend wurden weitere 65.54 g Methyl-4-(2-iodethyl)benzoat sowie 23.06 g (166.87 mmol) pulverisiertes Kaliumcarbonat zugesetzt und das Gemisch nochmals 48 h unter Rückfluss erhitzt. Nach dem Abkühlen des Reaktionsgemisches wurden die anorganischen Salze abfiltriert und das erhaltene Filtrat bis zur Trockene eingedampft. Der resultierende Rückstand wurde in Essigsäureethylester aufgenommen, zweimal mit 10%-iger wässriger Natriumchlorid-Lösung gewaschen, über Magnesiumsulfat getrocknet, filtriert und anschließend wieder bis zur Trockene eingedampft. Der erhaltene Rückstand wurde chromatographisch an Kieselgel (3 kg) gereinigt (Laufmittel: Petrolether/Essigsäureethylester 8:2 → 7:3). Es wurden 42 g (54.45 mmol, 49% d. Th.) der Zielverbindung erhalten.

LC-MS (Methode 3): Rₜ = 1.69 min; m/z = 771/773 (M+H)⁺.

¹H-NMR (400 MHz, DMSO-*d₆*, δ/ppm): 1.27 (t, 3H), 1.37-1.52 (m, 1H), 1.52-1.67 (m, 1H), 1.85-1.95 (m, 1H), 1.96-2.05 (m, 1H), 2.56-2.79 (m, 10H), 3.80 (s, 3H), 3.97-4.09 (m, 1H), 4.26 (q, 2H), 5.07 (m, 2H), 6.88 (t, 1H), 7.01-7.16 (m, 4H), 7.24 (t, 1H), 7.36-7.48 (m, 2H), 7.53 (d, 1H), 7.61 (d, 1H), 7.74 (d, 2H), 7.77-7.88 (m, 5H).

Analog zu Beispiel 92A wurden die folgenden Verbindungen hergestellt:

| **Beispiel** | **Name / Struktur / Edukte** | **Analytische Daten** |
|---|---|---|
| **93A** | Ethyl-5-({2-[4-(methoxycarbonyl)phenyl]ethyl}-{2-[2-({4-[5-(trifluormethyl)-1,3-benzoxazol-2-yl]-benzyl}oxy)phenyl]ethyl}amino)-5,6,7,8-tetrahydrochinolin-2-carboxylat (*Enantiomer 2*) | LC-MS (Methode 3): Rₜ = 1.63 min; m/z = 778 (M+H)⁺. |
| | 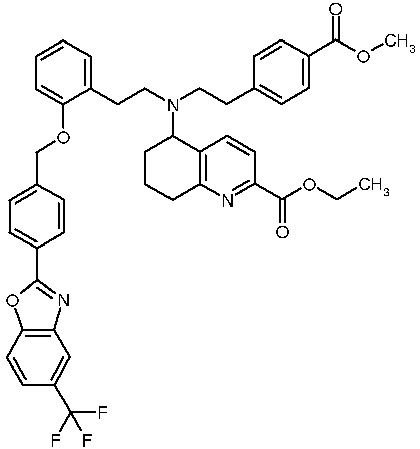 | ¹H-NMR (400 MHz, DMSO-*d₆,* δ/ppm): 1.26 (t, 3H), 1.43-1.55 (m, 1H), 1.56-1.69 (m, 1H), 1.89-2.09 (m, 2H), 2.58-2.86 (m, 10H), 3.74 (s, 3H), 4.03-4.12 (m, 1H), 4.26 (q, 2H), 5.02-5.20 (m, 2H), 6.86 (t, 1H), 7.00-7.14 (m, 4H), 7.16-7.26 (m, 1H), 7.44 (d, 1H), 7.49-7.60 (m, 3H), 7.72 (d, 2H), 7.82 (d, 1H), 8.01 (d, 1H), 8.13 (d, 2H), 8.23 (s, 1H). |
| | aus Ethyl-5-({2-[2-({4-[5-(trifluormethyl)-1,3-benzoxazol-2-yl]benzyl}oxy)phenyl]ethyl}-amino)-5,6,7,8-tetrahydrochinolin-2-carboxylat (Enantiomer 2, Beispiel 87A) und Methyl-4-(2-iodethyl)benzoat | |
| **94A** | Ethyl-5-([2-(2-{[4-(1,3-benzoxazol-2-yl)benzyl]-oxy}phenyl)ethyl]{2-[4-(methoxycarbonyl)-phenyl]ethyl}amino)-5,6,7,8-tetrahydrochinolin-2-carboxylat (*Enantiomer 2*) | LC-MS (Methode 3): Rₜ = 1.55 min; m/z = 710 (M+H)⁺. |
| | 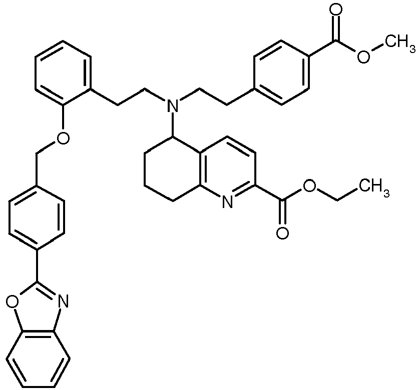 | ¹H-NMR (400 MHz, DMSO-*d₆,* δ/ppm): 1.26 (t, 3H), 1.41-1.55 (m, 1H), 1.55-1.71 (m, 1H), 1.89-2.08 (m, 2H), 2.58-2.84 (m, 10H), 3.76 (s, 3H), 4.03-4.13 (m, 1H), 4.26 (q, 2H), 5.03-5.16 (m, 2H), 6.86 (t, 1H), 6.99-7.09 (m, 2H), 7.13 (d, 2H), 7.21 (t, 1H), 7.38-7.46 (m, 3H), 7.49-7.57 (m, 3H), 7.73 (d, 2H), 7.77-7.85 (m, 2H), 8.11 (d, 2H). |
| | aus Ethyl-5-{[2-(2-{[4-(1,3-benzoxazol-2-yl)-benzyl]oxy}phenyl)ethyl]amino}5,6,7,8-tetrahydrochinolin-2-carboxylat (Enantiomer 2, Beispiel 88A) und Methyl-4-(2-iodethyl)benzoat | |
| **95A** | Ethyl-5-({2-[4-(methoxycarbonyl)phenyl]ethyl}-{2-[2-({4-[5-(trifluormethoxy)-1,3-benzoxazol-2-yl]benzyl}oxy)phenyl]ethyl}amino)-5,6,7,8-tetrahydrochinolin-2-carboxylat (*Enantiomer 2*) | LC-MS (Methode 3): Rₜ = 1.66 min; m/z = 794 (M+H)⁺. |
| | 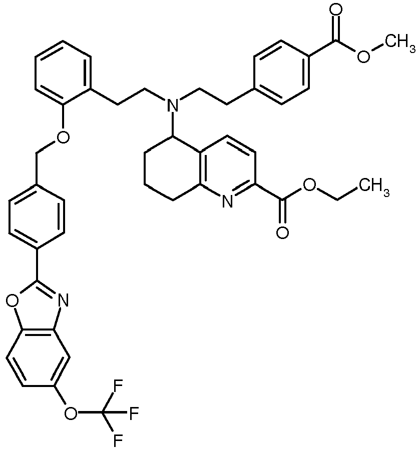 | ¹H-NMR (400 MHz, DMSO-*d₆,* δ/ppm): 1.26 (t, 3H), 1.42-1.55 (m, 1H), 1.55-1.70 (m, 1H), 1.89-2.08 (m, 2H), 2.57-2.85 (m, 10H), 3.75 (s, 3H), 4.02-4.12 (m, 1H), 4.25 (q, 2H), 5.05-5.15 (m, 2H), 6.86 (t, 1H), 7.03 (d, 1H), 7.06-7.15 (m, 3H), 7.21 (t, 1H), 7.40-7.50 (m, 2H), 7.50-7.58 (m, 3H), 7.73 (d, 2H), 7.87-7.95 (m, 2H), 8.10 (d, 2H). |
| | aus Ethyl-5-({2-[2-({4-[5-(trifluormethoxy)-1,3-benzoxazol-2-yl]benzyl}oxy)phenyl]ethyl}-amino)-5,6,7,8-tetrahydrochinolin-2-carboxylat (Enantiomer 2, Beispiel 89A) und Methyl-4-(2-iodethyl)benzoat | |
| **96A** | Ethyl-5-([2-(2-{[4-(5-cyano-1,3-benzoxazol-2-yl)-benzyl]oxy}phenyl)ethyl]{2-[4-(methoxycarbonyl)-phenyl]ethyl}amino)-5,6,7,8-tetrahydrochinolin-2-carboxylat (*Enantiomer 2*) | LC-MS (Methode 3): Rₜ = 1.50 min; m/z = 735 (M+H)⁺. |
| | 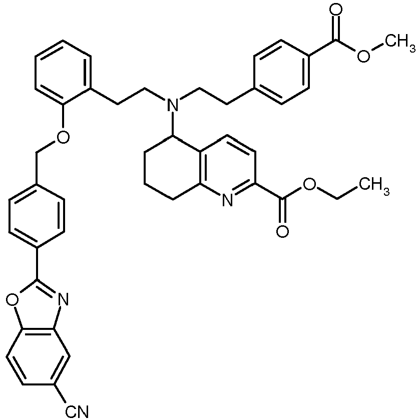 | ¹H-NMR (400 MHz, DMSO-*d₆,* δ/ppm): 1.26 (t, 3H), 1.41-1.55 (m, 1H), 1.55-1.71 (m, 1H), 1.89-2.10 (m, 2H), 2.58-2.86 (m, 10H), 3.75 (s, 3H), 4.00-4.13 (m, 1H), 4.25 (q, 2H), 5.03-5.17 (m, 2H), 6.87 (t, 1H), 6.99-7.14 (m, 4H), 7.21 (t, 1H), 7.44 (d, 1H), 7.49-7.60 (m, 3H), 7.71 (d, 2H), 7.92 (d, 1H), 8.00 (d, 1H), 8.12 (d, 2H), 8.42 (s, 1H). |
| | aus Ethyl-5-{[2-(2-{[4-(5-cyano-1,3-benzoxazol-2-yl)benzyl]oxy}phenyl)ethyl]ammo}-5,6,7,8-tetrahydrochinolin-2-carboxylat (Enantiomer 2, Beispiel 90A) und Methyl-4-(2-iodethyl)benzoat | |
| **97A** | Ethyl-5-({2-[4-(methoxycarbonyl)phenyl]ethyl}-[2-(2-{[4-(2-phenylethyl)benzyl]oxy}phenyl)ethyl]-amino)-5,6,7,8-tetrahydrochinolin-2-carboxylat (*Enantiomer 2*) | LC-MS (Methode 3): Rₜ = 1.58 min; m/z = 697 (M+H)⁺. |
| | 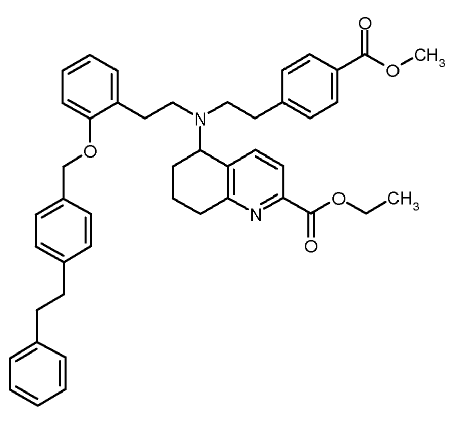 | ¹H-NMR (400 MHz, DMSO-*d₆,* δ/ppm): 1.29 (t, 3H), 1.40-1.53 (m, 1H), 1.53-1.68 (m, 1H), 1.88-2.06 (m, 2H), 2.57-2.87 (m, 14H), 3.83 (s, 3H), 4.01-4.10 (m, 1H), 4.29 (q, 2H), 4.91 (q, 2H), 6.83 (t, 1H), 6.95-7.07 (m, 2H), 7.10-7.22 (m, 10H), 7.23-7.29 (m, 2H), 7.40 (d, 1H), 7.46 (d, 1H), 7.80 (d, 2H). |
| | aus Ethyl-5-{[2-(2-{[4-(2-phenylethyl)benzyl]oxy}-phenyl)ethyl]amino}-5,6,7,8-tetrahydrochinolin-2-carboxylat (Enantiomer 2, Beispiel 91A) und Methyl-4-(2-iodethyl)benzoat | |

Analog zu den Beispielen 35A und 36A wurden die folgenden Verbindungen hergestellt:

| **Beispiel** | **Name / Struktur / Edukte** | **Analytische Daten** |
|---|---|---|
| **98A** | Ethyl-5-[(5-ethoxy-5-oxopentyl){2-[2-({4-[5-(trifluormethyl)-1,3-benzoxazol-2-yl]benzyl}oxy)-phenyl]ethyl}amino]-5,6,7,8-tetrahydrochinolin-2-carboxylat (*Enantiomer 2*) | LC-MS (Methode 3): Rₜ = 1.39 min; m/z = 744 (M+H)⁺. |
| | 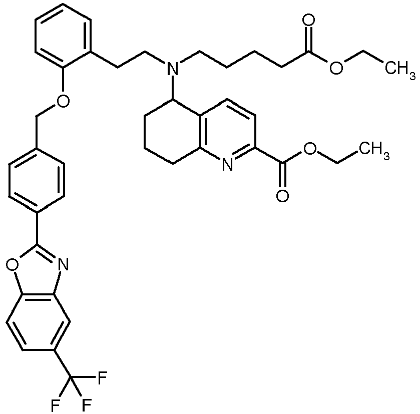 | ¹H-NMR (400 MHz, DMSO-*d₆,* δ/ppm): 1.09 (t, 3H), 1.25 (t, 4H), 1.30-1.56 (m, 5H), 1.56-1.70 (m, 1H), 1.90-2.05 (m, 2H), 2.08-2.20 (m, 2H), 2.35-2.64 (m, 3H, teilweise verdeckt durch DMSO-Signal), 2.65-2.89 (m, 4H), 3.90-4.03 (m, 3H), 4.26 (q, 2H), 5.05-5.18 (m, 2H), 6.87 (t, 1H), 7.00 (d, 1H), 7.10-7.22 (m, 2H), 7.57 (d, 2H), 7.66 (d, 1H), 7.79-7.91 (m, 2H), 8.04 (d, 1H), 8.20 (d, 2H), 8.26 (s, 1H). |
| | aus Ethyl-5-({2-[2-({4-[5-(trifluormethyl)-1,3-benzoxazol-2-yl]benzyl}oxy)phenyl]ethyl}amino)-5,6,7,8-tetrahydrochinolin-2-carboxylat (Enantiomer 2, Beispiel 87A) und Ethyl-5-brompentanoat | |
| **99A** | Ethyl-5-{[2-(2-{[4-(1,3-benzoxazol-2-yl)benzyl]-oxy}phenyl)ethyl](5-ethoxy-5-oxopentyl)amino}-5,6,7,8-tetrahydrochinolin-2-carboxylat (*Enantiomer 2*) | LC-MS (Methode 3): Rₜ = 1.27 min; m/z = 676 (M+H)⁺. |
| | 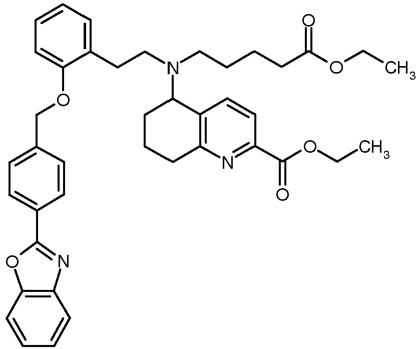 | ¹H-NMR (400 MHz, DMSO-*d₆,* δ/ppm): 1.10 (t, 3H), 1.26 (t, 4H), 1.31-1.57 (m, 5H), 1.58-1.71 (m, 1H), 1.90-2.05 (m, 2H), 2.10-2.20 (m, 2H), 2.35-2.64 (m, 3H, teilweise verdeckt durch DMSO-Signal), 2.64-2.88 (m, 4H), 3.92-4.02 (m, 3H), 4.26 (q, 2H), 5.02-5.18 (m, 2H), 6.86 (t, 1H), 7.00 (d, 1H), 7.10-7.23 (m, 2H), 7.39-7.49 (m, 2H), 7.53 (d, 2H), 7.66 (d, 1H), 7.78-7.92 (m, 3H), 8.18 (d, 2H). |
| | aus Ethyl-5-{[2-(2-{[4-(1,3-benzoxazol-2-yl)-benzyl]oxy}phenyl)ethyl]amino}-5,6,7,8-tetrahydrochinolin-2-carboxylat (Enantiomer 2, Beispiel 88A) und Ethyl-5-brompentanoat | |
| **100A** | Ethyl-5-[(5-ethoxy-5-oxopentyl){2-[2-({4-[5-(trifluormethoxy)-1,3-benzoxazol-2-yl]benzyl}-oxy)phenyl]ethyl}amino]-5,6,7,8-tetrahydro-chinolin-2-carboxylat (*Enantiomer 2*) | LC-MS (Methode 3): Rₜ = 1.43 min; m/z = 760 (M+H)⁺. |
| | 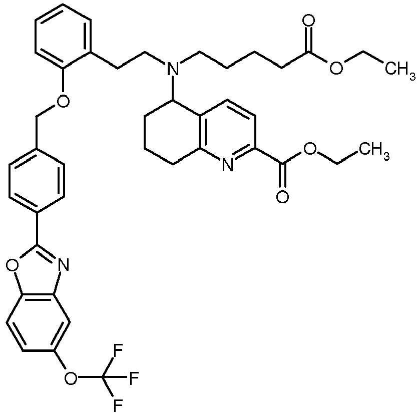 | ¹H-NMR (400 MHz, DMSO-*d₆,* δ/ppm): 1.09 (t, 3H), 1.25 (t, 3H), 1.30-1.56 (m, 6H), 1.56-1.71 (m, 1H), 1.89-2.05 (m, 2H), 2.09-2.20 (m, 2H), 2.35-2.64 (m, 3H, teilweise verdeckt durch DMSO-Signal), 2.65-2.88 (m, 4H), 3.90-4.03 (m, 3H), 4.27 (q, 2H), 5.04-5.18 (m, 2H), 6.88 (br. t, 1H), 7.00 (d, 1H), 7.09-7.23 (m, 2H), 7.47 (dd, 1H), 7.56 (d, 2H), 7.65 (d, 1H), 7.86 (d, 1H), 7.90-7.98 (m, 2H), 8.19 (d, 2H). |
| | aus Ethyl-5-({2-[2-({4-[5-(trifluormethoxy)-1,3-benzoxazol-2-yl]benzyl}oxy)phenyl]ethyl}amino)-5,6,7,8-tetrahydrochinolin-2-carboxylat (Enantiomer 2, Beispiel 89A) und Ethyl-5-brompentanoat | |
| **101A** | Ethyl-5-{[2-(2-{[4-(5-cyano-1,3-benzoxazol-2-yl)-benzyl]oxy}phenyl)ethyl](5-ethoxy-5-oxopentyl)-amino}-5,6,7,8-tetrahydrochinolin-2-carboxylat (*Enantiomer 2*) | LC-MS (Methode 3): Rₜ = 1.24 min; m/z = 701 (M+H)⁺. |
| | 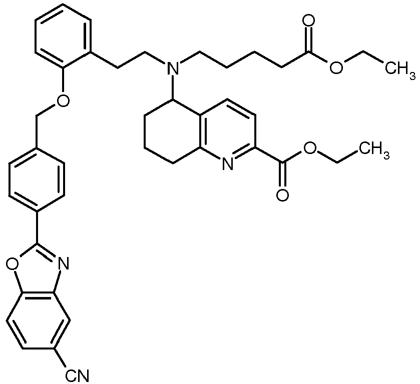 | ¹H-NMR (400 MHz, DMSO-*d₆,* δ/ppm): 1.10 (t, 3H), 1.26 (t, 3H), 1.30-1.72 (m, 7H), 1.90-2.05 (m, 2H), 2.09-2.20 (m, 2H), 2.35-2.64 (m, 3H, teilweise verdeckt durch DMSO-Signal), 2.65-2.88 (m, 4H), 3.91-4.02 (m, 3H), 4.26 (q, 2H), 5.11 (m, 2H), 6.87 (t, 1H), 6.99 (d, 1H), 7.10-7.24 (m, 2H), 7.56 (d, 2H), 7.65 (d, 1H), 7.87 (d, 1H), 7.93 (m, 1H), 8.04 (d, 1H), 8.19 (d, 2H), 8.44 (s, 1H). |
| | aus Ethyl-5-{[2-(2-{[4-(5-cyano-1,3-benzoxazol-2-yl)benzyl]oxy}phenyl)ethyl]ammo}-5,6,7,8-tetrahydrochinolin-2-carboxylat (Enantiomer 2, Beispiel 90A) und Ethyl-5-brompentanoat | |
| **102A** | Ethyl-5-{[2-(2-{[4-(5-chlor-1,3-benzoxazol-2-yl)-benzyl]oxy}phenyl)ethyl](5-ethoxy-5-oxopentyl)-amino}-5,6,7,8-tetrahydrochinolin-2-carboxylat (*Enantiomer 2*) | LC-MS (Methode 3): Rₜ = 1.39 min; m/z = 710/712 (M+H)⁺. |
| | 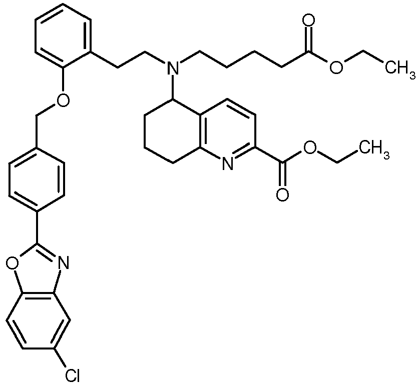 | |
| | aus Ethyl-5-{[2-(2-{[4-(5-chlor-1,3-benzoxazol-2-yl)benzyl]oxy}phenyl)ethyl]ammo}-5,6,7,8-tetrahydrochinolin-2-carboxylat (Enantiomer 2, Beispiel 62A) und Ethyl-5-brompentanoat | |

### Beispiel 103A

### rac-5-{[2-(5-Fluor-2-methoxyphenyl)ethyl]amino}-5,6,7,8-tetrahydrochinolin-2-carbonitril

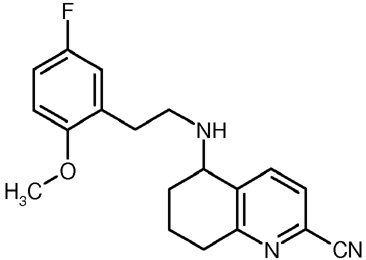

21.98 g (127.66 mmol) 5-Oxo-5,6,7,8-tetrahydrochinolin-2-carbonitril, 21.6 g (127.66 mmol) 2-(5-Fluor-2-methoxyphenyl)ethanamin [CAS Reg.-Nr. 1000533-03-8] und 3.64 g (19.15 mmol) *p*-Toluolsulfonsäure-Monohydrat wurden in 511 ml Toluol gelöst und die Lösung über Nacht unter Rückfluss unter Einsatz eines Wasserabscheiders gerührt. Anschließend wurden 200 ml Toluol abdestilliert und nach dem Abkühlen durch frisches Toluol ersetzt. Danach wurde die Reaktionslösung bis zur Trockene eingedampft und der resultierende Rückstand in 511 ml wasserfreiem Ethanol und 511 ml wasserfreiem THF aufgenommen. Die Lösung wurde unter Rühren bei einer Temperatur von 15° bis 20°C portionsweise mit 9.66 g (255.32 mmol) Natriumborhydrid versetzt (Vorsicht: Reaktionsgemisch schäumt). Danach wurde die Reaktionslösung bei der gleichen Temperatur über Nacht nachgerührt. Das Reaktionsgemisch wurde dann vorsichtig mit 10%-iger wässriger Natriumchlorid-Lösung versetzt und zweimal mit Essigsäureethylester extrahiert. Die vereinigten organischen Phasen wurden über Natriumsulfat getrocknet, filtriert und anschließend bis zur Trockene eingeengt. Der so erhaltene Rückstand wurde durch Säulenchromatographie an Kieselgel gereinigt (Laufmittel: Cyclohexan/Essigsäureethylester 2:1). Es wurden 19.5 g (59.93 mmol, 46% d. Th.) der Zielverbindung erhalten.

LC-MS (Methode 2): Rₜ = 1.55 min; m/z = 326 (M+H)⁺.

¹H-NMR (400 MHz, DMSO-*d₆*, δ/ppm): 1.63-1.81 (m, 2H), 1.84-2.04 (m, 2H), 2.12 (br. s, 1H), 2.63-2.93 (m, 6H), 3.74 (s, 3H), 3.80 (br. s, 1H), 6.87-7.08 (m, 3H), 7.78 (d, 1H), 7.94 (d, 1H).

### Beispiel 104A

### rac-5-{[2-(5-Fluor-2-hydroxyphenyl)ethyl]amino}-5,6,7,8-tetrahydrochinolin-2-carbonsäure

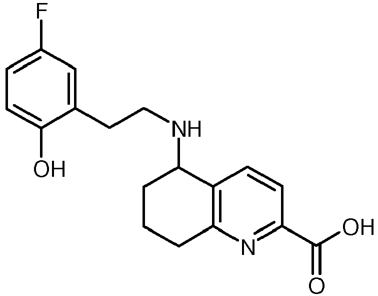

72.8 g (223.73 mmol) *rac*-5-{[2-(5-Fluor-2-methoxyphenyl)ethyl]amino}-5,6,7,8-tetrahydrochinolin-2-carbonitril wurden in 360 ml Bromwasserstoffsäure (48% in Wasser) aufgenommen und zunächst 12 h bei Siedetemperatur gerührt, anschließend auf Raumtemperatur abgekühlt und über Nacht bei dieser Temperatur stehengelassen. Danach wurde die Reaktionslösung mit 400 ml Wasser verdünnt und mit gesättigter Natriumhydrogencarbonat-Lösung auf pH 6 gestellt. Die entstandenen Kristalle wurden abgesaugt, mit Wasser gewaschen und im Vakuum bei 50°C getrocknet. Es wurden 59 g (178.59 mmol, 80% d. Th.) der Zielverbindung erhalten.

LC-MS (Methode 2): Rₜ = 1.30 min; m/z = 331 (M+H)⁺.

### Beispiel 105A

### rac-Ethyl-5-{[2-(5-fluor-2-hydroxyphenyl)ethyl]amino}-5,6,7,8-tetrahydrochinolin-2-carboxylat

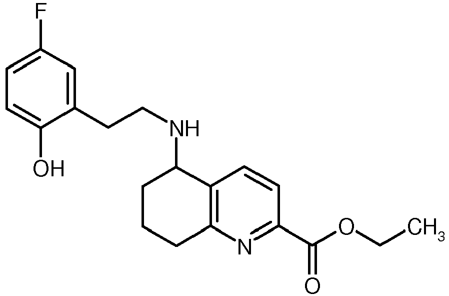

1.93 g (5.84 mmol) *rac*-5-{[2-(5-Fluor-2-hydroxyphenyl)ethyl]amino}-5,6,7,8-tetrahydrochinolin-2-carbonsäure wurden mit 40 ml wasserfreiem Ethanol und 4 ml einer 4 N Lösung von Chlorwasserstoff in Dioxan versetzt und über Nacht unter Rückfluss gerührt. Anschließend wurde die Reaktionslösung auf Raumtemperatur abgekühlt und zunächst mit Essigsäureethylester und dann langsam mit gesättigter wässriger Natriumhydrogencarbonat-Lösung versetzt. Die organische Phase wurde abgetrennt, über Magnesiumsulfat getrocknet, filtriert und bis zur Trockene eingeengt. Es wurden 1.67 g (4.66 mmol, 80% d. Th.) der Zielverbindung erhalten.

LC-MS (Methode 3): Rₜ = 0.60 min; m/z = 359 (M+H)⁺.

¹H-NMR (400 MHz, DMSO-*d₆*, δ/ppm): 1.32 (t, 3H), 1.69-1.82 (m, 2H), 1.83-1.93 (m, 1H), 1.94-2.08 (m, 1H), 2.64-2.77 (m, 4H), 2.79-2.91 (m, 2H), 3.81-3.90 (m, 1H), 4.33 (q, 2H), 6.68-6.75 (m, 1H), 6.77-6.85 (m, 1H), 6.87-6.94 (m, 1H), 7.83 (d, 1H), 7.91 (d, 1H), 10.56-10.73 (m, 1H).

### Beispiel 106A

### rac-Ethyl-5-{(tert.-butoxycarbonyl)[2-(5-fluor-2-hydroxyphenyl)ethyl]amino}-5,6,7,8-tetrahydrochinolin-2-carboxylat

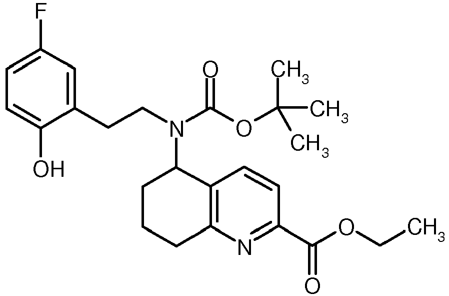

3.73 g (10.41 mmol) *rac*-Ethyl-5-{[2-(5-fluor-2-hydroxyphenyl)ethyl]amino}-5,6,7,8-tetrahydrochinolin-2-carboxylat wurden in 30 ml Dichlormethan gelöst und unter Rühren auf 0°C gekühlt. Anschließend wurde eine Lösung von 2.95 g (13.53 mmol) Di-*tert*.-butyldicarbonat in 10 ml Dichlormethan langsam zugetropft und das Reaktionsgemisch über Nacht bei Raumtemperatur gerührt. Danach wurde die Reaktionslösung bis zur Trockene eingeengt und der Rückstand mit Diethylether verrührt. Nach Filtration wurde der Filterkuchen mehrmals mit Diethylether gewaschen und abschließend an der Luft getrocknet. Es wurden 4.17 g (9.09 mmol, 87% d. Th.) der Zielverbindung erhalten.

LC-MS (Methode 3): Rₜ = 1.18 min; m/z = 459 (M+H)⁺.

¹H-NMR (400 MHz, CDCl₃, δ/ppm): 1.12 (br. s, 4H), 1.31 (t, 3H), 1.47 (s, 5H), 1.67-1.90 (m, 1H), 1.91-2.11 (m, 3H), 2.63-2.98 (m, 5H), 3.20-3.55 (m, 1H, teilweise verdeckt durch H₂O-Signal), 4.32 (q, 2H), 4.64-4.87 (m, 0.5H), 5.08-5.27 (m, 0.5H), 6.65-7.00 (m, 3H), 7.43-7.63 (m, 1H), 7.83 (d, 1H), 9.37 (s, 1H).

### Beispiel 107A

### rac-Ethyl-5-{(tert.-butoxycarbonyl)[2-(2-{[4-(5-chlor-1,3-benzoxazol-2-yl)benzyl]oxy}-5-fluorphenyl)ethyl]amino}-5,6,7,8-tetrahydrochinolin-2-carboxylat

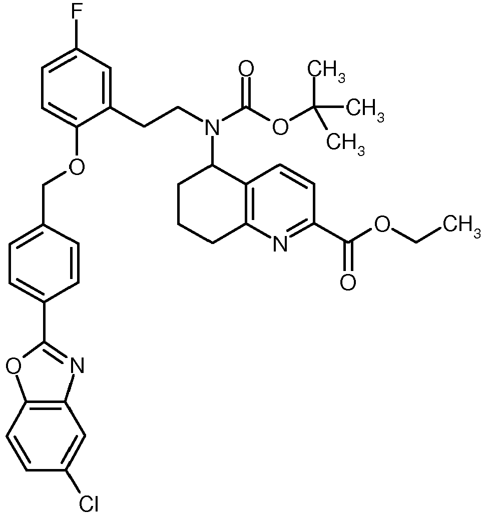

4.17 g (9.09 mmol) *rac*-Ethyl-5-{(*tert*.-butoxycarbonyl)[2-(5-fluor-2-hydroxyphenyl)ethyl]amino}-5,6,7,8-tetrahydrochinolin-2-carboxylat, 3.04 g (10.91 mmol) 5-Chlor-2-[4-(chlormethyl)phenyl]-1,3-benzoxazol und 3.14 g (22.74 mmol) Kaliumcarbonat in 120 ml Acetonitril wurden auf 110°C erhitzt und über Nacht bei dieser Temperatur gerührt. Nach dem Abkühlen wurde das Reaktionsgemisch filtriert, der Filterkuchen mehrmals mit Acetonitril nachgewaschen und die vereinigten Filtrate bis zur Trockene am Rotationsverdampfer eingeengt. Der erhaltene Rückstand wurde chromatographisch an Kieselgel gereinigt (Laufmittel: Cyclohexan/Essigsäureethylester 10:1 → 4:1). Es wurden 5.43 g (7.75 mmol, 85% d. Th.) der Zielverbindung erhalten.

LC-MS (Methode 3): Rₜ = 1.60 min; m/z = 700/702 (M+H)⁺.

### Beispiel 108A und Beispiel 109A

### Ethyl-5-{(tert.-butoxycarbonyl)[2-(2-{[4-(5-chlor-1,3-benzoxazol-2-yl)benzyl]oxy}-5-fluorphenyl)ethyl]amino}-5,6,7,8-tetrahydrochinolin-2-carboxylat (Enantiomer 1 und 2)

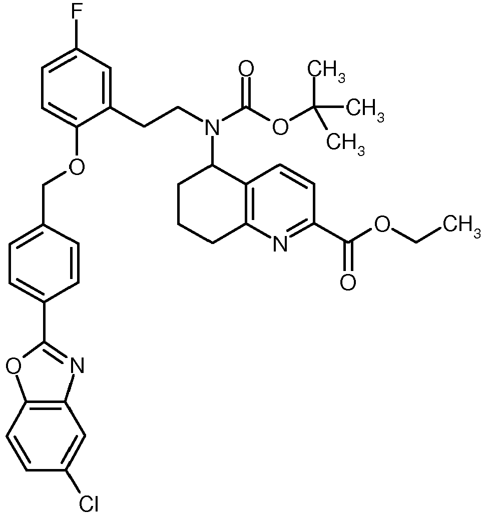

2.5 g (3.57 mmol) des racemischen Ethyl-5-{(ter-t.-butoxycarbonyl)[2-(2-{[4-(5-chlor-1,3-benzoxazol-2-yl)benzyl]oxy}-5-fluorphenyl)ethyl]amino}-5,6,7,8-tetrahydrochinolin-2-carboxylats (Beispiel 107A) wurden durch superkritische Flüssigchromatographie (SFC) an chiraler Phase in die Enantiomere aufgetrennt [Säule: Daicel Chiracel OD-H, 5 µm, 250 mm x 20 mm; Elutionsmittel: Kohlendioxid/Ethanol 75:25 (v/v); Fluss: 100 ml/min; Druck: 80 bar; UV-Detektion: 220 µm; Temperatur: 40°C]:

### Beispiel 108A (Enantiomer 1):

Ausbeute: 1020 mg
Rₜ = 3.497 min; chemische Reinheit >99.9%; >99% ee
[Säule: Chiralpak OD-H, 5 µm, 250 mm x 4.6 mm; Elutionsmittel: Kohlendioxid/Ethanol 70:30 (v/v); Fluss: 3 ml/min; UV-Detektion: 210 nm].
LC-MS (Methode 3): Rₜ = 1.60 min; m/z = 700/702 (M+H)⁺.

### Beispiel 109A (Enantiomer 2):

Ausbeute: 1040 mg
Rₜ = 4.97 min; chemische Reinheit >99%; >95% ee
[Säule: Chiralpak OD-H, 5 µm, 250 mm x 4.6 mm; Elutionsmittel: Kohlendioxid/Ethanol 70:30 (v/v); Fluss: 3 ml/nmin; UV-Detektion: 210 nm].
LC-MS (Methode 3): Rₜ = 1.60 min; m/z = 700/702 (M+H)⁺.

¹H-NMR (400 MHz, DMSO-*d₆*, δ/ppm): 1.09 (br. s, 4H), 1.27 (m, 3H), 1.43 (s, 5H), 1.50-1.62 (m, 0.5H), 1.63-1.75 (m, 0.5H), 1.76-1.97 (m, 3H), 2.59-2.80 (m, 2H), 2.81-3.04 (m, 3H), 3.20-3.40 (m, 0.5H, teilweise verdeckt durch H₂O-Signal), 3.42-3.57 (m, 0.5H), 4.27 (q, 2H), 4.39-4.60 (m, 0.5H), 5.00-5.11 (m, 0.5H), 5.11-5.26 (m, 2H), 6.90-6.98 (m, 0.5H), 6.99-7.17 (m, 2.5H), 7.44 (d, 0.5H), 7.51 (d, 1.5H), 7.59 (d, 1H), 7.69 (d, 1H), 7.76-7.89 (m, 2H), 7.93 (d, 1H), 8.06 (d, 1H), 8.16 (d, 1H).

Analog zu Beispiel 107A wurde die folgende Verbindung hergestellt:

| **Beispiel** | **Name / Struktur / Edukte** | **Analytische Daten** |
|---|---|---|
| **110A** | *rac*-Ethyl-5-{(*tert*.-butoxycarbonyl)[2-(2-{[3-chlor-4'-(trifluormethyl)biphenyl-4-yl]methoxy}-5-fluorphenyl)ethyl]amino}-5,6,7,8-tetrahydrochinolin-2-carboxylat | ¹11-NMR (400 MHz, DMSO-*d₆,* δ/ppm): 1.10 (br. s, 4H), 1.21-1.34 (m, 3H), 1.43 (s, 5H), 1.56-1.76 (m, 2H), 1.76-1.94 (m, 2H), 2.31-2.45 (m, 0.5H), 2.58-2.73 (m, 2H), 2.74-2.99 (m, 2.5H), 3.16-3.29 (m, 0.5H), 3.38-3.53 (m, 0.5H), 4.30 (q, 2H), 4.39-4.65 (m, 0.5H), 5.00-5.22 (m, 2.5H), 6.93 (d, 1H), 7.01-7.19 (m, 2H), 7.41 (d, 0.5H), 7.52 (d, 0.5H), 7.64 (t, 1H), 7.73 (br. s, 2H), 7.79-7.93 (m, 4H), 7.99-8.12 (m, 1H). |
| | 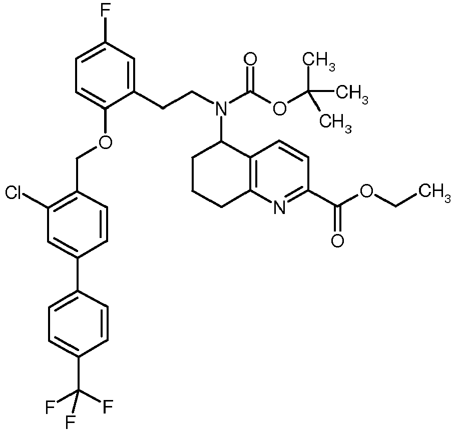 | |
| | aus *rac*-Ethyl-5-{(*tert*.-butoxycarbonyl)[2-(5-fluor-2-hydroxyphenyl)ethyl]amino1-5,6,7,8-tetrahydrochinolin-2-carboxylat und 4-(Brommethyl)-3-chlor-4'-(trifluormethyl)biphenyl | |

### Beispiel 111A und Beispiel 112A

### Ethyl-5-{(tert.-butoxycarbonyl)[2-(2- {[3-chlor-4'-(trifluormethyl)biphenyl-4-yl]methoxy}-5-fluorphenyl)ethyl]amino}-5,6,7,8-tetrahydrochinolin-2-carboxylat (Enantiomer 1 und 2)

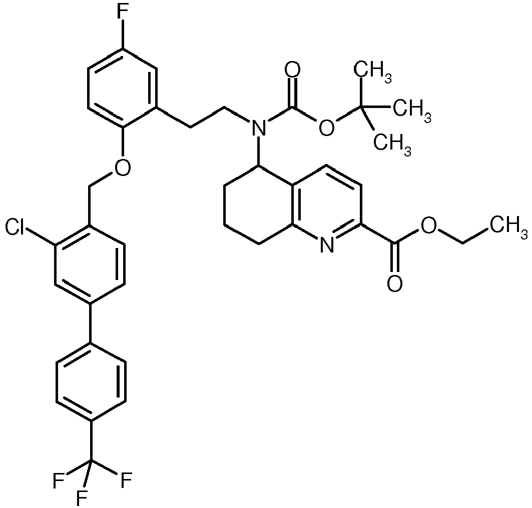

2.59 g (3.56 mmol) des racemischen Ethyl-5-{(*tert*.-butoxycarbonyl)[2-(2-{[3-chlor-4'-(trifluormethyl)biphenyl-4-yl]methoxy}-5-fluorphenyl)ethyl]amino}-5,6,7,8-tetrahydrochinolin-2-carboxylats (Beispiel 110A) wurden durch superkritische Flüssigchromatographie (SFC) an chiraler Phase in die Enantiomere aufgetrennt [Säule: Daicel Chiracel OD, 20 µm, 250 mm x 30 mm; Elutionsmittel: Kohlendioxid/Ethanol 80:20 (v/v); Fluss: 175 ml/min; Druck: 135 bar; UV-Detektion: 210 nm; Temperatur: 40°C]:

### Beispiel 111A (Enantiomer 1):

Ausbeute: 1130 mg
Rₜ = 2.24 min; chemische Reinheit >85%; >99% ee
[Säule: Chiralpak OD-H, 5 µm, 250 mm x 4.6 mm; Elutionsmittel: Kohlendioxid/Ethanol 70:30 (v/v); Fluss: 3 ml/min; UV-Detektion: 210 nm].
LC-MS (Methode 3): Rₜ = 1.65 min; m/z = 727/729 (M+H)⁺.

### Beispiel 112A (Enantiomer 2):

Ausbeute: 1170 mg
Rₜ = 3.33 min; chemische Reinheit >99%; >90% ee
[Säule: Chiralpak OD-H, 5 µm, 250 mm x 4.6 mm; Elutionsmittel: Kohlendioxid/Ethanol 70:30 (v/v); Fluss: 3 ml/min; UV-Detektion: 210 nm].
LC-MS (Methode 3): Rₜ = 1.65 min; m/z = 727/729 (M+H)⁺.

### Beispiel 113A

### Ethyl-5-{[2-(2-{[4-(5-chlor-1,3-benzoxazol-2-yl)benzyl]oxy}-5-fluorphenyl)ethyl]amino}-5,6,7,8-tetrahydrochinolin-2-carboxylat-Dihydrochlorid (Enantiomer 2)

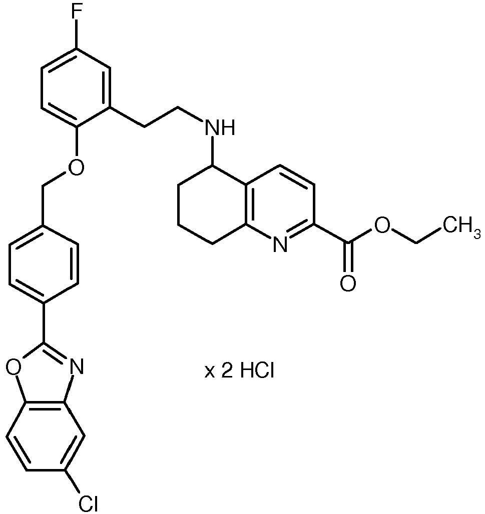

1025 mg (1.46 mmol) Ethyl-5-{(*tert*.-butoxycarbonyl)[2-(2-{[4-(5-chlor-1,3-benzoxazol-2-yl)-benzyl]oxy}-5-fluorphenyl)ethyl]amino}-5,6,7,8-tetrahydrochinolin-2-carboxylat (Enantiomer 2, Beispiel 109A) wurden mit 11 ml einer 4 N Lösung von Chlorwasserstoff in Dioxan versetzt und 2 h bei Raumtemperatur gerührt. Der ausgefallende Feststoff wurde abfiltriert, mehrmals mit Diethylether gewaschen und anschließend über Nacht im Hochvakuum bei 40°C getrocknet. Es wurden 980 mg (1.46 mmol, ca. 99% d. Th.) des Zielprodukts erhalten.

LC-MS (Methode 3): Rₜ = 1.01 min; m/z = 600/602 (M+H)⁺.

Analog zu Beispiel 113A wurden die folgenden Verbindungen hergestellt:

| **Beispiel** | **Name / Struktur / Edukt** | **Analytische Daten** |
|---|---|---|
| **114A** | Ethyl-5-{[2-(2-{[4-(5-chlor-1,3-benzoxazol-2-yl)-benzyl]oxy}-5-fluorphenyl)ethyl]amino}-5,6,7,8-tetrahydrochinolin-2-carboxylat-Dihydrochlorid (*Enantiomer 1*) | LC-MS (Methode 3): Rₜ = 1.01 min; m/z = 600/602 (M+H)⁺. |
| | 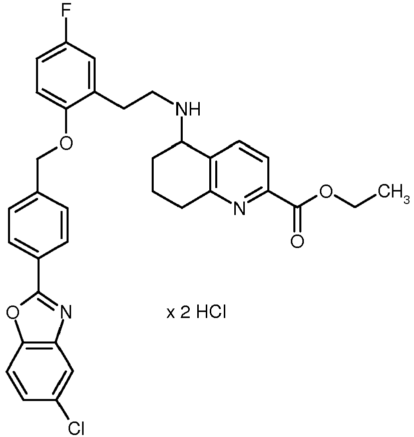 | |
| | aus Ethyl-5-{(*tert*.-butoxycarbonyl)[2-(2-{[4-(5-chlor-1,3-benzoxazol-2-yl)benzyl]oxy}-5-fluorphenyl)ethyl]amino}-5,6,7,8-tetrahydrochinolin-2-carboxylat (Enantiomer 1, Beispiel 108A) | |
| **115A** | Ethyl-5-{[2-(2-{[3-chlor-4'-(trifluormethyl)biphenyl-4-yl]methoxy}-5-fluorphenyl)ethyl]amino}-5,6,7,8-tetrahydrochinolin-2-carboxylat-Dihydrochlorid (*Enantiomer 1*) | |
| | 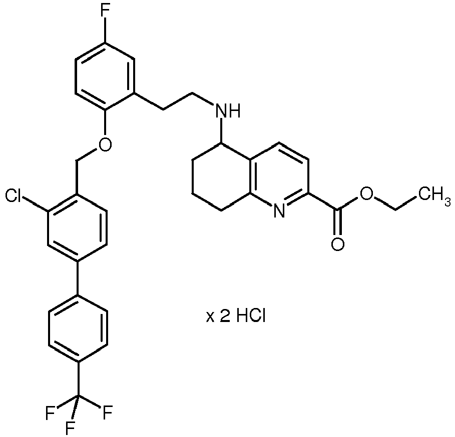 | |
| | aus Ethyl-5-{(*tert*.-butoxycarbonyl)[2-(2-{[3-chlor-4'-(trifluormethyl)biphenyl-4-yl]methoxy}-5-fluorphenyl)ethyl]amino}-5,6,7,8-tetrahydrochinolin-2-carboxylat (Enantiomer 1, Beispiel 111A) | |
| **116A** | Ethyl-5-{[2-(2-{[3-chlor-4'-(trifluormethyl)biphenyl-4-yl]methoxy}-5-fluorphenyl)ethyl]amino}-5,6,7,8-tetrahydrochinolin-2-carboxylat-Dihydrochlorid (*Enantiomer 2*) | |
| | 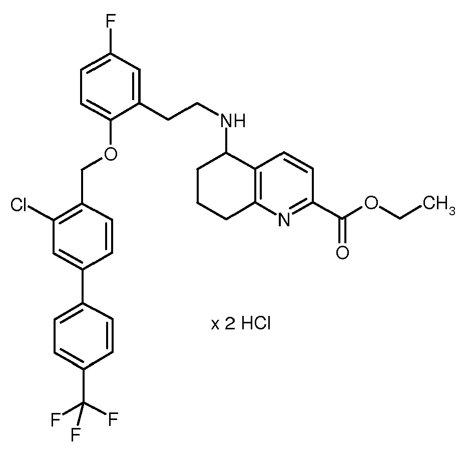 | |
| | aus Ethyl-5-{(*tert*.-butoxycarbonyl)[2-(2-{[3-chlor-4'-(trifluormethyl)biphenyl-4-yl]methoxy}-5-fluorphenyl)ethyl]amino}-5,6,7,8-tetrahydrochinolin-2-carboxylat (Enantiomer 2, Beispiel 112A) | |

### Beispiel 117A

### Ethyl-5-{[2-(2-{[4-(5-chlor-1,3-benzoxazol-2-yl)benzyl]oxy}-5-fluorphenyl)ethyl]amino}-5,6,7,8-tetrahydrochinolin-2-carboxylat (Enantiomer 2)

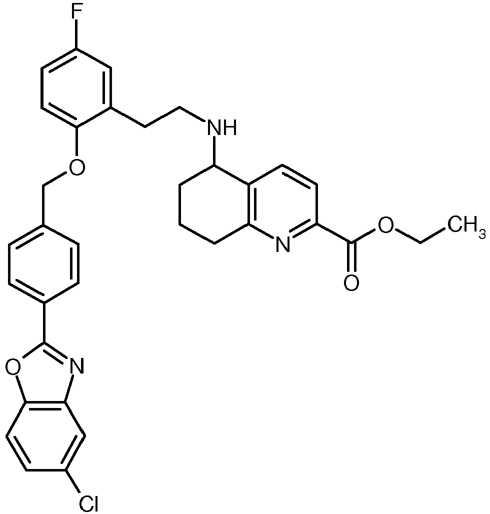

980 mg (1.46 mmol) Ethyl-5-{[2-(2-{[3-chlor-4'-(trifluormethyl)biphenyl-4-yl]methoxy}phenyl)-ethyl]amino}-5,6,7,8-tetrahydrochinolin-2-carboxylat-Dihydrochlorid (Enantiomer 2, Beispiel 113A) wurden in 20 ml THF aufgenommen, mit 0.81 ml Triethylamin versetzt und 1 h bei Raumtemperatur gerührt. Anschließend wurde die Reaktionslösung mit Essigsäureethylester und Wasser versetzt, die Phasen getrennt und die organische Phase noch einmal mit Essigsäureethylester extrahiert. Die vereinigten organischen Phasen wurden mit Wasser gewaschen, über Magnesiumsulfat getrocknet, filtriert und anschließend bis zur Trockene eingedampft. Es wurden 760 mg (1.27 mmol, 87% d. Th.) der Zielverbindung erhalten.

LC-MS (Methode 3): Rₜ = 1.03 min; m/z = 600/602 (M+H)⁺.

¹H-NMR (400 MHz, DMSO-*d₆*, δ/ppm): 1.28 (t, 3H), 1.62-1.78 (m, 2H), 1.80-2.01 (m, 2H), 2.03-2.17 (m, 1H), 2.70-2.92 (m, 6H), 3.65-3.89 (m, 1H), 4.28 (q, 2H), 5.21 (s, 2H), 6.94-7.15 (m, 3H), 7.48 (dd, 1H), 7.66 (d, 2H), 7.71-7.79 (m, 1H), 7.85 (d, 2H), 7.94 (d, 1H), 8.19 (d, 2H).

Analog zu Beispiel 117A wurde die folgende Verbindung hergestellt:

| **Beispiel** | **Name / Struktur / Edukt** | **Analytische Daten** |
|---|---|---|
| **118A** | Ethyl-5-{[2-(2-{[4-(5-chlor-1,3-benzoxazol-2-yl)-benzyl]oxy}-5-fluorphenyl)ethyl]amino}-5,6,7,8-tetrahydrochinolin-2-carboxylat (*Enantiomer 1*) | LC-MS (Methode 3): Rₜ = 1.05 min; m/z = 600/602 (M+H)⁺. |
| | 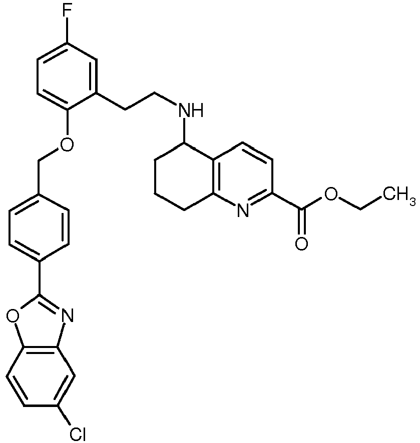 | ¹H-NMR (400 MHz, DMSO-*d₆,* δ/ppm): 1.28 (t, 3H), 1.62-1.77 (m, 2H), 1.81-2.02 (m, 2H), 2.04-2.17 (m, 1H), 2.72-2.91 (m, 6H), 3.71-3.87 (m, 1H), 4.28 (q, 2H), 5.21 (s, 2H), 6.95-7.15 (m, 3H), 7.49 (dd, 1H), 7.66 (d, 2H), 7.74 (d, 1H), 7.82-7.91 (m, 2H), 7.94 (d, 1H), 8.19 (d, 2H). |
| | aus Ethyl-5-{[2-(2-{[3-chlor-4'-(trifluormethyl)-biphenyl-4-yl]methoxy}phenyl)ethyl]amino}-5,6,7,8-tetrahydrochinolin-2-carboxylat-Dihydrochlorid (Enantiomer 1, Beispiel 114A) | |

Analog zu den Beispielen 35A und 36A wurden die folgenden Verbindungen hergestellt:

| **Beispiel** | **Name / Struktur / Edukte** | **Analytische Daten** |
|---|---|---|
| **119A** | Ethyl-5-{[2-(2-{[4-(5-chlor-1,3-benzoxazol-2-yl)-benzyl]oxy}-5-fluorphenyl)ethyl](5-ethoxy-5-oxo-pentyl)amino}-5,6,7,8-tetrahydrochinolin-2-carboxylat (*Enantiomer 1*) | LC-MS (Methode 3): Rₜ = 1.48 min; m/z = 728/730 (M+H)⁺. |
| | 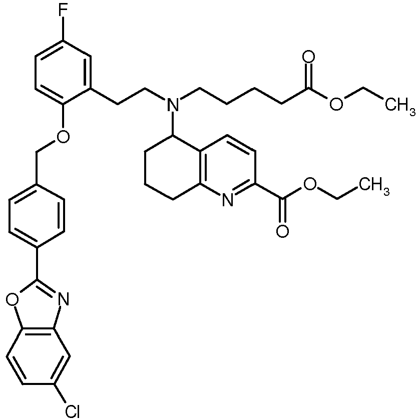 | ¹H-NMR (400 MHz, DMSO-*d₆,* δ/ppm): 1.10 (t, 3H), 1.26 (t, 3H), 1.30-1.70 (m, 6H), 1.90-2.05 (m, 2H), 2.08-2.18 (m, 2H), 2.35-2.45 (m, 2H), 2.57-2.64 (m, 2H), 2.72-2.83 (m, 4H), 3.91-4.02 (m, 3H), 4.26 (q, 2H), 5.02-5.15 (m, 2H), 6.96-7.08 (m, 3H), 7.46-7.57 (m, 3H), 7.65 (d, 1H), 7.78-7.88 (m, 2H), 7.95 (d, 1H), 8.17 (d, 2H). |
| | aus Ethyl-5-{[2-(2-{[4-(5-chlor-1,3-benzoxazol-2-yl)benzyl]oxy}-5-fluorphenyl)ethyl]amino}-5,6,7,8-tetrahydrochinolin-2-carboxylat (Enantiomer 1, Beispiel 118A) und Ethyl-5-brompentanoat | |
| **120A** | Ethyl-5-{[2-(2-{[4-(5-chlor-1,3-benzoxazol-2-yl)-benzyl]oxy}-5-fluorphenyl)ethyl](5-ethoxy-5-oxo-pentyl)amino}-5,6,7,8-tetrahydrochinolin-2-carboxylat (*Enantiomer 2*) | LC-MS (Methode 3): Rₜ = 1.49 min; m/z = 728/730 (M+H)⁺. |
| | 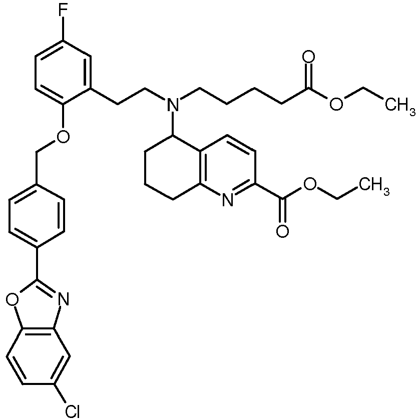 | ¹H-NMR (400 MHz, DMSO-*d₆,* δ/ppm): 1.10 (t, 3H), 1.26 (t, 3H), 1.30-1.69 (m, 6H), 1.90-2.03 (m, 2H), 2.10-2.17 (m, 2H), 2.35-2.46 (m, 2H), 2.56-2.64 (m, 2H), 2.72-2.83 (m, 4H), 3.91-4.01 (m, 3H), 4.26 (q, 2H), 5.02-5.16 (m, 2H), 6.95-7.07 (m, 3H), 7.46-7.57 (m, 3H), 7.65 (d, 1H), 7.79-7.88 (m, 2H), 7.94 (d, 1H), 8.17 (d, 2H). |
| | aus Ethyl-5-{[2-(2-{[4-(5-chlor-1,3-benzoxazol-2-yl)benzyl]oxy}-5-fluorphenyl)ethyl]amino}-5,6,7,8-tetrahydrochinolin-2-carboxylat (Enantiomer 2, Beispiel 117A) und Ethyl-5-brompentanoat | |

### Beispiel 121A

### Ethyl-5-([2-(2-{ [4-(5-chlor-1,3-benzoxazol-2-yl)benzyl]oxy}-5-fluorphenyl)ethyl]{2-[4-(methoxy-carbonyl)phenyl]ethyl}amino)-5,6,7,8-tetrahydrochinolin-2-carboxylat (Enantiomer 2)

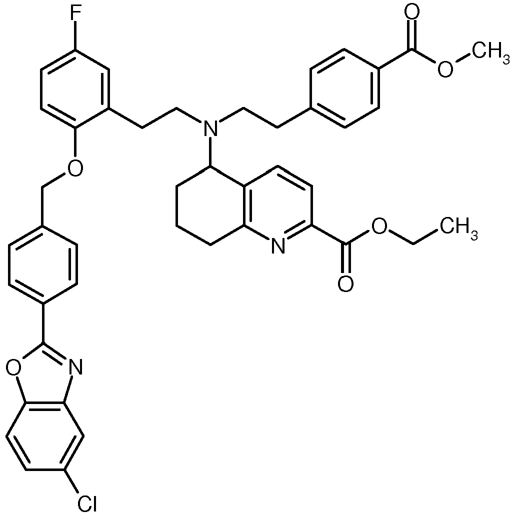

Eine Suspension aus 375 mg (0.63 mmol) Ethyl-5-{[2-(2-{[4-(5-chlor-1,3-benzoxazol-2-yl)-benzyl]oxy}-5-fluorphenyl)ethyl]amino}-5,6,7,8-tetrahydrochinolin-2-carboxylat (Enantiomer 2, Beispiel 117A), 272 mg (0.94 mmol) Methyl-4-(2-iodethyl)benzoat und 99 mg (0.94 mmol) wasserfreiem Natriumcarbonat in 10 ml trockenem Acetonitril wurde bei einer Badtemperatur von 110°C über Nacht gerührt. Anschließend wurden weitere 272 mg Methyl-4-(2-iodethyl)benzoat sowie 99 mg Natriumcarbonat zugesetzt und das Gemisch nochmals über Nacht unter Rückfluss erhitzt. Danach wurden abermals 272 mg Methyl-4-(2-iodethyl)benzoat und 99 mg Natriumcarbonat zugesetzt und das Gemisch wiederum über Nacht unter Rückfluss erhitzt. Nach dem Abkühlen des Reaktionsgemisches wurde Essigsäureethylester und Wasser zugesetzt, die organische Phase abgetrennt und die wässrige Phase noch dreimal mit Essigsäureethylester extrahiert. Die vereinigten organischen Phasen wurden über Magnesiumsulfat getrocknet, filtriert und bis zur Trockene eingedampft. Der erhaltene Rückstand wurde chromatographisch an Kieselgel gereinigt (Laufmittel: Petrolether/Essigsäureethylester 4:1 → 2:1). Es wurden 324 mg (0.42 mmol, 68% d. Th.) der Zielverbindung erhalten.

LC-MS (Methode 3): Rₜ = 1.63 min; m/z = 762/764 (M+H)⁺.

¹H-NMR (400 MHz, DMSO-*d₆*, δ/ppm): 1.26 (t, 3H), 1.41-1.55 (m, 1H), 1.55-1.69 (m, 1H), 1.89-2.08 (m, 2H), 2.57-2.83 (m, 10H), 3.76 (s, 3H), 4.02-4.12 (m, 1H), 4.26 (q, 2H), 5.02-5.12 (m, 2H), 6.95 (d, 1H), 7.03 (d, 2H), 7.11 (d, 2H), 7.41 (d, 1H), 7.45-7.56 (m, 4H), 7.72 (d, 2H), 7.82 (d, 1H), 7.92 (d, 1H), 8.09 (d, 2H).

Analog zu Beispiel 121A wurde die folgende Verbindung hergestellt:

| **Beispiel** | **Name / Struktur / Edukte** | **Analytische Daten** |
|---|---|---|
| **122A** | Ethyl-5-([2-(2-{[4-(5-chlor-1,3-benzoxazol-2-yl)-benzyl]oxy}-5-fluorphenyl)ethyl]{2-[4-(methoxy-carbonyl)phenyl]ethyl}amino)-5,6,7,8-tetrahydrochinolin-2-carboxylat (*Enantomer 1*) | LC-MS (Methode 3): Rₜ = 1.63 min; m/z = 762/764 (M+H)⁺. |
| | 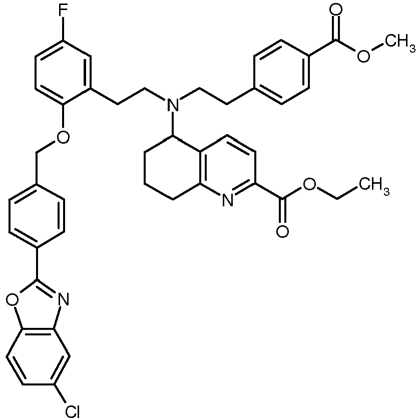 | ¹H-NMR (400 MHz, DMSO-*d₆,* δ/ppm): 1.26 (t, 3H), 1.41-1.55 (m, 1H), 1.55-1.69 (m, 1H), 1.89-2.08 (m, 2H), 2.57-2.81 (m, 10H), 3.76 (s, 3H), 4.02-4.11 (m, 1H), 4.26 (q, 2H), 5.02-5.12 (m, 2H), 6.95 (d, 1H), 7.02 (d, 2H), 7.11 (d, 2H), 7.41 (d, 1H), 7.45-7.56 (m, 4H), 7.72 (d, 2H), 7.82 (d, 1H), 7.92 (d, 1H), 8.09 (d, 2H). |
| | aus Ethyl-5-{[2-(2-{[4-(5-chlor-1,3-benzoxazol-2-yl)benzyl]oxy}-5-fluorphenyl)ethyl]amino}-5,6,7,8-tetrahydrochinolin-2-carboxylat (Enantiomer 1, Beispiel 118A) und Methyl-4-(2-iodethyl)benzoat | |

### Beispiel 123A

### Ethyl-5-([2-(2-{[3-chlor-4'-(trifluormethyl)biphenyl-4-yl]methoxy}-5-fluorphenyl)ethyl]{2-[4-(methoxycarbonyl)phenyl]ethyl}amino)-5,6,7,8-tetrahydrochinolin-2-carboxylat (Enantiomer 2)

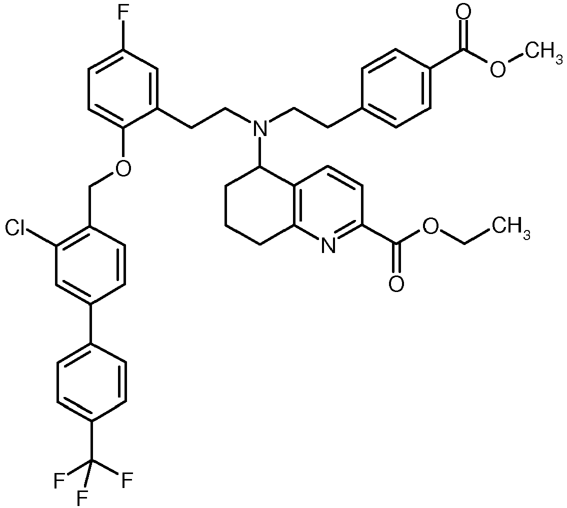

Eine Lösung von 740 mg (1.18 mmol) Ethyl-5-{[2-(2-{[3-chlor-4'-(trifluormethyl)biphenyl-4-yl]-methoxy}-5-fluorphenyl)ethyl]amino}-5,6,7,8-tetrahydrochinolin-2-carboxylat-Dihydrochlorid (Enantiomer 2, Beispiel 116A) in 20 ml trockenem Acetonitril wurde mit 685 mg (2.36 mmol) Methyl-4-(2-iodethyl)benzoat und 188 mg (1.77 mmol) wasserfreiem Natriumcarbonat versetzt und über Nacht unter Rückfluss erhitzt. Anschließend wurden weitere 342 mg Methyl-4-(2-iod-ethyl)benzoat zugegeben und das Gemisch über Nacht unter Rückfluss nachgerührt. Dieser Vorgang wurde noch zweimal an den darauffolgenden Tagen wiederholt. Danach wurden nochmals 342 mg Methyl-4-(2-iodethyl)benzoat sowie 188 mg wasserfreies Natriumcarbonat zugesetzt und das Gemisch wiederum über Nacht unter Rückfluss gerührt. Die Reaktionslösung wurde abschließend abermals mit 342 mg Methyl-4-(2-iodethyl)benzoat versetzt, über Nacht unter Rückfluss erhitzt und dann auf Raumtemperatur abgekühlt. Der Ansatz wurde filtriert, der Filterkuchen mit Acetonitril nachgewaschen und das Filtrat bis zur Trockene eingeengt. Der Rückstand wurde in Essigsäureethylester aufgenommen und mit Wasser versetzt. Die organische Phase wurde abgetrennt, über Magnesiumsulfat getrocknet, filtriert und bis zur Trockene eingedampft. Der erhaltene Rückstand wurde chromatographisch an Kieselgel gereinigt (Laufmittel: Cyclohexan/Essigsäureethylester 10:1 → 4:1). Es wurden 588 mg (0.40 mmol, 63% d. Th.) der Titelverbindung erhalten.

LC-MS (Methode 3): Rₜ = 1.68 min; m/z = 789/791 (M+H)⁺.

¹H-NMR (400 MHz, DMSO-*d₆*, δ/ppm): 1.27 (t, 3H), 1.38-1.51 (m, 1H), 1.51-1.68 (m, 1H), 1.87-2.05 (m, 2H), 2.57-2.80 (m, 10H), 3.81 (s, 3H), 4.00-4.08 (m, 1H), 4.26 (q, 2H), 5.00-5.10 (m, 2H), 6.92-6.98 (m, 1H), 7.01-7.15 (m, 4H), 7.35-7.42 (m, 2H), 7.53 (d, 1H), 7.61 (d, 1H), 7.74 (d, 2H), 7.77-7.88 (m, 5H).

Analog zu Beispiel 123A wurde die folgende Verbindung hergestellt:

| **Beispiel** | **Name / Struktur / Edukte** | **Analytische Daten** |
|---|---|---|
| **124A** | Ethyl-5-([2-(2-{[3-chlor-4'-(trifluormethyl)biphenyl-4-yl]methoxy}-5-fluorphenyl)ethyl]{2-[4-(methoxycarbonyl)phenyl]ethyl}amino)-5,6,7,8-tetrahydrochinolin-2-carboxylat (*Enantiomer 1*) | LC-MS (Methode 3): Rₜ = 1.68 min; m/z = 789/791 (M+H)⁺. |
| | 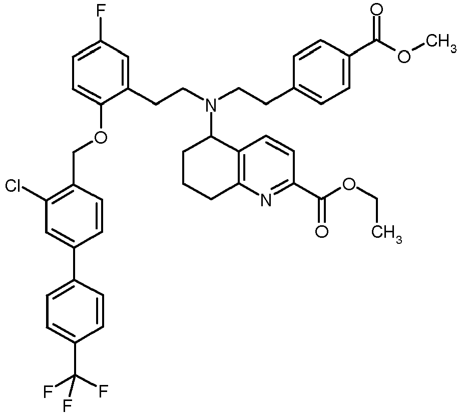 | ¹H-NMR (400 MHz, DMSO-*d₆,* δ/ppm): 1.27 (t, 3H), 1.42-1.69 (m, 2H), 1.86-2.05 (m, 2H), 2.58-2.79 (m, 10H), 3.81 (s, 3H), 3.99-4.08 (m, 1H), 4.26 (q, 2H), 5.00-5.10 (m, 2H), 6.91-6.99 (m, 1H), 7.03-7.15 (m, 4H), 7.35-7.42 (m, 2H), 7.53 (d, 1H), 7.58-7.64 (m, 1H), 7.74 (d, 2H), 7.77-7.89 (m, 5H). |
| | aus Ethyl-5-{[2-(2-{[3-chlor-4'-(trifluormethyl)-biphenyl-4-yl]methoxy}-5-fluorphenyl)ethyl]-amino}-5,6,7,8-tetrahydrochinolin-2-carboxylat-Dihydrochlorid (Enantiomer 1, Beispiel 115A) und Methyl-4-(2-iodethyl)benzoat | |

### Beispiel 125A

### Methyl-4-[(E/Z)-2-(4-fluorphenyl)vinyl]benzoat

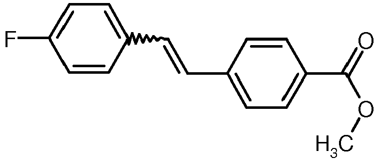

79.75 g (176.70 mmol) (4-Fluorbenzyl)(triphenyl)phosphoniumbromid und 29.59 g (180.24 mmol) Methyl-4-formylbenzoat wurden in 250 ml Methanol gelöst, auf 0°C abgekühlt und portionsweise mit 10.98 g (203.21 mmol) Natriummethanolat versetzt. Anschließend wurde das Reaktionsgemisch langsam auf Raumtemperatur erwärmt und über Nacht bei dieser Temperatur gerührt. Danach wurde die Reaktionslösung wieder auf 0°C abgekühlt, in Portionen mit weiteren 4.77 g (88.35 mmol) Natriummethanolat versetzt und nach Erwärmen auf Raumtemperatur nochmals über Nacht nachgerührt. Der ausgefallene Feststoff wurde abfiltriert, mit Methanol gewaschen und über Nacht bei 40°C im Trockenschrank unter Vakuum getrocknet. Es wurden 21.08 g (82.25 mmol, 46.5% d. Th.) der Zielverbindung erhalten. Das Filtrat wurde bis zur Trockene eingedampft und der erhaltene Rückstand chromatographisch an Kieselgel gereinigt (Laufmittel: Cyclohexan/Essigsäureethylester 10:1). Es wurden so weitere 23.75 g (92.67 mmol, 52% d. Th.) der Titelverbindung erhalten.

LC-MS (Methode 2): Rₜ = 2.80 min; m/z = 257 (M+H)⁺ (Fraktion 1); Rₜ = 2.82 min; m/z = 257 (M+H)⁺ (Fraktion 2).

### Beispiel 126A

### Methyl-4-[2-(4-fluorphenyl)ethyl]benzoat

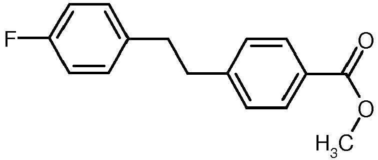

44 g (171.70 mmol) Methyl-4-[(*E*/*Z*)-2-(4-fluorphenyl)vinyl]benzoat in 500 ml THF wurden mit 2 g 10% Palladium auf Kohle versetzt und über Nacht bei Raumtemperatur unter einer Wasserstoffatmosphäre bei Normaldruck gerührt. Danach wurde nochmals 1 g 10% Palladium auf Kohle zugesetzt und das Gemisch erneut über Nacht bei Raumtemperatur unter einer Wasserstoffatmosphäre bei Normaldruck gerührt. Anschließend wurde das Reaktionsgemisch filtriert und das resultierende Filtrat bis zur Trockene eingeengt. Es wurden 35 g (135.5 mmol, 79% d. Th.) der Titelverbindung erhalten.

LC-MS (Methode 2): Rₜ = 2.76 min; m/z = 259 (M+H)⁺.

### Beispiel 127A

### {4-[2-(4-Fluorphenyl)ethyl]phenyl}methanol

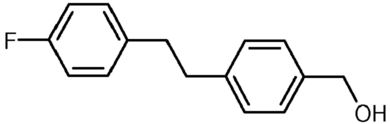

Zu einer Lösung von 35.4 g (136.98 mmol) Methyl-4-[2-(4-fluorphenyl)ethyl]benzoat in 500 ml trockenem THF wurden unter Rückfluss 45 ml einer 3.5 M Lösung von Lithiumaluminiumhydrid in Toluol langsam zugetropft. Nach Abschluss der Zugabe wurde das Reaktionsgemisch eine Stunde unter Rückfluss nachgerührt. Anschließend wurde das Reaktionsgemisch auf 0°C abgekühlt und langsam und vorsichtig mit 500 ml eisgekühlter 1 M Salzsäure versetzt. Danach wurden 750 ml Essigsäureethylester zugesetzt, die wässrige Phase abgetrennt und die organische Phase nacheinander je einmal mit 1 M Salzsäure und gesättigter Natriumchlorid-Lösung gewaschen. Die organische Phase wurde dann über Magnesiumsulfat getrocknet, filtriert und bis zur Trockene eingeengt. Es wurden 31.5 g (136.7 mmol, 99.9% d. Th.) der Titelverbindung erhalten.

LC-MS (Methode 3): Rₜ = 1.05 min; m/z = 213 (M+H-H₂O)⁺.

¹H-NMR (400 MHz, DMSO-*d₆*, δ/ppm): 2.84 (s, 4H), 4.44 (d, 2H), 5.09 (t, 1H), 7.08 (t, 2H), 7.13-7.27 (m, 6H).

### Beispiel 128A

### 1-(Chlormethyl)-4-[2-(4-fluorphenyl)ethyl]benzol

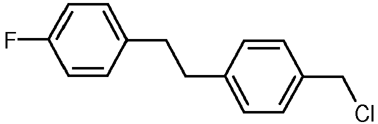

Zu einer Lösung von 31.5 g (136.7 mmol) {4-[2-(4-Fluorphenyl)ethyl]phenyl}methanol in 400 ml Dichlormethan wurden bei 0°C 14.96 ml Thionylchlorid in 100 ml Dichlormethan langsam zugetropft. Nach Abschluss der Zugabe wurde das Reaktionsgemisch auf Raumtemperatur erwärmt und zwei Stunden bei dieser Temperatur nachgerührt. Anschließend wurde das Reaktionsgemisch wieder auf 0°C abgekühlt und langsam und vorsichtig unter starkem Rühren mit 200 ml gesättigter wässriger Natriumhydrogencarbonat-Lösung versetzt. Darauffolgend wurde die Lösung so lange mit kleinen Portionen an festem Natriumhydrogencarbonat versetzt, bis ein pH-Wert von 6 eingestellt war. Die Phasen wurden dann getrennt, und die organische Phase wurde über Magnesiumsulfat getrocknet, filtriert und bis zur Trockene eingeengt. Es wurden 28.5 g (114.5 mmol, 84% d. Th.) der Titelverbindung erhalten.

¹H-NMR (400 MHz, DMSO-*d₆*, δ/ppm): 2.86 (s, 4H), 4.72 (s, 2H), 7.08 (t, 2H), 7.19-7.28 (m, 4H), 7.33 (d, 2H).

### Beispiel 129A

### rac-Ethyl-5-[(tert.-butoxycarbonyl){2-[2-({4-[2-(4-fluorphenyl)ethyl]benzyl}oxy)phenyl]ethyl}-amino]-5,6,7,8-tetrahydrochinolin-2-carboxylat

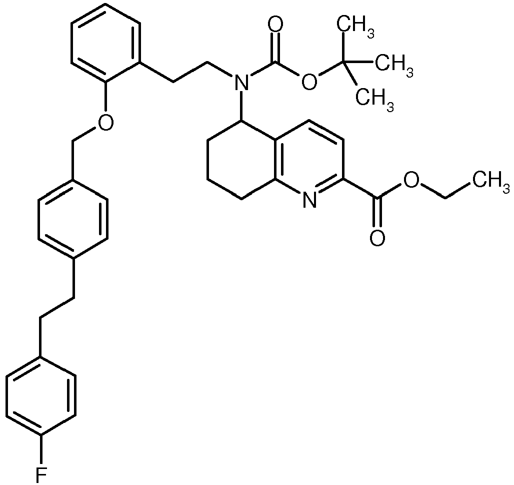

5.60 g (12.71 mmol) *rac*-Ethyl-5-{(*tert*.-butoxycarbonyl)[2-(2-hydroxyphenyl)ethyl]amino}-5,6,7,8-tetrahydrochinolin-2-carboxylat, 3.79 g (15.25 mmol) 1-(Chlormethyl)-4-[2-(4-fluorphenyl)ethyl]benzol und 2.64 g (19.07 mmol) Kaliumcarbonat in 200 ml Acetonitril wurden auf 110°C erhitzt und über Nacht bei dieser Temperatur gerührt. Nach dem Abkühlen wurde das Reaktionsgemisch filtriert, der Filterkuchen mehrmals mit Acetonitril nachgewaschen und die vereinigten Filtrate am Rotationsverdampfer bis zur Trockene eingeengt. Der erhaltene Rückstand wurde chromatographisch an Kieselgel gereinigt (Laufmittel: Cyclohexan/Essigsäureethylester 10:1 → 4:1). Es wurden 6.8 g (10.42 mmol, 82% d. Th.) der Zielverbindung erhalten.

LC-MS (Methode 3): Rₜ = 1.62 min; m/z = 653 (M+H)⁺.

### Beispiel 130A und Beispiel 131A

### Ethyl-5-[(tert.-butoxycarbonyl){2-[2-({4-[2-(4-fluorphenyl)ethyl]benzyl}oxy)phenyl]ethyl}-amino]-5,6,7,8-tetrahydrochinolin-2-carboxylat (Enantiomer 1 und 2)

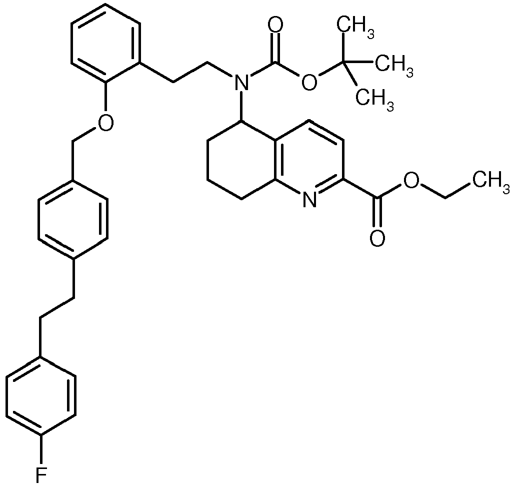

6.8 g (10.42 mmol) des racemischen Ethyl-5-[(*tert*.-butoxycarbonyl){2-[2-({4-[2-(4-fluorphenyl)-ethyl]benzyl}oxy)phenyl]ethyl}amino]-5,6,7,8-tetrahydrochinolin-2-carboxylats (Beispiel 129A) wurden durch superkritische Flüssigchromatographie (SFC) an chiraler Phase in die Enantiomere aufgetrennt [Säule: Chiracel OD-H, 20 µm, 250 mm x 30 mm; Elutionsmittel: Kohlendioxid/ Ethanol 83:17 (v/v); Fluss: 185 ml/min; Druck: 135 bar; UV-Detektion: 210 nm; Temperatur: 38°C]:

### Beispiel 130A (Enantiomer 1):

Ausbeute: 3240 mg
Rₜ = 2.83 min; chemische Reinheit >99.9%; >99% ee
[Säule: Chiralpak OD-H, 5 µm, 250 mm x 4.6 mm; Elutionsmittel: Kohlendioxid/Ethanol 70:30 (v/v); Fluss: 3 ml/min; UV-Detektion: 210 nm].
LC-MS (Methode 3): Rₜ = 1.57 min; m/z = 653 (M+H)⁺.

### Beispiel 131A (Enantiomer 2):

Ausbeute: 3180 mg
Rₜ = 4.12 min; chemische Reinheit >99%; >99% ee
[Säule: Chiralpak OD-H, 5 µm, 250 mm x 4.6 mm; Elutionsmittel: Kohlendioxid/Ethanol 70:30 (v/v); Fluss: 3 ml/nmin; UV-Detektion: 210 nm].
LC-MS (Methode 3): Rₜ = 1.57 min; m/z = 653 (M+H)⁺.

### Beispiel 132A

### Ethyl-5-({2-[2-({4-[2-(4-fluorphenyl)ethyl]benzyl}oxy)phenyl]ethyl}ammo)-5,6,7,8-tetrahydrochinolin-2-carboxylat-Dihydrochlorid (Enantiomer 2)

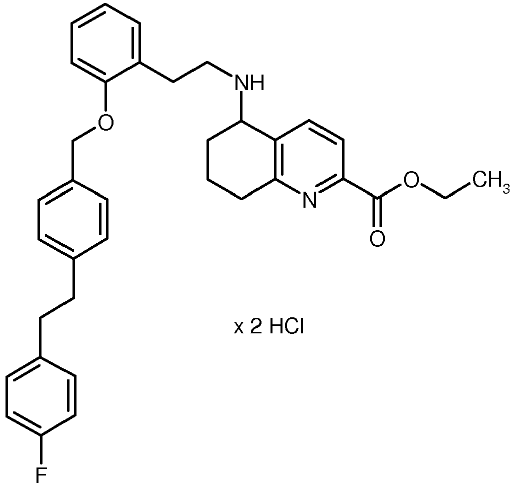

3180 mg (4.87 mmol) Ethyl-5-[(ter-t.-butoxycarbonyl){2-[2-({4-[2-(4-fluorphenyl)ethyl]benzyl}-oxy)phenyl]ethyl}amino]-5,6,7,8-tetrahydrochinolin-2-carboxylat (Enantiomer 2, Beispiel 131A) wurden mit 12 ml einer 4 N Lösung von Chlorwasserstoff in Dioxan versetzt und 2 h bei Raumtemperatur gerührt. Anschließend wurde das Reaktionsgemisch bis zur Trockene eingeengt. Es wurden 3290 mg des Zielprodukts erhalten, welches ohne weitere analytische Charakterisierung weiter umgesetzt wurde.

Analog zu Beispiel 132A wurde die folgende Verbindung hergestellt:

| **Beispiel** | **Name / Struktur / Edukt** | **Analytische Daten** |
|---|---|---|
| **133A** | Ethyl-5-({2-[2-({4-[2-(4-fluorphenyl)ethyl]benzyl}-oxy)phenyl]ethyl}amino)-5,6,7,8-tetrahydrochinolin-2-carboxylat-Dihydrochlorid (*Enantiomer 1*) | |
| | 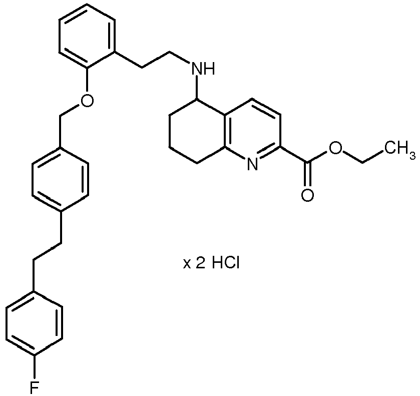 | |
| | aus Ethyl-5-[(*tert*.-butoxycarbonyl){2-[2-({4-[2-(4-fluorphenyl)ethyl]benzyl}oxy)phenyl]ethyl}amino]-5,6,7,8-tetrahydrochinolin-2-carboxylat (Enantiomer 1, Beispiel 130A) | |

### Beispiel 134A

### Ethyl-5-({2-[2-({4-[2-(4-fluorphenyl)ethyl]benzyl}oxy)phenyl]ethyl}ammo)-5,6,7,8-tetrahydro-chinolin-2-carboxylat (Enantiomer 2)

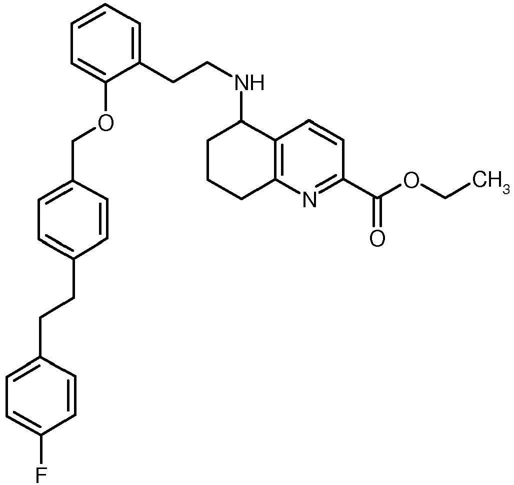

3290 mg (5.58 mmol) Ethyl-5-({2-[2-({4-[2-(4-fluorphenyl)ethyl]benzyl}oxy)phenyl]ethyl}-amino)-5,6,7,8-tetrahydrochinolin-2-carboxylat-Dihydrochlorid (Enantiomer 2, Beispiel 132A) wurden in 50 ml THF aufgenommen, mit 3.11 ml Triethylamin versetzt und eine Stunde bei Raumtemperatur gerührt. Anschließend wurde die Reaktionslösung mit Essigsäureethylester und Wasser versetzt, die Phasen getrennt und die wässrige Phase noch einmal mit Essigsäureethylester extrahiert. Die vereinigten organischen Phasen wurden nochmals mit Wasser gewaschen, über Magnesiumsulfat getrocknet, filtriert und anschließend bis zur Trockene eingedampft. Es wurden 2150 mg (3.89 mmol, 70% d. Th.) der Zielverbindung erhalten.

LC-MS (Methode 3): Rₜ = 1.06 min; m/z = 553 (M+H)⁺.

¹H-NMR (400 MHz, DMSO-*d₆*, δ/ppm): 1.30 (t, 3H), 1.60-1.74 (m, 2H), 1.78-1.89 (m, 1H), 1.89-2.06 (m, 2H), 2.65-2.92 (m, 10H), 3.76 (br. s, 1H), 4.31 (q, 2H), 5.04 (s, 2H), 6.86 (t, 1H), 6.99-7.11 (m, 3H), 7.13-7.27 (m, 6H), 7.31 (d, 2H), 7.76 (d, 1H), 7.85 (d, 1H).

Analog zu Beispiel 134A wurde die folgende Verbindung hergestellt:

| **Beispiel** | **Name / Struktur / Edukt** | **Analytische Daten** |
|---|---|---|
| **135A** | Ethyl-5-({2-[2-({4-[2-(4-fluorphenyl)ethyl]benzyl}-oxy)phenyl]ethyl}amino)-5,6,7,8-tetrahydro-chinolin-2-carboxylat (*Enantiomer 1*) | LC-MS (Methode 3): Rₜ = 1.02 min; m/z = 553 (M+H)⁺. |
| | 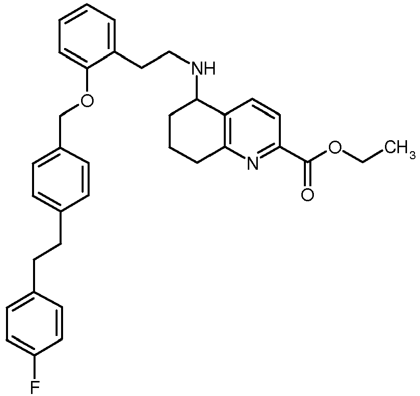 | ¹H-NMR (400 MHz, DMSO-*d₆*, δ/ppm): 1.30 (t, 3H), 1.60-1.75 (m, 2H), 1.79-1.89 (m, 1H), 1.90-2.05 (m, 2H), 2.65-2.91 (m, 10H), 3.76 (br. s, 1H), 4.31 (q, 2H), 5.04 (s, 2H), 6.87 (t, 1H), 6.98-7.11 (m, 3H), 7.13-7.27 (m, 6H), 7.31 (d, 2H), 7.76 (d, 1H), 7.85 (d, 1H). |
| | aus Ethyl-5-({2-[2-({4-[2-(4-fluorphenyl)ethyl]-benzyl}oxy)phenyl]ethyl}amino)-5,6,7,8-tetra-hydrochinolin-2-carboxylat-Dihydrochlorid (Enantiomer 1, Beispiel 133A) | |

### Beispiel 136A

### Ethyl-5-({2-[2-({4-[2-(4-fluorphenyl)ethyl]benzyl}oxy)phenyl]ethyl}{2-[4-(methoxycarbonyl)-phenyl]ethyl}amino)-5,6,7,8-tetrahydrochinolin-2-carboxylat (Enantiomer 2)

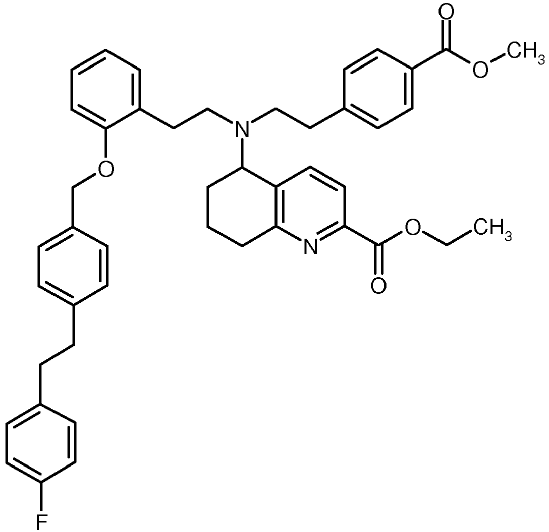

Eine Lösung von 1980 mg (3.58 mmol) Ethyl-5-({2-[2-({4-[2-(4-fluorphenyl)ethyl]benzyl}oxy)-phenyl]ethyl}amino)-5,6,7,8-tetrahydrochinolin-2-carboxylat (Enantiomer 2, Beispiel 134A) in 30 ml trockenem Acetonitril wurde mit 3118 mg (10.75 mmol) Methyl-4-(2-iodethyl)benzoat und 570 mg (5.37 mmol) wasserfreiem Natriumcarbonat versetzt und über Nacht unter Rückfluss erhitzt. Anschließend wurden weitere 379 mg Methyl-4-(2-iodethyl)benzoat zugegeben und das Gemisch nochmals über Nacht unter Rückfluss nachgerührt. Danach wurden erneut 379 mg Methyl-4-(2-iodethyl)benzoat zugesetzt. Das Gemisch wurde dann drei weitere Tage lang unter Rückfluss gerührt und schließlich auf Raumtemperatur abgekühlt. Der Ansatz wurde filtriert, der Filterkuchen mit Acetonitril nachgewaschen und das Filtrat bis zur Trockene eingeengt. Der erhaltene Rückstand wurde chromatographisch an Kieselgel gereinigt (Laufmittel: Cyclohexan/Essig-säureethylester 10:1 → 4:1 → 2:1). Es wurden 715 mg (2.40 mmol, Gehalt 97%, 67% d. Th.) der Titelverbindung erhalten.

LC-MS (Methode 3): Rₜ = 1.57 min; m/z = 715 (M+H)⁺.

[α]_{D}²⁰ = +60.75°, c = 0.40, Methanol.

Analog zu Beispiel 136A wurde die folgende Verbindung hergestellt:

| **Beispiel** | **Name / Struktur / Edukte** | **Analytische Daten** |
|---|---|---|
| **137A** | Ethyl-5-({2-[2-({4-[2-(4-fluorphenyl)ethyl]benzyl}-oxy)phenyl]ethyl}{2-[4-(methoxycarbonyl)-phenyl]ethyl}amino)-5,6,7,8-tetrahydrochinolin-2-carboxylat (*Enantiomer 1*) | LC-MS (Methode 3): Rₜ = 1.57 min; m/z = 715 (M+H)⁺. |
| | 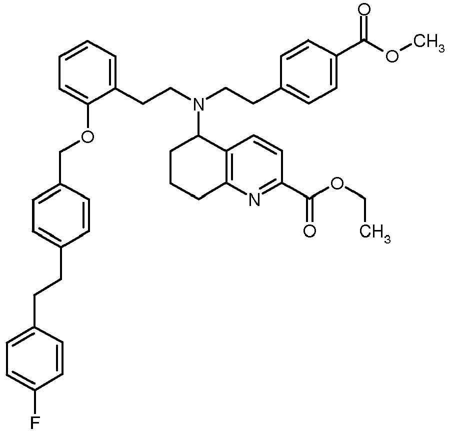 | [α]_{D}²⁰ = -52.1°, c = 0.325, Methanol. |
| | aus Ethyl-5-({2-[2-({4-[2-(4-fluorphenyl)ethyl]-benzyl}oxy)phenyl]ethyl}amino)-5,6,7,8-tetra-hydrochinolin-2-carboxylat (Enantiomer 1, Beispiel 135A) und Methyl-4-(2-iodethyl)benzoat | |

### Beispiel 138A

### rac-Ethyl-5-[(tert.-butoxycarbonyl){2-[5-fluor-2-({4-[2-(4-fluorphenyl)ethyl]benzyl}oxy)phenyl]-ethyl}amino]-5,6,7,8-tetrahydrochinolin-2-carboxylat

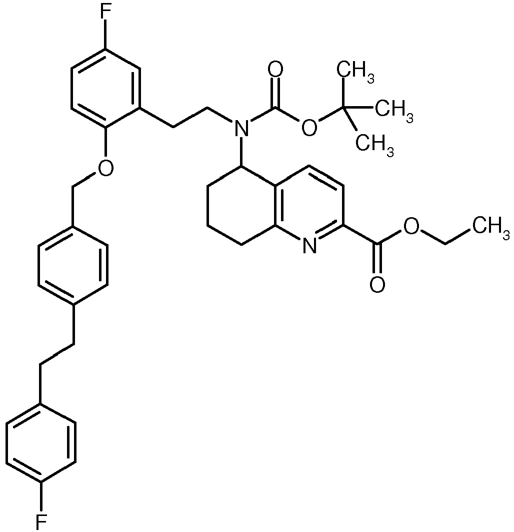

10 g (21.81 mmol) *rac*-Ethyl-5-{(*tert*.-butoxycarbonyl)[2-(5-fluor-2-hydroxyphenyl)ethyl]amino}-5,6,7,8-tetrahydrochinolin-2-carboxylat (Beispiel 106A), 5.98 g (23.99 mmol) 1-(Chlormethyl)-4-[2-(4-fluorphenyl)ethyl]benzol und 4.52 g (32.71 mmol) Kaliumcarbonat in 240 ml Acetonitril wurden auf 110°C erhitzt und über Nacht bei dieser Temperatur gerührt. Nach dem Abkühlen wurde das Reaktionsgemisch filtriert, der Filterkuchen mehrmals mit Acetonitril nachgewaschen und die vereinigten Filtrate am Rotationsverdampfer bis zur Trockene eingeengt. Der erhaltene Rückstand wurde chromatographisch an Kieselgel gereinigt (Laufmittel: Cyclohexan/Essigsäureethylester 10:1 → 4:1). Es wurden 14.11 g (21.03 mmol, 96% d. Th.) der Zielverbindung erhalten.

LC-MS (Methode 3): Rₜ = 1.57 min; m/z = 671 (M+H)⁺.

### Beispiel 139A und Beispiel 140A

### Ethyl-5-[(tert.-butoxycarbonyl){2-[5-fluor-2-({4-[2-(4-fluorphenyl)ethyl]benzyl}oxy)phenyl]-ethyl}amino]-5,6,7,8-tetrahydrochinolin-2-carboxylat (Enantiomer 1 und 2)

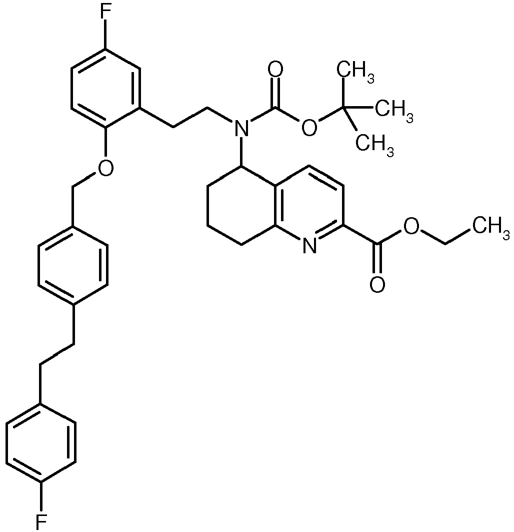

14.11 g (21.03 mmol) des racemischen Ethyl-5-[(*tert*.-butoxycarbonyl){2-[5-fluor-2-({4-[2-(4-fluorphenyl)ethyl]benzyl}oxy)phenyl]ethyl}amino]-5,6,7,8-tetrahydrochinolin-2-carboxylats (Beispiel 138A) wurden durch superkritische Flüssigchromatographie (SFC) an chiraler Phase in die Enantiomere aufgetrennt [Säule: Daicel Chiralpak AZ-H, 5 µm, 250 mm x 50 mm; Elutionsmittel: Kohlendioxid/Ethanol 70:30 (v/v); Fluss: 200 ml/min; Druck: 80 bar; UV-Detektion: 220 nm; Temperatur: 15°C]:

### Beispiel 139A (Enantiomer 1):

Ausbeute: 5690 mg
Rₜ = 3.98 min; chemische Reinheit >99.9%; >99% ee
[Säule: Daicel Chiralpak AZ-H, 5 µm, 250 mm x 4.6 mm; Elutionsmittel: Kohlendioxid/Ethanol 70:30 (v/v); Fluss: 3 ml/min; UV-Detektion: 220 nm].
LC-MS (Methode 3): Rₜ = 1.61 min; m/z = 671 (M+H)⁺.

### Beispiel 140A (Enantiomer 2):

Ausbeute: 6080 mg
Rₜ = 6.41 min; chemische Reinheit >99%; >99% ee
[Säule: Daicel Chiralpak AZ-H, 5 µm, 250 mm x 4.6 mm; Elutionsmittel: Kohlendioxid/Ethanol 70:30 (v/v); Fluss: 3 ml/min; UV-Detektion: 220 nm].
LC-MS (Methode 3): Rₜ = 1.61 min; m/z = 671 (M+H)⁺.

### Beispiel 141A

### Ethyl-5-({2-[5-fluor-2-({4-[2-(4-fluorphenyl)ethyl]benzyl}oxy)phenyl]ethyl}amino)-5,6,7,8-tetra-hydrochinolin-2-carboxylat-Dihydrochlorid (Enantiomer 2)

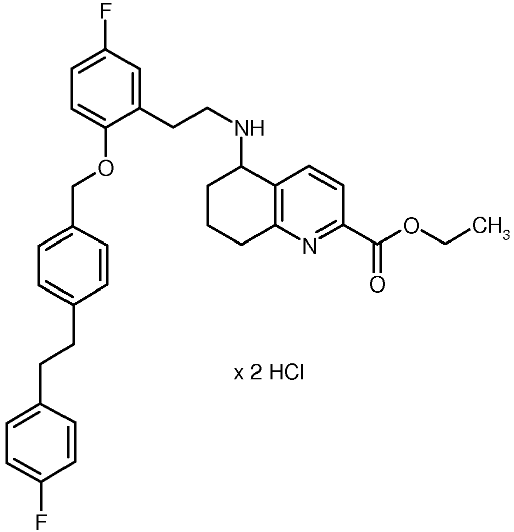

6080 mg (9.06 mmol) Ethyl-5-[(*tert*.-butoxycarbonyl){2-[5-fluor-2-({4-[2-(4-fluorphenyl)ethyl]-benzyl}oxy)phenyl]ethyl}amino]-5,6,7,8-tetrahydrochinolin-2-carboxylat (Enantiomer 2, Beispiel 140A) wurden mit 23 ml einer 4 N Lösung von Chlorwasserstoff in Dioxan versetzt und 2 h bei Raumtemperatur gerührt. Anschließend wurde das Reaktionsgemisch bis zur Trockene eingeengt. Es wurden 6240 mg des Zielprodukts erhalten, welches ohne weitere analytische Charakterisierung weiter umgesetzt wurde.

Analog zu Beispiel 141A wurde die folgende Verbindung hergestellt:

| **Beispiel** | **Name / Struktur / Edukt** | **Analytische Daten** |
|---|---|---|
| **142A** | Ethyl-5-({2-[5-fluor-2-({4-[2-(4-fluorphenyl)ethyl]-benzyl}oxy)phenyl]ethyl}amino)-5,6,7,8-tetra-hydrochinolin-2-carboxylat-Dihydrochlorid (*Enantiomer 1*) | |
| | 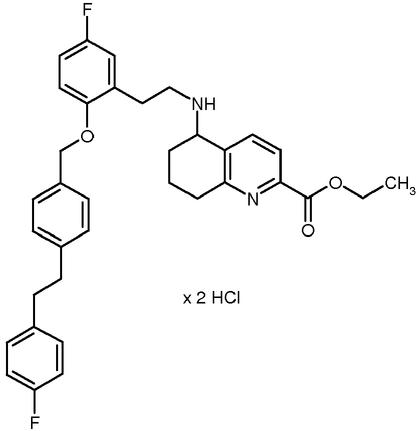 | |
| | aus Ethyl-5-[(*tert*.-butoxycarbonyl){2-[5-fluor-2-({4-[2-(4-fluorphenyl)ethyl]benzyl}oxy)phenyl]-ethyl}amino]-5,6,7,8-tetrahydrochinolin-2-carboxylat (Enantiomer 1, Beispiel 139A) | |

### Beispiel 143A

### Ethyl-5-({2-[5-fluor-2-({4-[2-(4-fluorphenyl)ethyl]benzyl}oxy)phenyl]ethyl}amino)-5,6,7,8-tetra-hydrochinolin-2-carboxylat (Enantiomer 2)

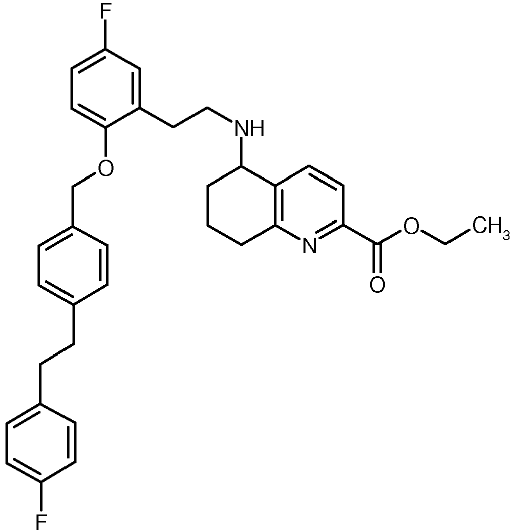

6240 mg (10.28 mmol) Ethyl-5-({2-[5-fluor-2-({4-[2-(4-fluorphenyl)ethyl]benzyl}oxy)phenyl]-ethyl}amino)-5,6,7,8-tetrahydrochinolin-2-carboxylat-Dihydrochlorid (Enantiomer 2, Beispiel 141A) wurden in 103 ml THF aufgenommen, mit 5.7 ml Triethylamin versetzt und eine Stunde bei Raumtemperatur gerührt. Anschließend wurde die Reaktionslösung mit Essigsäureethylester und Wasser versetzt, die Phasen getrennt und die wässrige Phase noch einmal mit Essigsäureethylester extrahiert. Die vereinigten organischen Phasen wurden nochmals mit Wasser gewaschen, über Magnesiumsulfat getrocknet, filtriert und schließlich bis zur Trockene eingedampft. Es wurden 3600 mg (6.31 mmol, 61% d. Th.) der Zielverbindung erhalten, welche ohne weitere analytische Charakterisierung weiter umgesetzt wurde.

Analog zu Beispiel 143A wurde die folgende Verbindung hergestellt:

| **Beispiel** | **Name / Struktur / Edukt** | **Analytische Daten** |
|---|---|---|
| **144A** | Ethyl-5-({2-[5-fluor-2-({4-[2-(4-fluorphenyl)ethyl]-benzyl}oxy)phenyl]ethyl}amino)-5,6,7,8-tetrahydrochinolin-2-carboxylat (*Enantiomer 1*) | |
| | 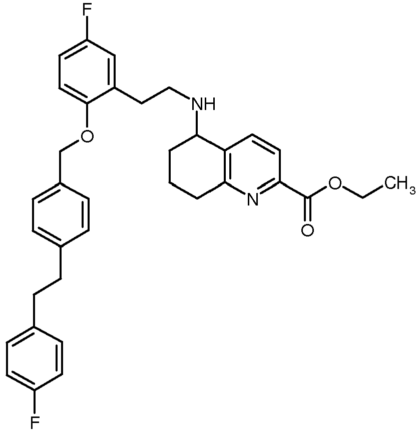 | |
| | aus Ethyl-5-({2-[5-fluor-2-({4-[2-(4-fluorphenyl)-ethyl]benzyl}oxy)phenyl]ethyl}amino)-5,6,7,8-tetrahydrochinolin-2-carboxylat-Dihydrochlorid (Enantiomer 1, Beispiel 142A) | |

### Beispiel 145A

### Ethyl-5-({2-[5-fluor-2-({4-[2-(4-fluorphenyl)ethyl]benzyl}oxy)phenyl]ethyl}{2-[4-(methoxy-carbonyl)phenyl]ethyl}amino)-5,6,7,8-tetrahydrochinolin-2-carboxylat (Enantiomer 2)

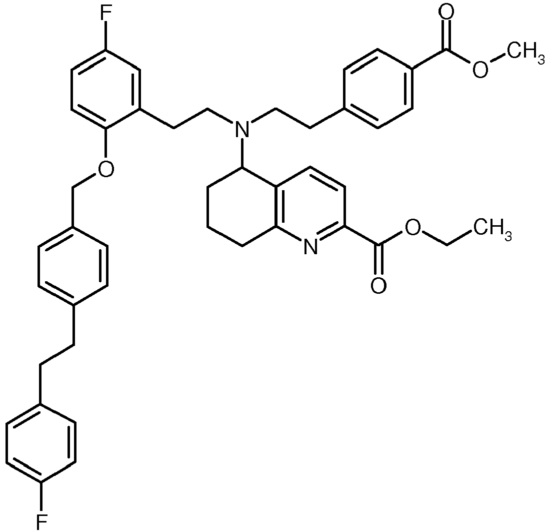

Eine Lösung von 200 mg (0.35 mmol) Ethyl-5-({2-[5-fluor-2-({4-[2-(4-fluorphenyl)ethyl]benzyl}-oxy)phenyl]ethyl}amino)-5,6,7,8-tetrahydrochinolin-2-carboxylat (Enantiomer 2, Beispiel 143A) in 3 ml trockenem Acetonitril wurde mit 305 mg (1.05 mmol) Methyl-4-(2-iodethyl)benzoat und 56 mg (0.53 mmol) wasserfreiem Natriumcarbonat versetzt und 4 Stunden bei 140°C in einer Mikrowellenapparatur (Biotage Initiator) gerührt. Anschließend wurde die Reaktionslösung abgekühlt und direkt mittels präparativer HPLC gereinigt (Eluent: Acetonitril/Wasser 9:1). Es wurden 75 mg (0.10 mmol, 29% d. Th.) der Titelverbindung erhalten.

LC-MS (Methode 3): Rₜ = 1.58 min; m/z = 733 (M+H)⁺.

¹H-NMR (400 MHz, DMSO-*d₆*, δ/ppm): 1.29 (t, 3H), 1.40-1.53 (m, 1H), 1.53-1.67 (m, 1H), 1.87-2.06 (m, 2H), 2.56-2.86 (m, 14H), 3.83 (s, 3H), 4.00-4.09 (m, 1H), 4.29 (q, 2H), 4.82-4.94 (m, 2H), 6.91 (d, 1H), 6.96-7.02 (m, 2H), 7.03-7.26 (m, 10H), 7.34-7.44 (m, 2H), 7.80 (d, 2H).

Analog zu Beispiel 145A wurde die folgende Verbindung hergestellt:

| **Beispiel** | **Name / Struktur / Edukte** | **Analytische Daten** |
|---|---|---|
| **146A** | Ethyl-5-({2-[5-fluor-2-({4-[2-(4-fluorphenyl)ethyl]-benzyl}oxy)phenyl]ethyl}{2-[4-(methoxycarbonyl)-phenyl]ethyl}amino)-5,6,7,8-tetrahydrochinolin-2-carboxylat (*Enantiomer 1*) | LC-MS (Methode 3): Rₜ = 1.59 min; m/z = 733 (M+H)⁺. |
| | 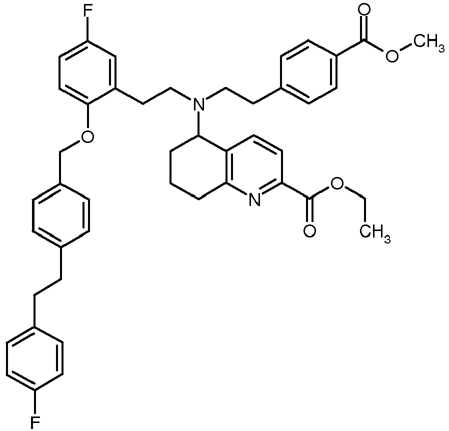 | ¹H-NMR (400 MHz, DMSO-*d₆*, δ/ppm): 1.29 (t, 3H), 1.39-1.52 (m, 1H), 1.53-1.68 (m, 1H), 1.88-2.07 (m, 2H), 2.57-2.85 (m, 14H), 3.83 (s, 3H), 4.00-4.10 (m, 1H), 4.29 (q, 2H), 4.81-4.94 (m, 2H), 6.86-6.94 (m, 1H), 6.99 (d, 2H), 7.03-7.26 (m, 10H), 7.39 (m, 2H), 7.80 (d, 2H). |
| | aus Ethyl-5-({2-[5-fluor-2-({4-[2-(4-fluorphenyl)-ethyl]benzyl}oxy)phenyl]ethyl}amino)-5,6,7,8-tetrahydrochinolin-2-carboxylat (Enantiomer 1, Beispiel 144A) und Methyl-4-(2-iodethyl)benzoat | |

### Beispiel 147A und Beispiel 69A

### Ethyl-5-{(tert.-butoxycarbonyl)[2-(2-hydroxyphenyl)ethyl]amino}-5,6,7,8-tetrahydrochinolin-2-carboxylat (Enantiomer 1 und 2)

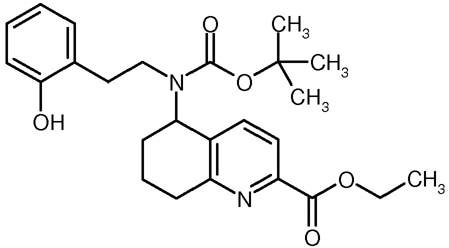

25 g (56.74 mmol) des racemischen Ethyl-5-{(*tert*.-butoxycarbonyl)[2-(2-hydroxyphenyl)ethyl]-amino}-5,6,7,8-tetrahydrochinolin-2-carboxylats (Beispiel 10A) wurden durch superkritische Flüssigchromatographie (SFC) an chiraler Phase in die Enantiomere aufgetrennt [Säule: Daicel Chiralpak AZ-H, 5 µm, 250 mm x 50 mm; Elutionsmittel: Kohlendioxid/Isopropanol 85:15 (v/v); Fluss: 400 ml/min; Druck: 80 bar; UV-Detektion: 220 nm; Temperatur: 37°C]:

### Beispiel 147A (Enantiomer 1):

Ausbeute: 11.3 g
Rₜ = 5.98 min; chemische Reinheit >99.9%; >99% ee
[Säule: Daicel Chiralpak OZ-H, 5 µm, 250 mm x 4.6 mm; Elutionsmittel: Isohexan/Ethanol 80:20 (v/v); Fluss: 1 ml/min; UV-Detektion: 220 nm].

### Beispiel 69A (Enantiomer 2):

Ausbeute: 11.9 g
Rₜ = 4.36 min; chemische Reinheit >99%; >92% ee
[Säule: Daicel Chiralpak OZ-H, 5 µm, 250 mm x 4.6 mm; Elutionsmittel: Isohexan/Ethanol 80:20 (v/v); Fluss: 1 ml/min; UV-Detektion: 220 nm].

### Beispiel 148A und Beispiel 74A

### Ethyl-5-{(tert.-butoxycarbonyl)[2-(2-{[3-chlor-4'-(trifluormethyl)biphenyl-4-yl]methoxy}phenyl)-ethyl]amino}-5,6,7,8-tetrahydrochinolin-2-carboxylat (Enantiomer 1 und 2)

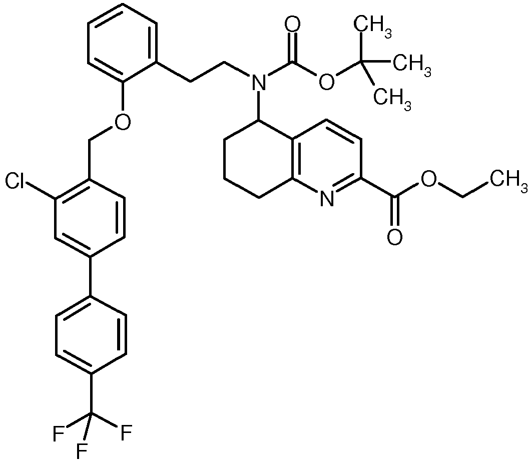

15 g (21.42 mmol) des racemischen Ethyl-5-{(*tert*.-butoxycarbonyl)[2-(2-{[3-chlor-4'-(trifluor-methyl)biphenyl-4-yl]methoxy}phenyl)ethyl]amino}-5,6,7,8-tetrahydrochinolin-2-carboxylats (Beispiel 22A) wurden durch superkritische Flüssigchromatographie (SFC) an chiraler Phase in die Enantiomere aufgetrennt [Säule: Chiralpak OD-H, 20 µm, 400 mm x 50 mm; Elutionsmittel: Kohlendioxid/Isopropanol 70:30 (v/v); Fluss: 400 ml/min; Druck: 80 bar; UV-Detektion: 220 nm; Temperatur: 37°C]:

### Beispiel 148A (Enantiomer 1):

Ausbeute: 5830 mg
Rₜ = 2.83 min; chemische Reinheit >99.9%; >99% ee
[Säule: Chiralpak OD-H, 5 µm, 250 mm x 4.6 mm; Elutionsmittel: Kohlendioxid/Isopropanol 70:30 (v/v); Fluss: 3 ml/min; UV-Detektion: 210 nm].

### Beispiel 74A (Enantiomer 2):

Ausbeute: 6330 mg
Rₜ = 5.30 min; chemische Reinheit >99%; >98% ee
[Säule: Chiralpak OD-H, 5 µm, 250 mm x 4.6 mm; Elutionsmittel: Kohlendioxid/Isopropanol 70:30 (v/v); Fluss: 3 ml/min; UV-Detektion: 210 nm].

### Beispiel 149A

### Methyl-4-{(E/Z)-2-[4-(trifluormethyl)phenyl]vinyl}benzoat

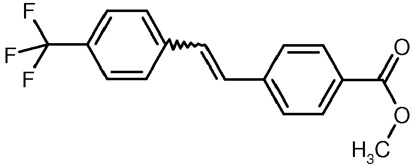

59.13 g (94.36 mmol) [4-(Trifluormethyl)benzyl](triphenyl)phosphoniumbromid und 15.80 g (96.24 mmol) Methyl-4-formylbenzoat wurden in 160 ml Methanol gelöst, auf 0°C abgekühlt und portionsweise mit 5.86 g (108.51 mmol) Natriummethanolat versetzt. Anschließend wurde das Reaktionsgemisch langsam auf Raumtemperatur erwärmt und über Nacht bei dieser Temperatur gerührt. Danach wurde die Reaktionslösung wieder auf 0°C abgekühlt, in Portionen mit weiteren 2.55 g (47.18 mmol) Natriummethanolat versetzt und nach Erwärmen auf Raumtemperatur nochmals über Nacht nachgerührt. Das Reaktionsgemisch wurde danach bis zur Trockene eingeengt und der Rückstand chromatographisch an Kieselgel gereinigt (Laufmittel: Cyclohexan/Essigsäureethylester 10:1). Es wurden 12.39 g (40.45 mmol, 43% d. Th.) der Titelverbindung erhalten.

LC-MS (Methode 2): Rₜ = 2.87 min; m/z = 307 (M+H)⁺.

### Beispiel 150A

### Methyl-4-{2-[4-(trifluormethyl)phenyl]ethyl}benzoat

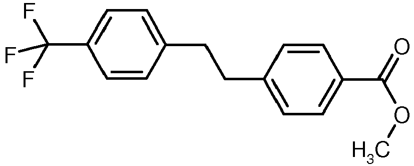

12.3 g (40.16 mmol) Methyl-4-{(*E*/*Z*)-2-[4-(trifluormethyl)phenyl]vinyl}benzoat in 150 ml THF und 150 ml Ethanol wurden mit 427 mg 10% Palladium auf Kohle versetzt und über Nacht bei Raumtemperatur unter einer Wasserstoffatmosphäre bei Normaldruck gerührt. Anschließend wurde das Reaktionsgemisch filtriert und das resultierende Filtrat bis zur Trockene eingeengt. Es wurden 11.52 g (37.37 mmol, 93% d. Th.) der Titelverbindung erhalten.

### LC-MS (Methode 2): Rₜ = 2.86 min; m/z = 309 (M+H)⁺.

¹H-NMR (400 MHz, DMSO-*d₆*, δ/ppm): 2.93-3.05 (m, 4H), 3.83 (s, 3H), 7.38 (d, 2H), 7.45 (d, 2H), 7.63 (d, 2H), 7.87 (d, 2H).

### Beispiel 151A

### (4-{2-[4-(Trifluormethyl)phenyl]ethyl}phenyl)methanol

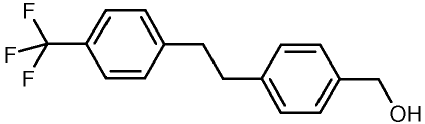

Zu einer Lösung von 11.5 g (37.30 mmol) Methyl-4-{2-[4-(trifluormethyl)phenyl]ethyl}benzoat in 150 ml trockenem THF wurden bei Raumtemperatur unter Argon 12.3 ml einer 1 M Lösung von Lithiumaluminiumhydrid in THF langsam zugetropft. Nach Abschluss der Zugabe wurde das Reaktionsgemisch eine Stunde bei Raumtemperatur nachgerührt. Anschließend wurde das Reaktionsgemisch auf 0°C abgekühlt und langsam und vorsichtig mit 150 ml eisgekühlter 1 M Salzsäure versetzt. Danach wurden ca. 250 ml Essigsäureethylester zugesetzt, die wässrige Phase abgetrennt und die organische Phase nacheinander je einmal mit 1 M Salzsäure und gesättigter Natriumchlorid-Lösung gewaschen. Die organische Phase wurde dann über Magnesiumsulfat getrocknet, filtriert und bis zur Trockene eingeengt. Es wurden 9.69 g (34.57 mmol, 93% d. Th.) der Titelverbindung erhalten.

### LC-MS (Methode 2): Rₜ = 2.55 min; m/z = 263 (M+H-H₂O)⁺.

¹H-NMR (400 MHz, DMSO-*d₆*, δ/ppm): 2.85-2.93 (m, 2H), 2.93-3.02 (m, 2H), 4.45 (d, 2H), 5.09 (t, 1H), 7.19 (q, 4H), 7.45 (d, 2H), 7.62 (d, 2H).

### Beispiel 152A

### 1-(Chlormethyl)-4-{2-[4-(trifluormethyl)phenyl]ethyl}benzol

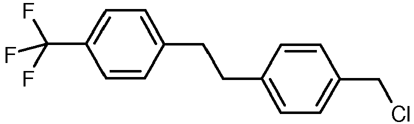

Zu einer Lösung von 9.69 g (34.57 mmol) (4-{2-[4-(Trifluormethyl)phenyl]ethyl}phenyl)methanol in 100 ml Dichlormethan wurden bei 0°C 3.78 ml Thionylchlorid in 30 ml Dichlormethan langsam zugetropft. Nach Abschluss der Zugabe wurde das Reaktionsgemisch auf Raumtemperatur erwärmt und zwei Stunden bei dieser Temperatur nachgerührt. Anschließend wurde das Reaktionsgemisch wieder auf 0°C abgekühlt und langsam und vorsichtig unter starkem Rühren mit 100 ml gesättigter wässriger Natriumhydrogencarbonat-Lösung versetzt, bis ein pH-Wert von 6 erreicht war. Die Phasen wurden dann getrennt, und die organische Phase wurde über Magnesiumsulfat getrocknet, filtriert und bis zur Trockene eingeengt. Es wurden 8.64 g (28.92 mmol, 84% d. Th.) der Titelverbindung erhalten.

GC-MS (Methode 5): Rₜ = 5.96 min; m/z = 298/300 (M+H)⁺.

### Beispiel 153A

### Ethyl-5-[(tert.-butoxycarbonyl)(2-{2-[(4-{2-[4-(trifluormethyl)phenyl]ethyl}benzyl)oxy]phenyl}-ethyl)amino]-5,6,7,8-tetrahydrochinolin-2-carboxylat (Enantiomer 2)

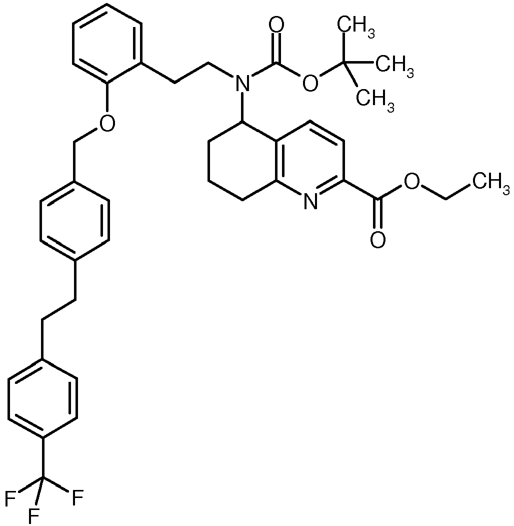

1 g (2.27 mmol) Ethyl-5-{(*tert*.-butoxycarbonyl)[2-(2-hydroxyphenyl)ethyl]amino}-5,6,7,8-tetra-hydrochinolin-2-carboxylat (Enantiomer 2, Beispiel 69A), 746 mg (2.50 mmol) 1-(Chlormethyl)-4-{2-[4-(trifluormethyl)phenyl]ethyl}benzol und 784 mg (5.68 mmol) Kaliumcarbonat in 25 ml Acetonitril wurden auf 110°C erhitzt und über Nacht bei dieser Temperatur gerührt. Nach dem Abkühlen wurde das Reaktionsgemisch filtriert, der Filterkuchen mehrmals mit Acetonitril nachgewaschen und die vereinigten Filtrate am Rotationsverdampfer bis zur Trockene eingeengt. Der erhaltene Rückstand wurde chromatographisch an Kieselgel gereinigt (Laufmittel: Cyclohexan/ Essigsäureethylester 20:1 → 10:1). Es wurden 1110 mg (1.48 mmol, 65% d. Th.) der Zielverbindung erhalten.

LC-MS (Methode 3): Rₜ = 1.66 min; m/z = 703 (M+H)⁺.

### Beispiel 154A

### Ethyl-5-[(2-{2-[(4-{2-[4-(trifluormethyl)phenyl]ethyl}benzyl)oxy]phenyl}ethyl)amino]-5,6,7,8-tetrahydrochinolin-2-carboxylat-Dihydrochlorid (Enantiomer 2)

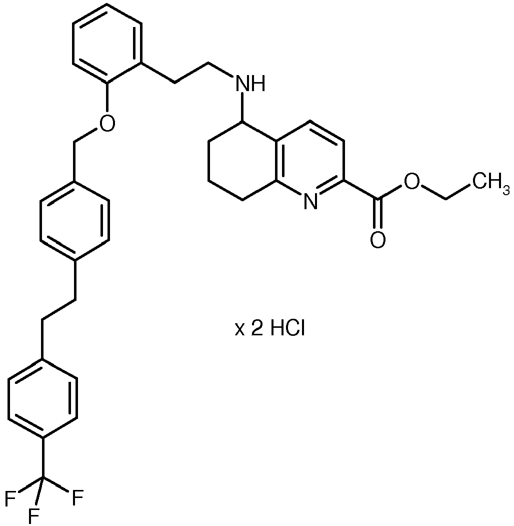

1100 mg (1.57 mmol) Ethyl-5-[(*tert*.-butoxycarbonyl)(2-{2-[(4-{2-[4-(trifluormethyl)phenyl]-ethyl}benzyl)oxy]phenyl}ethyl)amino]-5,6,7,8-tetrahydrochinolin-2-carboxylat (Enantiomer 2, Beispiel 153A) wurden mit 12 ml einer 4 N Lösung von Chlorwasserstoff in Dioxan versetzt und 2 h bei Raumtemperatur gerührt. Anschließend wurde das Reaktionsgemisch bis zur Trockene eingeengt. Es wurden 1045 mg des Zielprodukts erhalten, welches ohne weitere analytische Charakterisierung weiter umgesetzt wurde.

### Beispiel 155A

### Ethyl-5-[(2-{2-[(4-{2-[4-(trifluormethyl)phenyl]ethyl}benzyl)oxy]phenyl}ethyl)amino]-5,6,7,8-tetrahydrochinolin-2-carboxylat (Enantiomer 2)

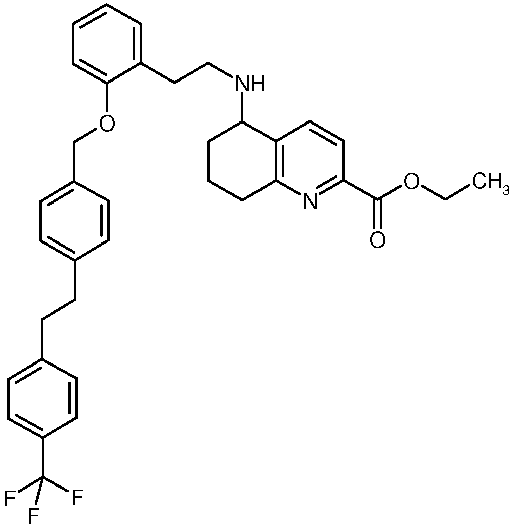

1045 mg (1.55 mmol) Ethyl-5-[(2-{2-[(4-{2-[4-(trifluormethyl)phenyl]ethyl}benzyl)oxy]phenyl}-ethyl)amino]-5,6,7,8-tetrahydrochinolin-2-carboxylat-Dihydrochlorid (Enantiomer 2, Beispiel 154A) wurden in 15 ml THF aufgenommen, mit 0.65 ml Triethylamin versetzt und eine Stunde bei Raumtemperatur gerührt. Anschließend wurde die Reaktionslösung mit Essigsäureethylester und Wasser versetzt, die Phasen getrennt und die wässrige Phase noch einmal mit Essigsäureethylester extrahiert. Die vereinigten organischen Phasen wurden nochmals mit Wasser gewaschen, über Magnesiumsulfat getrocknet, filtriert und anschließend bis zur Trockene eingeengt. Es wurden 800 mg (1.33 mmol, 86% d. Th.) der Zielverbindung erhalten.

LC-MS (Methode 3): Rₜ = 1.03 min; m/z = 603 (M+H)⁺.

### Beispiel 156A

### Ethyl-5-[{2-[4-(methoxycarbonyl)phenyl]ethyl}(2-{2-[(4-{2-[4-(trifluormethyl)phenyl]ethyl}-benzyl)oxy]phenyl}ethyl)amino]-5,6,7,8-tetrahydrochinolin-2-carboxylat (Enantiomer 2)

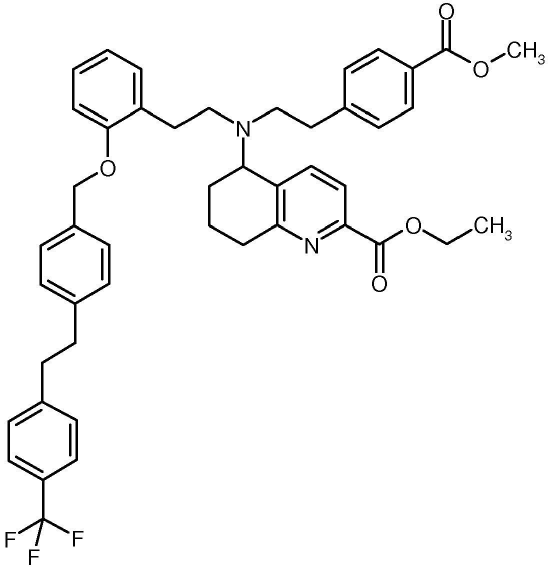

Eine Lösung von 208 mg (0.35 mmol) Ethyl-5-[(2-{2-[(4-{2-[4-(trifluormethyl)phenyl]ethyl}-benzyl)oxy]phenyl}ethyl)amino]-5,6,7,8-tetrahydrochinolin-2-carboxylat (Enantiomer 2, Beispiel 155A) in 3 ml trockenem Acetonitril wurde mit 300 mg (1.04 mmol) Methyl-4-(2-iodethyl)benzoat und 55 mg (0.52 mmol) wasserfreiem Natriumcarbonat versetzt und 4 h lang bei 140°C in einer Mikrowellenapparatur (Biotage Initiator) gerührt. Anschließend wurde die Reaktionslösung abgekühlt und direkt mittels präparativer HPLC gereinigt (Eluent: Acetonitril/Wasser 9:1). Es wurden 102 mg (0.13 mmol, 39% d. Th.) der Titelverbindung erhalten.

LC-MS (Methode 3): Rₜ = 1.64 min; m/z = 765 (M+H)⁺.

### Beispiel 157A

### rac-Ethyl-5-[(tert.-butoxycarbonyl)(2-{5-fluor-2-[(4-{2-[4-(trifluormethyl)phenyl]ethyl}benzyl)-oxy]phenyl}ethyl)amino]-5,6,7,8-tetrahydrochinolin-2-carboxylat

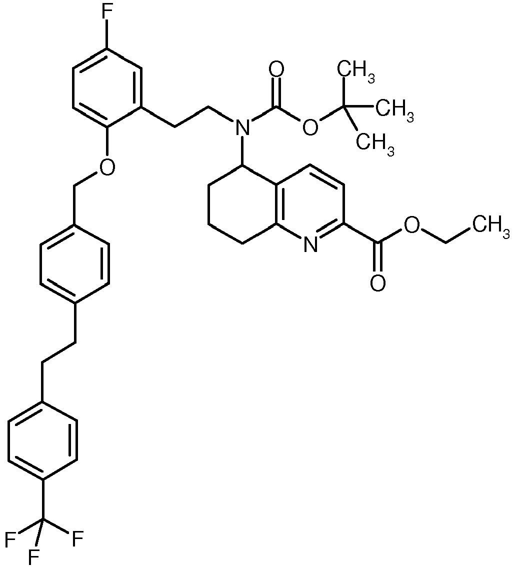

2 g (4.36 mmol) *rac*-Ethyl-5-{(*tert*.-butoxycarbonyl)[2-(5-fluor-2-hydroxyphenyl)ethyl]amino}-5,6,7,8-tetrahydrochinolin-2-carboxylat (Beispiel 106A), 1433 mg (4.80 mmol) 1-(Chlormethyl)-4-{2-[4-(trifluormethyl)phenyl]ethyl}benzol und 1507 mg (10.90 mmol) Kaliumcarbonat in 50 ml Acetonitril wurden auf 110°C erhitzt und über Nacht bei dieser Temperatur gerührt. Nach dem Abkühlen wurde das Reaktionsgemisch filtriert, der Filterkuchen mehrmals mit Acetonitril nachgewaschen und die vereinigten Filtrate am Rotationsverdampfer bis zur Trockene eingeengt. Der erhaltene Rückstand wurde chromatographisch an Kieselgel gereinigt (Laufmittel: Cyclohexan/ Essigsäureethylester 20:1 → 10:1). Es wurden 2490 mg (3.29 mmol, 95% d. Th.) der Zielverbindung erhalten.

LC-MS (Methode 3): Rₜ = 1.60 min; m/z = 721 (M+H)⁺.

### Beispiel 158A

### rac-Ethyl-5-[(2-{5-fluor-2-[(4-{2-[4-(trifluormethyl)phenyl]ethyl}benzyl)oxy]phenyl}ethyl)-amino]-5,6,7,8-tetrahydrochinolin-2-carboxylat-Dihydrochlorid

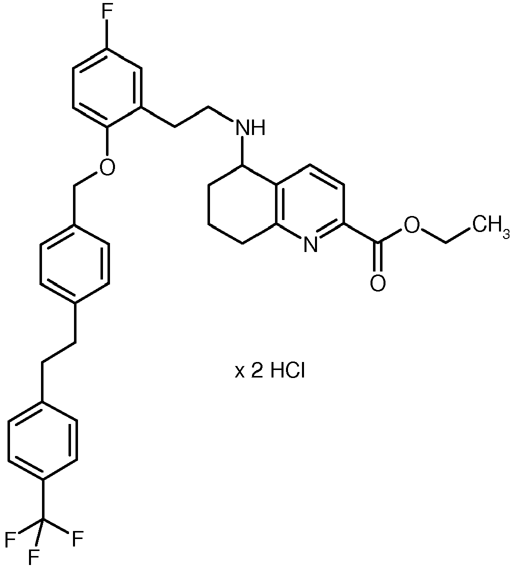

500 mg (0.69 mmol) *rac*-Ethyl-5-[(*tert*.-butoxycarbonyl)(2-{5-fluor-2-[(4-{2-[4-(trifluormethyl)-phenyl]ethyl}benzyl)oxy]phenyl}ethyl)amino]-5,6,7,8-tetrahydrochinolin-2-carboxylat (Beispiel 157A) wurden mit 5.2 ml einer 4 N Lösung von Chlorwasserstoff in Dioxan versetzt und 2 h bei Raumtemperatur gerührt. Anschließend wurde das Reaktionsgemisch bis zur Trockene eingeengt. Es wurden 479 mg des Zielprodukts erhalten, welches ohne weitere analytische Charakterisierung weiter umgesetzt wurde.

### Beispiel 159A

### rac-Ethyl-5-[(2-{5-fluor-2-[(4-{2-[4-(trifluormethyl)phenyl]ethyl}benzyl)oxy]phenyl}ethyl)-amino]-5,6,7,8-tetrahydrochinolin-2-carboxylat

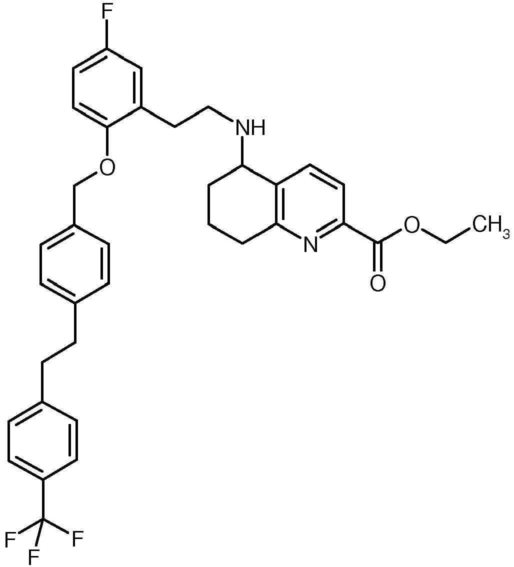

479 mg (0.64 mmol) *rac*-Ethyl-5-[(2-{5-fluor-2-[(4-{2-[4-(trifluormethyl)phenyl]ethyl}benzyl)-oxy]phenyl}ethyl)amino]-5,6,7,8-tetrahydrochinolin-2-carboxylat-Dihydrochlorid (Beispiel 158A) wurden in 4.7 ml THF aufgenommen, mit 0.27 ml Triethylamin versetzt und eine Stunde bei Raumtemperatur gerührt. Anschließend wurde die Reaktionslösung mit Essigsäureethylester und Wasser versetzt, die Phasen getrennt und die wässrige Phase noch einmal mit Essigsäureethylester extrahiert. Die vereinigten organischen Phasen wurden nochmals mit Wasser gewaschen, über Magnesiumsulfat getrocknet, filtriert und anschließend bis zur Trockene eingeengt. Es wurden 383 mg (0.62 mmol, 96% d. Th.) der Zielverbindung erhalten.

LC-MS (Methode 3): Rₜ = 1.04 min; m/z = 621 (M+H)⁺.

### Beispiel 160A

### rac-Ethyl-5-[(2-{5-fluor-2-[(4-{2-[4-(trifluormethyl)phenyl]ethyl}benzyl)oxy]phenyl}ethyl)-{2-[4-(methoxycarbonyl)phenyl]ethyl}amino]-5,6,7,8-tetrahydrochinolin-2-carboxylat

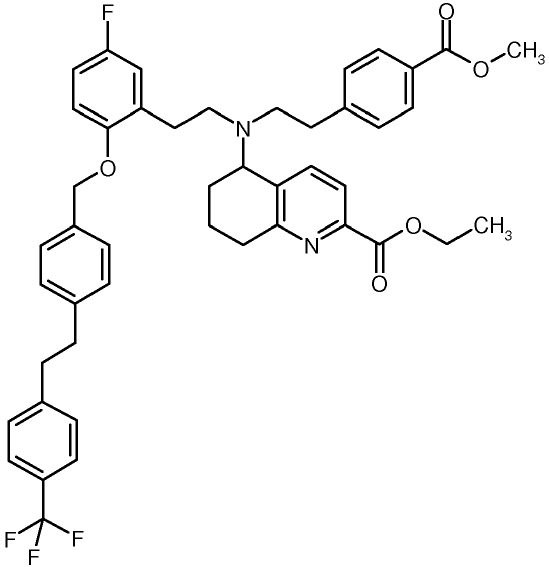

Eine Lösung von 380 mg (0.61 mmol) *rac*-Ethyl-5-[(2-{5-fluor-2-[(4-{2-[4-(trifluormethyl)-phenyl]ethyl}benzyl)oxy]phenyl}ethyl)amino]-5,6,7,8-tetrahydrochinolin-2-carboxylat (Beispiel 159A) in 5 ml trockenem Acetonitril wurde mit 533 mg (1.84 mmol) Methyl-4-(2-iodethyl)benzoat und 97 mg (0.92 mmol) wasserfreiem Natriumcarbonat versetzt und 4 h lang bei 140°C in einer Mikrowellenapparatur (Biotage Initiator) gerührt. Anschließend wurde die Reaktionslösung abgekühlt und direkt mittels präparativer HPLC gereinigt (Eluent: Acetonitril/Wasser 9:1). Es wurden 178 mg (0.23 mmol, 37% d. Th.) der Titelverbindung erhalten.

LC-MS (Methode 3): Rₜ = 1.62 min; m/z = 783 (M+H)⁺.

### Ausführungsbeispiele:

(nur Beispiele, welche unter die Ansprüche fallen, sind erfindungsgemäss)

### Beispiel 1

### (-)-5-{(4-Carboxybutyl)[2-(2-{[4-(5-methyl-1,3-benzoxazol-2-yl)benzyl]oxy}phenyl)ethyl]amino}-5,6,7,8-tetrahydrochinolin-2-carbonsäure (Enantiomer 1)

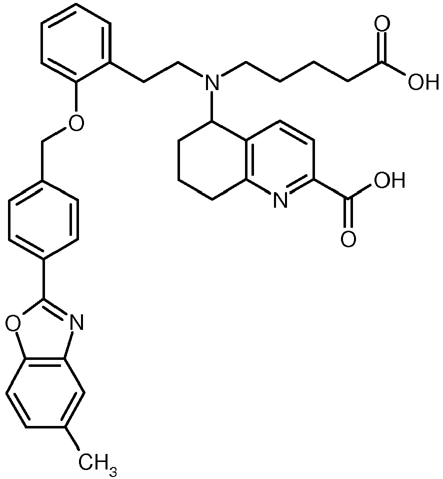

752 mg (1.09 mmol) (-)-Ethyl-5-{(5-ethoxy-5-oxopentyl)[2-(2-{[4-(5-methyl-1,3-benzoxazol-2-yl)-benzyl]oxy}phenyl)ethyl]amino}-5,6,7,8-tetrahydrochinolin-2-carboxylat (Enantiomer 1, Beispiel 35A) wurden in 9 ml THF und 4.6 ml Wasser aufgenommen und mit 137 mg (3.27 mmol) Lithiumhydroxid-Monohydrat versetzt. Der Ansatz wurde über Nacht bei 60°C gerührt. Nach vollständiger Umsetzung wurde das THF am Rotationsverdampfer entfernt und das verbliebene Gemisch mit Wasser verdünnt. Anschließend wurde mit Essigsäure auf pH 4-5 angesäuert und mehrfach mit Essigsäureethylester extrahiert. Die vereinigten organischen Phasen wurden über Magnesiumsulfat getrocknet, filtriert und bis zur Trockene eingeengt. Es wurden 590 mg (0.93 mmol, 86% d. Th.) der Titelverbindung als gelblicher Schaum erhalten.

LC-MS (Methode 3): Rₜ = 1.03 min; m/z = 634 (M+H)⁺.

¹H-NMR (400 MHz, DMSO-*d₆*): δ [ppm] = 1.10-1.71 (m, 7H), 1.89-2.04 (m, 2H), 2.05-2.17 (m, 2H), 2.35-2.64 (m, 3H, teilweise verdeckt durch DMSO-Signal), 2.45 (s, 3H), 2.65-2.88 (m, 4H), 3.94-4.05 (m, 1H), 5.08 (q, 2H), 6.87 (t, 1H), 6.99 (d, 1H), 7.13 (d, 1H), 7.18 (t, 1H), 7.25 (d, 1H), 7.52 (d, 2H), 7.61 (s, 1H), 7.68 (d, 2H), 7.85 (d, 1H), 8.16 (d, 2H), 11.31-12.96 (br. s, 2H).

[α]_{D}²⁰ = -61.61°, c = 0.455, Methanol.

### Beispiel 2

### (+)-5-{(4-Carboxybutyl)[2-(2-{[4-(5-methyl-1,3-benzoxazol-2-yl)benzyl]oxy}phenyl)ethyl]-amino}-5,6,7,8-tetrahydrochinolin-2-carbonsäure (Enantiomer 2)

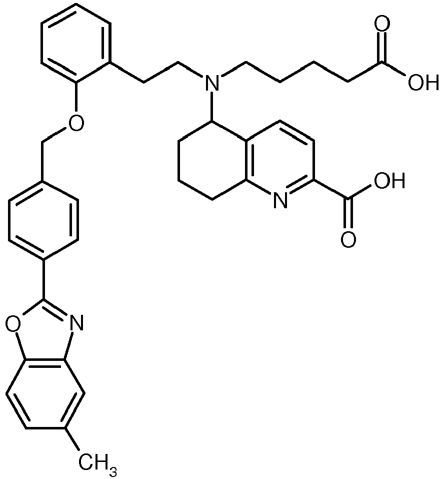

735 mg (1.07 mmol) (+)-Ethyl-5-{(5-ethoxy-5-oxopentyl)[2-(2-{[4-(5-methyl-1,3-benzoxazol-2-yl)benzyl]oxy}phenyl)ethyl]amino}-5,6,7,8-tetrahydrochinolin-2-carboxylat (Enantiomer 2, Beispiel 36A) wurden in 9 ml THF und 4.5 ml Wasser aufgenommen und mit 134 mg (3.20 mmol) Lithiumhydroxid-Monohydrat versetzt. Der Ansatz wurde über Nacht bei 60°C gerührt. Nach vollständiger Umsetzung wurde das THF am Rotationsverdampfer entfernt und das verbliebene Gemisch mit Wasser verdünnt. Anschließend wurde mit Essigsäure auf pH 4-5 angesäuert und mehrfach mit Essigsäureethylester extrahiert. Die vereinigten organischen Phasen wurden über Magnesiumsulfat getrocknet, filtriert und bis zur Trockene eingeengt. Es wurden 617 mg (0.97 mmol, 91% d. Th.) der Titelverbindung als gelblicher Schaum erhalten.

### LC-MS (Methode 3): Rₜ = 1.03 min; m/z = 634 (M+H)⁺.

¹H-NMR (400 MHz, DMSO-*d₆*): δ [ppm] = 1.32-1.70 (m, 7H), 1.89-2.03 (m, 2H), 2.07-2.16 (m, 2H), 2.39-2.64 (m, 3H, teilweise verdeckt durch DMSO-Signal), 2.46 (s, 3H), 2.65-2.87 (m, 4H), 3.95-4.03 (m, 1H), 5.08 (q, 2H), 6.87 (t, 1H), 6.99 (d, 1H), 7.13 (d, 1H), 7.18 (t, 1H), 7.25 (d, 1H), 7.52 (d, 2H), 7.61 (s, 1H), 7.67 (d, 2H), 7.85 (d, 1H), 8.16 (d, 2H), 11.30-12.97 (br. s, 2H).

[α]_{D}²⁰ = +62.89°, c = 0.380, Methanol.

Analog zu den Beispielen 1 und 2 wurden die folgenden Verbindungen hergestellt:

| **Beispiel** | **Name / Struktur / Edukt** | **Analytische Daten** |
|---|---|---|
| **3** | 5-[(2-{2-[(4-*tert*.-Butylbenzyl)oxy]phenyl}-ethyl)(4-carboxybutyl)amino]-5,6,7,8-tetrahydro-chinolin-2-carbonsäure (*Enantiomer 1*) | LC-MS (Methode 4): Rₜ = 1.00 min; m/z = 559 (M+H)⁺. |
| | 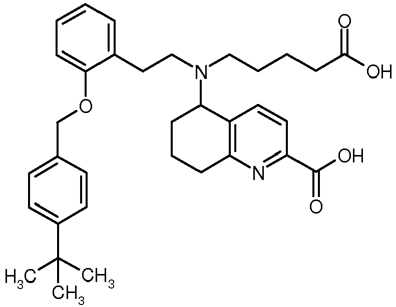 | ¹H-NMR (400 MHz, DMSO-*d₆*): δ [ppm] = 1.20-1.31 (m, 1H), 1.27 (s, 9H), 1.33-1.67 (m, 6H), 1.88-2.02 (m, 2H), 2.06-2.16 (m, 2H), 2.35-2.59 (m, 3H, teilweise verdeckt durch DMSO-Signal), 2.59-2.84 (m, 4H), 3.91-4.01 (m, 1H), 4.85-4.98 (m, 2H), 6.84 (t, 1H), 6.98 (d, 1H), 7.10 (d, 1H), 7.16 (t, 1H), 7.22 (d, 1H), 7.35 (d, 2H), 7.67 (d, 1H), 7.85 (d, 1H), 11.35-13.30 (br. s, ca. 2H). |
| | aus Ethyl-5-[(2-{2-[(4-*tert*.-butylbenzyl)oxy]-phenyl}ethyl)(5-ethoxy-5-oxopentyl)amino]-5,6,7,8-tetrahydrochinolin-2-carboxylat (*Enantiomer 1*) | |
| **4** | 5-[(2-{2-[(4-*tert*.-Butylbenzyl)oxy]phenyl}-ethyl)(4-carboxybutyl)amino]-5,6,7,8-tetrahydro-chinolin-2-carbonsäure (*Enantiomer 2*) | LC-MS (Methode 3): Rₜ = 1.00 min; m/z = 559 (M+H)⁺. |
| | 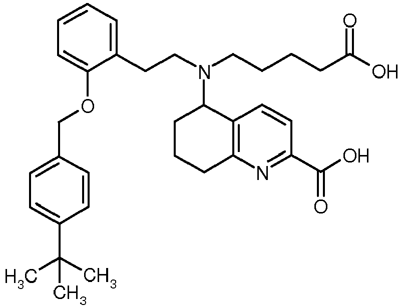 | ¹H-NMR (400 MHz, DMSO-*d₆*): δ [ppm] = 1.22-1.31 (m, 1H), 1.27 (s, 9H), 1.32-1.67 (m, 6H), 1.89-2.02 (m, 2H), 2.07-2.17 (m, 2H), 2.35-2.59 (m, 3H, teilweise verdeckt durch DMSO-Signal), 2.59-2.84 (m, 4H), 3.93-4.01 (m, 1H), 4.85-4.98 (m, 2H), 6.84 (t, 1H), 6.98 (d, 1H), 7.10 (d, 1H), 7.16 (t, 1H), 7.22 (d, 1H), 7.35 (d, 2H), 7.67 (d, 1H), 7.85 (d, 1H), 11.16-12.92 (br. s, ca. 2H). |
| | aus Ethyl-5-[(2-{2-[(4-*tert*.-butylbenzyl)oxy]-phenyl}ethyl)(5-ethoxy-5-oxopentyl)amino]-5,6,7,8-tetrahydrochinolin-2-carboxylat (*Enantiomer* 2) | |
| **5** | *rac*-5-{(4-Carboxybutyl)\|2-(2-{[4-(1-propionylpiperidin-4-yl)benzyl]oxy}phenyl)ethyl]amino}-5,6,7,8-tetrahydrochinolin-2-carbonsäure | LC-MS (Methode 3): Rₜ = 0.86 min; m/z = 642 (M+H)⁺. |
| | 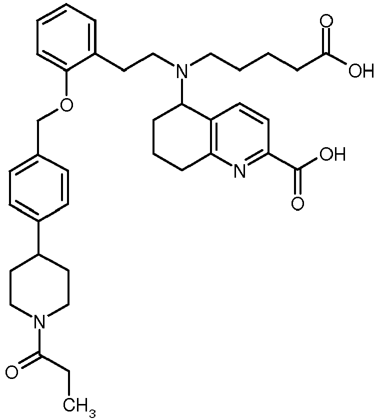 | ¹H-NMR (400 MHz, DMSO-*d₆*): δ [ppm] = 1.01 (t, 3H), 1.31-1.66 (m, 8H), 1.70-1.84 (m, 2H), 1.88-2.02 (m, 2H), 2.06-2.16 (m, 2H), 2.29-2.39 (m, 2H), 2.39-2.47 (m, 2H), 2.47-2.84 (m, 8H, teilweise verdeckt durch DMSO-Signal), 3.01-3.14 (m, 1H), 3.88-4.02 (m, 2H), 4.48-4.60 (m, 1H), 4.84-4.99 (m, 2H), 6.80-6.88 (m, 1H), 6.94-7.01 (m, 1H), 7.07-7.30 (m, 6H), 7.62-7.69 (m, 1H), 7.78-7.87 (m, 1H), 11.51-13.13 (br. s, ca. 2H). |
| | aus Ethyl-5-{(5-ethoxy-5-oxopentyl)[2-(2-{[4-(1-propionylpiperidin-4-yl)benzyl]oxy}phenyl)-ethyl]amino}-5,6,7,8-tetrahydrochinolin-2-carboxylat | |
| **6** | *rac*-5-[(4-Carboxybutyl)(2-{2-[(4-cyclohexylbenzyl)oxy]phenyl}ethyl)amino]-5,6,7,8-tetrahydrochinolin-2-carbonsäure | ¹H-NMR (400 MHz, DMSO-*d₆*): δ [ppm] = 1.14-1.65 (m, 13H), 1.65-1.83 (m, 5H), 1.87-2.02 (m, 2H), 2.07-2.16 (m, 2H), 2.35-2.84 (m, 7H, teilweise verdeckt durch DMSO-Signal), 3.92-4.01 (m, 1H), 4.83-4.96 (m, 2H), 6.84 (t, 1H), 6.98 (d, 1H), 7.10 (d, 1H), 7.13-7.22 (m, 5H), 7.66 (d, 1H), 7.84 (d, 1H), 11.06-13.55 (br. s, ca. 2H). |
| | 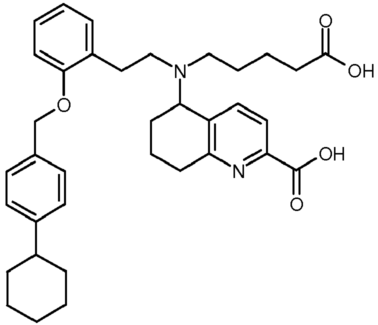 | |
| | aus Ethyl-5-[(2-{2-[(4-cyclohexylbenzyl)oxy]-phenyl}ethyl)(5-ethoxy-5-oxopentyl)amino]-5,6,7,8-tetrahydrochinolin-2-carboxylat | |
| **7** | 5-{(4-Carboxybutyl)[2-(2-{[3-chlor-4'-(trifluormethyl)biphenyl-4-yl]methoxy}phenyl)ethyl]-amino}-5,6,7,8-tetrahydrochinolin-2-carbonsäure (*Enantiomer 1*) | LC-MS (Methode 3): Rₜ = 1.18 min; m/z = 681 (M+H)⁺. |
| | 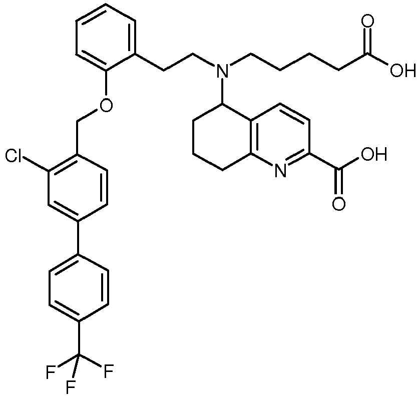 | ¹H-NMR (400 MHz, DMSO-*d₆*):δ [ppm] = 1.20-1.30 (m, 1H), 1.31-1.62 (m, 6H), 1.86-1.99 (m, 2H), 2.06-2.16 (m, 2H), 2.35-2.46 (m, 2H), 2.46-2.60 (m, 1H, teilweise verdeckt durch DMSO-Signal), 2.60-2.83 (m, 4H), 3.90-3.99 (m, 1H), 5.00-5.14 (m, 2H), 6.89 (t, 1H), 7.03 (d, 1H), 7.14 (d, 1H), 7.21 (t, 1H), 7.54 (d, 1H), 7.65 (d, 1H), 7.70 (dd, 1H), 7.78-7.89 (m, 4H), 7.95 (d, 2H), 11.29-12.89 (br. s, ca. 2H). |
| | aus Ethyl-5-{[2-(2-{[3-chlor-4'-(trifluormethyl)-biphenyl-4-yl]methoxy}phenyl)ethyl](5-ethoxy-5-oxopentyl)amino}-5,6,7,8-tetrahydrochinolin-2-carboxylat (*Enantiomer 1*) | |
| **8** | 5-{(4-Carboxybutyl)[2-(2-{[3-chlor-4'-(trifluormethyl)biphenyl-4-yl]methoxy}phenyl)ethyl]-amino}-5,6,7,8-tetrahydrochinolin-2-carbonsäure (*Enantiomer 2)* | LC-MS (Methode 3): Rₜ = 1.17 min; m/z = 681 (M+H)⁺. |
| | 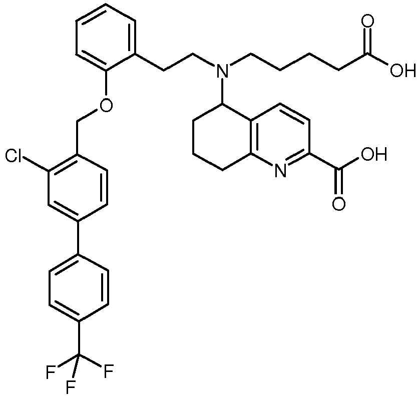 | ¹H-NMR (400 MHz, DMSO-*d₆*):δ [ppm] = 1.21-1.29 (m, 1H), 1.32-1.64 (m, 6H), 1.86-2.00 (m, 2H), 2.06-2.15 (m, 2H), 2.36-2.47 (m, 2H), 2.47-2.60 (m, 1H, teilweise verdeckt durch DMSO-Signal), 2.60-2.84 (m, 4H), 3.90-4.00 (m, 1H), 5.01-5.14 (m, 2H), 6.89 (t, 1H), 7.03 (d, 1H), 7.14 (d, 1H), 7.21 (t, 1H), 7.54 (d, 1H), 7.65 (d, 1H), 7.70 (dd, 1H), 7.79-7.89 (m, 4H), 7.95 (d, 2H), 11.35-12.90 (br. s, 2H). |
| | aus Ethyl-5-{[2-(2-{[3-chlor-4'-(trifluormethyl)-biphenyl-4-yl]methoxy}phenyl)ethyl](5-ethoxy-5-oxopentyl)amino}-5,6,7,8-tetrahydrochinolin-2-carboxylat (*Enantiomer 2)* | |
| **9** | 5-[(4-Carboxybutyl)(2-{2-[(5-phenylpentyl)oxy]-phenyl}ethyl)amino]-5,6,7,8-tetrahydrochinolin-2-carbonsäure (*Enantiomer 1*) | LC-MS (Methode 3): Rₜ = 1.03 min; m/z = 559 (M+H)⁺. |
| | 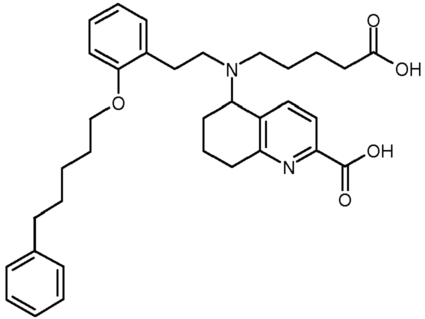 | ¹H-NMR (400 MHz, DMSO-*d₆*): δ [ppm] = 1.09-1.77 (m, 16H), 1.94-2.08 (m, 2H), 2.12-2.22 (m, 2H), 2.38-2.64 (m, 3H, teilweise verdeckt durch DMSO-Signal), 2.65-2.76 (m, 1H), 2.78-2.88 (m, 2H), 3.69-3.79 (m, 1H), 3.79-3.88 (m, 1H), 3.95-4.05 (m, 1H), 6.76-6.88 (m, 2H), 7.07 (d, 1H), 7.10-7.20 (m, 4H), 7.22-7.29 (m, 2H), 7.68 (d, 1H), 7.82 (d, 1H), 11.05-13.54 (br. s, ca. 2H). |
| | aus Ethyl-5-[(5-ethoxy-5-oxopentyl)(2-{2-[(5-phenylpentyl)oxy]phenyl}ethyl)amino]-5,6,7,8-tetrahydrochinolin-2-carboxylat (*Enantiomer 1*) | |
| **10** | 5-[(4-Carboxybutyl)(2-{2-[(5-phenylpentyl)oxy]-phenyl}ethyl)amino]-5,6,7,8-tetrahydrochinolin-2-carbonsäure (*Enantiomer 2*) | LC-MS (Methode 3): Rₜ = 1.02 min; m/z = 559 (M+H)⁺. |
| | 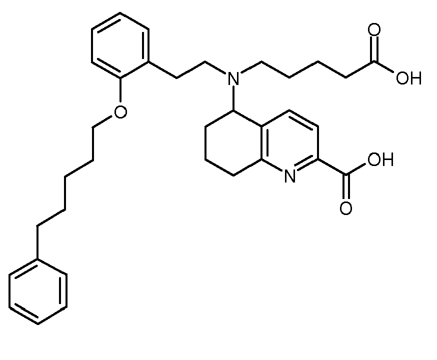 | ¹H-NMR (400 MHz, DMSO-*d₆*): δ [ppm] = 1.08-1.78 (m, 16H), 1.94-2.07 (m, 2H), 2.11-2.23 (m, 2H), 2.39-2.65 (m, 3H, teilweise verdeckt durch DMSO-Signal), 2.65-2.76 (m, 1H), 2.77-2.89 (m, 2H), 3.69-3.80 (m, 1H), 3.80-3.89 (m, 1H), 3.95-4.05 (m, 1H), 6.75-6.89 (m, 2H), 7.07 (d, 1H), 7.10-7.20 (m, 4H), 7.21-7.30 (m, 2H), 7.68 (d, 1H), 7.82 (d, 1H), 11.03-12.99 (br. s, ca. 2H). |
| | aus Ethyl-5-[(5-ethoxy-5-oxopentyl)(2-{2-[(5-phenylpentyl)oxy]phenyl}ethyl)amino]-5,6,7,8-tetrahydrochinolin-2-carboxylat (*Enantiomer* 2) | |
| **11** | *rac*-5-[(4-Carboxybutyl){2-[2-({4-[*trans*-4-(trifluormethyl)cyclohexyl]benzyl}oxy)phenyl]-ethyl}amino]-5,6,7,8-tetrahydrochinolin-2-carbonsäure | LC-MS (Methode 3): Rₜ = 1.12 min; m/z = 653 (M+H)⁺. |
| | 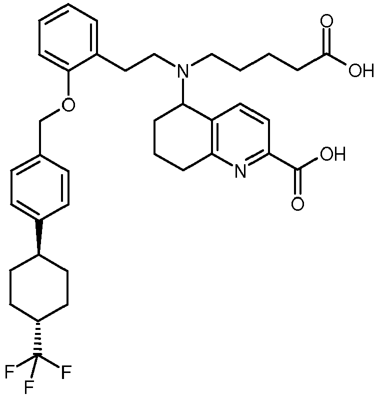 | ¹H-NMR (400 MHz, DMSO-*d₆*): δ [ppm] = 1.14-1.65 (m, 12H), 1.79-2.02 (m, 6H), 2.05-2.16 (m, 2H), 2.28-2.59 (m, 4H, teilweise verdeckt durch DMSO-Signal), 2.59-2.70 (m, 1H), 2.70-2.84 (m, 3H), 3.91-3.99 (m, 1H), 4.83-4.98 (m, 2H), 6.84 (t, 1H), 6.98 (d, 1H), 7.10 (d, 1H), 7.12-7.25 (m, 5H), 7.66 (d, 1H), 7.84 (d, 1H), 11.39-13.00 (br. s, 2H). |
| | aus Ethyl-5-[(5-ethoxy-5-oxopentyl){2-[2-({4-[*trans*-4-(trifluormethyl)cyclohexyl]benzyl}oxy)-phenyl]ethyl}amino]-5,6,7,8-tetrahydrochinolin-2-carboxylat | |

### Beispiel 12

### rac-5-{(2-{2-[(4-tert.-Butylbenzyl)oxy]phenyl}ethyl)[2-(4-carboxyphenyl)ethyl]amino}-5,6,7,8-tetrahydrochinolin-2-carbonsäure

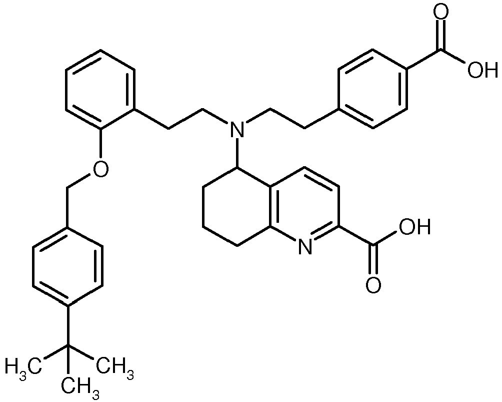

35 mg (0.05 mmol) Ethyl-5-[(2-{2-[(4-*tert*.-butylbenzyl)oxy]phenyl}ethyl){2-[4-(methoxycarbonyl)phenyl]ethyl}amino]-5,6,7,8-tetrahydrochinolin-2-carboxylat (Beispiel 43A) wurden in 1 ml THF und 1 ml Wasser aufgenommen und mit 7 mg (0.16 mmol) Lithiumhydroxid-Monohydrat versetzt. Der Ansatz wurde über Nacht bei 50°C gerührt. Nach vollständiger Umsetzung wurde das THF am Rotationsverdampfer entfernt und das verbliebene Gemisch mit Wasser verdünnt. Anschließend wurde mit 1 M Salzsäure angesäuert und mehrfach mit einem 1:1-Gemisch aus Essigsäureethylester und Dichlormethan extrahiert. Die vereinigten organischen Phasen wurden über Magnesiumsulfat getrocknet, filtriert und bis zur Trockene eingeengt. Es wurden 29 mg (0.04 mmol, Gehalt 91%, 81% d. Th.) der Titelverbindung als gelblicher Feststoff erhalten.

LC-MS (Methode 4): Rₜ = 1.29 min; m/z = 607 (M+H)⁺.

¹H-NMR (400 MHz, DMSO-*d₆*): δ [ppm] = 0.77-0.90 (m, 0.5H), 1.12-1.31 (m, 10H), 1.42-1.87 (m, 2H), 1.88-2.14 (m, 2H), 2.22-2.34 (m, 0.5H), 2.41-3.07 (m, 7H, teilweise verdeckt durch DMSO-Signal), 3.98-4.11 (m, 0.5H), 4.84-5.13 (m, 2H), 5.13-5.26 (m, 0.5H), 6.79-6.89 (m, 0.5H), 6.95-7.52 (m, 11H), 7.55-7.62 (m, 0.5H), 7.74-7.88 (m, 2H), 7.90-8.00 (m, 1H), 8.54-8.68 (m, 0.5H), 10.39-10.58 (m, 0.5H), 11.69-14.09 (br. s, ca. 1H).

### Beispiel 13

### 5-{(4-Carboxybutyl)[2-(2-{[4-(2-phenylethyl)benzyl]oxy}phenyl)ethyl]amino}-5,6,7,8-tetrahydrochinolin-2-carbonsäure (Enantiomer 1)

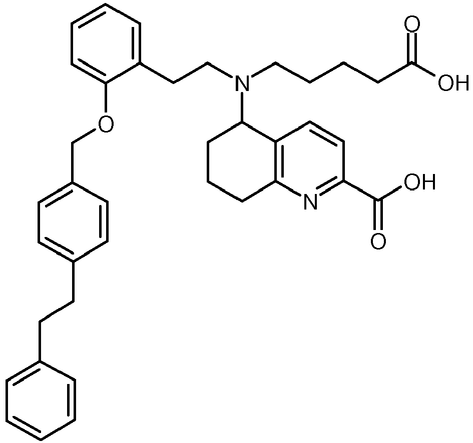

259 mg (0.39 mmol) Ethyl-5-{(5-ethoxy-5-oxopentyl)[2-(2-{[4-(2-phenylethyl)benzyl]oxy}-phenyl)ethyl]amino}-5,6,7,8-tetrahydrochinolin-2-carboxylat (Enantiomer 1, Beispiel 50A) wurden in 4 ml Dioxan aufgenommen, mit 2 ml einer 2 M Lösung von Kaliumhydroxid in Wasser versetzt und über Nacht bei Raumtemperatur gerührt. Nach vollständiger Umsetzung wurde das Reaktionsgemisch mit 0.75 ml Essigsäure und mit 1 N Salzsäure leicht sauer gestellt und dann bis zur Trockene eingeengt. Der erhaltene Rückstand wurde mittels präparativer HPLC gereinigt. Es wurden 183 mg (0.30 mmol, 77% d. Th.) der Titelverbindung erhalten.

LC-MS (Methode 4): Rₜ = 1.02 min; m/z = 607 (M+H)⁺.

¹H-NMR (400 MHz, DMSO-*d₆*): δ [ppm] = 1.30-1.68 (m, 6H), 1.89-2.04 (m, 2H), 2.08-2.18 (m, 2H), 2.39-2.47 (m, 2H), 2.47-2.58 (m, 1H, verdeckt durch DMSO-Signal), 2.58-2.84 (m, 5H), 2.86 (s, 4H), 3.92-4.01 (m, 1H), 4.82-4.97 (m, 2H), 6.84 (t, 1H), 6.97 (d, 1H), 7.10 (d, 1H), 7.13-7.21 (m, 6H), 7.21-7.30 (m, 4H), 7.66 (d, 1H), 7.83 (d, 1H), 11.20-13.00 (br. s, ca. 2H).

Analog zu Beispiel 13 wurden die folgenden Verbindungen hergestellt:

| **Beispiel** | **Name / Struktur / Edukt** | **Analytische Daten** |
|---|---|---|
| **14** | 5-{(4-Carboxybutyl)[2-(2-{[4-(2-phenylethyl)-benzyl]oxy}phenyl)ethyl]amino}-5,6,7,8-tetra-hydrochinolin-2-carbonsäure (*Enantiomer 2*) | LC-MS (Methode 1): Rₜ = 1.08 min; m/z = 607 (M+H)⁺. |
| | 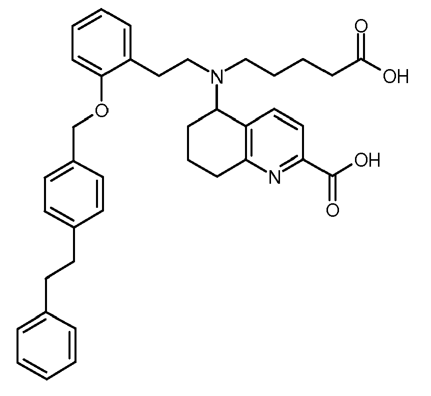 | ¹H-NMR (400 MHz, DMSO-*d₆*): δ [ppm] = 1.31-1.69 (m, 6H), 1.90-2.02 (m, 2H), 2.08-2.17 (m, 2H), 2.39-2.46 (m, 2H), 2.46-2.58 (m, 1H, verdeckt durch DMSO-Signal), 2.58-2.83 (m, 5H), 2.86 (s, 4H), 3.92-4.01 (m, 1H), 4.83-4.97 (m, 2H), 6.84 (t, 1H), 6.97 (d, 1H), 7.10 (d, 1H), 7.13-7.21 (m, 6H), 7.21-7.30 (m, 4H), 7.66 (d, 1H), 7.82 (d, 1H), 11.36-12.90 (br. s, ca. 2H). |
| | aus Ethyl-5-{(5-ethoxy-5-oxopentyl)[2-(2-{[4-(2-phenylethyl)benzyl]oxy}phenyl)ethyl]amino}-5,6,7,8-tetrahydrochinolin-2-carboxylat (*Enantiomer 2*) | |
| **15** | 5-[(4-Carboxybutyl){2-[2-({4-[2-(4-fluorphenyl)-ethyl]benzyl}oxy)phenyl]ethyl}amino]-5,6,7,8-tetrahydrochinolin-2-carbonsäure (*Enantiomer 1*) | LC-MS (Methode 4): Rₜ = 1.03 min; m/z = 625 (M+H)⁺. |
| | 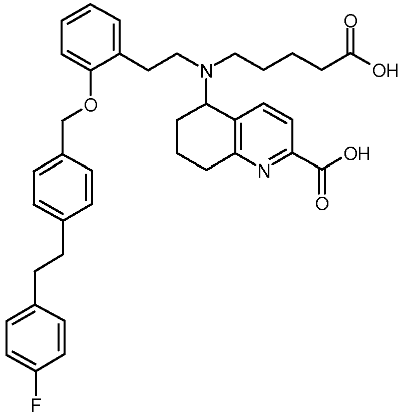 | ¹H-NMR (400 MHz, DMSO-*d₆*): δ [ppm] = 1.31-1.54 (m, 5H), 1.54-1.69 (m, 1H), 1.89-2.02 (m, 2H), 2.08-2.17 (m, 2H), 2.37 (s, 2H), 2.40-2.70 (m, 2H, teilweise verdeckt durch DMSO-Signal), 2.71 (s, 2H), 2.77-2.83 (m, 2H), 2.85 (s, 4H), 3.92-4.00 (m, 1H), 4.82-4.97 (m, 2H), 6.84 (t, 1H), 6.97 (d, 1H), 7.04-7.12 (m, 3H), 7.13-7.21 (m, 5H), 7.22-7.29 (m, 2H), 7.66 (d, 1H), 7.82 (d, 1H). |
| | aus Ethyl-5-[(5-ethoxy-5-oxopentyl){2-[2-({4-[2-(4-fluorphenyl)ethyl]benzyl}oxy)phenyl]ethyl}-amino]-5,6,7,8-tetrahydrochinolin-2-carboxylat (*Enantiomer 1*) | |
| **16** | 5-[(4-Carboxybutyl){2-[2-({4-[2-(4-fluorphenyl)-ethyl]benzyl}oxy)phenyl]ethyl}amino]-5,6,7,8-tetrahydrochinolin-2-carbonsäure (*Enantiomer 2*) | LC-MS (Methode 4): Rₜ = 1.03 min; m/z = 625 (M+H)⁺. |
| | 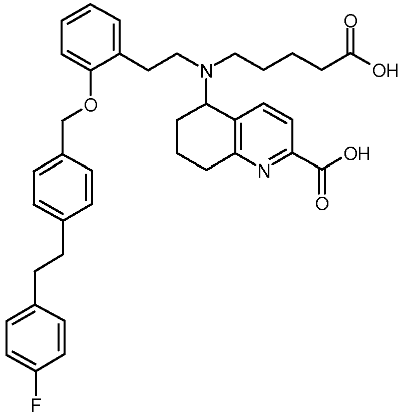 | |
| | aus Ethyl-5-[(5-ethoxy-5-oxopentyl){2-[2-({4-[2-(4-fluorphenyl)ethyl]benzyl}oxy)phenyl]ethyl}-amino]-5,6,7,8-tetrahydrochinolin-2-carboxylat (*Enantiomer* 2) | |
| **17** | 5-{(4-Carboxybutyl)[2-(2-{[4'-(trifluormethyl)-biphenyl-4-yl]methoxy}phenyl)ethyl]amino}-5,6,7,8-tetrahydrochinolin-2-carbonsäure (*Enantiomer 1*) | LC-MS (Methode 1): Rₜ = 1.04 min; m/z = 647 (M+H)⁺. |
| | 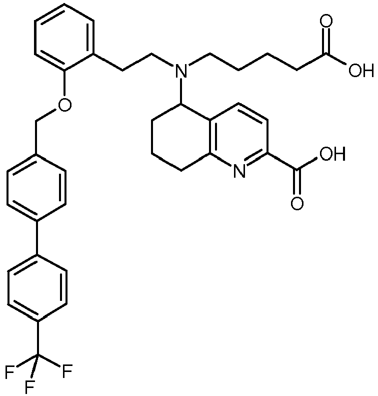 | ¹H-NMR (400 MHz, DMSO-*d₆*): δ [ppm] = 1.34-1.68 (m, 6H), 1.88-2.03 (m, 2H), 2.08-2.18 (m, 2H), 2.41-2.63 (m, 4H, teilweise verdeckt durch DMSO-Signal), 2.64-2.74 (m, 1H), 2.74-2.85 (m, 3H), 3.94-4.02 (m, 1H), 4.97-5.11 (m, 2H), 6.86 (t, 1H), 7.00 (d, 1H), 7.13 (d, 1H), 7.18 (t, 1H), 7.42 (d, 2H), 7.64-7.73 (m, 3H), 7.79-7.93 (m, 5H), 11.26-12.64 (br. s, ca. 2H). |
| | aus Ethyl-5-{(5-ethoxy-5-oxopentyl)[2-(2-{[4'-(trifluormethyl)biphenyl-4-yl]methoxy}phenyl)-ethyl]amino}-5,6,7,8-tetrahydrochinolin-2-carboxylat (*Enantiomer 1*) | |
| **18** | 5-{(4-Carboxybutyl)[2-(2-{[4'-(trifluormethyl)-biphenyl-4-yl]methoxy}phenyl)ethyl]amino}-5,6,7,8-tetrahydrochinolin-2-carbonsäure (*Enantiomer 2*) | LC-MS (Methode 4): Rₜ = 1.05 min; m/z = 647 (M+H)⁺. |
| | 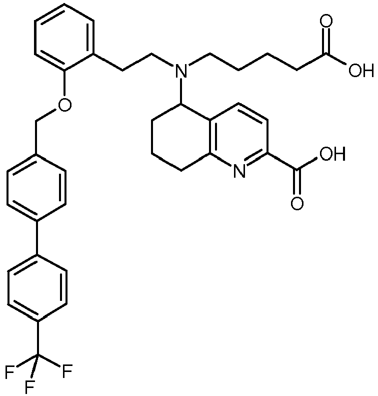 | ¹H-NMR (400 MHz, DMSO-*d₆*): δ [ppm] = 1.33-1.68 (m, 6H), 1.88-2.03 (m, 2H), 2.09-2.18 (m, 2H), 2.40-2.63 (m, 4H, teilweise verdeckt durch DMSO-Signal), 2.64-2.74 (m, 1H), 2.74-2.85 (m, 3H), 3.94-4.03 (m, 1H), 4.96-5.10 (m, 2H), 6.86 (t, 1H), 7.00 (d, 1H), 7.13 (d, 1H), 7.18 (t, 1H), 7.42 (d, 2H), 7.64-7.74 (m, 3H), 7.78-7.93 (m, 5H), 11.20-12.71 (br. s, ca. 2H). |
| | aus Ethyl-5-{(5-ethoxy-5-oxopentyl)[2-(2-{[4'-(trifluormethyl)biphenyl-4-yl]methoxy}phenyl)-ethyl]amino}-5,6,7,8-tetrahydrochinolin-2-carboxylat (*Enantiomer 2*) | |

### Beispiel 19

### 5-{[2-(4-Carboxyphenyl)ethyl][2-(2-{[4-(5-chlor-1,3-benzoxazol-2-yl)benzyl]oxy}phenyl)ethyl]-amino}-5,6,7,8-tetrahydrochinolin-2-carbonsäure (Enantiomer 1)

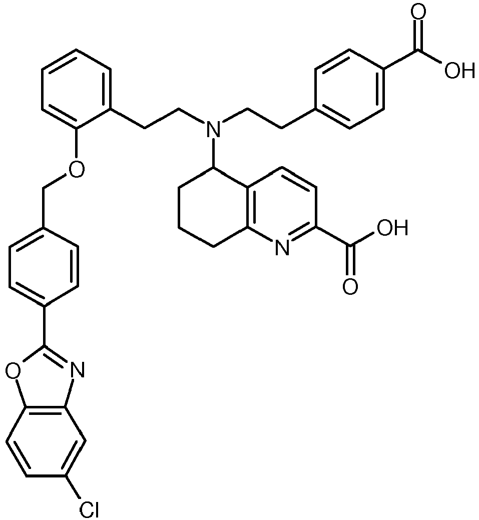

68 mg (0.09 mmol) Ethyl-5-([2-(2-{[4-(5-chlor-1,3-benzoxazol-2-yl)benzyl]oxy}phenyl)ethyl]{2-[4-(methoxycarbonyl)phenyl]ethyl}amino)-5,6,7,8-tetrahydrochinolin-2-carboxylat (Enantiomer 1, Beispiel 63A) wurden in 4 ml THF und 2 ml Wasser aufgenommen und mit 12 mg (0.27 mmol) Lithiumhydroxid-Monohydrat versetzt. Der Ansatz wurde über Nacht bei 60°C gerührt. Nach vollständiger Umsetzung wurde das THF am Rotationsverdampfer entfernt und das verbliebene Gemisch mit Wasser verdünnt. Anschließend wurde mit Essigsäure auf pH 4-5 angesäuert und mehrfach mit Essigsäureethylester extrahiert. Die vereinigten organischen Phasen wurden über Magnesiumsulfat getrocknet, filtriert und bis zur Trockene eingeengt. Es wurden 33 mg (0.04 mmol, 48% d. Th.) der Titelverbindung erhalten.

LC-MS (Methode 3): Rₜ = 1.26 min; m/z = 702/704 (M+H)⁺.

¹H-NMR (400 MHz, DMSO-*d₆*): δ [ppm] = 1.41-1.55 (m, 1H), 1.55-1.70 (m, 1H), 1.89-2.08 (m, 2H), 2.59-2.87 (m, 10H), 4.01-4.14 (m, 1H), 5.01-5.15 (m, 2H), 6.86 (t, 1H), 7.01 (d, 1H), 7.06 (d, 1H), 7.14 (d, 2H), 7.20 (t, 1H), 7.42-7.57 (m, 5H), 7.76 (d, 2H), 7.83 (d, 1H), 7.93 (d, 1H), 8.11 (d, 2H), 12.03-13.45 (br. s, ca. 2H).

### Beispiel 20

### 5-{[2-(4-Carboxyphenyl)ethyl][2-(2-{[4-(5-chlor-1,3-benzoxazol-2-yl)benzyl]oxy}phenyl)ethyl]-amino}-5,6,7,8-tetrahydrochinolin-2-carbonsäure (Enantiomer 2)

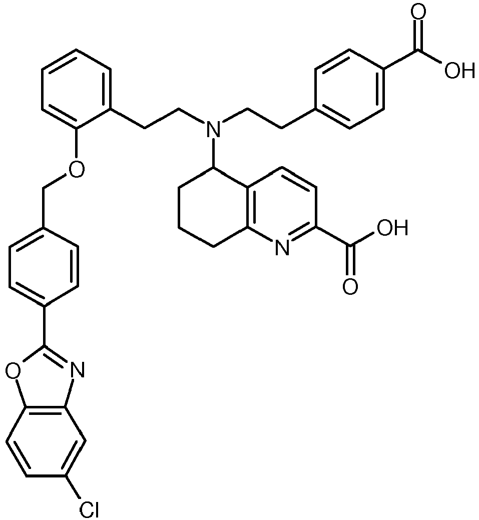

45 mg (0.06 mmol) Ethyl-5-([2-(2-{[4-(5-chlor-1,3-benzoxazol-2-yl)benzyl]oxy}phenyl)ethyl]{2-[4-(methoxycarbonyl)phenyl]ethyl}amino)-5,6,7,8-tetrahydrochinolin-2-carboxylat (Enantiomer 2, Beispiel 64A) wurden in 4 ml THF und 2 ml Wasser aufgenommen und mit 8 mg (0.18 mmol) Lithiumhydroxid-Monohydrat versetzt. Der Ansatz wurde über Nacht bei 60°C gerührt. Nach vollständiger Umsetzung wurde das THF am Rotationsverdampfer entfernt und das verbliebene Gemisch mit Wasser verdünnt. Anschließend wurde mit Essigsäure auf pH 4-5 angesäuert und mehrfach mit Essigsäureethylester extrahiert. Die vereinigten organischen Phasen wurden über Magnesiumsulfat getrocknet, filtriert und bis zur Trockene eingeengt. Es wurden 13 mg (0.02 mmol, 32% d. Th.) der Titelverbindung erhalten.

LC-MS (Methode 3): Rₜ = 1.26 min; m/z = 702/704 (M+H)⁺.

¹H-NMR (400 MHz, DMSO-*d₆*): δ [ppm] = 1.40-1.55 (m, 1H), 1.55-1.70 (m, 1H), 1.88-2.08 (m, 2H), 2.58-2.87 (m, 10H), 4.02-4.13 (m, 1H), 5.01-5.15 (m, 2H), 6.86 (t, 1H), 7.02 (d, 1H), 7.06 (d, 1H), 7.14 (d, 2H), 7.20 (t, 1H), 7.42-7.57 (m, 5H), 7.76 (d, 2H), 7.84 (d, 1H), 7.93 (d, 1H), 8.11 (d, 2H), 11.69-13.84 (br. s, ca. 2H).

Analog zu Beispiel 20 wurden die folgenden Verbindungen hergestellt:

| **Beispiel** | **Name / Struktur / Edukt** | **Analytische Daten** |
|---|---|---|
| **21** | 5-{[2-(4-Carboxyphenyl)ethyl][2-(2-{[4-(5-methyl-1,3-benzoxazol-2-yl)benzyl]oxy}phenyl)ethyl]-amino}-5,6,7,8-tetrahydrochinolin-2-carbonsäure (*Enantiomer 1*) | LC-MS (Methode 3): Rₜ = 1.25 min; m/z = 682 (M+H)⁺. |
| | 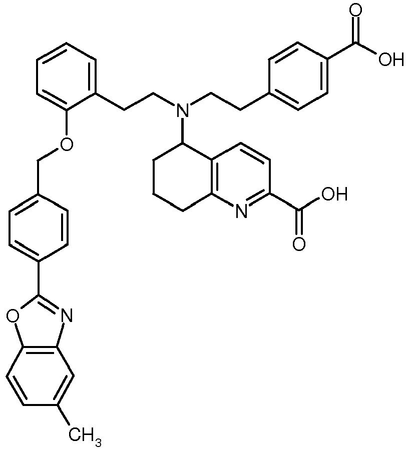 | ¹H-NMR (400 MHz, DMSO-*d₆*): δ [ppm] = 1.40-1.70 (m, 2H), 1.87-2.11 (m, 2H), 2.45 (s, 3H), 2.59-2.87 (m, 10H), 4.02-4.16 (m, 1H), 5.07 (m, 2H), 6.86 (t, 1H), 7.02 (m, 2H), 7.15 (m, 4H), 7.42-7.55 (m, 4H), 7.61 (s, 1H), 7.66 (d, 1H), 7.77 (d, 2H), 8.11 (d, 2H), 12.01-13.58 (br. s, ca. 2H). |
| | aus Ethyl-5-({2-[4-(methoxycarbonyl)phenyl]ethyl}-[2-(2-{[4-(5-methyl-1,3-benzoxazol-2-yl)benzyl]-oxy}phenyl)ethyl]amino)-5,6,7,8-tetrahydro-chinolin-2-carboxylat (Enantiomer 1, Beispiel 67A) | [α]_{D}²⁰ = -38.06°, c = 0.310,Methanol. |
| **22** | 5-{[2-(4-Carboxyphenyl)ethyl][2-(2-{[4-(5-methyl-1,3-benzoxazol-2-yl)benzyl]oxy}phenyl)ethyl]-amino}-5,6,7,8-tetrahydrochinolin-2-carbonsäure (*Enantiomer 2*) | LC-MS (Methode 3): Rₜ = 1.25 min; m/z = 682 (M+H)⁺. |
| | 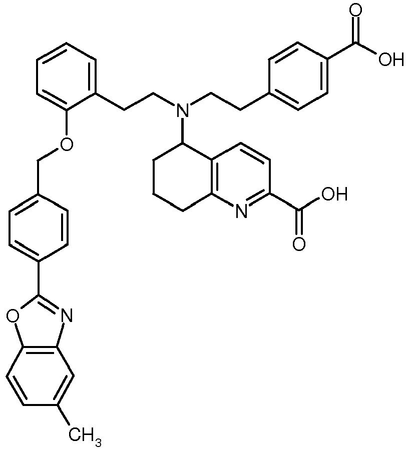 | ¹H-NMR (400 MHz, DMSO-*d₆*): δ [ppm] = 1.41-1.70 (m, 2H), 1.87-2.10 (m, 2H), 2.45 (s, 3H), 2.59-2.88 (m, 10H), 4.03-4.14 (m, 1H), 5.07 (m, 2H), 6.86 (t, 1H), 6.97-7.10 (m, 2H), 7.11-7.29 (m, 4H), 7.42-7.55 (m, 4H), 7.61 (s, 1H), 7.66 (d, 1H), 7.78 (d, 2H), 8.11 (d, 2H), 11.78-13.52 (br. s, ca. 2H). |
| | aus Ethyl-5-({2-[4-(methoxycarbonyl)phenyl]ethyl}-[2-(2-{[4-(5-methyl-1,3-benzoxazol-2-yl)benzyl]-oxy}phenyl)ethyl]amino)-5,6,7,8-tetrahydrochinolin-2-carboxylat (Enantiomer 2, Beispiel 68A) | |

### Beispiel 23

### 5-{[2-(4-Carboxyphenyl)ethyl][2-(2-{[3-chlor-4'-(trifluormethyl)biphenyl-4-yl]methoxy}phenyl)-ethyl]amino}-5,6,7,8-tetrahydrochinolin-2-carbonsäure (Enantiomer 2)

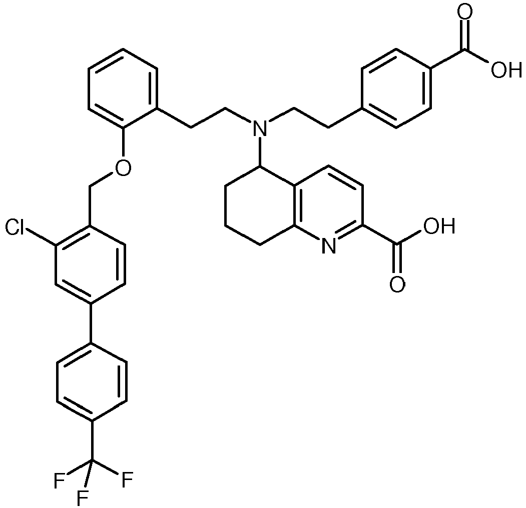

42 g (54.46 mmol) Ethyl-5-([2-(2-{[3-chlor-4'-(trifluormethyl)biphenyl-4-yl]methoxy}phenyl)-ethyl]{2-[4-(methoxycarbonyl)phenyl]ethyl}amino)-5,6,7,8-tetrahydrochinolin-2-carboxylat (Enantiomer 2, Beispiel 92A) wurden in 429 ml Dioxan gelöst, mit 163 ml 1 N Natronlauge versetzt und anschließend über Nacht bei Raumtemperatur gerührt. Nach vollständiger Umsetzung wurde das Dioxan am Rotationsverdampfer entfernt und das verbliebene Gemisch mit ca. 750 ml Wasser verdünnt. Es wurde dann mit Essigsäure auf pH 4-5 angesäuert. Der ausgefallene Feststoff wurde abgesaugt und mehrmals mit Wasser gewaschen (insgesamt ca. 250 ml Wasser). Danach wurde der Feststoff in 750 ml Wasser aufgenommen und über Nacht bei Raumtemperatur gerührt. Nach erneutem Absaugen wurde der Feststoff nochmals mit Wasser nachgewaschen und dann über Nacht im Hochvakuum über Phosphorpentoxid als Trocknungsmittel getrocknet. Danach wurde das Trocknungsmittel entfernt und der Feststoff weitere 24 h bei 40°C getrocknet. Es wurden auf diese Weise 35 g (48 mmol, 88% d. Th.) der Titelverbindung erhalten.

LC-MS (Methode 3): Rₜ = 1.39 min; m/z = 729/731 (M+H)⁺.

¹H-NMR (400 MHz, DMSO-*d₆*): δ [ppm] = 1.37-1.68 (m, 2H), 1.85-2.06 (m, 2H), 2.59-2.83 (m, 10H), 3.98-4.10 (m, 1H), 4.99-5.15 (m, 2H), 6.87 (t, 1H), 7.05 (d, 2H), 7.12 (d, 2H), 7.23 (t, 1H), 7.38-7.48 (m, 2H), 7.54 (d, 1H), 7.62 (d, 1H), 7.71-7.91 (m, 7H), 11.60-13.85 (br. s, ca. 2H).

[α]_{D}²⁰ = +61.75°, c = 0.420, Methanol.

Analog zu Beispiel 20 und Beispiel 23 wurden die folgenden Verbindungen hergestellt:

| **Beispiel** | **Name / Struktur / Edukt** | **Analytische Daten** |
|---|---|---|
| **24** | 5-([2-(4-Carboxyphenyl)ethyl]{2-[2-({4-[5-(trifluormethyl)-1,3-benzoxazol-2-yl]benzyl}oxy)phenyl]-ethyl}amino)-5,6,7,8-tetrahydrochinolin-2-carbonsäure (*Enantiomer 2*) | LC-MS (Methode 3): Rₜ = 1.32 min; m/z = 736 (M+H)⁺. |
| | 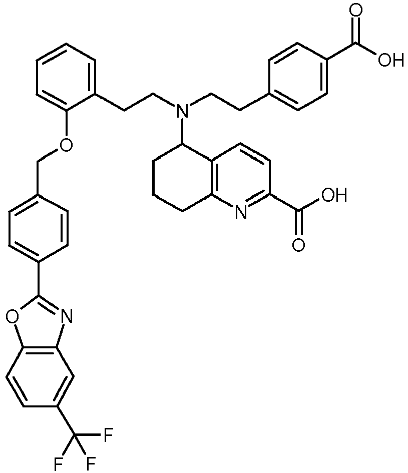 | ¹H-NMR (400 MHz, DMSO-*d₆*): δ [ppm] 1.41-1.55 (m, 1H), 1.55- 1.71 (m, 1H), 1.88-2.10 (m, 2H), 2.58-2.89 (m, 10H), 4.01-4.14 (m, 1H), 5.03-5.16 (m, 2H), 6.86 (t, 1H), 6.99-7.10 (m, 2H), 7.14 (d, 2H), 7.21 (t, 1H), 7.46 (d, 1H), 7.49-7.60 (m, 3H), 7.72-7.86 (m, 3H), 8.03 (d, 1H), 8.14 (d, 2H), 8.24 (s, 1H), 12.01-13.42 (br. s, ca. 2H). |
| | aus Ethyl-5-({2-[4-(methoxycarbonyl)phenyl]ethyl}-{2-[2-({4-[5-(trifluormethyl)-1,3-benzoxazol-2-yl]-benzyl}oxy)phenyl]ethyl}amino)-5,6,7,8-tetrahydro-chinolin-2-carboxylat (Enantiomer 2, Beispiel 93A) | |
| **25** | 5-[(4-Carboxybutyl){2-[2-({4-[5-(trifluormethyl)-1,3-benzoxazol-2-yl]benzyl}oxy)phenyl]ethyl}-amino]-5,6,7,8-tetrahydrochinolin-2-carbonsäure (*Enantiomer 2*) | LC-MS (Methode 3): Rₜ = 1.07 min; m/z = 688 (M+H)⁺. |
| | 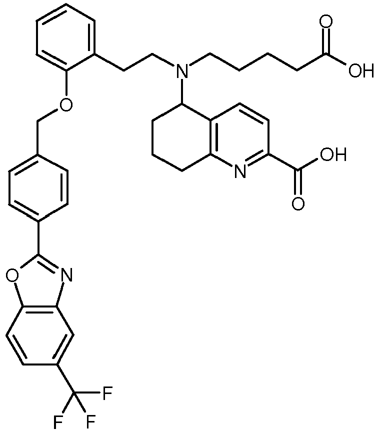 | ¹H-NMR (400 MHz, DMSO-*d₆*): δ [ppm] 1.30-1.71 (m, 6H), 1.90-2.04 (m, 2H), 2.07-2.18 (m, 2H), 2.39-2.65 (m, 4H, teilweise verdeckt durch DMSO-Signal), 2.65-2.91 (m, 4H), 3.87-4.07 (m, 1H), 5.10 (q, 2H), 6.87 (t, 1H), 7.00 (d, 1H), 7.10-7.23 (m, 2H), 7.56 (d, 2H), 7.66 (d, 1H), 7.85 (d, 2H), 8.04 (d, 1H), 8.16-8.30 (m, 3H), 11.10-13.31 (br. s, ca. 2H). |
| | aus Ethyl-5-[(5-ethoxy-5-oxopentyl){2-[2-({4-[5-(trifluormethyl)-1,3-benzoxazol-2-yl]benzyl}oxy)-phenyl]ethyl}amino]-5,6,7,8-tetrahydrochinolin-2-carboxylat (Enantiomer 2, Beispiel 98A) | |
| **26** | 5-{[2-(2-{[4-(1,3-Benzoxazol-2-yl)benzyl]oxy}-phenyl)ethyl][2-(4-carboxyphenyl)ethyl]amino}-5,6,7,8-tetrahydrochinolin-2-carbonsäure (*Enantiomer 2)* | LC-MS (Methode 3): Rₜ = 1.17 min; m/z = 668 (M+H)⁺. |
| | 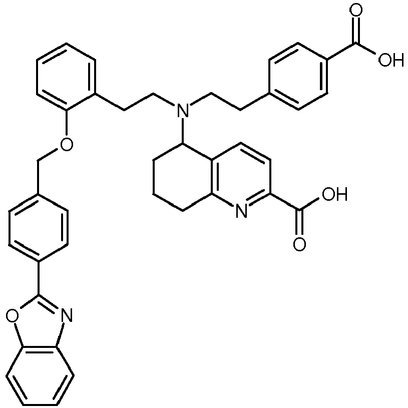 | ¹H-NMR (400 MHz, DMSO-*d₆*): δ [ppm] = 1.41-1.54 (m, 1H), 1.55-1.69 (m, 1H), 1.86-2.09 (m, 2H), 2.58-2.86 (m, 10H), 4.05-4.13 (m, 1H), 5.00-5.15 (m, 2H), 6.86 (t, 1H), 6.97-7.10 (m, 2H), 7.12-7.26 (m, 3H), 7.37-7.56 (m, 6H), 7.72-7.88 (m, 4H), 8.13 (d, 2H), 11.90-13.36 (br. s, ca. 2H). |
| | aus Ethyl-5-([2-(2-{[4-(1,3-benzoxazol-2-yl)benzyl]-oxy}phenyl)ethyl]{2-[4-(methoxycarbonyl)-phenyl]ethyl}amino)-5,6,7,8-tetrahydrochinolin-2-carboxylat (Enantiomer 2, Beispiel 94A) | |
| **27** | 5-{[2-(2-{[4-(1,3-Benzoxazol-2-yl)benzyl]oxy}-phenyl)ethyl](4-carboxybutyl)amino}-5,6,7,8-tetrahydrochinolin-2-carbonsäure (*Enantiomer 2)* | LC-MS (Methode 3): Rₜ = 0.95 min; m/z = 620 (M+H)⁺. |
| | 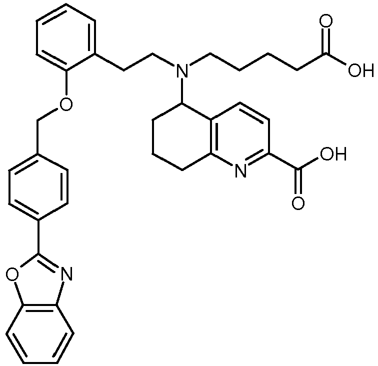 | ¹H-NMR (400 MHz, DMSO-*d₆*): δ [ppm] = 1.34-1.70 (m, 6H), 1.90-2.04 (m, 2H), 2.12 (t, 2H), 2.39-2.64 (m, 4H, teilweise verdeckt durch DMSO-Signal), 2.65-2.87 (m, 4H), 3.90-4.06 (m, 1H), 5.09 (q, 2H), 6.87 (t, 1H), 6.99 (d, 1H), 7.09-7.23 (m, 2H), 7.37-7.49 (m, 2H), 7.53 (d, 2H), 7.67 (d, 1H), 7.77-7.89 (m, 3H), 8.18 (d, 2H), 11.00-13.58 (br. s, ca. 2H). |
| | aus Ethyl-5-{[2-(2-{[4-(1,3-benzoxazol-2-yl)-benzyl]oxy}phenyl)ethyl](5-ethoxy-5-oxopentyl)-amino}-5,6,7,8-tetrahydrochinolin-2-carboxylat (Enantiomer 2, Beispiel 99A) | |
| **28** | 5-[(4-Carboxybutyl){2-[2-({4-[5-(trifluormethoxy)-1,3-benzoxazol-2-yl]benzyl}oxy)phenyl]ethyl}-amino]-5,6,7,8-tetrahydrochinolin-2-carbonsäure (*Enantiomer 2*) | LC-MS (Methode 3): Rₜ = 1.09 min; m/z = 704 (M+H)⁺. |
| | 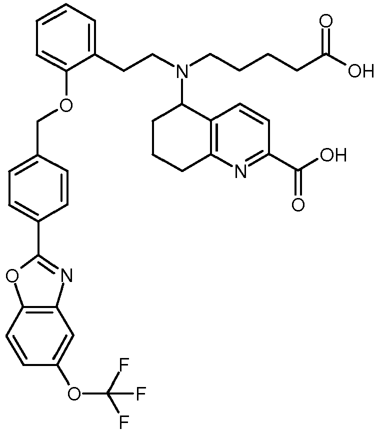 | ¹H-NMR (400 MHz, DMSO-*d₆*): δ [ppm] 1.32-1.71 (m, 6H), 1.88-2.05 (m, 2H), 2.07-2.17 (m, 2H), 2.39-2.64 (m, 4H, teilweise verdeckt durch DMSO-Signal), 2.64-2.88 (m, 4H), 3.93-4.05 (m, 1H), 5.10 (q, 2H), 6.87 (t, 1H), 6.99 (d, 1H), 7.09-7.23 (m, 2H), 7.46 (dd, 1H), 7.54 (d, 2H), 7.66 (d, 1H), 7.85 (d, 1H), 7.89-7.98 (m, 2H), 8.18 (d, 2H), 11.10-13.04 (br. s, ca. 2H). |
| | aus Ethyl-5-[(5-ethoxy-5-oxopentyl){2-[2-({4-[5-(trifluormethoxy)-1,3-benzoxazol-2-yl]benzyl}oxy)-phenyl]ethyl}amino]-5,6,7,8-tetrahydrochinolin-2-carboxylat (Enantiomer 2, Beispiel 100A) | |
| **29** | 5-([2-(4-Carboxyphenyl)ethyl]{2-[2-({4-[5-(tri-fluormethoxy)-1,3-benzoxazol-2-yl]benzyl}oxy)-phenyl]ethyl}amino)-5,6,7,8-tetrahydrochinolin-2-carbonsäure (*Enantiomer 2*) | LC-MS (Methode 3): Rₜ = 1.34 min; m/z = 752 (M+H)⁺. |
| | 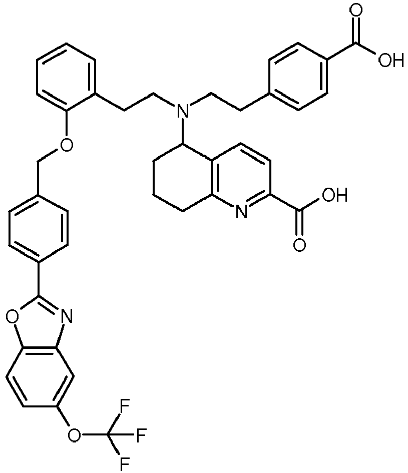 | ¹H-NMR (400 MHz, DMSO-*d₆*): δ [ppm] 1.42-1.55 (m, 1H), 1.55-1.71 (m, 1H), 1.88-2.11 (m, 2H), 2.59-2.87 (m, 10H), 3.99-4.13 (m, 1H), 5.09 (q, 2H), 6.88 (t, 1H), 6.98-7.09 (m, 2H), 7.15 (d, 2H), 7.20 (t, 1H), 7.41-7.58 (m, 5H), 7.77 (d, 2H), 7.87-7.96 (m, 2H), 8.12 (d, 2H), 11.89-13.63 (br. s, ca. 2H). |
| | aus Ethyl-5-({2-[4-(methoxycarbonyl)phenyl]-ethyl}{2-[2-({4-[5-(trifluormethoxy)-1,3-benzoxazol-2-yl]benzyl}oxy)phenyl]ethyl}amino)-5,6,7,8-tetrahydrochinolin-2-carboxylat (Enantiomer 2, Beispiel 95A) | |
| **30** | 5-{[2-(4-Carboxyphenyl)ethyl][2-(2-{[4-(5-cyano-1,3-benzoxazol-2-yl)benzyl]oxy}phenyl)ethyl]-amino}-5,6,7,8-tetrahydrochinolin-2-carbonsäure (*Enantiomer 2*) | LC-MS (Methode 3): Rₜ = 1.14 min; m/z = 693 (M+H)⁺. |
| | 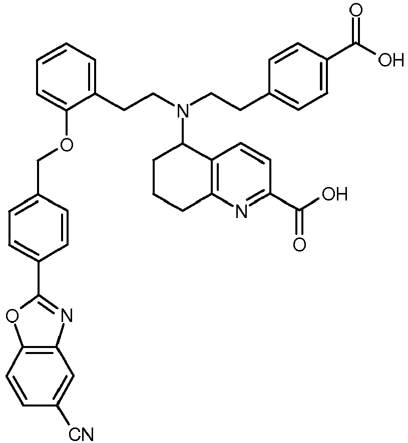 | ¹H-NMR (400 MHz, DMSO-*d₆*): δ [ppm] = 1.42-1.55 (m, 1H), 1.56-1.70 (m, 1H), 1.89-2.10 (m, 2H), 2.59-2.90 (m, 10H), 4.02-4.13 (m, 1H), 5.10 (m, 2H), 6.86 (t, 1H), 6.99-7.09 (m, 2H), 7.13 (d, 2H), 7.21 (t, 1H), 7.47 (d, 1H), 7.50-7.60 (m, 3H), 7.74 (d, 2H), 7.91 (dd, 1H), 8.01 (d, 1H), 8.13 (d, 2H), 8.41 (s, 1H), 11.96-13.51 (br. s, ca. 2H). |
| | aus Ethyl-5-([2-(2-{[4-(5-cyano-1,3-benzoxazol-2-yl)benzyl]oxy}phenyl)ethyl]{2-[4-(methoxycarbonyl)phenyl]ethyl}amino)-5,6,7,8-tetrahydrochinolin-2-carboxylat (Enantiomer 2, Beispiel 96A) | |
| **31** | 5-{(4-Carboxybutyl)[2-(2-{[4-(5-cyano-1,3-benzoxazol-2-yl)benzyl]oxy}phenyl)ethyl]amino}-5,6,7,8-tetrahydrochinolin-2-carbonsäure (*Enantiomer 2*) | LC-MS (Methode 3): Rₜ = 0.93 min; m/z = 645 (M+H)⁺. |
| | 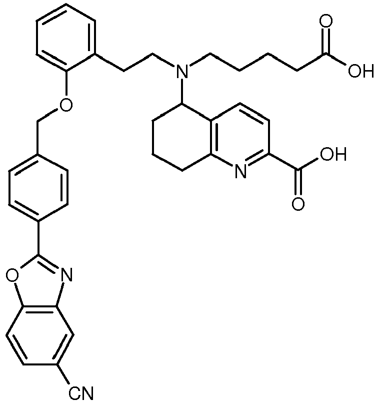 | ¹H-NMR (400 MHz, DMSO-*d₆*): δ [ppm] = 1.31-1.77 (m, 6H), 1.90-2.05 (m, 2H), 2.05-2.18 (m, 2H), 2.39-2.64 (m, 4H, teilweise verdeckt durch DMSO-Signal), 2.65-2.88 (m, 4H), 3.92-4.05 (m, 1H), 5.10 (q, 2H), 6.87 (t, 1H), 6.99 (d, 1H), 7.10-7.22 (m, 2H), 7.55 (d, 2H), 7.67 (d, 1H), 7.85 (d, 1H), 7.93 (d, 1H), 8.04 (d, 1H), 8.19 (d, 2H), 8.44 (s, 1H), 11.38-12.79 (br. s, ca. 2H). |
| | aus Ethyl-5-{[2-(2-{[4-(5-cyano-1,3-benzoxazol-2-yl)benzyl]oxy}phenyl)ethyl](5-ethoxy-5-oxopentyl)amino}-5,6,7,8-tetrahydrochinolin-2-carboxylat (Enantiomer 2, Beispiel 101A) | |
| **32** | 5-{(4-Carboxybutyl)[2-(2-{[4-(5-chlor-1,3-benzoxazol-2-yl)benzyl]oxy}phenyl)ethyl]amino}-5,6,7,8-tetrahydrochinolin-2-carbonsäure (*Enantiomer 2*) | LC-MS (Methode 3): Rₜ = 1.04 min; m/z = 654/656 (M+H)⁺. |
| | 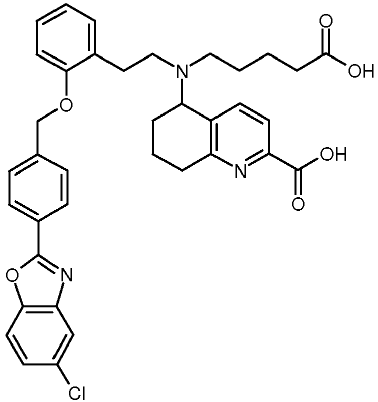 | ¹H-NMR (400 MHz, DMSO-*d₆*): δ [ppm] = 1.32-1.70 (m, 6H), 1.89-2.04 (m, 2H), 2.12 (t, 2H), 2.39-2.65 (m, 4H, teilweise verdeckt durch DMSO-Signal), 2.65-2.88 (m, 4H), 3.92-4.04 (m, 1H), 5.09 (q, 2H), 6.88 (t, 1H), 6.99 (d, 1H), 7.09-7.24 (m, 2H), 7.44-7.57 (m, 3H), 7.66 (d, 1H), 7.85 (d, 2H), 7.94 (d, 1H), 8.17 (d, 2H), 11.17-13.29 (br. s, ca. 2H). |
| | aus Ethyl-5-{[2-(2-{[4-(5-chlor-1,3-benzoxazol-2-yl)benzyl]oxy}phenyl)ethyl](5-ethoxy-5-oxopentyl)amino}-5,6,7,8-tetrahydrochinolin-2-carboxylat (Enantiomer 2, Beispiel 102A) | |
| **33** | 5-{[2-(4-Carboxyphenyl)ethyl][2-(2-{[3-chlor-4'-(trifluormethyl)biphenyl-4-yl]methoxy}-5-fluorphenyl)ethyl]amino}-5,6,7,8-tetrahydrochinolin-2-carbonsäure (*Enantiomer 1*) | LC-MS (Methode 3): Rₜ = 1.39 min; m/z = 747/749 (M+H)⁺. |
| | 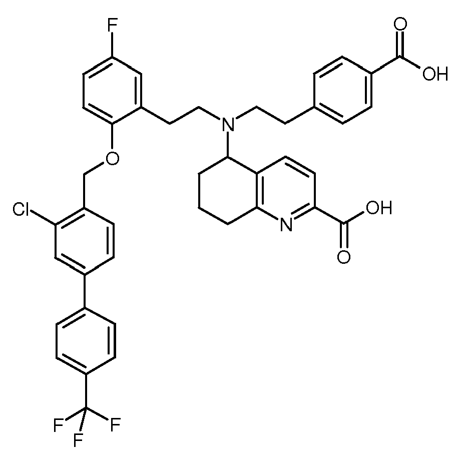 | ¹H-NMR (400 MHz, DMSO-*d₆*): δ [ppm] = 1.38-1.67 (m, 2H), 1.84-2.05 (m, 2H), 2.57-2.80 (m, 10H), 3.98-4.08 (m, 1H), 5.00-5.11 (m, 2H), 6.90-7.00 (m, 1H), 7.01-7.15 (m, 4H), 7.41 (s, 2H), 7.54 (d, 1H), 7.63 (dd, 1H), 7.72-7.89 (m, 7H), 12.07-13.44 (br. s, ca. 2H). |
| | aus Ethyl-5-([2-(2-{[3-chlor-4'-(trifluormethyl)-biphenyl-4-yl]methoxy}-5-fluorphenyl)ethyl]{2-[4-(methoxycarbonyl)phenyl]ethyl}amino)-5,6,7,8-tetrahydrochinolin-2-carboxylat (Enantiomer 1, Beispiel 124A) | |
| **34** | 5-{[2-(4-Carboxyphenyl)ethyl][2-(2-{[3-chlor-4'-(trifluormethyl)biphenyl-4-yl]methoxy}-5-fluorphenyl)ethyl]amino}-5,6,7,8-tetrahydrochinolin-2-carbonsäure (*Enantiomer 2*) | LC-MS (Methode 3): Rₜ = 1.39 min; m/z = 747/749 (M+H)⁺. |
| | 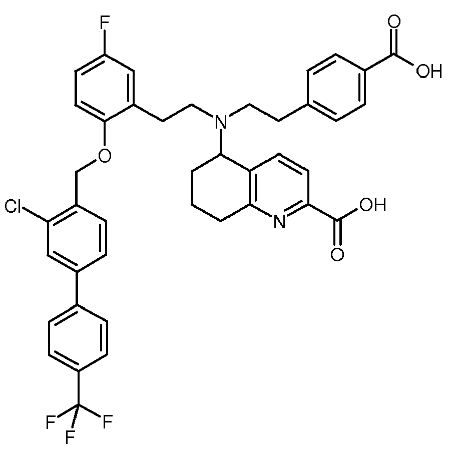 | ¹H-NMR (400 MHz, DMSO-*d₆*): δ [ppm] = 1.38-1.66 (m, 2H), 1.85-2.06 (m, 2H), 2.57-2.79 (m, 10H), 3.99-4.08 (m, 1H), 5.00-5.11 (m, 2H), 6.92-7.00 (m, 1H), 7.01-7.16 (m, 4H), 7.41 (s, 2H), 7.54 (d, 1H), 7.63 (dd, 1H), 7.72-7.90 (m, 7H), 12.21-13.18 (br. s, ca. 2H). |
| | aus Ethyl-5-([2-(2-{[3-chlor-4'-(trifluormethyl)-biphenyl-4-yl]methoxy}-5-fluorphenyl)ethyl]{2-[4-(methoxycarbonyl)phenyl]ethyl}amino)-5,6,7,8-tetrahydrochinolin-2-carboxylat (Enantiomer 2, Beispiel 123A) | |
| **35** | 5-{[2-(4-Carboxyphenyl)ethyl][2-(2-{[4-(5-chlor-1,3-benzoxazol-2-yl)benzyl]oxy}-5-fluorphenyl)-ethyl]amino}-5,6,7,8-tetrahydrochinolin-2-carbonsäure (*Enantiomer 1*) | LC-MS (Methode 3): Rₜ = 1.27 min; m/z = 720/722 (M+H)⁺. |
| | 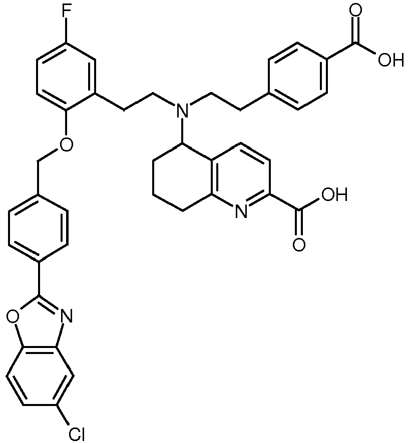 | ¹H-NMR (400 MHz, DMSO-*d₆*): δ [ppm] = 1.41-1.70 (m, 2H), 1.88-2.08 (m, 2H), 2.57-2.85 (m, 10H), 4.01-4.12 (m, 1H), 5.00-5.12 (m, 2H), 6.96 (d, 1H), 7.02 (d, 2H), 7.13 (d, 2H), 7.40-7.57 (m, 5H), 7.75 (d, 2H), 7.83 (d, 1H), 7.93 (d, 1H), 8.11 (d, 2H), 11.88-13.55 (br. s, ca. 2H). |
| | aus Ethyl-5-([2-(2-{[4-(5-chlor-1,3-benzoxazol-2-yl)benzyl]oxy}-5-fluorphenyl)ethyl]{2-[4-(methoxycarbonyl)phenyl]ethyl}amino)-5,6,7,8-tetrahydrochinolin-2-carboxylat (Enantiomer 1, Beispiel 122A) | |
| **36** | 5-{(4-Carboxybutyl)[2-(2-{[4-(5-chlor-1,3-benzoxazol-2-yl)benzyl]oxy}-5-fluorphenyl)ethyl]-amino}-5,6,7,8-tetrahydrochinolin-2-carbonsäure (*Enantiomer 1*) | LC-MS (Methode 3): Rₜ = 1.10 min; m/z = 672/674 (M+H)⁺. |
| | 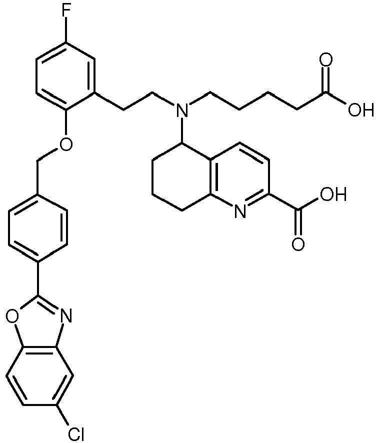 | |
| | aus Ethyl-5-{[2-(2-{[4-(5-chlor-1,3-benzoxazol-2-yl)benzyl]oxy}-5-fluorphenyl)ethyl](5-ethoxy-5-oxopentyl)amino}-5,6,7,8-tetrahydrochinolin-2-carboxylat (Enantiomer 1, Beispiel 119A) | |
| **37** | 5-{(4-Carboxybutyl)[2-(2-{[4-(5-chlor-1,3-benzoxazol-2-yl)benzyl]oxy}-5-fluorphenyl)ethyl]-amino}-5,6,7,8-tetrahydrochinolin-2-carbonsäure (*Enantiomer 2*) | LC-MS (Methode 3): Rₜ = 1.10 min; m/z = 672/674 (M+H)⁺. |
| | 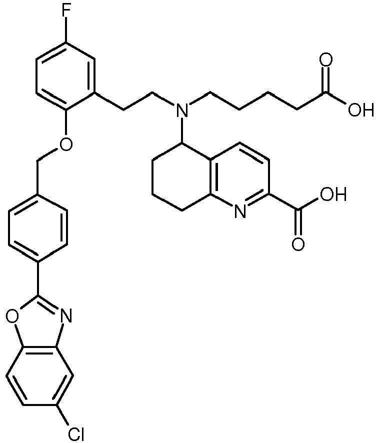 | |
| | aus Ethyl-5-{[2-(2-{[4-(5-chlor-1,3-benzoxazol-2-yl)benzyl]oxy}-5-fluorphenyl)ethyl](5-ethoxy-5-oxopentyl)amino}-5,6,7,8-tetrahydrochinolin-2-carboxylat (Enantiomer 2, Beispiel 120A) | |

### Beispiel 38

### 5-{[2-(4-Carboxyphenyl)ethyl][2-(2-{[4-(2-phenylethyl)benzyl]oxy}phenyl)ethyl]amino}-5,6,7,8-tetrahydrochinolin-2-carbonsäure (Enantiomer 2)

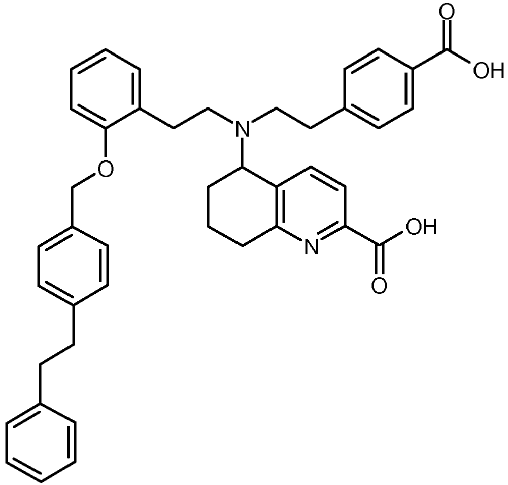

3.64 g (5.22 mmol) Ethyl-5-({2-[4-(methoxycarbonyl)phenyl]ethyl}[2-(2-{[4-(2-phenylethyl)-benzyl]oxy}phenyl)ethyl]amino)-5,6,7,8-tetrahydrochinolin-2-carboxylat (Enantiomer 2, Beispiel 97A) wurden in 40 ml Dioxan und 20 ml Wasser aufgenommen, mit 658 mg (15.67 mmol) Lithiumhydroxid-Monohydrat versetzt und anschließend über Nacht bei Raumtemperatur gerührt. Nach vollständiger Umsetzung wurde das Dioxan am Rotationsverdampfer entfernt und das verbliebene Gemisch mit Wasser verdünnt. Anschließend wurde mit Essigsäure auf pH 4-5 angesäuert. Der ausgefallene Feststoff wurde abgesaugt und mehrmals mit Wasser gewaschen. Danach wurde der Feststoff in Wasser aufgenommen und bei Raumtemperatur gerührt. Nach erneutem Absaugen wurde der Feststoff nochmals mit Wasser nachgewaschen und dann über Nacht im Hochvakuum bei 40°C getrocknet. Es wurden 3.24 g (4.95 mmol, 95% d. Th.) der Titelverbindung erhalten.

LC-MS (Methode 3): Rₜ = 1.27 min; m/z = 655 (M+H)⁺.

¹H-NMR (400 MHz, DMSO-*d₆*): δ [ppm] = 1.38-1.53 (m, 1H), 1.54-1.68 (m, 1H), 1.89-2.08 (m, 2H), 2.57-2.87 (m, 14H), 4.01-4.10 (m, 1H), 4.90 (q, 2H), 6.83 (t, 1H), 6.93-7.06 (m, 2H), 7.09-7.30 (m, 12H), 7.39-7.50 (m, 2H), 7.80 (d, 2H), 12.03-13.45 (br. s, ca. 2H).

[α]_{D}²⁰ = +64.36°, c = 0.380, Methanol.

### Beispiel 39

### 5-{[2-(4-Carboxyphenyl)ethyl][2-(2-{[4-(5-chlor-1,3-benzoxazol-2-yl)benzyl]oxy}-5-fluorphenyl)-ethyl]amino}-5,6,7,8-tetrahydrochinolin-2-carbonsäure (Enantiomer 2)

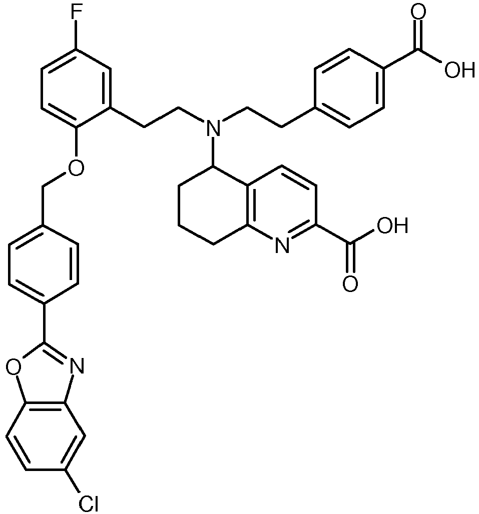

5.4 g (7.08 mmol) Ethyl-5-([2-(2-{[4-(5-chlor-1,3-benzoxazol-2-yl)benzyl]oxy}-5-fluorphenyl)-ethyl]{2-[4-(methoxycarbonyl)phenyl]ethyl}amino)-5,6,7,8-tetrahydrochinolin-2-carboxylat (Enantiomer 2, Beispiel 121A) wurden in 50 ml Dioxan gelöst, mit 21 ml 1N Natronlauge versetzt und anschließend über Nacht bei Raumtemperatur gerührt. Nach vollständiger Umsetzung wurde das Dioxan am Rotationsverdampfer entfernt und das verbliebene Gemisch mit Wasser verdünnt. Anschließend wurde mit Essigsäure auf pH 4-5 angesäuert. Der ausgefallene Feststoff wurde abgesaugt, mehrmals mit Wasser gewaschen und dann über Nacht an der Luft getrocknet. Es wurden 4.8 g (6.66 mmol, 94% d. Th.) der Titelverbindung erhalten.

LC-MS (Methode 3): Rₜ = 1.28 min; m/z = 720/722 (M+H)⁺.

¹H-NMR (400 MHz, DMSO-*d₆*): δ [ppm] = 1.40-1.72 (m, 2H), 1.88-2.11 (m, 2H), 2.59-2.84 (m, 10H), 4.02-4.13 (m, 1H), 5.00-5.14 (m, 2H), 6.96 (d, 1H), 7.02 (d, 2H), 7.13 (d, 2H), 7.41-7.57 (m, 5H), 7.75 (d, 2H), 7.83 (d, 1H), 7.93 (d, 1H), 8.11 (d, 2H), 12.05-13.41 (br. s, ca. 2H).

[α]_{D}²⁰ =+58.77°, c = 0.405, DMSO.

### Beispiel 40

### 5-([2-(4-Carboxyphenyl)ethyl]{2-[2-({4-[2-(4-fluorphenyl)ethyl]benzyl}oxy)phenyl]ethyl}amino)-5,6,7,8-tetrahydrochinolin-2-carbonsäure (Enantiomer 2)

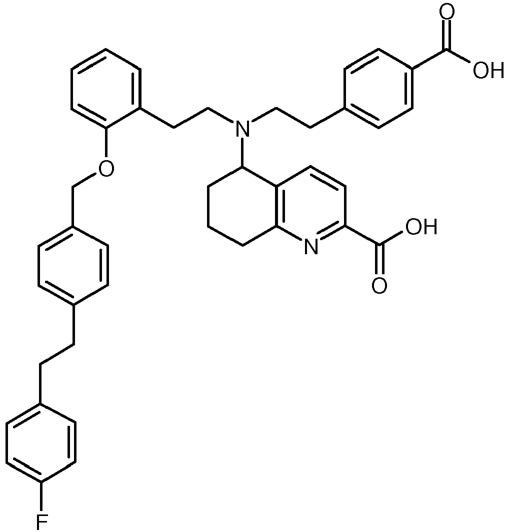

1.76 g (2.46 mmol) Ethyl-5-({2-[2-({4-[2-(4-fluorphenyl)ethyl]benzyl}oxy)phenyl]ethyl}{2-[4-(methoxycarbonyl)phenyl]ethyl}amino)-5,6,7,8-tetrahydrochinolin-2-carboxylat (Enantiomer 2, Beispiel 136A) wurden in 50 ml Dioxan gelöst, mit 7.4 ml 1 N Natronlauge versetzt und über Nacht bei Raumtemperatur gerührt. Anschließend wurden weitere 0.2 ml 1 M Natronlauge zudosiert und das Gemisch zwei Stunden bei Raumtemperatur nachgerührt. Nach vollständiger Umsetzung wurde das Dioxan am Rotationsverdampfer entfernt und das verbliebene Gemisch mit Wasser verdünnt. Anschließend wurde mit Essigsäure auf pH 4-5 angesäuert. Der ausgefallene Feststoff wurde abgesaugt, mehrmals mit Wasser gewaschen und dann drei Tage lang im Trockenschrank unter Vakuum bei 40°C getrocknet. Es wurden 673 mg (2.31 mmol, 94% d. Th.) der Titelverbindung erhalten.

LC-MS (Methode 3): Rₜ = 1.33 min; m/z = 673 (M+H)⁺.

¹H-NMR (400 MHz, DMSO-*d₆*): δ [ppm] = 1.39-1.53 (m, 1H), 1.54-1.70 (m, 1H), 1.87-2.07 (m, 2H), 2.57-2.89 (m, 14H), 3.98-4.11 (m, 1H), 4.90 (q, 2H), 6.84 (t, 1H), 6.96-7.28 (m, 13H), 7.38-7.50 (m, 2H), 7.79 (d, 2H), 11.79-13.60 (br. s, ca. 2H).

[α]_{D}²⁰ = +85.73°, c = 0.285, DMSO.

Analog zu Beispiel 40 wurden die folgenden Verbindungen hergestellt:

| **Beispiel** | **Name / Struktur / Edukt** | **Analytische Daten** |
|---|---|---|
| **41** | 5-([2-(4-Carboxyphenyl)ethyl]{2-[2-({4-[2-(4-fluorphenyl)ethyl]benzyl}oxy)phenyl]ethyl}amino)-5,6,7,8-tetrahydrochinolin-2-carbonsäure (*Enantiomer 1*) | LC-MS (Methode 3): Rₜ = 1.33 min; m/z = 673 (M+H)⁺. |
| | 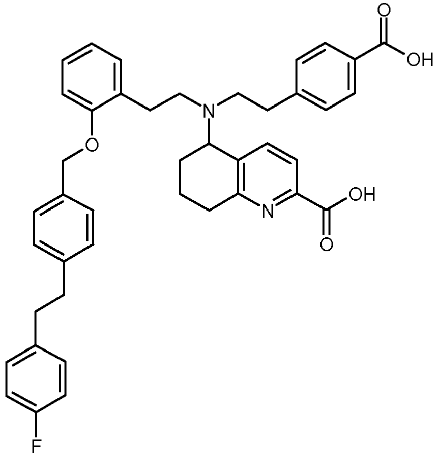 | ¹H-NMR (400 MHz, DMSO-*d₆*): δ [ppm] = 1.40-1.53 (m, 1H), 1.53-1.69 (m, 1H), 1.88-2.08 (m, 2H), 2.57-2.88 (m, 14H), 4.00-4.10 (m, 1H), 4.90 (q, 2H), 6.83 (t, 1H), 6.94-7.28 (m, 13H), 7.39-7.50 (m, 2H), 7.80 (d, 2H), 12.08-13.73 (br. s, ca. 2H). |
| | aus Ethyl-5-({2-[2-({4-[2-(4-fluorphenyl)ethyl]-benzyl}oxy)phenyl]ethyl}{2-[4-(methoxycarbonyl)-phenyl]ethyl}amino)-5,6,7,8-tetrahydrochinolin-2-carboxylat (Enantiomer 1, Beispiel 137A) | [α]_{D}²⁰ = -84.29°, c = 0.305,DMSO. |
| **42** | 5-([2-(4-Carboxyphenyl)ethyl]{2-[5-fluor-2-({4-[2-(4-fluorphenyl)ethyl]benzyl}oxy)phenyl]ethyl}-amino)-5,6,7,8-tetrahydrochinolin-2-carbonsäure (*Enantiomer 1*) | LC-MS (Methode 3): Rₜ = 1.29 min; m/z = 691 (M+H)⁺. |
| | 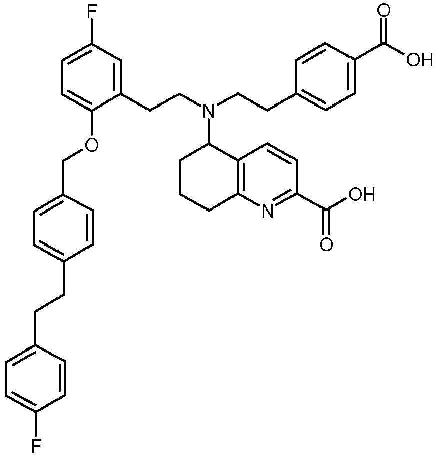 | ¹H-NMR (400 MHz, DMSO-*d₆*): δ [ppm] = 1.40-1.53 (m, 1H), 1.54-1.68 (m, 1H), 1.87-2.08 (m, 2H), 2.57-2.88 (m, 14H), 3.97-4.11 (m, 1H), 4.88 (q, 2H), 6.92 (d, 1H), 6.99 (d, 2H), 7.03-7.29 (m, 10H), 7.42 (s, 2H), 7.79 (d, 2H), 11.93-13.43 (br. s, ca. 2H). [α]_{D}²⁰ = -79.32°, c = 0.295, DMSO. |
| | aus Ethyl-5-({2-[5-fluor-2-({4-[2-(4-fluorphenyl)-ethyl]benzyl}oxy)phenyl]ethyl}{2-[4-(methoxycarbonyl)phenyl]ethyl}amino)-5,6,7,8-tetrahydrochinolin-2-carboxylat (Enantiomer 1, Beispiel 146A) | |

### Beispiel 43

### 5-([2-(4-Carboxyphenyl)ethyl]{2-[5-fluor-2-({4-[2-(4-fluorphenyl)ethyl]benzyl}oxy)phenyl]-ethyl}amino)-5,6,7,8-tetrahydrochinolin-2-carbonsäure (Enantiomer 2)

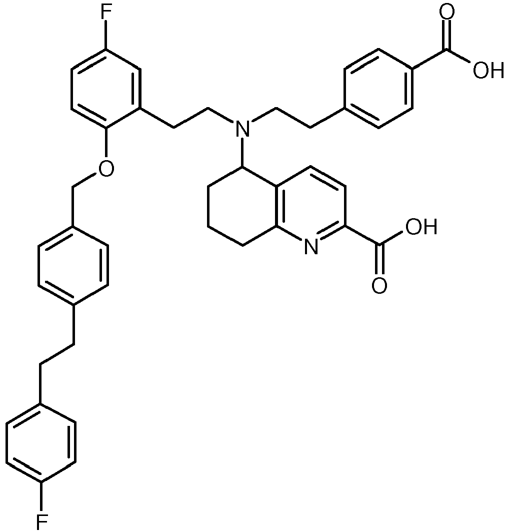

75 mg (0.10 mmol) Ethyl-5-({2-[5-fluor-2-({4-[2-(4-fluorphenyl)ethyl]benzyl}oxy)phenyl]ethyl}-{2-[4-(methoxycarbonyl)phenyl]ethyl}amino)-5,6,7,8-tetrahydrochinolin-2-carboxylat (Enantiomer 2, Beispiel 145A) wurden in 2 ml Dioxan gelöst, mit 0.3 ml 1 N Natronlauge versetzt und über Nacht bei Raumtemperatur gerührt. Nach vollständiger Umsetzung wurde das Dioxan am Rotationsverdampfer entfernt und das verbliebene Gemisch mit Wasser verdünnt. Anschließend wurde mit Essigsäure auf pH 4-5 angesäuert. Der ausgefallene Feststoff wurde abgesaugt, mehrmals mit Wasser gewaschen und dann drei Tage lang im Trockenschrank unter Vakuum bei 40°C getrocknet. Es wurden 58 mg (0.08 mmol, 78% d. Th.) der Titelverbindung erhalten.

LC-MS (Methode 3): Rₜ = 1.29 min; m/z = 691 (M+H)⁺.

¹H-NMR (400 MHz, DMSO-*d₆*): δ [ppm] = 1.41-1.53 (m, 1H), 1.54-1.69 (m, 1H), 1.88-2.07 (m, 2H), 2.58-2.88 (m, 14H), 3.99-4.10 (m, 1H), 4.88 (q, 2H), 6.92 (d, 1H), 6.99 (d, 2H), 7.03-7.27 (m, 10H), 7.41 (s, 2H), 7.79 (d, 2H), 12.25-13.34 (br. s, ca. 2H).

[α]_{D}²⁰ =+77.21°, c = 0.335, DMSO.

### Beispiel 44

### 5-{[2-(4-Carboxyphenyl)ethyl](2-{2-[(4-{2-[4-(trifluormethyl)phenyl]ethyl}benzyl)oxy]phenyl}-ethyl)amino}-5,6,7,8-tetrahydrochinolin-2-carbonsäure (Enantiomer 2)

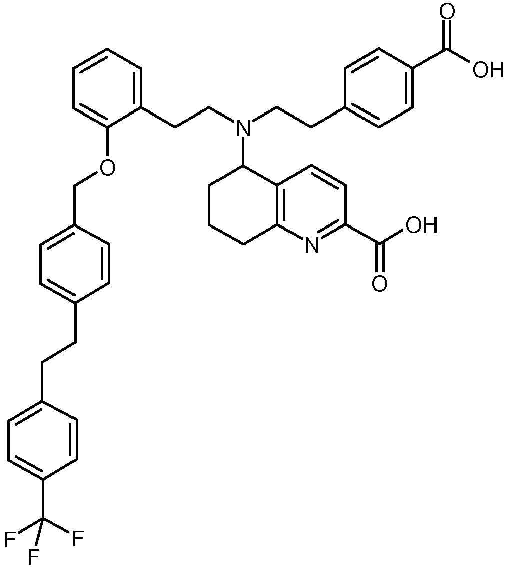

98 mg (0.13 mmol) Ethyl-5-[{2-[4-(methoxycarbonyl)phenyl]ethyl}(2-{2-[(4-{2-[4-(trifluormethyl)phenyl]ethyl}benzyl)oxy]phenyl}ethyl)amino]-5,6,7,8-tetrahydrochinolin-2-carboxylat (Enantiomer 2, Beispiel 156A) wurden in 2.5 ml Dioxan gelöst, mit 0.4 ml 1 N Natronlauge versetzt und anschließend über Nacht bei Raumtemperatur gerührt. Nach vollständiger Umsetzung wurde das Dioxan am Rotationsverdampfer entfernt und das verbliebene Gemisch mit Wasser verdünnt. Anschließend wurde mit Essigsäure auf pH 4-5 angesäuert. Der ausgefallene Feststoff wurde abgesaugt, mehrmals mit Wasser gewaschen und dann drei Tage lang im Trockenschrank unter Vakuum bei 40°C getrocknet. Es wurden 71 mg (73% d. Th.) der Titelverbindung erhalten.

LC-MS (Methode 3): Rₜ = 1.33 min; m/z = 723 (M+H)⁺.

¹H-NMR (400 MHz, DMSO-*d₆*): δ [ppm] = 1.40-1.43 (m, 1H), 1.54-1.69 (m, 1H), 1.88-2.09 (m, 2H), 2.57-2.99 (m, 14H), 3.97-4.11 (m, 1H), 4.90 (q, 2H), 6.83 (t, 1H), 7.00 (dd, 2H), 7.09-7.27 (m, 7H), 7.37-7.52 (m, 4H), 7.61 (d, 2H), 7.80 (d, 2H), 11.77-13.56 (br. s, ca. 2H).

### Beispiel 45

### rac-5-{[2-(4-Carboxyphenyl)ethyl](2-{5-fluor-2-[(4-{2-[4-(trifluormethyl)phenyl]ethyl}benzyl)-oxy]phenyl}ethyl)amino}-5,6,7,8-tetrahydrochinolin-2-carbonsäure

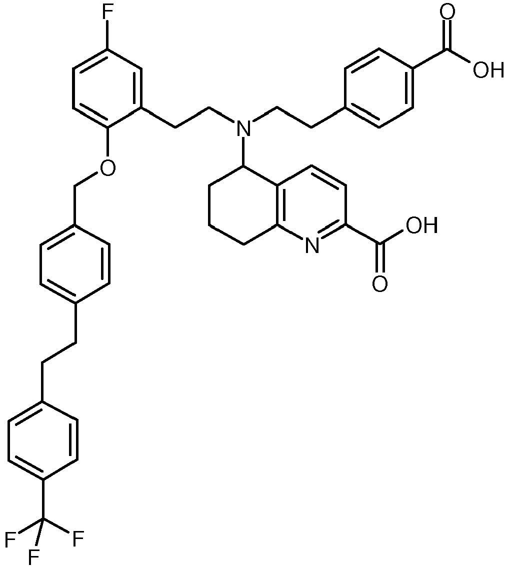

173 mg (0.22 mmol) *rac*-Ethyl-5-[(2-{5-fluor-2-[(4-{2-[4-(trifluormethyl)phenyl]ethyl}benzyl)-oxy]phenyl}ethyl){2-[4-(methoxycarbonyl)phenyl]ethyl}amino]-5,6,7,8-tetrahydrochinolin-2-carboxylat (Beispiel 160A) wurden in 4 ml Dioxan gelöst, mit 0.7 ml 1 N Natronlauge versetzt und anschließend über Nacht bei Raumtemperatur gerührt. Nach vollständiger Umsetzung wurde das Dioxan am Rotationsverdampfer entfernt und das verbliebene Gemisch mit Wasser verdünnt. Anschließend wurde mit Essigsäure auf pH 4-5 angesäuert. Der ausgefallene Feststoff wurde abgesaugt, mehrmals mit Wasser gewaschen und dann drei Tage lang im Trockenschrank unter Vakuum bei 40°C getrocknet. Es wurden 134 mg (75% d. Th.) der Titelverbindung erhalten.

LC-MS (Methode 3): Rₜ = 1.34 min; m/z = 741 (M+H)⁺.

¹H-NMR (400 MHz, DMSO-*d₆*): δ [ppm] = 1.37-1.70 (m, 2H), 1.85-2.09 (m, 2H), 2.57-2.98 (m, 14H), 3.96-4.15 (m, 1H), 4.80-4.99 (m, 2H), 6.85-7.05 (m, 3H), 7.16 (br. s, 6H), 7.42 (br. s, 4H), 7.61 (d, 2H), 7.79 (d, 2H).

### B. Bewertung der pharmakologischen Wirksamkeit

Die pharmakologische Wirkung der erfindungsgemäßen Verbindungen kann in folgenden Assays gezeigt werden:

### B-1. Stimulation der rekombinanten löslichen Guanylatcyclase (sGC) in vitro

Die Untersuchungen zur Stimulation der rekombinanten löslichen Guanylatcyclase (sGC) durch die erfindungsgemäßen Verbindungen mit und ohne Natriumnitroprussid sowie mit und ohne den Häm-abhängigen sGC-Inhibitor 1*H*-1,2,4-Oxadiazolo[4,3-a]chinoxalin-1-on (ODQ) werden nach der in folgender Literaturstelle im Detail beschriebenen Methode durchgeführt: M. Hoenicka, E. M. Becker, H. Apeler, T. Sirichoke, H. Schroeder, R. Gerzer und J.-P. Stasch, "Purified soluble guanylyl cyclase expressed in a baculovirus/Sf9 system: Stimulation by YC-1, nitric oxide, and carbon oxide", J. Mol. Med. 77 (1999), 14-23. Die Häm-freie Guanylatcyclase wird durch Zugabe von Tween 20 zum Probenpuffer (0.5% in der Endkonzentration) erhalten.

Die Aktivierung der sGC durch eine Prüfsubstanz wird als x-fache Stimulation der Basalaktivität angegeben. Das Ergebnis für Beispiel 2 ist in Tabelle 1A, das für Beispiel 23 in Tabelle 1B und das für Beispiel 39 in Tabelle 1C gezeigt:

**Tabelle 1A: Stimulation (x-fach) der rekombinanten löslichen Guanylatcyclase (sGC) in vitro durch Beispiel 2**

| **Konzentration Beispiel 2 [µM]** | **Häm-haltige sGC** | | | **Häm-freie sGC** |
|---|---|---|---|---|
| | **Basal (n=7)** | **+ 0.01 µM DEA/NO (n=5)** | **+ 10 µM ODQ (n=6)** | **Basal (n=6)** |
| 0 | 1.0 ± 0.0 | 7.0 ± 1.3 | 2.8 ± 0.3 | 1.0 ± 0.0 |
| 0.01 | 13.7 ± 1.9 | 20.2 ± 3.8 | 63.9 ± 12.2 | 5.2 ± 0.6 |
| 0.1 | 31.2 ± 3.5 | 42.7 ± 7.2 | 129.2 ± 18.9 | 19.9 ± 2.2 |
| 1.0 | 40.2 ± 4.0 | 56.5 ± 10.5 | 172.2 ± 26.3 | 82.7 ± 10.5 |
| 10 | 51.6 ± 6.4 | 62.7 ± 11.0 | 189.5 ± 28.2 | 169.5 ± 23.6 |

**Tabelle 1B: Stimulation (x-fach) der rekombinanten löslichen Guanylatcyclase (sGC) in vitro durch Beispiel 23**

| **Konzentration Beispiel 23 [µM]** | **Häm-haltige sGC** | | | **Häm-freie sGC** |
|---|---|---|---|---|
| | **Basal (n=7)** | **+ 0.1 µM DEA/NO (n=5)** | **+ 10 µM ODQ (n=6)** | **Basal (n=7)** |
| 0 | 1.0 ± 0.0 | 14.4 ± 3.0 | 2.0 ± 0.8 | 1.0 ± 0.0 |
| 0.01 | 8.3 ± 1.1 | 24.5 ± 4.4 | 16.9 ± 3.3 | 52.5 ± 3.8 |
| 0.1 | 54.2 ± 11.9 | 77.1 ± 9.2 | 105.4 ± 18.0 | 184.3 ± 15.4 |
| 1.0 | 108.7 ± 16.3 | 155.2 ± 20.7 | 216.1 ± 28.9 | 284.7 ± 18.8 |
| 10 | 135.7 ± 20.0 | 180.4 ± 22.9 | 227.0 ± 31.7 | 310.4 ± 22.6 |
| 100 | 180.5 ± 21.2 | 241.0 ± 34.4 | 261.7 ± 32.1 | 342.0 ± 27.6 |

**Tabelle 1C: Stimulation (x-fach) der rekombinanten löslichen Guanylatcyclase (sGC) in vitro durch Beispiel 39**

| **Konzentration Beispiel 39 [µM]** | **Häm-haltige sGC** | | | **Häm-freie sGC** |
|---|---|---|---|---|
| | **Basal (n=6)** | **+ 0.1 µM DEA/NO (n=5)** | **+ 10 µM ODQ (n=6)** | **Basal (n=6)** |
| 0 | 1.0 ± 0.0 | 33.4 ± 2.1 | 1.7 ± 0.2 | 1.0 ± 0.0 |
| 0.01 | 17.4 ± 2.4 | 46.5 ± 2.8 | 42.0 ± 4.9 | 39.0 ± 5.8 |
| 0.1 | 73.0 ± 7.7 | 115.5 ± 11.9 | 145.6 ± 14.7 | 176.9 ± 29.9 |
| 1.0 | 96.6 ± 8.5 | 145.2 ± 15.9 | 182.5 ± 17.6 | 284.9 ± 54.5 |
| 10 | 108.6 ± 9.0 | 159.4 ± 17.9 | 188.7 ± 16.5 | 311.5 ± 55.9 |
| 100 | 143.6 ± 13.4 | 192.1 ± 24.6 | 208.5 ± 19.0 | 309.4 ± 54.8 |

| | | | | |
|---|---|---|---|---|
| [DEA/NO = 2-(*N,N*-Diethylamino)diazenolat-2-oxid; ODQ = 1*H*-1,2,4-Oxadiazolo[4,3-a]chinoxalin-1-on]. | | | | |

Aus den Tabellen 1A, 1B und 1C ist ersichtlich, dass eine Stimulation sowohl des Häm-haltigen als auch des Häm-freien Enzyms erreicht wird. Weiterhin zeigt die Kombination von Beispiel 2, Beispiel 23 bzw. Beispiel 39 mit 2-(*N,N*-Diethylamino)diazenolat-2-oxid (DEA/NO), einem NO-Donor, keinen synergistischen Effekt, d.h. die Wirkung von DEA/NO wird nicht potenziert, wie dies bei einem über einen Häm-abhängigen Mechanismus wirkenden sGC-Aktivator zu erwarten wäre. Darüber hinaus wird die Wirkung des erfindungsgemäßen sGC-Aktivators durch 1*H*-1,2,4-Oxadiazolo[4,3-a]chinoxalin-l-on (ODQ), einen Häm-abhängigen Inhibitor der löslichen Guanylatcyclase, nicht blockiert, sondern sogar gesteigert. Die Ergebnisse in den Tabellen 1A, 1B und 1C belegen somit den Wirkmechanismus der erfindungsgemäßen Verbindungen als Aktivatoren der löslichen Guanylatcyclase.

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative Ergebnisse zu den erfindungsgemäßen Verbindungen sind in Tabelle 2 aufgeführt:

**Tabelle 2: sGC-aktivierende Wirkung in der CHO-Reporterzelle in vitro**

| **Beispiel Nr.** | **MEC [nM]** |
|---|---|
| 1 | 160 |
| 2 | 1.1 |
| 3 | 100 |
| 4 | 0.5 |
| 5 | 100 |
| 7 | 0.3 |
| 8 | 3 |
| 9 | 10 |
| 10 | 300 |
| 11 | 0.3 |
| 12 | 0.3 |
| 13 | 0.3 |
| 14 | 30 |
| 15 | 0.3 |
| 16 | 3 |
| 17 | 1000 |
| 18 | 0.3 |
| 19 | 30 |
| 20 | 3 |
| 21 | 100 |
| 22 | 1 |
| 23 | 0.7 |
| 24 | 1 |
| 25 | 0.3 |
| 26 | 1 |
| 27 | 1 |
| 28 | 1 |
| 29 | 3 |
| 30 | 1 |
| 31 | 10 |
| 32 | 0.3 |
| 33 | 10 |
| 34 | 1 |
| 35 | 3 |
| 36 | 30 |
| 37 | 1 |
| 38 | 0.3 |
| 39 | 1 |
| 42 | 3 |
| 43 | 0.3 |

| | |
|---|---|
| (MEC = minimale effektive Konzentration). | |

### B-3. Gefäßrelaxierende Wirkung in vitro

Kaninchen werden durch intravenöse Injektion von Thiopental-Natrium narkotisiert bzw. getötet (ca. 50 mg/kg) und entblutet. Die *Arteria Saphena* wird entnommen und in 3 mm breite Ringe geteilt. Die Ringe werden einzeln auf je einem triangelförmigen, am Ende offenen Häkchenpaar aus 0.3 mm starkem Spezialdraht (Remanium^{®}) montiert. Jeder Ring wird unter Vorspannung in 5 ml-Organbäder mit 37°C warmer, carbogenbegaster Krebs-Henseleit-Lösung folgender Zusammensetzung gebracht: NaCl 119 mM; KCl 4.8 mM; CaCl₂ x 2 H₂O 1 mM; MgSO₄ x 7 H₂O 1.4 mM; KH₂PO₄ 1.2 mM; NaHCO₃ 25 mM; Glucose 10 mM; Rinderserumalbumin 0.001%. Die Kontraktionskraft wird mit Statham UC2-Zellen erfasst, verstärkt und über A/D-Wandler (DAS-1802 HC, Keithley Instruments, München) digitalisiert sowie parallel auf Linienschreibern registriert. Kontraktionen werden durch Zugabe von Phenylephrin induziert.

Nach mehreren (allgemein 4) Kontrollzyklen wird die zu untersuchende Substanz in jedem weiteren Durchgang in steigender Dosierung zugesetzt und die Höhe der unter dem Einfluss der Testsubstanz erzielten Kontraktion mit der Höhe der im letzten Vordurchgang erreichten Kontraktion verglichen. Daraus wird die Konzentration errechnet, die erforderlich ist, um die in der Vorkontrolle erreichte Kontraktion auf 50% zu reduzieren (IC₅₀-Wert). Das Standard-Applikationsvolumen beträgt 5 µl. Der DMSO-Anteil in der Badlösung entspricht 0.1%.

Repräsentative Ergebnisse zu den erfindungsgemäßen Verbindungen sind in Tabelle 3 aufgeführt:

**Tabelle 3: Gefäßrelaxierende Wirkung in vitro**

| **Beispiel Nr.** | **IC₅₀ [nM]** |
|---|---|
| 1 | 571 |
| 2 | 3.6 |
| 4 | 0.3 |
| 13 | 0.1 |
| 14 | 41.8 |
| 15 | 0.2 |
| 16 | 15.5 |
| 18 | 0.4 |

### B-4. Bronchodilatierende Wirkung in vitro und in vivo

### B-4.1 Bronchorelaxation in vitro

Es werden Bronchialringe (2-3 Segmente) von Ratte, Maus oder Meerschweinchen entnommen und einzeln auf je ein triangelförmiges, am Ende offenes Häkchenpaar aus 0.3 mm starkem Spezialdraht (Remanium®) montiert. Jeder Ring wird unter Vorspannung in 5 ml-Organbäder mit 37°C warmer, carbogenbegaster Pufferlösung (z.B. Krebs-Henseleit-Lösung) gebracht. Die Bronchialringe werden mit Methacholin (1 µM) vorkontrahiert, um dann die Bronchorelaxation durch Zugabe von steigenden Konzentrationen (10⁻⁹ bis 10⁻⁶ M) der jeweiligen Testsubstanz zu untersuchen. Die Ergebnisse werden als prozentuale Relaxation in Bezug auf die Vorkonstriktion durch Methacholin ausgewertet.

### B-4.2 Tierexperimentelle Prüfung zur Untersuchung der Wirkung auf die Bronchokonstriktion im Asthmamodell

Alle Tiere (Ratten, Mäuse) werden vor Beginn des Provokationstests intragastral mit der Schlundsonde oder inhalativ behandelt. Dabei erhalten die Tiere der Behandlungsgruppen die Prüfsubstanz, die Kontrolltiere erhalten entsprechend eine Vehikel-Lösung. Nach Ablauf der Wartephase werden die Tiere narkotisiert und intubiert. Nach Legen eines Oesophagus-Katheters und Erreichen eines Steady state der Atmung wird zunächst die Lungenfunktion vor Provokation gemessen. Messparameter sind u.a. die Lungenresistance (RL) und die dynamische Compliance (Cdyn) sowie Atemzugvolumen (VT) und Atemfrequenz (f). Die Datenspeicherung und die statistische Auswertung erfolgen mit speziell für diese Lungenfunktionstests entwickelten Rechnerprogrammen (Notocord HEM).

Dann folgt eine definierte inhalative Exposition der Versuchstiere gegenüber einem Methacholin (MCh)-Aerosol (Modell einer unspezifisch induzierten asthmatischen Bronchokonstriktion). Während und 3 min nach der Exposition wird die Aufzeichnung der Lungenfunktionsparameter fortgesetzt. MCh-Konzentration und -Dosis in der Inhalationsluft werden mit einem entwickelten Feedback-Dosiskontrollsystem gesteuert und überwacht (mittels Messung der Aerosolkonzentration und des Minutenvolumens). Bei Erreichen der Zieldosis wird der Test beendet. Anhand des Resistanceanstiegs wird im Vergleich mit der scheinbehandelten Positivkontrolle die inhibitorische Wirkung der Testsubstanzen ermittelt.

### Studie im allergischen Asthmamodell:

Alle Tiere bis auf die Negativkontrolle werden mit dem Allergen Ovalbumin und Adjuvans (Alum) systemisch sensibilisiert. Die Negativkontrollgruppe erhält stattdessen physiologische KochsalzLösung (NaCl). Alle Gruppen werden dann mit Ovalbumin provoziert. In der Studie werden 6 Behandlungsgruppen - 2 Prüfsubstanzen in je 3 Dosisgruppen - eingesetzt, dazu wird eine mit Dexamethason i.p. behandelte Referenzgruppe, eine scheinbehandelte und -provozierte Negativkontrollgruppe und eine scheinbehandelte und mit Ovalbumin provozierte Positivkontrollgruppe eingesetzt. Sensibilisierungs-, Behandlungs- und Challengeprotokoll: An Tag 0, 14 und 21 werden alle Tiere mit Ovalbumin und Adjuvans i.p. sensibilisiert, die Negativkontrolle wird mit NaCl behandelt. An Tag 28 und 29 werden die Tiere mit einer intratrachealen Gabe einer Ovalbumin-Lösung provoziert. Die Prüfsubstanzen werden 1 h vor jeder intratrachealen Allergenprovokation intragastral oder inhalativ verabreicht. Eine Referenzgruppe wird 18 h und 1 h vor jeder intratrachealen Allergenprovokation mit Dexamethason i.p. behandelt. Die Positiv- und die Negativkontrollgruppe werden entsprechend mit dem Vehikel behandelt.

### Atemwegshyperreaktivität und inflammatorische Antwort:

Zunächst werden die Tiere auf Hyperreaktivität der Atemwege gegenüber unspezifischen Stimuli untersucht. Dazu wird ca. 24 h nach Ovalbumin-Challenge ein Hyperreaktivitätstest in Form einer stufenweise ansteigenden inhalativen Methacholin-Provokation durchgeführt.

Die Tiere werden narkotisiert, orotracheal intubiert und die Lungenfunktion vor Provokation bodyplethysmographisch gemessen (incl. Parametern wie Atemzugvolumen, Atemfrequenz, dynamischer Compliance und Lungenresistance). Nach Abschluss der Messungen wird für jedes Tier die Dosiswirkungskurve erstellt und die Hyperreaktivität der Positivkontrolle gegenüber der Negativkontrolle bzw. deren Hemmung in den Behandlungsgruppen ausgewertet.

Danach werden die Tiere schmerzlos getötet, Blutproben entnommen und die Lungen lavagiert (BAL). Aus der Lavageflüssigkeit wird die Gesamtzellzahl und das Differentialzellbild incl. Anzahl der Eosinophilen in der BAL bestimmt. Die restlichen Mengen an BAL-Flüssigkeit werden zunächst eingefroren. Es können dann gegebenenfalls nachträglich noch weitere Parameter (z.B. Zytokine) bestimmt werden. Das Lungengewebe wird für eine optionale histopathologische Untersuchung asserviert.

### B-5. Isoliert perfundiertes Herz nach Langendorff

Männliche Wistar-Ratten (Stamm HsdCpb:WU) mit einem Körpergewicht von 200-250 g werden mit Narcoren^{®} (100 mg/kg) narkotisiert. Nach Eröffnen des Thorax wird das Herz frei präpariert, ausgeschnitten und durch Kanulieren der Aorta an eine Langendorff-Apparatur angeschlossen. Das Herz wird retrograd mit einer Krebs-Henseleit-Pufferlösung (begast mit 95% O₂ und 5% CO₂, pH 7.4, 35°C; Zusammensetzung in mmol/l: NaCl 118; KCl 3; NaHCO₃ 22; KH₂PO₄ 1.2; MgSO₄ 1.2; CaCl₂ 1.8; Glucose 10; Na-Pyruvat 2) mit 9 ml/min flusskonstant perfundiert. Für die Erfassung der Kontraktionskraft des Herzens wird durch eine Öffnung im linken Herzohr ein an einem PE-Schlauch befestigter und mit Wasser gefüllter Ballon aus dünner Kunststofffolie in den linken Ventrikel eingeführt. Der Ballon wird mit einem Druckaufnehmer verbunden. Der enddiastolische Druck wird auf 5-10 mmHg über das Ballonvolumen eingestellt. Der Perfusionsdruck wird mit Hilfe eines zweiten Druckaufnehmers detektiert. Die Daten werden über einen Brückenverstärker an einen Computer gesendet und registriert.

Nach 40 min Äquilibrierungszeit wird die jeweilige Testsubstanz in einer Endkonzentration von 10⁻⁷ mol/l der Perfusionslösung für 20 min hinzugefügt, was als Zeichen der koronaren Dilatation zu einer Reduktion des Perfusionsdruckes führt. Danach werden die Herzen für weitere 120 min ohne Testsubstanz perfundiert (Auswaschphase). Für die Bestimmung der Reversibilität der Perfusionsdrucksenkung (Wash out-Score) wird der Wert des Perfusionsdruckes nach 60 min der Auswaschphase auf die maximale Perfusionsdrucksenkung durch die Testsubstanz bezogen und prozentuell dargestellt. Der so erhaltene Wash out-Score wird als Maß für die Verweildauer der Testsubstanz am Wirkort gewertet.

### B-6. Hämodynamik im narkotisierten Ferkel

Es werden 2-6 kg schwere, gesunde Göttinger Minipigs^{®} Ellegaard (Ellegaard, Dänemark) beiderlei Geschlechts verwendet. Die Tiere werden sediert durch i.m.-Gabe von ca. 25 mg/kg Ketamin und ca. 10 mg/kg Azaperon. Die Narkose-Einleitung erfolgt durch i.v.-Gabe von ca. 2 mg/kg Ketamin und ca. 0.3 mg/kg Midazolam. Die Narkose-Erhaltung erfolgt durch i.v.-Gabe von ca. 7.5-30 mg/kg/h Ketamin und ca. 1-4 mg/kg/h Midazolam (Infusionsrate 1-4 ml/kg/h) sowie ca. 150 µg/kg/h Pancuroniumbromid (z.B. Pancuronium-Actavis). Nach der Intubation werden die Tiere über die Beatmungsmaschine mit konstantem Atemvolumen beatmet (10-12 ml/kg, 35 Atemzüge/min; Avea^{®}, Viasys Healthcare, USA, oder Engström Carestation, GE Healthcare, Freiburg, Deutschland), so dass eine endtidale CO₂-Konzentration von etwa 5% erreicht wird. Die Beatmung erfolgt mit Raumluft, angereichert mit ca. 40% Sauerstoff (Normoxie). Zur Messung der hämodynamischen Parameter, wie z.B. pulmonal-arterieller Druck (PAP), Blutdruck (BP) und Herzfrequenz (HR), werden Katheter in die A. carotis zur Messung des Blutdrucks und ein Swan-Ganz^{®}-Katheter über die V. jugularis in die Pulmonalarterie eingeschwemmt. Die hämodynamischen Signale werden mittels Druckaufnehmern (Combitransducer, B. Braun, Melsungen, Deutschland) / Verstärkern und Ponemah^{®} als Datenerfassungssoftware aufgezeichnet und ausgewertet.

Nach Beendigung der Instrumentierung der Tiere wird zur Erhöhung des pulmonal-arteriellen Drucks eine Dauerinfusion mit einem Thromboxan A₂-Analogon gestartet. Es werden ca. 0.3-0.75 µg/kg/min 9,11-Dideoxy-9α,11α-epoxymethanoprostaglandin F_{2α} (U-44069; Sigma, Kat.-Nr. D0400, oder Cayman Chemical Company, Kat.-Nr. 16440), gelöst in physiologischer KochsalzLösung, infundiert, um einen Anstieg des mittleren pulmonal-arteriellen Drucks auf Werte über 25 mmHg zu erreichen. 30 Minuten nach Start der Infusion stellt sich ein Plateau ein, und das Experiment wird gestartet.

Die Testsubstanzen werden als i.v.-Infusion oder per Inhalation verabreicht. Zur Herstellung der Inhalationslösung wird folgendermaßen vorgegangen: Für ein 4 kg schweres Tier wird zur Herstellung der Stammlösung (300 µg/kg) 1.2 mg der Testverbindung eingewogen und in 3 ml Gesamtvolumen (1% DMSO, 99% 0.2%-ige Zitronensäurelösung, 1 N Natronlauge zur Adjustierung des pH auf 8) gelöst. Die Lösung wird dann mit 0.2%-iger Zitronensäure, die zuvor mit Natronlauge auf pH 8 eingestellt wurde, bis zur eingesetzten Konzentration verdünnt. Pro Versuch werden pro 4 kg-Tier 3 ml der Testverbindungslösung mittels des Aeroneb^{®} Pro-Verneblersystems im Inhalationsschenkel des Beatmungskreislaufs vernebelt. Die mittlere Vernebelungszeit beträgt ca. 7 min nach Start der Vernebelung.

### B-7. Inhalative Gabe von sGC-Aktivatoren in PAH-Tiermodellen

Die Experimente werden in narkotisierten Göttinger Minischweinen, narkotisierten Ratten oder wachen, telemetrisch instrumentierten Hunden durchgeführt. Akute pulmonale Hypertonie wird z.B. durch Infusion eines Thromboxan A₂-Analogons, durch akute oder mehrwöchige Hypoxiebehandlung und/oder durch Monocrotalin-Gabe induziert. Die Vernebelung der Testsubstanzen erfolgt unter Verwendung des Nebutec^{®}- oder Aeroneb^{®} Pro-Verneblersystems, über Pulver- und/ oder Lösungsapplikatoren zur experimentellen intratrachealen Applikation (Liquid MicroSprayer^{®}, Dry Powder Insufflator™, MicroSprayer^{®}, Penn-Century Inc., Wyndmoor, PA, USA) oder nach Feststoffvernebelung, die in den Inspirationsschenkel der Beatmung geschaltet werden. Die Substanzen werden in Abhängigkeit von der molekularen Struktur als Feststoffe oder Lösungen eingesetzt. Die hämodynamischen Signale werden mittels Druckaufnehmern / Verstärkern (Combitransducer B. Braun, Melsungen, Deutschland oder CardioMEMS Inc., Atlanta, GA, USA) und Ponemah^{®} oder CardioMems^{®} als Datenerfassungssoftware aufgezeichnet und ausgewertet. Nach Langzeitexperimenten (z.B. Monocrotalin-Ratte) kann auch eine histologische Auswertung erfolgen.

### B-8. Radiotelemetrische Messung von Blutdruck und Herzfrequenz an wachen Ratten

Für die im Folgenden beschriebenen Messungen an wachen Ratten wird ein im Handel erhältliches Telemetriesystem der Firma Data Sciences International DSI, USA, eingesetzt. Das System besteht aus 3 Hauptkomponenten: (*1*) Implantierbare Sender (Physiotel^{®} Telemetrietransmitter), (2) Empfänger (Physiotel^{®} Receiver), die über einen Multiplexer (DSI Data Exchange Matrix) mit einem (3) Datenakquisitionscomputer verbunden sind. Die Telemetrieanlage ermöglicht eine kontinuierliche Erfassung von Blutdruck, Herzfrequenz und Körperbewegung an wachen Tieren in ihrem gewohnten Lebensraum.

Die Untersuchungen werden an ausgewachsenen weiblichen Wistar-Ratten mit einem Körpergewicht von > 200 g durchgeführt. Die Versuchstiere werden nach Senderimplantation einzeln in Makrolon-Käfigen Typ 3 gehalten. Sie haben freien Zugang zu Standardfutter und Wasser. Der Tag/Nacht-Rhythmus im Versuchslabor wird per Raumbeleuchtung um 6:00 Uhr morgens und um 19:00 Uhr abends gewechselt.

### Senderimplantation:

Die eingesetzten Telemetriesender (TA11 PA-C40, DSI) werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobarbital (Nembutal^{®}, Sanofi, 50 mg/kg i.p.) narkotisiert und an der Bauchseite weiträumig rasiert und desinfiziert. Nach Eröffnung des Bauchraumes entlang der Linea alba wird der flüssigkeitsgefüllte Messkatheter des Systems oberhalb der Bifurcation nach cranial in die Aorta descendens eingesetzt und mit Gewebekleber (VetBonD™, 3M) befestigt. Das Sendergehäuse wird intraperitoneal an der Bauchwandmuskulatur fixiert und die Wunde schichtweise verschlossen. Postoperativ wird zur Infektionsprophylaxe ein Antibiotikum (Oxytetracyclin^{®} 10%, 60 mg/kg s.c., 0.06 ml/100 g Körpergewicht, Beta-Pharma GmbH, Deutschland) sowie ein Analgetikum (Rimadyl^{®}, 4 mg/kg s.c., Pfizer, Deutschland) verabreicht.

### Substanzen und Lösungen:

Wenn nicht anders beschrieben, werden die zu untersuchenden Substanzen jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert. Eine Lösungsmittel-behandelte Gruppe von Tieren wird als Kontrolle eingesetzt.

### Versuchsablauf:

Die Telemetrie-Messeinrichtung ist für 24 Tiere konfiguriert. Jeder Versuch wird unter einer Versuchsnummer registriert.

Den in der Anlage lebenden instrumentierten Ratten ist jeweils eine eigene Empfangsantenne zugeordnet (1010 Receiver, DSI). Die implantierten Sender sind über einen eingebauten Magnetschalter von außen aktivierbar und werden bei Versuchsvorlauf auf Sendung geschaltet. Die ausgestrahlten Signale können durch ein Datenakquisitionssystem (Dataquest™ A.R.T. for Windows, DSI) online erfasst und entsprechend aufgearbeitet werden. Die Ablage der Daten erfolgt jeweils in einem hierfür eröffneten Ordner, der die Versuchsnummer trägt.

Im Standardablauf werden über je 10 Sekunden Dauer gemessen: (*1*) systolischer Blutdruck (SBP), (2) diastolischer Blutdruck (DBP), (3) arterieller Mitteldruck (MAP), (4) Herzfrequenz (HR) und (5) Aktivität (ACT).

Die Messwerterfassung wird rechnergesteuert in 5 Minuten-Abständen wiederholt. Die als Absolutwert erhobenen Quelldaten werden im Diagramm mit dem aktuell gemessenen Barometerdruck (Ambient Pressure Reference Monitor, APR-1) korrigiert und in Einzeldaten abgelegt. Weitere technische Details sind in der umfangreichen Dokumentation der Herstellerfirma (DSI) aufgeführt.

Wenn nicht anders beschrieben, erfolgt die Verabreichung der Prüfsubstanzen am Versuchstag um 9:00 Uhr. Im Anschluss an die Applikation werden die oben beschriebenen Parameter über 24 Stunden gemessen.

### Auswertung:

Nach Versuchsende werden die erhobenen Einzeldaten mit der Analysis-Software (Dataquest™ A.R.T. 4.1 Analysis) sortiert. Als Leerwert wird der Zeitpunkt 2 Stunden vor Substanz-Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten-Mittelwert) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden in Papierform nach Nummern sortiert in Ordnern abgelegt.

### Literatur:

K. Witte, K. Hu, J. Swiatek, C. Müssig, G. Ertl und B. Lemmer, Experimental heart failure in rats: effects on cardiovascular circadian rhythms and on myocardial β-adrenergic signaling, Cardiovasc. Res. 47 (2), 350-358 (2000).

### B-9. Testung des Desaturierungspotentials von Substanzen (Ventilations-Perfusions-Mismatch)

Es werden 4-5 kg schwere, gesunde Göttinger Minipigs^{®} Ellegaard (Ellegaard, Dänemark) beiderlei Geschlechts verwendet. Die Tiere werden sediert durch i.m.-Gabe von ca. 25 mg/kg Ketamin und ca. 10 mg/kg Azaperon. Die Narkose-Einleitung erfolgt durch i.v.-Gabe von ca. 2 mg/kg Ketamin und ca. 0.3 mg/kg Midazolam. Die Narkose-Erhaltung erfolgt durch i.v.-Gabe von ca. 7.5-30 mg/kg/h Ketamin und ca. 1-4 mg/kg/h Midazolam (Infusionsrate 1-4 ml/kg/h) sowie ca. 150 µg/kg/h Pancuroniumbromid (z.B. Pancuronium-Actavis). Nach der Intubation werden die Tiere über die Beatmungsmaschine mit konstantem Atemvolumen beatmet (50-60 ml, 30 Atemzüge/min; Avea^{®}, Viasys Healthcare, USA, oder Engström Carestation, GE Healthcare, Freiburg, Deutschland), so dass eine endtidale CO₂-Konzentration von etwa 5% erreicht wird. Die Beatmung erfolgt mit Raumluft, angereichert mit ca. 40% Sauerstoff (Normoxie), und wird so angepasst, dass ein positiver end-expiratorischer Druck von 5 cm Wassersäule erreicht wird. Zur Messung der hämodynamischen Parameter, wie z.B. pulmonal-arterieller Druck (PAP), Blutdruck (BP) und Herzfrequenz (HR), werden Katheter in die A. carotis zur Messung des Blutdrucks und ein Swan-Ganz^{®}-Katheter über die V. jugularis in die Pulmonalarterie eingeschwemmt. Die hämodynamischen Signale werden mittels Druckaufnehmern (Combitransducer, B. Braun, Melsungen, Deutschland) / Verstärkern und Ponemah^{®} als Datenerfassungssoftware aufgezeichnet und ausgewertet. Ein 4 French-Oximetrie-Katheter (Edwards Lifesciences, Irvine, CA, USA) wird in der linken Femoralarterie platziert und mit einem Vigilance Monitor (Edwards Lifesciences, Irvine, CA, USA) verbunden, um die arterielle Sauerstoffsättigung (SaO₂) zu messen.

Alle hämodynamischen Parameter werden kontinuierlich gemessen; zur Auswertung werden die Mittelwerte stabiler Intervalle von mindestens 1 min (bei Extremwerten, z.B. maximaler PAP-Anstieg) und/oder 3 min (bei Basalbedingungen) gebildet. Die Blutgase (Stat Profile pHOx plus L; Nova Biomedical, Waltham, MA, USA) werden 3 min nach Start jedes unilateralen Bronchookklusionszyklus bestimmt. Rechtsseitige Einzel-Lungenventilation wird dadurch erreicht, dass der Trachealtubus in den rechten Hauptbronchus vorgeschoben und der linke Teil der Lunge durch Inflation eines Ballons von der Ventilation abgeschlossen wird. Die Platzierung des Tubus wird mittels Auskultation bestätigt. In jedem Tier werden mehrere Zyklen von 10 min Dauer Univentilation durchgeführt, jeweils unterbrochen von 30 min Biventilation. Die ersten Zyklen werden als Kontrollzyklen verwendet, um die Reproduzierbarkeit der Zyklen zu gewährleisten. Anschließend wird der Einfluss von Lösungsmittel (Vehikel) und darin gelöster Testsubstanz nach intravenöser und/oder inhalativer Gabe auf folgende Hauptparameter gemessen: Blutdruck (BP), Pulmonaldruck (PAP) und arterielle Sauerstoffsättigung (SaO₂). Ziel dieses Tiermodells ist es, eine Substanz zu identifizieren, die eine möglichst große Reduktion des PAP bzw. des Hypoxie-bedingten PAP-Anstiegs bewirkt (gewünschte Wirkung), ohne dabei durch Dilatation von Pulmonalarterien in nicht-ventilierten Lungenbereichen zu einem Anstieg der Sauerstoffentsättigung zu führen (unerwünschte Wirkung).

### Literatur:

E.M. Becker et al., "V/Q mismatch" bei sekundärer pulmonaler Hypertonie - Riociguat im Vergleich, Pneumologie 65 (Suppl. 2), S122-S123 (2011).

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 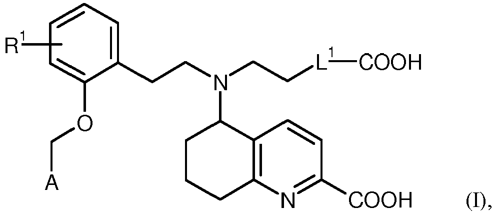 in welcher
R¹ für Wasserstoff oder Fluor steht,
L¹ für Ethan-1,2-diyl oder 1,4-Phenylen steht,
und
A für eine Gruppe der Formel 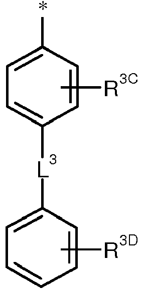
steht, worin
* die jeweilige Verknüpfungsstelle mit dem Rest des Moleküls kennzeichnet,
L³ eine Bindung, -O-, -CH₂-, -CH₂-CH₂- oder -CH=CH- bedeutet,
und
R^{3C} einen Substituenten ausgewählt aus der Reihe Fluor, Chlor, Brom, Cyano, (C₁-C₄)-Alkyl, Difluormethyl, Trifluormethyl, (C₁-C₄)-Alkoxy, Difluormethoxy und Trifluormethoxy bedeutet,
und R^{3D} einen Substituenten ausgewählt aus der Reihe Wasserstoff, Fluor, Chlor, Brom, Cyano, (C₁-C₄)-Alkyl, Difluormethyl, Trifluormethyl, (C₁-C₄)-Alkoxy, Difluormethoxy und Trifluormethoxy bedeutet,
sowie ihre Salze, Solvate und Solvate der Salze.

2. Verbindung der Formel (I) nach Anspruch 1, in welcher
R¹ für Wasserstoff oder Fluor steht,
L¹ für Ethan-1,2-diyl oder 1,4-Phenylen steht,
und
A für eine Gruppe der Formel 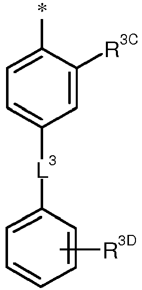
steht, worin
* die jeweilige Verknüpfungsstelle mit dem Rest des Moleküls kennzeichnet,
L³ eine Bindung, -CH₂-CH₂- oder -CH=CH- bedeutet,
R^{3C} Fluor, Chlor, Methyl oder Trifluormethyl bedeutet,
und
R^{3D} Wasserstoff, Fluor, Chlor, Cyano, Methyl, Trifluormethyl, Methoxy oder Trifluormethoxy bedeutet,
sowie ihre Salze, Solvate und Solvate der Salze.

3. Verbindung der Formel (I) nach Anspruch 1 oder 2, in welcher
R¹ für Wasserstoff oder Fluor steht,
L¹ für Ethan-1,2-diyl oder 1,4-Phenylen steht,
und
A für eine Gruppe der Formel 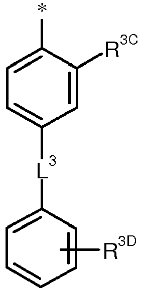
steht, worin
* die jeweilige Verknüpfungsstelle mit dem Rest des Moleküls kennzeichnet,
L³ eine Bindung, -CH₂-CH₂- oder -CH=CH- bedeutet,
R^{3C} Fluor, Chlor, Methyl oder Trifluormethyl bedeutet,
und
R^{3D} Wasserstoff, Fluor, Chlor, Cyano, Methyl, Trifluormethyl oder Trifluormethoxy bedeutet,
sowie ihre Salze, Solvate und Solvate der Salze.

4. Verbindung der Formel (I) nach Anspruch 1, 2 oder 3, in welcher
R¹ für Wasserstoff oder Fluor steht,
L¹ für Ethan-1,2-diyl oder 1,4-Phenylen steht,
und
A für eine Gruppe der Formel 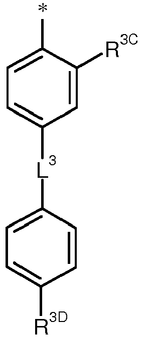
steht, worin
* die jeweilige Verknüpfungsstelle mit dem Rest des Moleküls kennzeichnet,
L³ eine Bindung oder -CH₂-CH₂- bedeutet,
R^{3C} Chlor bedeutet,
und
R^{3D} Wasserstoff, Fluor oder Trifluormethyl bedeutet,
sowie ihre Salze, Solvate und Solvate der Salze.

5. 5-{[2-(4-Carboxyphenyl)ethyl][2-(2-{[3-chlor-4'-(trifluormethyl)biphenyl-4-yl]methoxy}-phenyl)ethyl]amino}-5,6,7,8-tetrahydrochinolin-2-carbonsäure der folgenden Formel 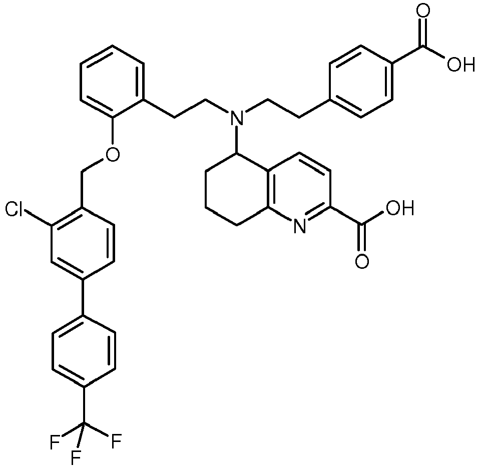 sowie ihre Salze, Solvate und Solvate der Salze.

6. 5-{(4-Carboxybutyl)[2-(2-{[3-chlor-4'-(trifluormethyl)biphenyl-4-yl]methoxy}phenyl)ethyl]-amino}-5,6,7,8-tetrahydrochinolin-2-carbonsäure der folgenden Formel 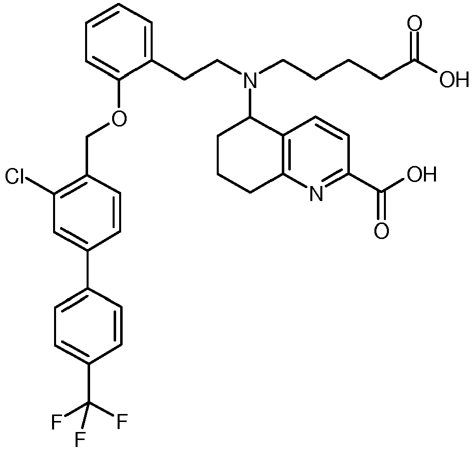 sowie ihre Salze, Solvate und Solvate der Salze.

7. Verfahren zur Herstellung einer Verbindung der Formel (I), wie in den Ansprüchen 1 bis 6 definiert, **dadurch gekennzeichnet, dass** man entweder
[A] eine Verbindung der Formel (II) 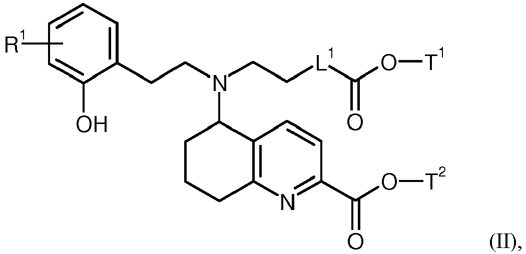 in welcher R¹ und L¹ die in den Ansprüchen 1 bis 6 angegebenen Bedeutungen haben und
T¹ und T² gleich oder verschieden sind und für (C₁-C₄)-Alkyl stehen,
in Gegenwart einer Base mit einer Verbindung der Formel (III) 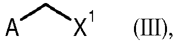 in welcher A die in den Ansprüchen 1 bis 6 angegebenen Bedeutungen hat und
X¹ für eine Abgangsgruppe wie beispielsweise Chlor, Brom, Iod, Mesylat, Triflat oder Tosylat steht,
umsetzt oder
[B] eine Verbindung der Formel (IV) 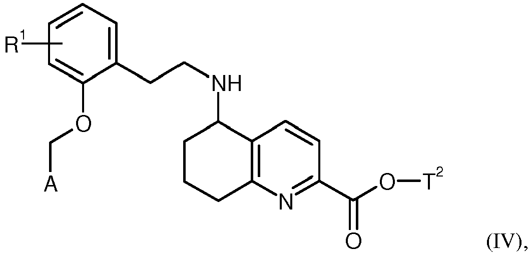 in welcher R¹ und A die in den Ansprüchen 1 bis 6 angegebenen Bedeutungen haben und
T² für (C₁-C₄)-Alkyl steht,
in Gegenwart einer Base mit einer Verbindung der Formel (V) 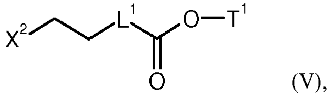 in welcher L¹ die in den Ansprüchen 1 bis 6 angegebenen Bedeutungen hat,
T¹ für (C₁-C₄)-Alkyl steht,
und
X² für eine Abgangsgruppe wie beispielsweise Chlor, Brom, Iod, Mesylat, Triflat oder Tosylat steht,
umsetzt,
und die jeweils resultierende Verbindung der Formel (VI) 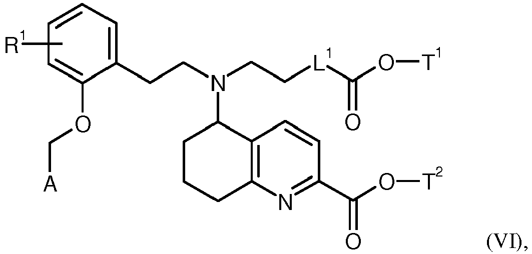 in welcher R¹, A, L¹, T¹ und T² die oben angegebenen Bedeutungen haben,
dann durch Hydrolyse der Ester-Gruppierungen -C(O)OT¹ und -C(O)OT² in die entsprechende Dicarbonsäure der Formel (I) überführt
und die so erhaltenen Verbindungen der Formel (I) gegebenenfalls in ihre Enantiomere und/oder Diastereomere trennt und/oder gegebenenfalls mit den entsprechenden (i) Lösungsmitteln und/oder (ii) Basen oder Säuren zu ihren Solvaten, Salzen und/oder Solvaten der Salze umsetzt.

8. Verbindung, wie in einem der Ansprüche 1 bis 6 definiert, zur Behandlung und/oder Prävention von Krankheiten.

9. Verbindung, wie in einem der Ansprüche 1 bis 6 definiert, zur Verwendung in einem Verfahren zur Behandlung und/oder Prävention von primären und sekundären Formen der pulmonalen Hypertonie, Herzinsuffizienz, Angina pectoris, Hypertonie, thromboembolischen Erkrankungen, Ischämien, Gefäßerkrankungen, Mikrozirkulationsstörungen, Niereninsuffizienz, fibrotischen Erkrankungen und Arteriosklerose.

10. Verwendung einer Verbindung, wie in einem der Ansprüche 1 bis 6 definiert, zur Herstellung eines Arzneimittels zur Behandlung und/oder Prävention von primären und sekundären Formen der pulmonalen Hypertonie, Herzinsuffizienz, Angina pectoris, Hypertonie, thromboembolischen Erkrankungen, Ischämien, Gefäßerkrankungen, Mikrozirkulationsstörungen, Niereninsuffizienz, fibrotischen Erkrankungen und Arteriosklerose.

11. Arzneimittel enthaltend eine Verbindung, wie in einem der Ansprüche 1 bis 6 definiert, in Kombination mit einem oder mehreren inerten, nicht-toxischen, pharmazeutisch geeigneten Hilfsstoffen.

12. Arzneimittel enthaltend eine Verbindung, wie in einem der Ansprüche 1 bis 6 definiert, in Kombination mit einem oder mehreren weiteren Wirkstoffen ausgewählt aus der Gruppe bestehend aus organischen Nitraten, NO-Donatoren, PDE 5-Inhibitoren, Prostacyclin-Analoga, IP-Rezeptor-Agonisten, Endothelin-Rezeptor-Antagonisten, Guanylatcyclase-Stimulatoren, Tyrosinkinase-Inhibitoren, anti-obstruktiv wirkenden Mitteln, entzündungshemmmenden und/oder immunsuppressiven Mitteln, antithrombotisch wirkenden Mitteln, den Blutdruck senkenden Mitteln sowie den Fettstoffwechsel verändernden Mitteln.

13. Arzneimittel nach Anspruch 11 oder 12 zur Behandlung und/oder Prävention von primären und sekundären Formen der pulmonalen Hypertonie, Herzinsuffizienz, Angina pectoris, Hypertonie, thromboembolischen Erkrankungen, Ischämien, Gefäßerkrankungen, Mikrozirkulationsstörungen, Niereninsuffizienz, fibrotischen Erkrankungen und Arteriosklerose.

## Claims

1. Compound of the formula (I) 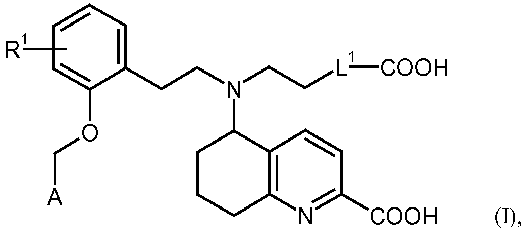 in which
R¹ represents hydrogen or fluorine,
L¹ represents ethane-1,2-diyl or 1,4-phenylene,
and
A represents a group of the formula 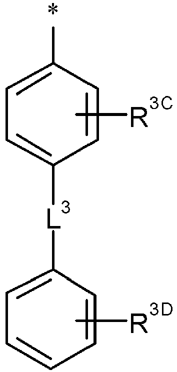
in which
* denotes the respective point of attachment to the remainder of the molecule,
L³ represents a bond, -O-, -CH₂-, -CH₂-CH₂- or -CH=CH-,
and
R^{3C} represents a substituent selected from the group consisting of fluorine, chlorine, bromine, cyano, (C₁-C₄)-alkyl, difluoromethyl, trifluoromethyl, (C₁-C₄)-alkoxy, difluoromethoxy and trifluoromethoxy,
and R^{3D} represents a substituent selected from the group consisting of hydrogen, fluorine, chlorine, bromine, cyano, (C₁-C₄)-alkyl, difluoromethyl, trifluoromethyl, (C₁-C₄)-alkoxy, difluoromethoxy and trifluoromethoxy,
and salts, solvates and solvates of the salts thereof.

2. Compound of the formula (I) according to Claim 1 in which
R¹ represents hydrogen or fluorine,
L¹ represents ethane-1,2-diyl or 1,4-phenylene,
and
A represents a group of the formula 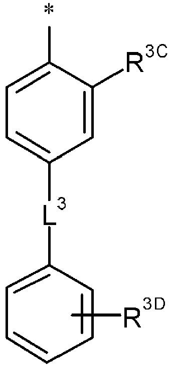
in which
* denotes the respective point of attachment to the remainder of the molecule,
L³ represents a bond, -CH₂-CH₂- or -CH=CH-,
R^{3C} represents fluorine, chlorine, methyl or trifluoromethyl,
and
R^{3D} represents hydrogen, fluorine, chlorine, cyano, methyl, trifluoromethyl, methoxy or trifluoromethoxy,
and salts, solvates and solvates of the salts thereof.

3. Compound of the formula (I) according to Claim 1 or 2 in which
R¹ represents hydrogen or fluorine,
L¹ represents ethane-1,2-diyl or 1,4-phenylene,
and
A represents a group of the formula 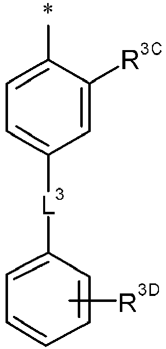
in which
* denotes the respective point of attachment to the remainder of the molecule,
L³ represents a bond, -CH₂-CH₂- or -CH=CH-,
R^{3C} represents fluorine, chlorine, methyl or trifluoromethyl,
and
R^{3D} represents hydrogen, fluorine, chlorine, cyano, methyl, trifluoromethyl or trifluoromethoxy,
and salts, solvates and solvates of the salts thereof.

4. Compound of the formula (I) according to Claim 1, 2 or 3 in which
R¹ represents hydrogen or fluorine,
L¹ represents ethane-1,2-diyl or 1,4-phenylene,
and
A represents a group of the formula 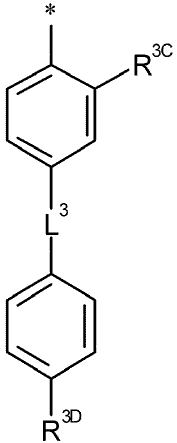
in which
* denotes the respective point of attachment to the remainder of the molecule,
L³ represents a bond or -CH₂-CH₂-,
R^{3C} represents chlorine,
and
R^{3D} represents hydrogen, fluorine or trifluoromethyl,
and salts, solvates and solvates of the salts thereof.

5. 5-{[2-(4-carboxyphenyl)ethyl][2-(2-{[3-chloro-4'-(trifluoromethyl)biphenyl-4-yl]methoxy}phenyl)ethyl]amino}-5,6,7,8-tetrahydroquinoline-2-carboxylic acid of the formula below 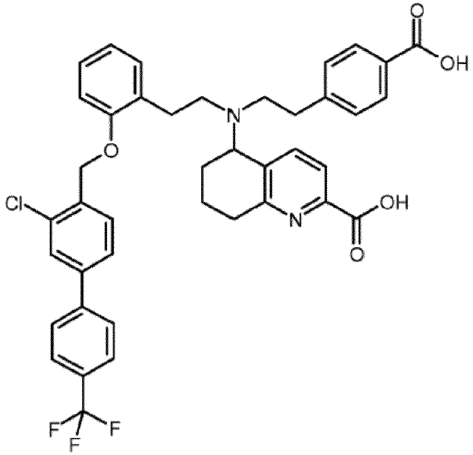 and salts, solvates and solvates of the salts thereof.

6. 5-{(4-Carboxybutyl)[2-(2-{[3-chloro-4'-(trifluoromethyl)biphenyl-4-yl]methoxy}phenyl)ethyl]amino}-5,6,7,8-tetrahydroquinoline-2-carboxylic acid of the formula below 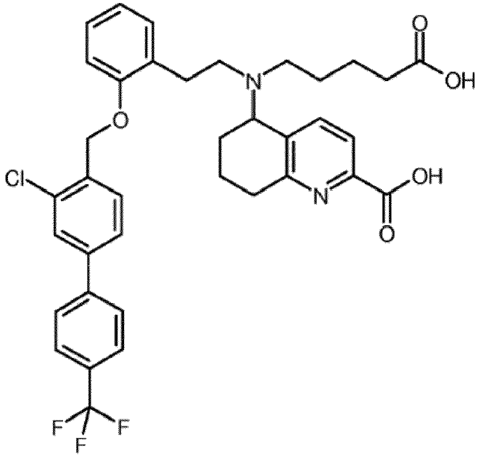 and salts, solvates and solvates of the salts thereof.

7. Process for preparing a compound of the formula (I) as defined in any of Claims 1 to 6, **characterized in that** either
[A] a compound of the formula (II) 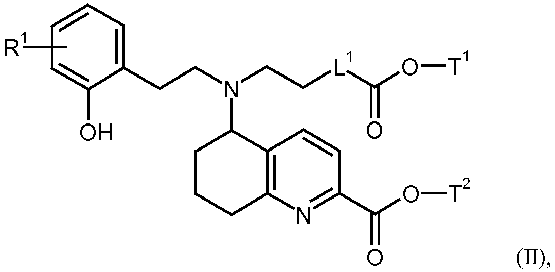 in which R¹ and L¹ have the meanings given in any of Claims 1 to 6 and
T¹ and T² are identical or different and represent (C₁-C₄)-alkyl,
is reacted in the presence of a base with a compound of the formula (III) 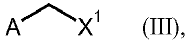 in which A has the meanings given in any of Claims 1 to 6 and
X¹ represents a leaving group such as, for example, chlorine, bromine, iodine, mesylate, triflate or tosylate,
or
[B] a compound of the formula (IV) 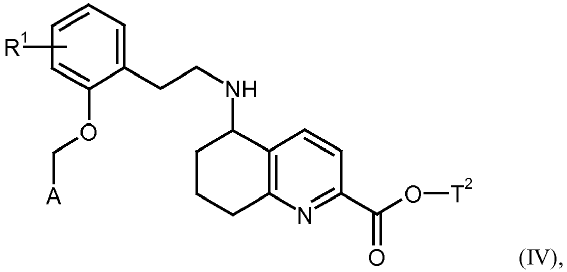 in which R¹ and A have the meanings given in any of Claims 1 to 6 and
T² represents (C₁-C₄)-alkyl,
is reacted in the presence of a base with a compound of the formula (V) 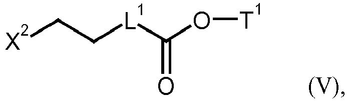 in which L¹ has the meanings given in any of Claims 1 to 6,
T¹ represents (C₁-C₄)-alkyl,
and
X² represents a leaving group such as, for example, chlorine, bromine, iodine, mesylate, triflate or tosylate,
and the respective resulting compound of the formula (VI) 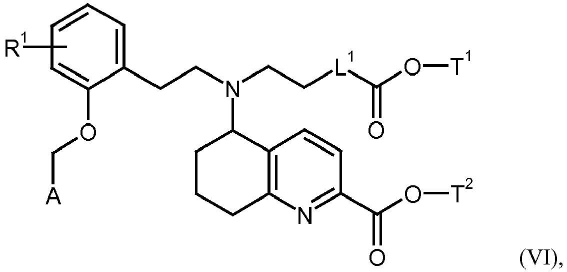 in which R¹, A, L¹, T¹ and T² have the meanings given above,
is then converted by hydrolysis of the ester groupings -C(O)OT¹ and -C(O)OT² into the corresponding dicarboxylic acid of the formula (I)
and the compounds of the formula (I) obtained in this manner are optionally separated into their enantiomers and/or diastereomers and/or optionally converted with the appropriate (i) solvents and/or (ii) bases or acids into their solvates, salts and/or solvates of the salts.

8. Compound as defined in any of Claims 1 to 6 for the treatment and/or prevention of diseases.

9. Compound as defined in any of Claims 1 to 6 for use in a method for the treatment and/or prevention of primary and secondary forms of pulmonary hypertension, heart failure, angina pectoris, hypertension, thromboembolic disorders, ischaemias, vascular disorders, impaired microcirculation, renal insufficiency, fibrotic disorders and arteriosclerosis.

10. Use of a compound as defined in any of Claims 1 to 6 for the preparation of medicament for the treatment and/or prevention of primary and secondary forms of pulmonary hypertension, heart failure, angina pectoris, hypertension, thromboembolic disorders, ischaemias, vascular disorders, impaired microcirculation, renal insufficiency, fibrotic disorders and arteriosclerosis.

11. Medicament, comprising a compound as defined in any of Claims 1 to 6 in combination with one or more inert non-toxic pharmaceutically suitable auxiliaries.

12. Medicament, comprising a compound as defined in any of Claims 1 to 6 in combination with one or more further active compounds selected from the group consisting of organic nitrates, NO donors, PDE 5 inhibitors, prostacyclin analogues, IP receptor agonists, endothelin receptor antagonists, guanylate cyclase stimulators, tyrosine kinase inhibitors, anti-obstructive agents, anti-inflammatory and/oder immunsuppressive agents, antithrombotic agents, agents for lowering blood pressure and agents that alter fat metabolism.

13. Medicament according to Claim 11 or 12 for the treatment and/or prevention of primary and secondary forms of pulmonary hypertension, heart failure, angina pectoris, hypertension, thromboembolic disorders, ischaemias, vascular disorders, impaired microcirculation, renal insufficiency, fibrotic disorders and arteriosclerosis.

## Revendications

1. Composé de formule (I) 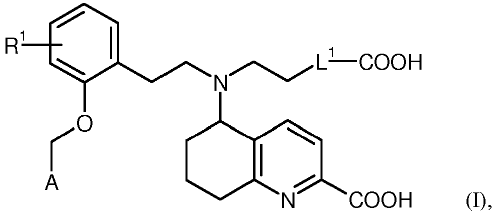 dans laquelle
R¹ représente hydrogène ou fluor,
L¹ représente éthane-1,2-diyle ou 1,4-phénylène et
A représente un groupe de formule 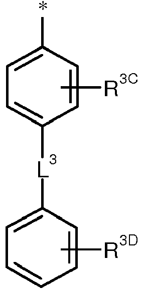
dans laquelle
* caractérise le site de liaison avec le reste de la molécule,
L³ signifie une liaison, -O-, -CH₂-, -CH₂-CH₂- ou -CH=CH- et
R^{3C} signifie un substituant choisi dans la série fluor, chlore, brome, cyano, (C₁-C₄)-alkyle, difluorométhyle, trifluorométhyle, (C₁-C₄)-alcoxy, difluorométhoxy et trifluorométhoxy et
R^{3D} signifie un substituant choisi dans la série hydrogène, fluor, chlore, brome, cyano, (C₁-C₄)-alkyle, difluorométhyle, trifluorométhyle, (C₁-C₄)-alcoxy, difluorométhoxy et trifluorométhoxy,
ainsi que ses sels, solvates et solvates des sels.

2. Composé de formule (I) selon la revendication 1, dans laquelle
R¹ représente hydrogène ou fluor,
L¹ représente éthane-1,2-diyle ou 1,4-phénylène et
A représente un groupe de formule 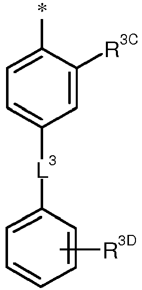
dans laquelle
* caractérise le site de liaison avec le reste de la molécule,
L³ signifie une liaison, -CH₂-CH₂- ou -CH=CH-,
R^{3C} signifie fluor, chlore, méthyle ou trifluorométhyle et
R^{3D} signifie hydrogène, fluor, chlore, cyano, méthyle, trifluorométhyle, méthoxy ou trifluorométhoxy,
ainsi que ses sels, solvates et solvates des sels.

3. Composé de formule (I) selon la revendication 1 ou 2, dans laquelle
R¹ représente hydrogène ou fluor,
L¹ représente éthane-1,2-diyle ou 1,4-phénylène et
A représente un groupe de formule 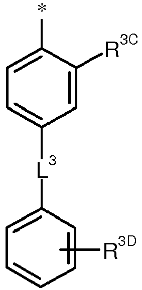
dans laquelle
* caractérise le site de liaison avec le reste de la molécule,
L³ signifie une liaison, -CH₂-CH₂- ou -CH=CH-,
R^{3C} signifie fluor, chlore, méthyle ou trifluorométhyle et
R^{3D} signifie hydrogène, fluor, chlore, cyano, méthyle, trifluorométhyle ou trifluorométhoxy,
ainsi que ses sels, solvates et solvates des sels.

4. Composé de formule (I) selon la revendication 1, 2 ou 3, dans laquelle
R¹ représente hydrogène ou fluor,
L¹ représente éthane-1,2-diyle ou 1,4-phénylène et
A représente un groupe de formule 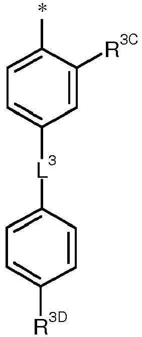
dans laquelle
* caractérise le site de liaison avec le reste de la molécule,
L³ signifie une liaison ou -CH₂-CH₂-,
R^{3C} signifie chlore et
R^{3D} signifie hydrogène, fluor ou trifluorométhyle,
ainsi que ses sels, solvates et solvates des sels.

5. Acide 5-{[2-(4-carboxyphényl)éthyl][2-(2-{[3-chloro-4'-(trifluorométhyl)biphényl-4-yl]méthoxy}-phényl)éthyl]amino}-5,6,7,8-tétrahydroquinoléine-2-carboxylique présentant la formule suivante 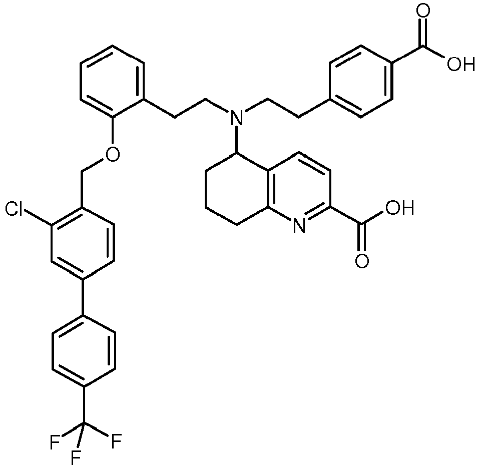 ainsi que ses sels, solvates et solvates des sels.

6. Acide 5-{(4-carboxybutyl)[2-(2-{[3-chloro-4'-(trifluorométhyl)biphényl-4-yl]méthoxy}phényl)éthyl]-amino}-5,6,7,8-tétrahydroquinoléine-2-carboxylique présentant la formule suivante 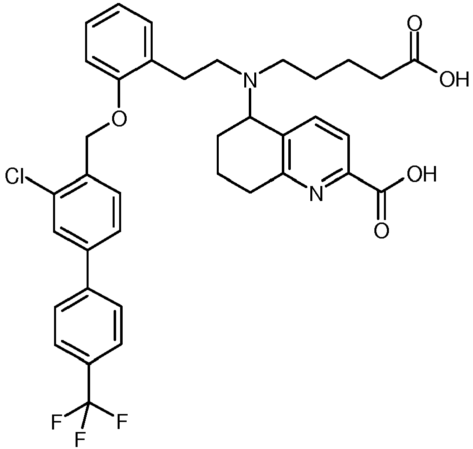 ainsi que ses sels, solvates et solvates des sels.

7. Procédé pour la préparation d'un composé de formule (I), tel que défini dans les revendications 1 à 6, **caractérisé en ce qu'**on transforme soit
[A] un composé de formule (II) 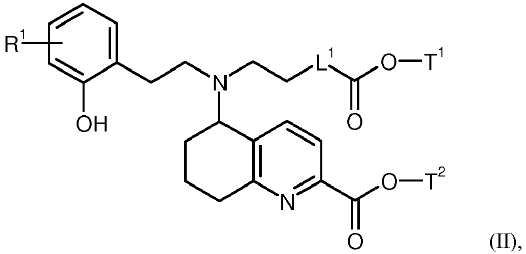 dans laquelle R¹ et L¹ présentent les significations indiquées dans les revendications 1 à 6 et
T¹ et T² sont identiques ou différents et représentent C₁-C₄-alkyle,
en présence d'une base avec un composé de formule (III) 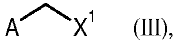 dans laquelle A présente les significations indiquées dans les revendications 1 à 6 et
X¹ représente un groupe partant tel que par exemple chlore, brome, iode, mésylate, triflate ou tosylate,
soit
[B] un composé de formule (IV) 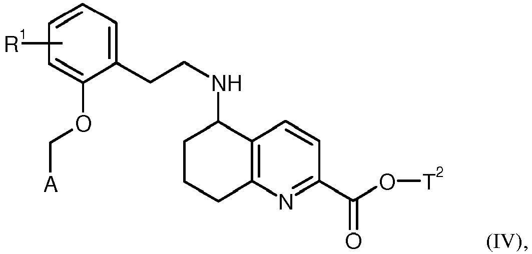 dans laquelle R¹ et A présentent les significations indiquées dans les revendications 1 à 6 et
T² représente C₁-C₄-alkyle,
en présence d'une base avec un composé de formule (V) 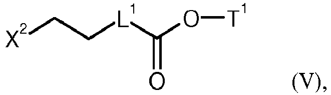 dans laquelle L¹ présente les significations indiquées dans les revendications 1 à 6,
T¹ représente C₁-C₄-alkyle et
X¹ représente un groupe partant tel que par exemple chlore, brome, iode, mésylate, triflate ou tosylate,
et on transforme ensuite le composé qui en résulte à chaque fois, de formule (VI) 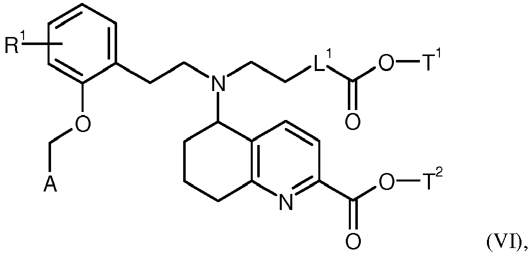 dans laquelle R¹, A, L¹, T¹ et T² présentent les significations indiquées ci-dessus, par hydrolyse des groupes ester -C(O)OT¹ et -C(O)OT² en acide dicarboxylique correspondant de formule (I) et
on sépare les composés de formule (I) ainsi obtenus le cas échéant en leurs énantiomères et/ou diastéréo-isomères et/ou on les transforme le cas échéant avec (i) des solvants et/ou (ii) des bases ou acides correspondants en leurs solvates, sels et/ou solvates des sels.

8. Composé, tel que défini dans l'une quelconque des revendications 1 à 6, pour le traitement et/ou la prévention de maladies.

9. Composé, tel que défini dans l'une quelconque des revendications 1 à 6, destiné à être utilisé dans un procédé pour le traitement et/ou la prévention de formes primaires et secondaires de l'hypertonie pulmonaire, de l'insuffisance cardiaque, de l'angine de poitrine, de l'hypertonie, des maladies thromboemboliques, des ischémies, des maladies vasculaires, des troubles microcirculatoires, de l'insuffisance rénale, de maladies fibrotiques et de l'artériosclérose.

10. Utilisation d'un composé, tel que défini dans l'une quelconque des revendications 1 à 6, pour la préparation d'un médicament pour le traitement et/ou la prévention de formes primaires et secondaires de l'hypertonie pulmonaire, de l'insuffisance cardiaque, de l'angine de poitrine, de l'hypertonie, des maladies thromboemboliques, des ischémies, des maladies vasculaires, des troubles microcirculatoires, de l'insuffisance rénale, de maladies fibrotiques et de l'artériosclérose.

11. Médicament contenant un composé, tel que défini dans l'une quelconque des revendications 1 à 6, en combinaison avec un ou plusieurs adjuvants inertes, non toxiques, pharmaceutiquement appropriés.

12. Médicament contenant un composé, tel que défini dans l'une quelconque des revendications 1 à 6, en combinaison avec une ou plusieurs autres substances actives choisies dans le groupe constitué par les nitrates organiques, les donneurs de NO, les inhibiteurs de PDE5, les analogues de prostacycline, les agonistes du récepteur d'IP, les antagonistes du récepteur de l'endothéline, les stimulateurs de la guanylate-cyclase, les inhibiteurs de la tyrosine-kinase, les agents à action anti-obstructive, les agents d'inhibition d'inflammation et/ou immunosuppresseurs, les agents à effet antithrombotique, les agents hypotenseurs ainsi que les agents modifiant le métabolisme des lipides.

13. Médicament selon la revendication 11 ou 12 pour le traitement et/ou la prévention de formes primaires et secondaires de l'hypertonie pulmonaire, de l'insuffisance cardiaque, de l'angine de poitrine, de l'hypertonie, des maladies thromboemboliques, des ischémies, des maladies vasculaires, des troubles microcirculatoires, de l'insuffisance rénale, de maladies fibrotiques et de l'artériosclérose.
